(19) Europäisches Patentamt
European Patent Office
Office européen des brevets

(11) Veröffentlichungsnummer: **0 304 758 B1**

(12) # EUROPÄISCHE PATENTSCHRIFT

(45) Veröffentlichungstag der Patentschrift: **04.09.91 Patentblatt 91/36**

(51) Int. Cl.$^5$: **C07C 251/32**

(21) Anmeldenummer: **88113231.0**

(22) Anmeldetag: **16.08.88**

(54) **N,N'- Diacylaminale.**

Die Akte enthält technische Angaben, die nach dem Eingang der Anmeldung eingereicht wurden und die nicht in dieser Patentschrift enthalten sind.

(30) Priorität: **25.08.87 DE 3728277**

(43) Veröffentlichungstag der Anmeldung: **01.03.89 Patentblatt 89/09**

(45) Bekanntmachung des Hinweises auf die Patenterteilung: **04.09.91 Patentblatt 91/36**

(84) Benannte Vertragsstaaten: **BE CH DE ES FR GB IT LI NL**

(56) Entgegenhaltungen:
**EP-A- 0 201 999**
**EP-A- 0 206 004**

(73) Patentinhaber: **BAYER AG**
**W-5090 Leverkusen 1 Bayerwerk (DE)**

(72) Erfinder: **Lunkenheimer, Winfried, Dr.**
**Bismarckstrasse 29**
**W-5600 Wuppertal 1 (DE)**
Erfinder: **Berg, Dieter, Dr.**
**Gellertweg 27**
**W-5600 Wuppertal 1 (DE)**
Erfinder: **Brandes, Wilhelm, Dr.**
**Eichendorffstrasse 3**
**W-5653 Leichlingen (DE)**

**EP 0 304 758 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N,N'-Diacylaminale, mehrere Verfahren zu ihrer Herstellung und ihre Verwendung in Schädlingsbekämpfungsmitteln, insbesondere als Fungizide.

Es ist bereits bekannt, daß bestimmte substituierte 2-Cyan-2-oximino-acetamide gute fungizide Wirksamkeit besitzen (vergl. z.B. EP-OS 0201999 und DE-OS 3602243). Die Wirkung dieser Verbindungen ist jedoch, insbesondere bei niedrigen Aufwandmengen und -konzentrationen, nicht immer voll befriedigend.

Es wurden neue N,N'-Diacylaminale der allgemeinen Formel (I)

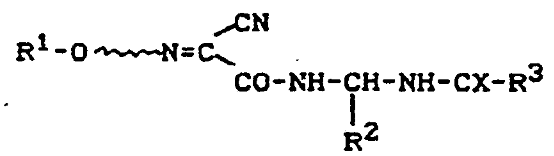

(I)

in welcher

$R^1$ für gegebenenfalls ein- bis fünffach, gleich oder verschieden substituiertes, geradkettiges oder verzweigtes Alkyl mit 1 bis 6 Kohlenstoffatomen steht, wobei folgende Substituenten infrage kommen : Fluor, Chlor, Brom, Iod, Cyano, —$COOR^I$, —$CONR^{II}R^{III}$, —$OR^{IV}$, Acyl mit 2 bis 9 Kohlenstoffatomen, gegebenenfalls ein- bis fünffach, gleich oder verschieden substituiertes Phenyl, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen ; weitere Alkylsubstituenten sind gegebenenfalls ein- bis fünffach, gleich oder verschieden durch Alkyl mit 1 bis 4 Kohlenstoffatomen Substituiertes Cycloalkyl mit 3 bis 6 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff-und Schwefelatomen ; $R^1$ steht ferner für jeweils gegebenenfalls ein- bis dreifach, gleich oder verschieden substituiertes, geradkettiges oder verzweigtes Alkenyl oder Alkinyl mit 2 bis 6 Kohlenstoffatomen, wobei als Substituenten genannt seien : gegebenenfalls ein- bis fünffach, gleich oder verschieden durch Halogen und Alkyl mit 1 bis 4 Kohlenstoffatomen substituiertes Phenyl ; $R^1$ steht außerdem für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ;

$R^2$ für Wasserstoff oder für gegebenenfalls ein- bis fünffach, gleich oder verschieden substituiertes, geradkettiges oder verzweigtes Alkyl mit 1 bis 6 Kohlenstoffatomen steht, wobei folgende Substituenten infrage kommen : Cyano, Alkoxy und Alkylthio mit jeweils 1 bis 4 Kohlenstoffatomen, —$COOR^I$, Acylamino mit 2 bis 9 Kohlenstoffatomen, gegebenenfalls ein- bis fünffach, gleich oder verschieden substituiertes Phenyl, wobei als Substituenten die bei $R^1$ bereits genannten Phenylsubstituenten infrage kommen ; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen, gegebenenfalls ein-bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff- und Schwefelatomen ; $R^2$ steht ferner für jeweils gegebenenfalls ein- bis dreifach, gleich oder verschieden substituiertes, geradkettiges oder verzweigtes Alkenyl oder Alkinyl mit 2 bis 6 Kohlenstoffatomen, wobei als Substituenten genannt seien : gegebenenfalls ein-bis fünffach, gleich oder verschieden durch Halogen und Alkyl mit 1 bis 4 Kohlenstoffatomen substituiertes Phenyl ; $R^2$ steht außerdem für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ; $R^2$ steht weiterhin für gegebenenfalls ein- bis fünffach, gleich oder verschieden substituiertes Phenyl, wobei als Substituenten die bei $R^1$ bereits, genannten Phenylsubstituenten infrage kommen ; $R^2$ steht ferner für gegebenenfalls ein-bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff- und Schwefelatome ;

$R^3$ für Wasserstoff oder für gegebenenfalls ein- bis fünffach, gleich oder verschieden substituiertes, geradkettiges oder verzweigtes Alkyl mit 1 bis 6 Kohlenstoffatomen steht, wobei folgende Substituenten infrage kommen : Fluor, Chlor, Brom, Iod, Cyano, Alkoxy, Alkylthio, Alkylsulfinyl und Alkylsulfonyl mit jeweils 1 bis 4 Kohlenstoffatomen, Acyloxy und Acylamino mit jeweils 2 bis 9 Kohlenstoffatomen, —$COOR^I$, gegebenenfalls ein- bis fünffach, gleich oder verschieden substituiertes Phenyl, wobei als Substituenten die bei $R^1$ bereits genannten Phenylsubstituenten infrage kommen ; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6

Kohlenstoffatomen sowie gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff- und Schwefelatomen ; $R^3$ steht ferner für jeweils gegebenenfalls ein- bis dreifach, gleich oder verschieden substituiertes, geradkettiges oder verzweigtes Alkenyl oder Alkinyl mit jeweils 2 bis 6 Kohlenstoffatomen, wobei als Substituenten genannt seien : gegebenenfalls ein- bis fünffach, gleich oder verschieden durch Halogen und Alkyl mit 1 bis 4 Kohlenstoffatomen substituiertes Phenyl; $R^3$ steht weiterhin für jeweils gegebenenfalls mit Methylen oder Ethylen überbrücktes und/oder mit 1 oder 2 Benzol-, Cyclopentan-oder Cyclohexanringen anelliertes, jeweils gegebenenfalls ein- bis fünffach, gleich oder verschieden substituiertes Cycloalkyl mit 3 bis 6 Kohlenstoffatomen oder Cycloalkenyl mit 5 bis 7 Kohlenstoffatomen, wobei als Substituenten genannt seien : Halogen, Alkyl und Alkoxy mit jeweils 1 bis 4 Kohlenstoffatomen, Acyloxy und Acylamino mit jeweils 2 bis 5 Kohlenstoffatomen, die Oxogruppe, Phenyl, Hydroxycarbonyl und Alkoxycarbonyl mit 1 bis 4 Kohlenstoffatomen im Alkoxyteil ; $R^3$ steht außerdem für gegebenenfalls mit 1 oder 2 Benzol- oder Cyclohexanringen anelliertes, gegebenenfalls ein-bis fünffach, gleich oder verschieden substituiertes Phenyl, wobei als Substituenten genannt seien : Halogen, Hydroxy, Nitro, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen, wie Fluor- und Chloratome, Acylamino und Acylalkylamino mit jeweils 2 bis 5 Kohlenstoffatomen im Acylteil und 1 bis 4 Kohlenstoffatomen im Alkylteil, Hydroxycarbonyl und Alkoxycarbonyl mit 1 bis 4 Kohlenstoffatomen im Alkoxyteil ; $R^3$ steht ferner für gegebenenfalls mit 1 oder 2 Benzol-, Cyclopentan- oder Cyclohexanringen anelliertes, gegebenenfalls ein- bis fünffach, gleich oder verschieden substituiertes 5- oder 6-gliedriges Heterocyclyl mit 1 bis 3 gleichen oder verschiedenen Heteroatomen, wie Stickstoff-, Sauerstoff- und Schwefelatomen, wobei als Substituenten genannt seien : Halogen, Alkyl mit 1 bis 4 Kohlenstoffatomen, Acyl mit 2 bis 9 Kohlenstoffatomen, Phenyl, die Oxogruppe und Hydroxy ; $R^3$ steht schließlich auch für Alkoxycarbonyl mit 1 bis 4 Kohlenstoffatomen im Alkoxyteil, für $-OR^4$, $-SR^4$ oder $-NR^5R^6$ ;

$R^4$ für gegebenenfalls ein- bis fünffach, gleich oder verschieden substituiertes, geradkettiges oder verzweigtes Alkyl mit 1 bis 6 Kohlenstoffatomen steht, wobei folgende Substituenten infrage kommen : Halogen, $-OR^{IV}$, $-SR^{IV}$, $-COOR^{I}$, $-CONR^{II}R^{III}$, CN, $-NR^{II}R^{III}$, Acyl mit 2 bis 9 Kohlenstoffatomen, jeweils gegebenenfalls einfach bis fünffach, gleich oder verschieden durch Halogen und Alkyl mit 1 bis 4 Kohlenstoffatomen substituiertes Phenyl oder Phenoxy, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff- und Schwefelatomen ; $R^4$ steht ferner für Alkenyl oder Alkinyl mit jeweils 2 bis 6 Kohlenstoffatomen, für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ;

$R^5$ für Wasserstoff oder für gegebenenfalls gleich oder verschieden, ein- oder zweifach substituiertes, geradkettiges oder verzweigtes Alkyl mit 1 bis 4 Kohlenstoffatomen steht, wobei als Substituenten genannt seien : Halogen, Cyano, $-COOR^{I}$, $-CONR^{II}R^{III}$, $-NR^{II}R^{III}$, $-OR^{IV}$, $-S(O)_nR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, gleich oder verschieden durch Halogen, Alkyl und Alkoxy mit jeweils 1 bis 4 Kohlenstoffatomen substituierter 5- oder 6-gliedriger Heterocyclus mit 1 bis 3 gleichen oder verschiedenen Heteroatomen, z.B. Stickstoff, Sauerstoff und Schwefel, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ; $R^5$ steht ferner für geradkettiges oder verzweigtes Alkenyl oder Alkinyl mit jeweils 2 bis 6 Kohlenstoffatomen ; für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden substituiertes Cycloalkyl mit 3 bis 6 Kohlenstoffatomen, wobei als Substituenten genannt seien : Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden durch Halogen und Alkyl mit 1 bis 4 Kohlenstoffatomen substituiertes Phenyl oder Pyrrolidon ; $R^5$ steht weiterhin für gegebenenfalls ein- bis fünffach, gleich oder verschieden durch Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis fünffach, gleich oder verschieden substituierten 5- oder 6-gliedrigen Heterocyclus

mit 1 bis 3 gleichen oder verschiedenen Heteroatomen, wie insbesondere Stickstoff-, Sauerstoff- und Schwefelatomen ; als Substituenten seien genannt : Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil ;

$R^6$ für die Bedeutungen von $R^5$ oder die Gruppierung —$OR^{IV}$ steht ;

oder

$R^5$ und $R^6$ gemeinsam mit dem Stickstoffatom, an das sie gebunden sind, für einen gegebenenfalls ein- bis fünffach, gleich oder verschieden substituierten mono-, bi- oder tricyclischen Heterocyclus oder Spiroheterocyclus mit ein bis drei weiteren, gleichen oder verschiedenen Heteroatomen, wie Sauerstoff-, Stickstoff- oder Schwefelatome stehen, wobei als Substituenten genannt seien : geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen im geradkettigen oder verzweigten Alkylteil, jeweils gegebenenfalls ein- oder zweifach, gleich oder verschieden durch Halogen oder geradkettiges oder verzweigtes Alkyl oder Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil ;

$R^I$ für Wasserstoff oder geradkettiges oder verzweigtes Alkyl mit 1 bis 6 Kohlenstoffatomen steht,

$R^{II}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten genannt seien : Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 2 bis 5 gleichen oder verschiedenen Halogenatomen ; $R^{II}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen ;

$R^{III}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen ; $R^{III}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen ;

$R^{IV}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen ; $R^{IV}$ steht ferner für Acyl mit 2 bis 9 Kohlenstoffatomen ;

n für die Zahlen 0, 1 oder 2 steht und

X für Sauerstoff oder Schwefel steht, gefunden.

Die Verbindungen der Formel (I) können in verschiedenen geometrischen Isomeren vorkommen, je nach Anordnung der Substituenten an der C=N-Gruppierunng (E- bzw. Z-Isomere).

Gegebenenfalls besitzen die Verbindungen ein oder mehrere asymmetrische Kohlenstoffatome ; sie können somit auch als Enantiomere oder Diastereomere vorliegen, die in unterschiedlichen Mengenverhältnissen anfallen können. Vorwiegend fallen sie als Racemate an.

Weiterhin wurde gefunden, daß man die neuen N,N'-Diacylaminale der allgemeinen Formel (I),

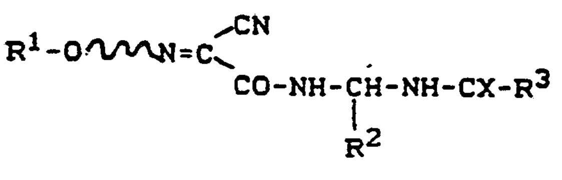

(I)

in welcher

$R^1$, $R^2$ und $R^3$ die oben angegebene Bedeutung haben, erhält, wenn man

a) N-(2-Cyan-2-oximino-acetyl)-aminale der Formel (II)

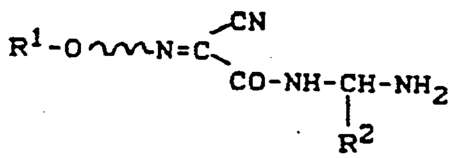 (II)

in welcher

$R^1$ und $R^2$ die oben angegebene Bedeutung haben und

HY für das Äquivalent einer anorganischen oder organischen Säure steht,

mit einem Acylierungsreagenz der Formeln (IIIa) oder (IIIb)

$$Z\text{-}CX\text{-}R^3 \qquad (IIIa)$$

$$X{=}C{=}N\text{-}R^6 \qquad (IIIb)$$

in welchen

Z für eine übliche Abgangsgruppe steht, wie Halogen, —O-CO-$R^3$, —O-CO-O$R^4$, —O$R^4$, —S$R^4$, Carboxymethoxy oder Carboxymethylthio und

$R^3$, $R^4$, $R^6$ und X die oben angegebene Bedeutung haben,

gegebenenfalls in Gegenwart einer Base und in Gegenwart eines Verdünnungsmittels und gegebenenfalls in Gegenwart eines Katalysators umsetzt;

oder

b) Isocyanate der Formel (IV),

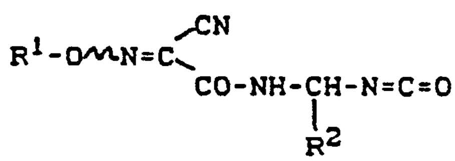 (IV)

in welcher

$R^1$ und $R^2$ die oben angegebene Bedeutung haben,

mit einem protischen Nukleophil der Formeln (Va) oder (Vb)

$$HO\text{-}CO\text{-}R^3 \qquad (Va)$$

$$H - R^7 \qquad (Vb)$$

in welchen

$R^7$ für —O$R^4$, —S$R^4$ oder —N$R^5R^6$ steht und

$R^3$, $R^4$, $R^5$ und $R^6$ die oben angegebene Bedeutung haben,

in Gegenwart eines Verdünnungsmittels und gegebenenfalls in Gegenwart eines Katalysators umsetzt.

Schließlich wurde gefunden, daß die neuen N,N'-Diacylaminale u.a. starke fungizide Eigenschaften aufweisen. Dabei zeigen überraschenderweise die erfindungsgemäßen Verbindungen eine höhere Wirkung als die aus dem Stand der Technik bekannten substituierten 2-Cyan-2-oximino-acetamide, welche konstitutionell bzw. wirkungsmäßig naheliegende Verbindungen sind. Die erfindungsgemäßen Stoffe stellen somit eine Bereicherung der Technik dar.

Im folgenden können alle aliphatischen Reste, wie Alkyl, Alkoxy, Alkenyl usw., allein oder in Zusammensetzungen, wie Alkoxyalkyl, geradkettig oder verzweigt sein, weiterhin können die aliphatischen Reste im allgemeinen vorzugsweise ein- bis fünffach, besonders bevorzugt ein- bis dreifach oder ganz besonders bevorzugt ein- oder zweifach, gleich oder verschieden substituiert sein; ebenso sind alle Ringsysteme gegebenenfalls ein- bis fünffach, besonders bevorzugt ein- bis dreifach oder ganz besonders bevorzugt ein- oder zweifach, gleich oder verschieden substituiert, wenn es nicht anders angegeben oder nicht ausdrücklich

beschrieben ist.

Besonders bevorzugt sind N,N'-Diacylaminale der allgemeinen Formel (I), bei denen

$R^1$ für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen : Fluor, Chlor, Brom, Iod, Cyano, $—COOR^I$, $—CONR^{II}R^{III}$, $—OR^{IV}$, Acyl mit 2 bis 9 Kohlenstoffatomen, gegebenenfalls ein- bis dreifach, gleich oder verschieden durch Fluor, Chlor, Methyl und Methoxy substituiertes Phenyl, jeweils gegebenenfalls ein- bis dreifach durch Methyl substituiertes Cyclopropyl oder Cyclohexyl, gegebenenfalls ein- bis dreifach, gleich oder verschieden durch Alkyl mit 1 oder 2 Kohlenstoffatomen substituiertes Heterocyclen der Formeln

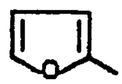 , 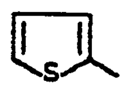 , 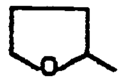 , 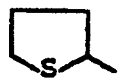 , 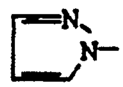

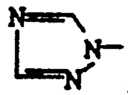 , 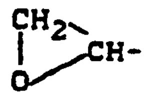 , 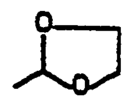 und 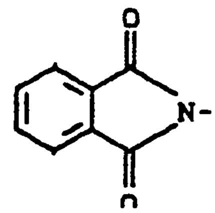 ;

$R^1$ steht ferner für gegebenenfalls ein- oder zweifach durch Methyl substituiertes Allyl oder Propargyl, für jeweils gegebenenfalls ein- bis dreifach durch Methyl substituiertes Cyclopropyl, Cyclohexyl, Cyclopentenyl oder Cyclohexenyl ;

$R^2$ für Wasserstoff oder für gegebenenfalls ein- bis dreifach, gleich oder verschieden substituiertes, geradkettiges oder verzweigtes Alkyl mit 1 bis 4 Kohlenstoffatomen, steht, wobei folgende Substituenten infrage kommen : Cyano, Methoxy, Ethoxy, Methylthio, Ethylthio, $—COOR^I$, Acylamino mit 2 bis 9 Kohlenstoffatomen, gegebenenfalls ein- bis dreifach, gleich oder verschieden durch Halogen, Methyl und Methoxy substituiertes Phenyl, jeweils gegebenenfalls ein- bis dreifach durch Methyl substituiertes Cyclopropyl oder Cyclohexyl sowie gegebenenfalls ein- bis dreifach, gleich oder verschieden durch Alkyl mit 1 oder 2 Kohlenstoffatomen substituierte Heterocyclen der Formeln

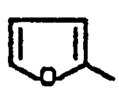 , 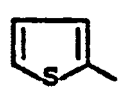 , 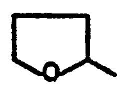 , 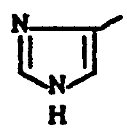 , 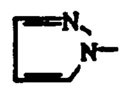

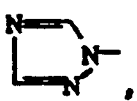 , 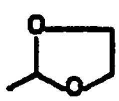 und 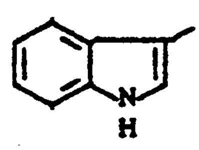 ;

$R^2$ steht ferner für jeweils gegebenenfalls durch Phenyl, das gegebenenfalls ein- bis dreifach, gleich oder verschieden durch Halogen und Methyl substituiert sein kann, substituiertes Allyl, Allenyl, Vinyl, Propargyl oder Ethinyl ; $R^2$ steht außerdem für Cyclopropyl, Cyclohexyl, Cyclopentenyl oder Cyclohexenyl ; $R^2$ steht weiterhin für gegebenenfalls ein- bis dreifach, gleich oder verschieden durch Halogen, Methyl und Methoxy substituiertes Phenyl, für gegebenenfalls ein-bis dreifach, gleich oder verschieden durch Alkyl mit 1 oder 2 Kohlenstoffatomen substituierte Heterocyclen der Formeln

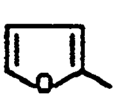 , 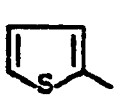 , 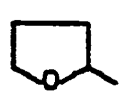 , 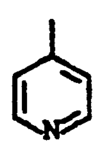 , 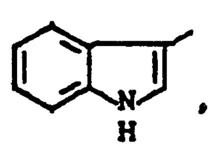 ,

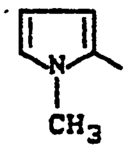 , 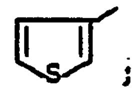 ;

$R^3$ für Wasserstoff oder für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen : Fluor, Chlor, Brom, Iod, Cyano, Alkoxy, Alkylthio, Alkylsulfinyl und Alkylsulfonyl mit jeweils 1 oder 2 Kohlenstoffatomen, Acyloxy und Acylamino mit jeweils 2 bis 9 Kohlenstoffatomen, —COOR$^I$, gegebenenfalls ein- bis dreifach, gleich oder verschieden durch Halogen und Methyl substituiertes Phenyl, Cyclopropyl, Cyclopentyl oder Cyclohexyl, gegebenenfalls ein- bis dreifach, gleich oder verschieden durch Alkyl mit 1 oder 2 Kohlenstoffatomen substituierte Heterocyclen der Formeln

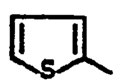 , 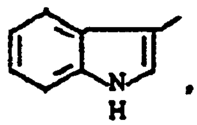 , 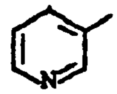 , 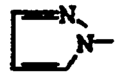 ,

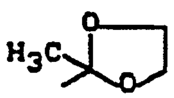 , 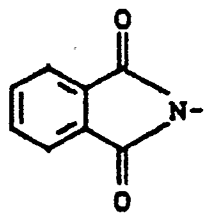 , 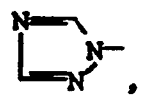 , 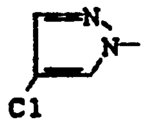 ,

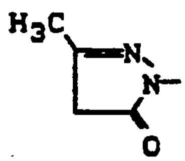 , 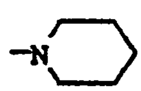 , 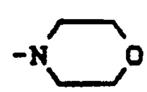 ;

$R^3$ steht ferner für jeweils gegebenenfalls durch Phenyl, das gegebenenfalls ein- bis dreifach, gleich oder verschieden durch Halogen und Methyl substituiert sein kann, oder durch Methyl substituiertes Vinyl oder Ethinyl ; $R^3$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis dreifach, gleich oder verschieden substituiertes Cyclopropyl, Cyclopentyl, Cyclohexyl, Cyclopentenyl oder Cyclohexenyl, wobei als Substituenten genannt seien : Halogen, Methyl, Methoxy, Acyloxy und Acylamino mit jeweils 2 bis 5 Kohlenstoffatomen, die Oxogruppe, Phenyl, Hydroxycarbonyl, Methoxy- und Ethoxycarbonyl ; $R^3$ steht außerdem für gegebenenfalls mit 1 oder 2 Benzol- oder Cyclohexanringen anelliertes, gegebenenfalls ein- bis dreifach, gleich oder verschieden substituiertes Phenyl, wobei als Substituenten genannt seien : Fluor, Chlor, Hydroxy, Nitro, Methyl, Methoxy, Acylamino und N-Alkyl-acyl-amino mit jeweils 2 bis 5 Kohlenstoffatomen im Acylteil und 1 oder 2 Kohlenstoffatomen im Alkylteil, Hydroxycarbonyl, Methoxy- und Ethoxycarbonyl ; $R^3$ steht ferner für gegebenenfalls mit 1 oder 2 Benzol- oder Cyclohexanringen anelliertes, gegebenenfalls ein- bis dreifach, gleich oder verschieden substituiertes 5- oder 6-gliedriges Heterocyclyl mit 1 bis 3 gleichen oder verschiedenen Heteroatomen, wie Stickstoff-, Sauerstoff- und Schwefelatomen, wobei als Substituenten genannt seien : Acyl mit 2 bis 5 Kohlenstoffatomen, Chlor, Brom, Methyl, Ethyl, Phenyl, Oxo und Hydroxy ; $R^3$ steht schließlich auch für Alkoxycarbonyl mit 1 bis 4 Kohlenstoffatomen im Alkoxyteil, für —OR$^4$, —SR$^4$ oder —NR$^5$R$^6$ ;

$R^4$ für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen : Fluor, Chlor, —OR$^{IV}$, —SR$^{IV}$, —COOR$^I$, —CONR$^{II}$R$^{III}$, CN, NR$^{II}$R$^{III}$, Alkylcarbonyl mit 1 bis 4 Kohlenstoffatomen im geradkettigen oder verzweigten Alkylteil, jeweils gegebenenfalls ein- bis dreifach, gleich oder verschieden durch Halogen und Methyl substituiertes Phenyl oder Phenoxy, jeweils gegebenenfalls ein- bis dreifach durch Methyl substituiertes Cyclopropyl, Cyclohexyl, Cyclopentenyl oder Cyclohexenyl, gegebenenfalls ein- bis dreifach, gleich oder verschieden durch Halogen, Methyl und Ethyl substituiertes 3- bis 6-gliedriges Heterocyclyl mit 1 bis 3 gleichen oder verschiedenen Heteroatomen, wie Stickstoff-, Sauerstoff- und Schwefelatomen ; $R^4$

steht ferner für gegebenenfalls ein- oder zweifach durch Methyl substituiertes Allyl oder Propargyl sowie für jeweils gegebenenfalls ein- bis dreifach durch Methyl substituiertes Cyclopropyl, Cyclohexyl, Cyclopentenyl oder Cyclohexenyl ;

$R^5$ für Wasserstoff oder für gegebenenfalls ein- oder zweifach, gleich oder verschieden substituiertes geradkettiges oder verzweigtes Alkyl mit 1 bis 4 Kohlenstoffatomen steht, wobei als Substituenten vorzugsweise genannt seien : Halogen, Cyano, $—COOR^I$, $—CONR^{II}R^{III}$, $NR^{II}R^{III}$, $—OR^{IV}$, $—S(O)_nR^{IV}$, Acyl mit 2 bis 9 Kohlenstoffatomen, gegebenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituierter 5- oder 6-gliedrigen Heterocyclus mit 1 bis 3 gleichen oder verschiedenen Heteroatomen, insbesondere Stickstoff, Sauerstoff und Schwefel, und jeweils gegebenenfalls durch geradkettiges oder verzweigtes Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ; $R^5$ steht ferner für geradkettiges oder verzweigtes Alkenyl oder Alkinyl mit jeweils 2 bis 6 Kohlenstoffatomen, für gegebenenfalls durch geradkettiges oder verzweigtes Alkyl mit 1 bis 4 Kohlenstoffatomen substituiertes Cycloalkenyl mit 5 bis 7 Kohlenstoffatomen, für gegebenenfalls substituiertes Cycloalkyl mit 3 bis 6 Kohlenstoffatomen, wobei als Substituenten genannt seien : Halogen, geradkettiges oder verzweigtes Alkyl und Alkoxy mit jeweils 1 bis 4 Kohlenstoffatomen, Cyano, Amino, Carbamoyl, jeweils geradkettiges oder verzweigtes Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden durch Halogen und Alkyl mit 1 bis 4 Kohlenstoffatomen substituiertes Phenyl oder Pyrrolidon; $R^5$ steht weiterhin für gegebenenfalls ein- bis dreifach, gleich oder vrschieden durch Halogen, geradkettiges oder verzweigtes Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis dreifach, gleich oder verschieden substituierten 5- oder 6-gliedrigen Heterocyclus mit 1 bis 3 gleichen oder verschiedenen Heteroatomen, wie insbesondere Stickstoff, Sauerstoff oder Schwefel ; als Substituenten für die Heterocyclen seien genannt : Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkyl ;

$R^6$ für die Bedeutungen von $R^5$ oder die Gruppierung $—OR^{IV}$ steht,

oder

$R^5$ und $R^6$ gemeinsam mit dem Stickstoffatom, an das sie gebunden sind, für einen gegebenenfalls ein- bis fünffach, gleich oder verschieden substituierten mono-, bi- oder tricyclischen Heterocyclus oder Spiroheterocyclus stehen, wobei als Heterocyclen genannt seien :

Oxazolidin, Pyrrolidin, Imidazolidin, Piperidin, Piperazin, Morpholin, Thiomorpholin, 1,3-Oxazan oder 1,3-Diazan ; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt :

geradkettiges oder verzweigtes Alkyl oder Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen in geradkettigen oder verzweigten Alkylteil, Carbamoyl, Alkyl- oder Dialkylcarbamoyl mit jeweils 1 bis 4 Kohlenstoffatomen in jedem Alkylteil, ferner jeweils gegebenenfals ein- oder zweifach, gleich oder verschieden durch Halogen und geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

$R^I$ für geradkettiges oder verzweigtes Alkyl mit 1 bis 4 Kohlenstoffatomen steht,

$R^{II}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien : Fluor, Chlor, Methyl, Methoxy und Trifluormethyl ; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl oder Dialkylcarbamoylalkyl mit jeweils 1 oder 2 Kohlenstoffatomen in jedem Alkylteil, oder für gegebenenfalls ein- bis dreifach, gleich oder verschieden durch Methyl und Ethyl substituiertes Cycloalkyl mit 3 bis 6 Kohlenstoffatomen steht,

$R^{III}$ für die Bedeutungen von $R^{II}$ steht,

$R^{IV}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien : Fluor, Chlor, Methyl, Methoxy und Trifluormethyl ; $R^{IV}$ steht ferner für

Acyl mit 2 bis 9 Kohlenstoffatomen ;

n für die Zahlen 0, 1 oder 2 steht und

X für Sauerstoff oder Schwefel steht.

Ganz besonders bevorzugt sind N,N'-Diacylaminale der allgemeinen Formel (I), bei denen

$R^1$ für gegebenenfalls ein- oder zweifach, gleich oder verschieden substituiertes Alkyl mit 1 oder 2 Kohlenstoffatomen steht, wobei folgende Substituenten infrage kommen : Fluor, Chlor, Cyano, $—COOR^I$, $—CONR^{II}R^{III}$, $—OR^{IV}$, Alkylcarbonyl mit 1 bis 4 Kohlenstoffatomen im geradkettiges oder verzweigten Alkylteil, gegebenenfalls ein- bis dreifach, gleich oder verschieden durch Fluor, Chlor und Methyl substituiertes Phenyl, jeweils gegebenenfalls ein- bis dreifach durch Methyl substituiertes Cyclopropyl oder Cyclohexyl und die folgenden Heterocyclen :

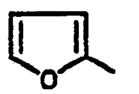 , 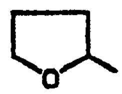 , 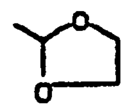 , 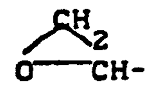

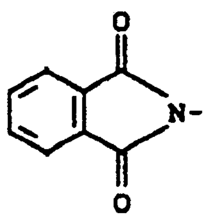 , 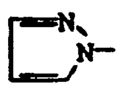 und 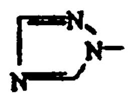 ;

$R^1$ steht ferner für Allyl oder Propargyl, für jeweils gegebenenfalls ein- bis dreifach durch Methyl substituiertes Cyclopropyl, Cyclohexyl, Cyclopentenyl oder Cyclohexenyl ;

$R^2$ für Wasserstoff oder für gegebenenfalls ein- oder zweifach, gleich oder verschieden substituiertes Alkyl mit 1 bis 4 Kohlenstoffatomen steht, wobei folgende Substituenten infrage kommen : Cyano, Methoxy, Ethoxy, Methylthio, Ethylthio, $—COOR^I$, Alkylcarbonylamino mit 1 bis 4 Kohlenstoffatomen im geradkettigen oder verzweigten Alkylteil, gegebenenfalls ein- bis dreifach, gleich oder verschieden durch Halogen, Methyl und Methoxy substituiertes Phenyl, jeweils gegebenenfalls ein- bis dreifach durch Methyl substituiertes Cyclopropyl oder Cyclohexyl und die folgenden Heterocyclen :

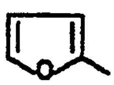 , 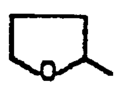 , 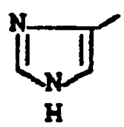 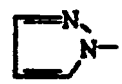 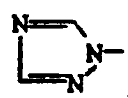 ,

und 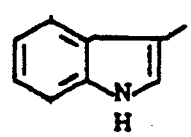 ;

$R^2$ steht ferner für jeweils gegebenenfalls durch Phenyl, das gegebenenfalls ein- bis dreifach, gleich oder verschieden durch Halogen und Methyl substituiert sein kann, substituiertes Vinyl, Allyl, Allenyl, Ethinyl oder Propargyl ;

$R^2$ steht außerdem für Cyclopropyl, Cyclohexyl, Cyclopentenyl oder Cyclohexenyl ;

$R^2$ steht weiterhin für gegebenenfalls ein- bis dreifach, gleich oder verschieden durch Halogen, Methyl und Methoxy substituiertes Phenyl oder für die folgenden Heterocyclen :

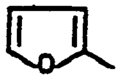, 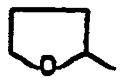, 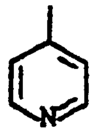, 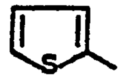 und 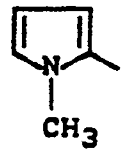 ;

$R^3$ für Wasserstoff oder für gegebenenfalls ein- oder zweifach, gleich oder verschieden substituiertes, geradkettiges oder verzweigtes Alkyl mit 1 bis 4 Kohlenstoffatomen steht, wobei folgende Substituenten infrage kommen : Fluor, Chlor, Brom, Iod, Cyano, Alkoxy, Alkylthio, Alkylsulfinyl und Alkylsulfonyl mit jeweils 1 oder 2 Kohlenstoffatomen, Alkylcarbonyloxy mit 1 bis 4 Kohlenstoffatomen im geradkettigen oder verzweigten Alkylteil, Formamido, Alkylcarbonylamino mit 1 oder 2 Kohlenstoffatomen im gegebenenfalls durch Fluor substituierten Alkylteil, gegebenenfalls durch Chlor substituiertes Benzoylamino, Alkoxycarbonylamino mit 1 bis 4 Kohlenstoffatomen im geradkettigen oder verzweigten Alkoxyteil, —$COOR^1$, gegebenenfalls ein- bis dreifach, gleich oder verschieden durch Halogen und Methyl substituiertes Phenyl, Cyclopropyl, Cyclopentyl oder Cyclohexyl und die folgenden Heterocyclen :

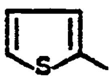, 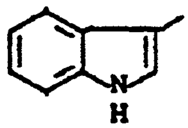, 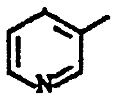, 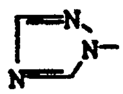, 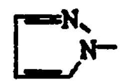,

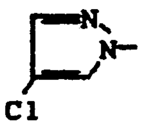, 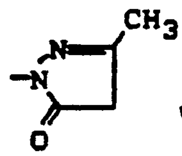 und 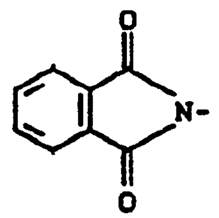 ;

$R^3$ steht ferner für jeweils gegebenenfalls einfach oder zweifach, gleich oder verschieden durch Phennyl, das gegebenenfalls ein- bis dreifach, gleich oder verschieden durch Halogen und Methyl substituiert sein kann, oder durch Methyl substituiertes Vinyl oder Ethinyl ;

$R^3$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis dreifach, gleich oder verschieden substituiertes Cyclopropyl, Cyclopentyl, Cyclohexyl, Cyclopentenyl oder Cyclohexenyl, wobei als Substituenten genannt seien : Halogen, Methyl, die Oxogruppe, Phenyl, Hydroxycarbonyl, Methoxy- und Ethoxycarbonyl ; im einzelnen seien als Ringe genannt :

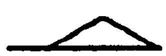, 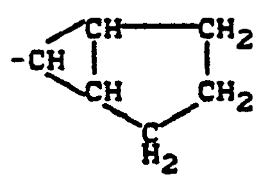, 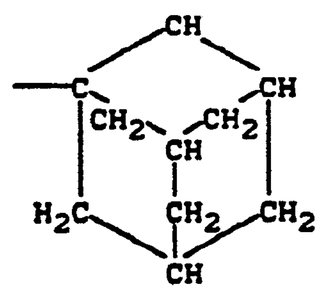,

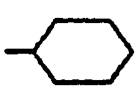, 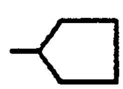, 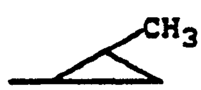, 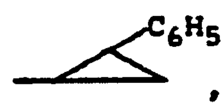,

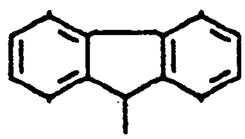 , 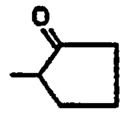 , 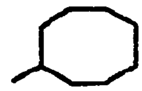 ,

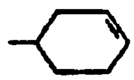 , 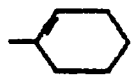 ;

$R^3$ steht außerdem für gegebenenfalls mit 1 oder 2 Benzol-, Cyclopentan- oder Cyclohexanringen anelliertes, gegebenenfalls ein- bis dreifach, gleich oder verschieden substituiertes Phenyl, wobei als Substituenten genannt seien : Fluor, Chlor, Hydroxy, Nitro, Methyl, Methoxy, Acetoxy, Acetamido, Hydroxycarbonyl, Methoxy- und Ethoxycarbonyl ;

$R^3$ steht ferner für gegebenenfalls mit 1 oder 2 Benzol- oder Cyclohexanringen anelliertes, gegebenenfalls ein- bis dreifach, gleich oder verschieden substituiertes 5- oder 6-gliedriges Heterocyclyl mit 1 bis 3 gleichen oder verschiedenen Heteroatomen, wie Stickstoff-, Sauerstoff- und Schwefelatomen, wobei als Substituenten genannt seien : Acyl mit 2 bis 5 Kohlenstoffatomen, Chlor, Brom, Methyl, Ethyl, Phenyl, die Oxogruppe und Hydroxy ; im einzelnen seien als Heterocyclen genannt :

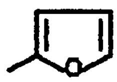 , 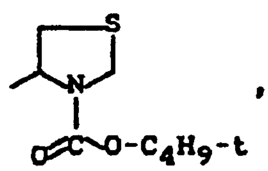 , 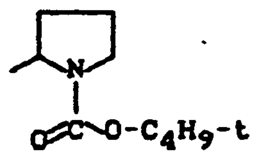 ,

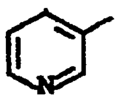 , 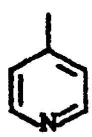 , 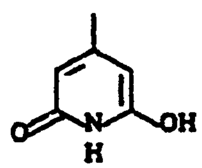 , 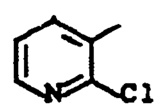 ,

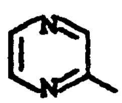 , 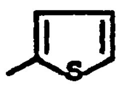 , 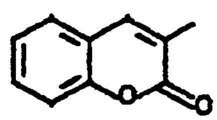 , 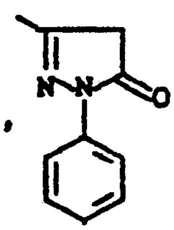 ,

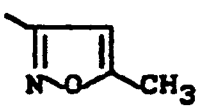 ;

$R^3$ steht schließlich auch für Alkoxycarbonyl mit 1 bis 4 Kohlenstoffatomen im geradkettigen oder verzweigten Alkoxyteil, für —$OR^4$, —$SR^4$ oder —$NR^5R^6$ ;

$R^4$ für gegebenenfalls ein- oder zweifach, gleich oder verschieden substituiertes, geradkettiges oder verzweigtes Alkyl mit 1 oder 2 Kohlenstoffatomen steht, wobei folgende Substituenten infrage kommen : Fluor, Chlor, Methoxy, Ethoxy, Methylthio, Alkoxycarbonyl mit 1 bis 4 Kohlenstoffatomen im geradkettigen oder verzweigten Alkylteil, Carbamoyl, Methylcarbamoyl, Dimethylcarbamoyl, Cyano, Dimethylamino, Diethylamino, Acetyl, Pivaloyl, jeweils gegebenenfalls ein- bis dreifach, gleich oder verschieden durch Halogen und Methyl substituiertes Phenyl oder Phenoxy, jeweils gegebenenfalls ein- bis dreifach durch Methyl substituiertes Cyclopropyl oder Cyclohexyl ;

$R^4$ steht ferner für gegebenenfalls ein- bis dreifach, gleich oder verschieden durch Halogen, Methyl und Ethyl substituiertes 3- bis 6-gliedriges Heterocyclyl mit 1 bis 3 gleichen oder verschiedenen Heteroatomen, wie Stickstoff-, Sauerstoff- und Schwefelatomen ; im einzelnen seien als Heterocyclen genannt :

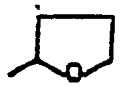, 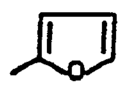, 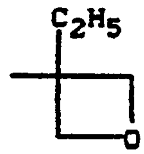, 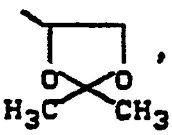, 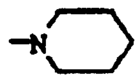,

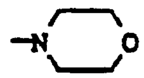, 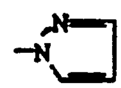, 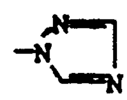;

$R^4$ steht außerdem für jeweils gegebenenfalls ein- oder zweifach durch Methyl substituiertes Allyl oder Propargyl oder für jeweils gegebenenfalls ein- bis dreifach durch Methyl substituiertes Cyclohexyl ;

$R^5$ für Wasserstoff, geradkettiges oder verzweigtes Alkyl mit 1 bis 4 Kohlenstoffatomen oder ein- oder zweifach, gleich oder verschieden substituiertes Alkyl mit 1 bis 3 Kohlenstoffatomen steht, wobei als Substituenten genannt seien : Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, Alkoxycarbonyl mit 1 bis 4 Kohlenstoffatomen im geradkettigen oder verzweigten Alkoxyteil, Carbamoyl, Methylcarbamoyl, Dimethylcarbamoyl, Cyano, Dimethylamino, Diethylamino, gegebenenfalls ein- bis dreifach, gleich oder verschieden durch Chlor und Methyl substituiertes Phenyl, 2-Furyl, 2-Pyridyl, 1-Morpholino, Cyclopropyl oder Cyclohexyl ;

$R^5$ steht ferner ganz besonders bevorzugt für Allyl oder Propargyl, für 1-Cyclohexenyl oder für ein- bis dreifach, gleich oder verschieden substituiertes Cyclohexyl, wobei als Substituenten genannt seien : Chlor, Methyl, Ethyl, Methoxy, Cyano, Amino, Alkyl- und Dialkylamino mit jeweils 1 oder 2 Kohlenstoffatomen in jedem Alkylteil, Carbamoyl, Alkyl- und Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil oder 1-Pyrrolidin-2-on ;

$R^5$ steht außerdem ganz besonders bevorzugt für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkylthio, Alkylsulfinyl und Alkylsulfonyl mit jeweils 1 bis 4 Kohlenstoffatomen substituiertes 1,3,4-Thiadiazol-5-yl oder für die Gruppierung

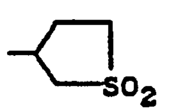,

$R^6$ für Wasserstoff, geradkettiges oder verzweigtes Alkyl mit 1 bis 4 Kohlenstoffatomen oder für ein- oder zweifach, gleich oder verschieden substituiertes Alkyl mit 1 bis 3 Kohlenstoffatomen steht, wobei als Substituenten genannt seien : Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, Alkoxycarbonyl mit 1 bis 4 Kohlenstoffatomen im geradkettigen oder verzweigten Alkoxyteil, Carbamoyl, Methylcarbamoyl, Dimethylcarbamoyl, Cyano, Dimethylamino, Diethylamino, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl ;

$R^6$ steht ferner ganz besonders bevorzugt für Allyl oder Propargyl, für 1-Cyclohexenyl oder für ein- bis dreifach, gleich oder verschieden substituiertes Cyclohexyl, wobei als Substituenten genannt seien : Chlor, Methyl, Ethyl, Methoxy, Cyano, Amino, Alkyl- oder Dialkylamino mit jeweils 1 oder 2 Kohlenstoffatomen in jedem Alkylteil, Carbamoyl, Alkyl- oder Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil oder 1-Pyrrolidin-2-on ;

$R^6$ steht außerdem ganz besonders bevorzugt für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes

2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkylthio, Alkylsulfinyl und Alkylsulfonyl mit jeweils 1 bis 4 Kohlenstoffatomen substituiertes 1,3,4-Thiadiazol-5-yl oder für die Gruppierung

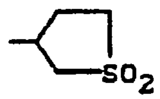 ,

$R^6$ steht weiterhin ganz besonders bevorzugt für Hydroxy, Alkoxy mit 1 oder 2 Kohlenstoffatomen, für gegebenenfalls ein- bis dreifach, gleich oder verschieden durch Chlor und Methyl substituiertes Benzyloxy, für 1-Adamantyl, 2-Norbornyl, 1- oder 2-Decalyl oder 1- oder 2-Tetralyl ;

oder

$R^5$ und $R^6$ gemeinsam mit dem Stickstoffatom, an das sie gebunden sind, für folgende mono-, bi- oder tricyclischen Heterocyclen oder Spiroheterocyclen stehen :

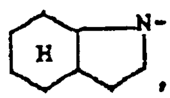 , 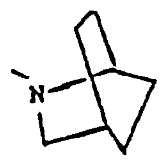 , 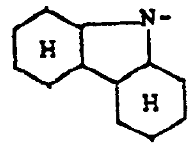 ,

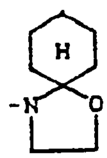 , 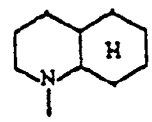 ,

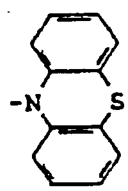 , 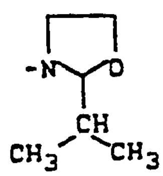 , 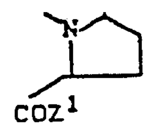

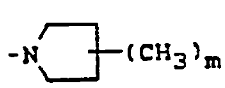 , 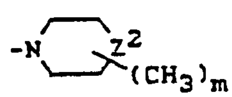

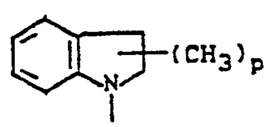 , 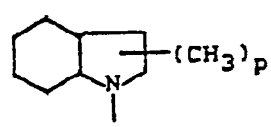 .

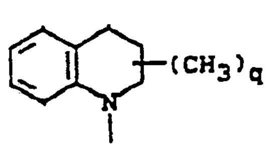 , 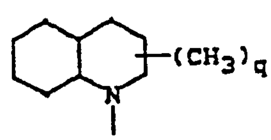 ,

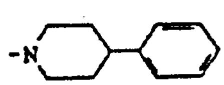 , 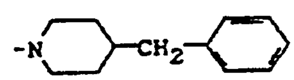 ,

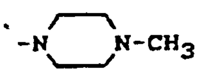 , 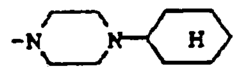 , 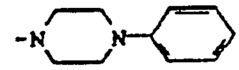 ,

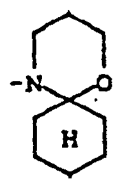 , 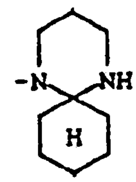 , 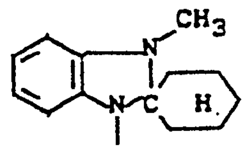 ,

wobei

$Z^1$ für Hydroxy, Methoxy, Ethoxy, Amino, Methylamino, Ethylamino, Dimethylamino oder Ethylmethylamino steht,

$Z^2$ für Sauerstoff oder die $CH_2$-Gruppe steht,

m für die Zahlen 0, 1, 2, 3 oder 4 steht,

p für die Zahlen 0, 1 oder 2 steht,

q für die Zahlen 0, 1, 2 oder 3 steht,

$R^I$ für Methyl, Ethyl, n- oder i-Propyl und n-, i-, s- oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen,

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien; und

X für Sauerstoff oder Schwefel steht.

Als Beispiele für erfindungsgemäße Verbindungen seien außer den bei den Herstellungsbeispielen genannten Verbindungen die in der folgenden Tabelle aufgeführten Stoffe der Formel (I) genannt:

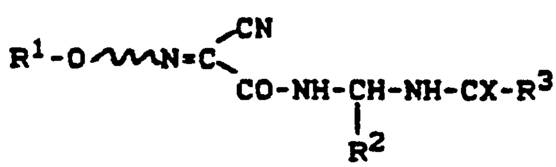 (I)

| $R^1$ | $R^2$ | $R^3$ | X |
|---|---|---|---|
| $CH_3$ | H | H | O |
| $CH_3$ | H | $-CH_2-O-CH_3$ | O |
| $CH_3$ | H | $-CHCl_2$ | O |
| $CH_3$ | H | $-CH_2$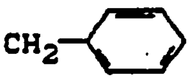 | O |
| $CH_3$ | H | 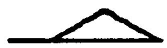 | O |
| $CH_3$ | H | 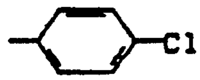 | O |
| $CH_3$ | H | 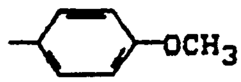 | O |
| $CH_3$ | H | 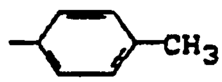 | O |
| $CH_3$ | H | 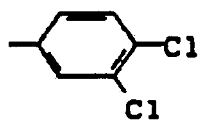 | O |
| $CH_3$ | H | $-CH_2$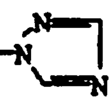 | O |
| $CH_3$ | H | 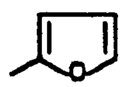 | O |
| $CH_3$ | H | $-O-CH(CH_3)_2$ | O |
| $CH_3$ | H | $-O-CH_2$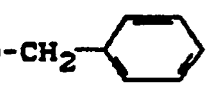 | O |
| $CH_3$ | H | $-O$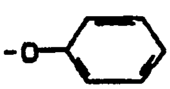 | O |
| $CH_3$ | H | $-N(C_2H_5)_2$ | O |

| $R^1$ | $R^2$ | $R^3$ | X |
|---|---|---|---|
| $CH_3$ | H | -NH 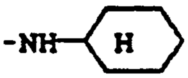 | O |
| $CH_3$ | H | $-S-C_2H_5$ | O |
| $CH_3$ | H | $-NH-CH_3$ | S |
| $CH_3$ | H | -NH 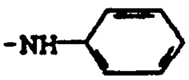 | O |
| $CH_3$ | $CH_3$ | $CH_3$ | O |
| $CH_3$ | $-CH_2$ 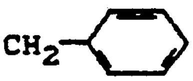 | $CH_3$ | O |
| $-CH_2-C{\equiv}N$ | H | $CH_3$ | O |
| $-CH_2$ 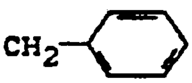 | H | $CH_3$ | O |
| $CH_3$ | H | $-CO-OC_2H_5$ | O |
| $CH_3$ | H | $-O-CH_2-CCl_3$ | O |
| $CH_3$ | H | $-O-CH_2CH_2-OCH_3$ | O |
| $CH_3$ | H | $-O-CH(CH_3)-CO-OC_2H_5$ | O |
| $CH_3$ | H | $-O-CH_2CH_2-CN$ | O |
| $CH_3$ | H | $-O-CH_2-CO-NH_2$ | O |
| $CH_3$ | H | $-O-CH_2CH_2-N(CH_3)_2$ | O |
| $CH_3$ | H | $-O-CH_2CH_2-$ 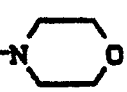 | O |

| $R^1$ | $R^2$ | $R^3$ | X |
|---|---|---|---|
| $CH_3$ | H | $-O-CH_2-$ 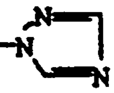 | O |
| $CH_3$ | H | $-O-CH_2$ 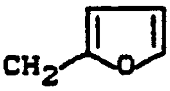 | O |
| $CH_3$ | H | $-O-CH_2$ 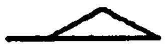 | O |
| $CH_3$ | H | $-O-CH_2-CH_2-O$ 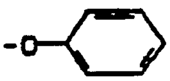 | O |
| $CH_3$ | H | $-O-CH_2-CH=CH_2$ | O |
| $CH_3$ | H | $-O-CH_2-C\equiv CH$ | O |
| $CH_3$ | H | $-O$ 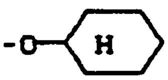 | O |
| $CH_3$ | H | $-CH_2-S-CH_3$ | O |
| $CH_3$ | H | $-CH_2-SO_2-CH_3$ | O |
| $CH_3$ | H | $-CH=CH_2$ | O |
| $CH_3$ | H | $-CH_2-C\equiv N$ | O |
| $CH_3$ | H | $-CH_2-O-CO-CH_3$ | O |
| $CH_3$ | H | $-CH_2-NH-CO-CH_3$ | O |
| $CH_3$ | H | $-CH_2-CO-OCH_3$ | O |
| $CH_3$ | H | $-CH_2$ 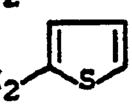 | O |
| $CH_3$ | H | 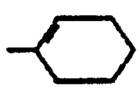 | O |
| $CH_3$ | H | $-CH_2-CH_2$ 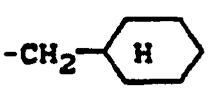 | O |

| $R^1$ | $R^2$ | $R^3$ | X |
|---|---|---|---|
| $CH_3$ | H | 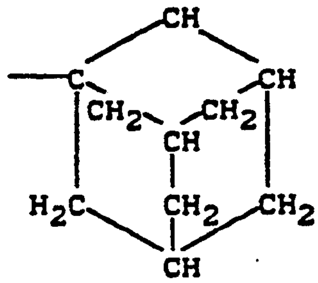 | O |
| $CH_3$ | H | 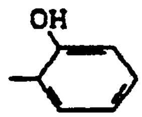 | O |
| $CH_3$ | H | 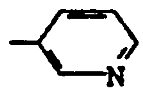 | O |
| $CH_3$ | H | 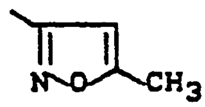 | O |
| $CH_3$ | H | 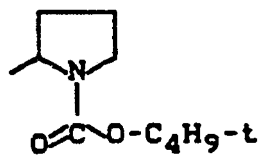 | O |
| $CH_3$ | H | $-NH_2$ | O |
| $CH_3$ | H | 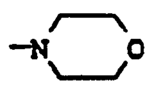 | O |
| $CH_3$ | H | $-NH-CH_2-CO-OC_2H_5$ | O |
| $CH_3$ | H | $-NH-CH_2-CN$ | O |
| $CH_3$ | H | $-NH-CH_2-$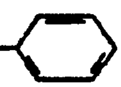 | O |
| $CH_3$ | H | -NH-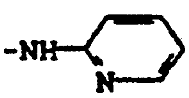 | O |

| $R^1$ | $R^2$ | $R^3$ | X |
|---|---|---|---|
| $CH_3$ | H | $-NH-CH_2-CH=CH_2$ | O |
| $CH_3$ | H | $-NH-CH_2-C\equiv CH$ | O |
| $CH_3$ | H | $-NH-CH_2-CO-N(C_2H_5)_2$ | O |
| $CH_3$ | H | $-N(CH_3)_2$ | S |
| $CH_3$ | H | $-OC_2H_5$ | S |

Verwendet man beispielsweise N-Aminomethyl-(E)-2-cyan-2-methoximinoacetamid-hydrochlorid als Ausgangsstoff, Acetylchlorid als Acylierungsreagenz und Triethylamin als Base und 4-Dimethylaminopyridin als Katalysator, so kann der Ablauf des erfindungsgemäßen Verfahrens (a) durch das folgende Formelschema wiedergegeben werden :

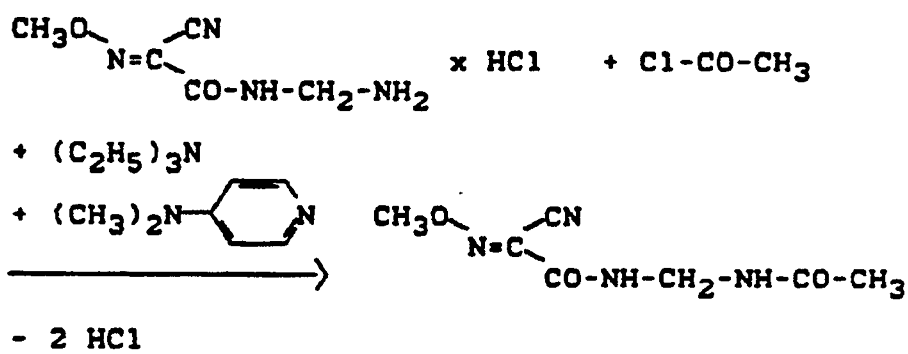


Verwendet man beispielsweise (E)-2-Cyan-2-methoximinoacetaminomethylisocyanat und Methylamin als Ausgangsstoffe, so kann der Ablauf des erfindunsggemäßen Verfahrens (b) durch das folgende Formelschema wiedergegeben werden :

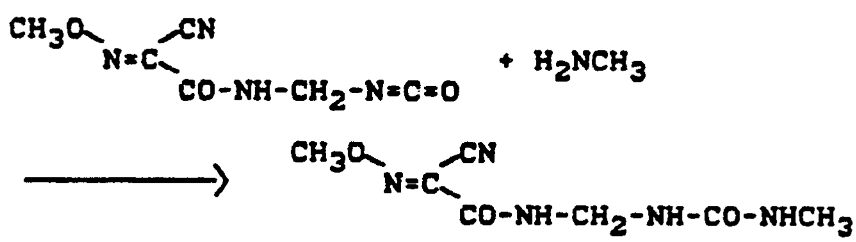


Die für die Durchführung des erfindungsgemäßen Verfahrens (a) als Ausgangsstoffe zu verwendenden 2-Cyan-2-oximino-acetamide sind durch die Formel (II) allgemein definiert. In dieser Formel haben $R^1$ und $R^2$ die Bedeutungen, die bereits im Zusammenhang mit der Beschreibung der erfindungsgemäßen Stoffe der Formel (I) für diese Substituenten genannt wurden, auch die vorzugsweisen, besonders bevorzugten und ganz besonders bevorzugten Bedeutungen von den Seiten 12-45. HY steht vorzugsweise für das Äquivalent einer Mineralsäure, wie z.B. Chlorwasserstoffsäure oder einer Carbonsäure, wie z.B. Oxalsäure.

Die 2-Cyan-2-oximino-acetamide der Formel (II) sind noch nicht bekannt. Sie können jedoch in bekannter Art und Weise hergestellt werden, indem man Isocyanate der Formel (IV),

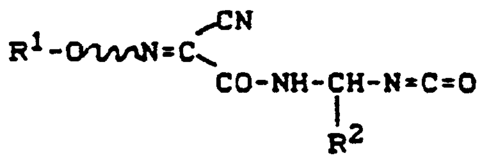

(IV)

in welcher

$R^1$ und $R^2$ die oben angegebene Bedeutung haben, in üblicher Weise hydrolisiert und das entstehende Amin in üblicher Weise als Salz isoliert.

Die Hydrolyse der Isocyanate der Formel (IV) wird in Gegenwart eines Verdünnungsmittels durchgeführt. Hierbei kommen vorzugsweise infrage : Wasser ; Nitrile, wie Acetonitril ; Ether, wie Tetrahydrofuran, Dioxan oder Dimethylether oder Ketone, wie Aceton.

Die Reaktionstemperaturen können bei der Hydrolyse der Isocyanate der Formel (IV) in einem größeren Bereich variiert werden. Im allgemeinen arbeitet man zwischen −20°C und 150°C, vorzugsweise zwischen 20°C und 80°C.

Bei der Durchführung der Hydrolyse der Isocyanate der Formel (IV) werden diese vorzugsweise in situ (zur Herstellung vergleiche die Angaben zum erfindungsgemäßen Verfahren (b)) mit überschüssigem Wasser umgesetzt. Die Isolierung des entstehenden Amins als Salz erfolgt durch Hinzufügen der Säure, z.B. Chlorwasserstoffsäure und anschließende übliche Aufarbeitung.

Die außerdem zur Durchführung des erfindungsgemäßen Verfahrens (a) als Ausgangsstoffe zu verwendenden Acylierungsreagenzien sind durch die Formeln (IIIa) und (IIIb) allgemein definiert. In der Formel (IIIa) steht X vorzugsweise für Sauerstoff oder Schwefel und Z steht vorzugsweise für eine Abgangsgruppe. Hierzu gehören vorzugsweise Chlor, Brom, Alkoxy und Alkylthio mit jeweils 1 bis 4 Kohlenstoffatomen, Carboxymethoxy, Carboxymethylthio, die Gruppierungen —O-CO-$R^3$, —O-CO-O$R^4$ und —S$R^4$. Dabei haben $R^3$ und $R^4$ vorzugsweise die Bedeutungen, die bereits im Zusammenhang mit der Beschreibung der erfindungsgemäßen Stoffe der Formel (I) für diese Substituenten vorzugsweise genannt wurden. In der Formel (IIIb) haben X und $R^6$ vorzugsweise die Bedeutungen, die bereits im Zusammenhang mit der Beschreibung der erfindungsgemäßen Stoffe der Formel (I) für diese Substituenten vorzugsweise genannt wurden.

Die Acylierungsreagenzien der Formeln (IIIa) und (IIIb), d.h. Carbonsäurehalogenide, Carbonsäureanhydride, Halogenameisensäurester und -thiolester, Trithiocarbonate, Pyrocarbonate, Carbamidsäurehalogenide, Carbamate, Thiolcarbamate, Dithiocarbamate, Isocyanate oder Isothiocyanate sind allgemein bekannte Verbindungen der organischen Chemie bzw. nach allgemein üblichen Methoden erhältlich.

Die für die Durchführung des erfindungsgemäßen Verfahrens (b) als Ausgangsstoffe und für das erfindunsggemäße Verfahren (a) als Vorprodukte zu verwendenden Isocyanate sind durch die Formel (IV) allgemein definiert. In dieser Formel haben $R^1$ und $R^2$ die Bedeutungen, die bereits im Zusammenhang mit der Beschreibung der erfindungsgemäßen Stoffe der Formel (I) für diese Substituenten genannt wurden, auch die vorzugsweisen, besonders bevorzugten und ganz besonders bevorzugten Bedeutungen von den Seiten 12-45.

Die Isocyanate der Formel (IV) sind noch nicht bekannt. Sie können jedoch in bekannter Art und Weise hergestellt werden, indem man z.B.

α) Amide der allgemeinen Formel (VI),

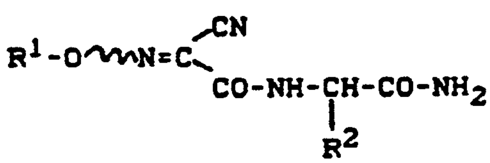

(VI)

in welcher

$R^1$ und $R^2$ die oben angegebene Bedeutung haben, in üblicher Weise, gegebenenfalls in Gegenwart eines Verdünnungsmittels oxidiert ("Hofmann-Abbau"), wobei die entstehenden Isocyanate der Formel (IV) gegebenenfalls auch ohne Isolierung direkt weiter umgesetzt werden können ;

oder

β) Azide der allgemeinen Formel (VII),

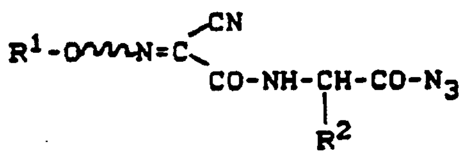 (VII)

in welcher

$R^1$ und $R^2$ die oben angegebene Bedeutung haben, in Gegenwart eines Verdünnungsmittels erwärmt ("Curtius-Abbau"), wobei die entstehenden Isocyanate der Formel (IV) gegebenenfalls auch ohne Isolierung direkt weiter umgesetzt werden können.

Als Oxidationsmittel für die Verfahrensvariante (α) seien vorzugsweise genannt : Natriumhypochlorit oder -bromit, Bleitetraacetat sowie I,I-Bis(trifluoracetoxy)-

iodbenzol [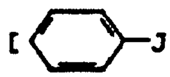$-J(OCOCF_3)_2$ = J. Chem.Soc.Chem.Com. 1982, S. 280].

Als Verdünnungsmittel kommen für die Verfahrensvariante (α) inerte organische Lösungsmittel infrage, wie beispielsweise Ether, wie Tetrahydrofuran, Dioxan oder Dimethylether oder Nitrile, wie Acetonitril. Für den Fall einer direkten weiteren Umsetzung der Isocyanate der Formel (IV) kommen als Verdünnungsmittel auch Wasser oder Alkohole infrage oder deren Mischungen mit inerten organischen Lösungsmitteln, wie beispielsweise Wasser/Acetonitril.

Die Reaktionstemperaturen können bei der Oxidation gemäß der Verfahrensvariante (α) in einem größeren Bereich variiert werden. Im allgemeinen arbeitet man zwischen −20°C und 120°C, vorzugsweise zwischen 0°C und 40°C.

Bei der Durchführung der Oxidation gemäß der Verfahrensvariante (α) wird vorzugsweise mit einem kleinen Überschuß an Oxidationsmittel gearbeitet, wobei in der Regel die Isocyanate der Formel (IV) ohne Isolierung direkt weiter umgesetzt werden.

Als Verdünnungsmittel für die Verfahrensvariante (β) kommen inerte organische Lösungsmittel infrage, wie beispielsweise aromatische Kohlenwasserstoffe, wie Toluol oder Chlorbenzol ; Ether, wie Dioxan oder halogenierte Kohlenwasserstoffe, wie Chloroform oder Methylenchlorid. Für den Fall einer direkten weiteren Umsetzung der Isocyanate der Formel (IV) kommen als Verdünnungsmittel auch Wasser oder Alkohole infrage oder deren Mischungen mit inerten organischen Lösungsmitteln.

Die Reaktionstemperaturen können bei der Durchführung der Verfahrensvariante (β) in einem größeren Bereich variiert werden. Im allgemeinen arbeitet man zwischen 0°C und 150°C, vorzugsweise zwischen 60°C und 100°C.

Bei der Durchführung der Verfahrensvariante (β) werden die entstehenden Isocyanate der Formel (IV) in der Regel ohne Isolierung direkt weiter umgesetzt.

Die Amide der Formel (VI) und die Azide der Formel (VII) sind bekannt (vergleiche hierzu z.B. EP-OS 0206004 und DE-OS 3602243) bzw. können sie nach den dort beschriebenen Verfahren erhalten werden.

Die außerdem für die Durchführung des erfindungsgemäßen Verfahrens (b) als Ausgangsstoffe zu verwendenden protischen Nukleophile sind durch die Formeln (Va) und (Vb) allgemein definiert. In der Formel (Va) steht $R^3$ vorzugsweise für die Bedeutungen, die bereits im Zusammenhang mit der Beschreibung der erfindungsgemäßen Stoffe der Formel (I) für diesen Substituenten vorzugsweise gennant wurden. In der Formel (Vb) steht $R^7$ vorzugsweise für die Gruppierungen $—OR^4$, $—SR^4$ oder $—NR^5R^6$. Dabei haben $R^4$, $R^5$ und $R^6$ vorzugsweise die Bedeutungen, die bereits im Zusammenhang mit der Beschreibung der erfindungsgemäßen Stoffe der Formel (I) für diese Substituenten vorzugsweise genannt wurden.

Die protischen Nukleophile der Formeln (Va) und (Vb), d.h. Carbonsäuren, Alkohole, Thiole und Amine sind allgemein bekannte Verbindungen der organischen Chemie bzw. nach allgemein üblichen Methoden erhältlich.

Als Verdünnungsmittel kommen für das erfindungsgemäße Verfahren (a) inerte organische Lösungsmittel infrage. Hierzu gehören vorzugsweise aromatische Kohlenwasserstoffe, wie Toluol ; halogenierte Kohlenwasserstoffe, wie Methylenchlorid oder Chloroform ; Ether, wie Tetrahydrofuran oder 1,2-Dimethoxyethan ; Ester, wie Essigsäureethylester ; Nitrile, wie Acetonitril ; Ketone, wie Aceton ; tertiäre Amine, wie Pyridin ; Amide, wie Dimethylformamid oder auch ein entsprechender Überschuß an Acylierungsreagenz der Formeln (IIIa) und (IIIb).

Das erfindungsgemäße Verfahren (a) wird in Gegenwart einer Base durchgeführt. Hierbei kommen übliche organische und anorganische Basen infrage. Vorzugsweise genannt seien teriäre Amine, wie Triethylamin oder

Pyridin ; Alkoholate, wie Natriummethylat und Alkalicarbonate, wie Kaliumcarbonat.

Das erfindungsgemäße Verfahren (a) wird gegebenenfalls in Gegenwart eines Katalysators durchgeführt. Beispielsweise genannt seien tertiäre Amine, wie 4-Dimethylaminopyridin, 1,8-Diazabicyclo[5,4,0]-undec-7-en (DBU) oder 1,4-Diazabicyclo[2,2,2]-octan (DABCO) ; ferner Imidazol und Dimethylformamid.

Die Reaktionstemperaturen können bei der Durchführung des erfindungsgemäßen Verfahrens (a) in einem größeren Bereich variiert werden. Im allgemeinen arbeitet man zwischen −20°C und 120°C, vorzugsweise zwischen 0°C und 40°C.

Bei der Durchführung des erfindungsgemäßen Verfahrens (a) arbeitet man vorzugsweise in äquimolaren Mengen, wobei jedoch auch die 2-Cyan-2-oximino-acetamide der Formel (II) oder die Acylierungsreagenzien der Formeln (IIIa) und (IIIb) im Überschuß eingesetzt werden können. Die Reaktionsdurchführung, Aufarbeitung und Isolierung der Reaktionsprodukte der Formel (I) erfolgt in allgemein üblicher Weise.

Als Verdünnungsmittel kommen für das erfindungsgemäße Verfahren (b) inerte organische Lösungsmittel infrage. Hierzu gehören vorzugsweise aromatische Kohlenwasserstoffe, wie Toluol ; halogenierte Kohlenwasserstoffe, wie Methylenchlorid ; Ether, wie Tetrahydrofuran oder Dimethoxyethan ; Ester, wie Essigsäureethylester ; Nitrile, wie Acetonitril oder auch ein entsprechender Überschuß an protischem Nucleophil der Formeln (Va) und (Vb).

Das erfindungsgemäße Verfahren (b) wird gegebenenfalls in Gegenwart eines Katalysators durchgeführt. Beispielsweise seien die beim Verfahren (a) bereits aufgeführten Katalysatoren genannt.

Die Reaktionstemperaturen können bei der Durchführung des erfindungsgemäßen Verfahrens (b) in einem größeren Bereich variiert werden. Im allgemeinen arbeitet man zwischen −20°C und 150°C, vorzugsweise zwischen 20°C und 80°C.

Bei der Durchführung des erfindungsgemäßen Verfahrens (b) wird das Isocyanat der Formel (IV) vorzugsweise in situ mit einem Überschuß an Nucleophil der Formeln (Va) oder (Vb) umgesetzt, wobei diese gleichzeitig als Lösungsmittel verwendet werden. Es ist aber auch möglich, die isolierten Isocyanate der Formel (IV) in äquimolaren Mengen mit den Nucleophilen der Formeln (Va) und (Vb) umzusetzen. Die Reaktionsführung, Aufarbeitung und Isolierung der Reaktionsprodukte der Formel (I) erfolgt in allgemein üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet.

Zum Beispiel werden fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt.

Pythium-Arten, wie beispielsweise Pythium ultimum ;
Phytophthora-Arten, wie beispielsweise Phytophthora infestans ;
Pseudoperonospora-Arten, wie beispielsweise Pseudoperonospora humuli oder Pseudoperonospora cubensis ;
Plasmopara-Arten, wie beispielsweise Plasmopara viticola ;
Peronospora-Arten, wie beispielsweise Peronospora pisi oder P. brassicae ;
Erysiphe-Arten, wie beispielsweise Erysiphe graminis ;
Sphaerotheca-Arten, wie beispielsweise Sphaerotheca fuliginea ;
Podosphaera-Arten, wie beispielsweise Podosphaera leucotricha ;
Venturia-Arten, wie beispielsweise Venturia inaequalis ;
Pyrenophora-Arten, wie beispielsweise Pyrenophora teres oder P. graminea (Konidienform : Drechslera, Syn : Helminthosporium) ;
Cochliobolus-Arten, wie beispielsweise Cochliobolus sativus (Konidienform : Drechslera, Syn : Helminthosporium) ;
Uromyces-Arten, wie beispielsweise Uromyces appendiculatus ;
Puccinia-Arten, wie beispielsweise Puccinia recondita ;
Tilletia-Arten, wie beispielsweise Tilletia caries ;
Ustilago-Arten, wie beispielsweise Ustilago nuda oder Ustilago avenae ;
Pellicularia-Arten, wie beispielsweise Pellicularia sasakii ;
Pyricularia-Arten, wie beispielsweise Pyricularia oryzae ;
Fusarium-Arten, wie beispielsweise Fusarium culmorum ;
Botrytis-Arten, wie beispielsweise Botrytis cinerea ;
Septoria-Arten, wie beispielsweise Septoria nodorum ;
Leptosphaeria-Arten, wie beispielsweise Leptosphaeria nodorum ;
Cercospora-Arten, wie beispielsweise Cercospora canescens ;

Pseudocercosporella-Arten, wie beispielsweise Pseudocerco sporella herpotrichoides.

Die gute Pflanzenverträglichkeit der Wirkstoffe in der zur Bekämpfung von Pflanzenkrankheiten notwendigen Konzentrationen erlaubt eine Behandlung von oberirdischen Pflanzenteilen, von Pflanz- und Saatgut, und des Bodens.

In Schädlingsbekämpfungsmitteln können die erfindungsgemäßen Wirkstoffe mit besonders gutem Erfolg zur Bekämpfung von Phytophthora-Arten, wie beispielsweise Phytophthora infestans, an Tomaten eingesetzt werden ; sowie auch zur Bekämpfung von Plasmopara-Arten, wie beispielsweise Plasmopara viticola, an Reben.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche beinormaler Temperatur und unter Normaldruck gasförmig sind, z.B. Aerosol-Treibgase, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage : z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von

0,0001 bis 0,02 Gew.-%, am Wirkungsort erforderlich.

Herstellungsbeispiele

Beispiel 1

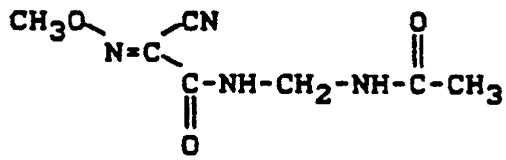

(Verfahren a)

7,7 g (0,04 Mol) N-Aminomethyl-(E)-2-cyan-2-methoximinoacetamid-hydrochlorid werden in 150 ml Methylenchlorid suspendiert, bei 0°C 3,2 g (0,04 Mol) Acetylchlorid und anschließend eine Lösung von 8,08 g Triethylamin und 0,49 g (0,004 Mol) 4-Dimethylaminopyridin in 40 ml Methylenchlorid zugetropft. Man rührt 1 Stunde bei 0°C und 18 Stunden bei Raumtemperatur, wäscht je zweimal mit 1 molarer Citronensäure, 10-prozentiger Natriumhydrogencarbonat- und gesättigter Kochsalzlösung, trocknet über Natriumsulfat und dampft im Vakuum ein.

Man erhält 3,7 g als erste Rohfraktion.

Die vereinigten Waschwässer werden 5 mal mit Methylenchlorid ausgeschüttelt, die vereinigten Extrakte über Natriumsulfat getrocknet und eingedampft. Der Rückstand (2,8 g) wird zusammen mit der 1.Fraktion aus Isopropanol/Petrolether umkristallisiert. Man erhält 4,95 g (62% der Theorie) N-Acetamidomethyl-(E)-2-cyan-2-methoximinoacetamid vom Schmelzpunkt 162-163°C.

Herstellung des Ausgangsproduktes

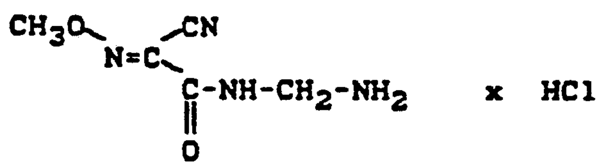

Eine Lösung von 12,4 g (0,067 Mol) N$^{\alpha}$-[(E)-2-Cyan-2-methoximinoacetyl]-glycinamid in 150 ml Acetonitril/Wasser (1 : 1) wird mit 31 g (0,07 Mol) I,I-Bis-(trifluoracetoxy)-iodbenzol (98%ig) versetzt und das Gemisch unter Durchleiten von Stickstoff 5 Stunden bei Raumtemperatur gerührt. Man dampft im Vakuum ein, trocknet den öligen Rückstand durch zweimaliges Abdampfen mit Essigester, löst ihn in 70 ml Essigester und gibt 10 ml einer 25 prozentigen etherischen Chlorwasserstofflösung hinzu. Der Niederschlag wird mit Ether im Mörser verrieben und aus Acetonitril/Ether umkristallisiert.

Man erhält 9,8 g (76% der Theorie) N-Aminomethyl-(E)-2-cyan-2-methoximinoacetamid-hydrochlorid vom Schmelzpunkt 122-124°C (Zersetzung).

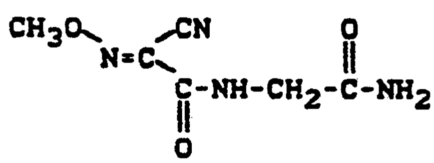

In eine Lösung von 56,6 g (0,252 Mol) N-[(E)-2-Cyan-2-methoximinoacetyl]-glycinethylester (95 prozentig) in 300 ml Isopropanol leitet man bei 0 bis 10°C bis zur Sättigung Ammoniak ein und läßt die Mischung 4 Tage bei Raumtemperatur stehen. Während dieser Zeit wird noch 2 mal Ammoniak eingeleitet. Nach Abkühlung auf 0°C wird der Niederschlag abgesaugt, mit kaltem Isopropanol gewaschen und bei Raumtemperatur getrocknet.

Man erhält 32 g (69% der Theorie) N$^{\alpha}$-[(E)-2-Cyan-2-methoximinoacetyl]-glycinamid vom Schmelzpunkt 170-172°C.

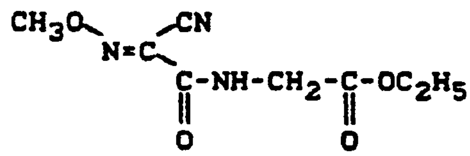

Es werden 60,5 g (0,427 Mol) Glycinethylester-hydrochlorid in 450 ml Dichlormethan suspendiert, 86,2 g (0,853 Mol) Triethylamin und 5,2 g (0,043 Mol) 4-Dimethylaminopyridin werden dazugegeben, das Reaktionsgemisch wird 15 Minuten bei 20°C gerührt und eine Lösung von 62,5 g (0,427 Mol) 2-Cyan-2-methyl-oximinoacetylchlorid (E-Isomeres) wird bei 0°C in einer Stunde zugetropft. Anschließend wird das Reaktionsgemisch eine Stunde lang bei 0°C und dann 5 Stunden bei Raumtemperatur gerührt und die Lösung 2 Tage bei Raumtemperatur stehengelassen. Nach Waschen mit 1 M Salzsäure (2 mal je 300 ml), gesättiger Natriumhydrogencarbonatlösung (2 mal je 200 ml) und Wasser (2 mal je 300 ml) wird die Lösung über Natriumsulfat getrocknet und im Vakuum eingedampft.

Man erhält 81,0 g (89% der Theorie) $N^{\alpha}$-(2-Cyan-2-methoximino-acetyl)-glycinethylester (E-Isomeres) als braunes Öl mit dem Brechungsindex $n_D^{23}$ = 1,4699.

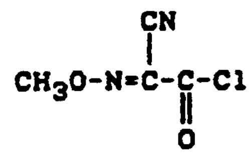

20 g (0,12 Mol) des Kaliumsalzes von 2-Cyan-2-methoximinoacetat (E-Isomeres) werden in 250 ml trockenem Ether suspendiert und nach Zugabe von einigen Tropfen Dimethylformamid bei 0°C tropfenweise mit 76,2 g (0,6 Mol) Oxalylchlorid versetzt. Das Reaktionsgemisch wird 2 Stunden bei 0°C gerührt, filtriert und das Filtrat im Vakuum bei Raumtemperatur eingedampft.

Man erhält 13,7 g (77% der Theorie) 2-Cyan-2-methoximinoacetylchlorid (E-Isomeres) als gelbes Öl, das sofort weiter umgesetzt wird.

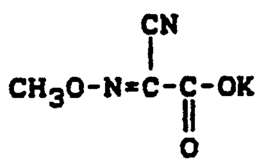

In eine Lösung von 124,8 g (0,672 Mol) 84%igem 2-Cyan-2-methoximino-essigsäureethylesters (E-Isomeres) in 500 ml Ethanol wird bei 20°C eine Lösung von 45,1 g (0,806 Mol) Kaliumhydroxid in 500 ml Wasser getropft und das Reaktionsgemisch eine Stunde bei 40°C gerührt. Die Lösung wird im Vakuum bei 40°C eingedampft, der Rückstand mit Methanol 30 Minuten verrührt, abgesaugt, mit Ethanol, Acetonitril und Dichlormethan gewaschen und bei Raumtemperatur getrocknet.

Man erhält 58,6 g (53% der Theorie) des Kaliumsalzes von 2-Cyan-2-methoximino-acetat (E-Isomeres).

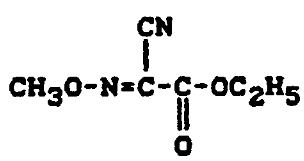

In eine Suspension von 164 g (1 Mol) 2-Cyan-2-hydroximinoessigsäureethylester, Natriumchlorid (G. Kinast, Liebigs Ann. Chem., 1981, 1561) und 138 g pulverisiertem Kaliumcarbonat in 1,5 l Aceton werden 161 g (1,25 Mol) Dimethylsulfat (98%ig) in 30 Minuten getropft und das Reaktionsgemisch 3 Stunden unter Rückfluß erhitzt. Nach Abkühlen wird über Kieselgur filtriert und eingedampft.

Man erhält 124,8 g 2-Cyan-2-methoximino-essigsäure-ethylester (E-Isomeres) als rotbraunes Öl von 85% Reinheit (GC). Die Ausbeute beträgt demnach 68% der Theorie. Nach Chromatographie an der fünffachen Menge Kieselgel 60 mit Chloroform erhält man ein 93%iges hellgelbes Öl.

In analoger Weise und gemäß den erfindungsgemäßen Verfahren (a) und (b) werden die nachfolgenden

substituierten Aminal-Derivate der allgemeinen Formel (I) erhalten :

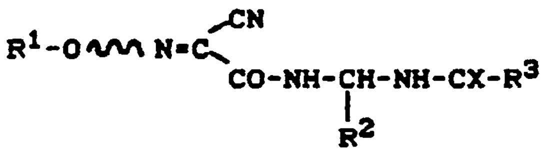

(I)

| Bsp. Nr. | $R^1$ | $R^2$ | X | $R^3$ | Schmelzpunkt (°C) |
|---|---|---|---|---|---|
| 2 | $CH_3$ | H | O | $-C_2H_5$ | 118-20 (E-Isomeres) |
| 3 | $CH_3$ | H | O | $-C_3H_7$-i | 145-47 (E-Isomeres) |
| 4 | $CH_3$ | H | O | 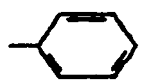 | 165-67 (E-Isomeres) |
| 5 | $CH_3$ | H | O | $-OCH_3$ | 99-102 (E-Isomeres) |
| 6 | $CH_3$ | H | O | $-OC_2H_5$ | 76-78 (E-Isomeres) |
| 7 | $CH_3$ | H | O | $-NHCH_3$ | 141-42 (E-Isomeres) |
| 8 | $CH_3$ | H | O | $-CH_2OCH_3$ | 89-91 (E-Isomeres) |
| 9 | $CH_3$ | H | O | -H | 130-31 (E-Isomeres) |
| 10 | $CH_3$ | H | O | 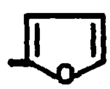 | 138-39 (E-Isomeres) |
| 11 | $CH_3$ | H | O | $-CH_2-Cl$ | 133-35 (E-Isomeres) |
| 12 | $CH_3$ | H | O | $-CH_2OCOCH_3$ | 111-13 (E-Isomeres) |
| 13 | $CH_3$ | H | O | $-CHCl_2$ | 148-50 (E-Isomeres) |

| Bsp. Nr. | $R^1$ | $R^2$ | X | $R^3$ | Schmelzpunkt (°C) |
|---|---|---|---|---|---|
| 14 | $CH_3$ | H | O | $-CH_2-$ 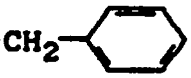 | 125-27 (E-Isomeres) |
| 15 | $CH_3$ | H | O | 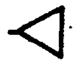 | 156-58 (E-Isomeres) |
| 16 | $CH_3$ | H | O | $-COOC_2H_5$ | 95-97 (E-Isomeres) |
| 17 | $CH_3$ | H | O | $-CF_3$ | 120-21 (E-Isomeres) |
| 18 | $CH_3$ | H | O | $-CH_2-$ 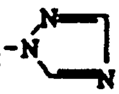 | 202-05 (E-Isomeres) |
| 19 | $CH_3$ | H | O | $-CH_2-NH-CO-O-CH_2-$ 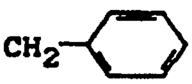 | 133-35 (E-Isomeres) |
| 20 | $CH_3$ | H | O | $-CH_2-NH-CO-O-C(CH_3)_3$ | amorph (E-Isomeres) |
| 13 | $CH_3$ | H | O | $-CH_2-NH-CO-OC_2H_5$ | 119-20 (E-Isomeres) |

Anwendungsbeispiele

In den folgenden Anwendungsbeispielen werden die nachstehend aufgeführten Verbindungen als Vergleichssubstanzen eingesetzt:

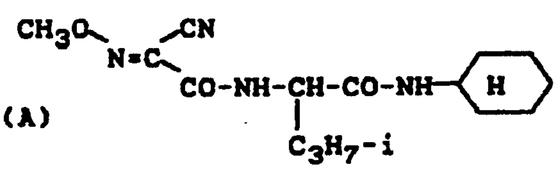

(A)

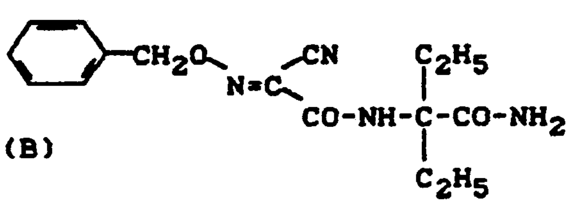

(B)

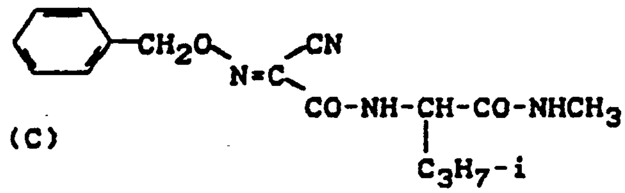

(C)

(bekannt aus DE-OS 35 21 131).

Beispiel A

Plasmopara-Test (Reben)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 22°C und ca. 80% Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen : 1, 2, 3, 4, 5, 6 und 7.

Beispiel B

Phytophthora-Test (Tomate)/protektiv

Lösungsmittel : 4,7 Gewichtsteile Aceton
Emulgator : 0,3 Gewichtsteile Alkylarylpolyglkol ether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen : 1, 2, 3, 4, 5, 6 und 7.

**Patentansprüche**

**Patentansprüche für folgende Vertragsstaaten : BE, CH, DE, FR, GB, IT, LI, NL**

1. N,N'-Diacylaminale der allgemeinen Formel (I)

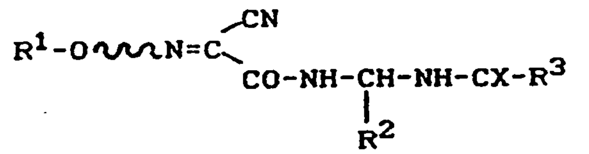

(I)

in welcher

$R^1$ für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen : Fluor, Chlor, Brom, lod, Cyano, —$COOR^I$, —$CONR^{II}R^{III}$, —$OR^{IV}$, Acyl mit 2 bis 9 Kohlenstoffatomen, gegebenenfalls ein- bis fünffach, gleich oder verschieden substituiertes Phenyl, wobei als Phenylsubstituenten genannt seien : Halogen, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen ; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen ; $R^1$ steht ferner für jeweils gegebenenfalls ein- bis dreifach, gleich oder verschieden substituiertes, geradkettiges oder verzweigtes Alkenyl oder Alkinyl mit 2 bis 6 Kohlenstoffatomen, wobei als Substituenten genannt seien : gegebenenfalls ein- bis fünffach, gleich oder verschieden durch Halogen und Alkyl mit 1 bis 4 Kohlenstoffatomen substituiertes Phenyl ; $R^1$ steht außerdem für jeweils gegebenenfalls ein-bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ;

$R^2$ für Wasserstoff oder für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Cyano, Alkoxy und Alkylthio mit jeweils 1 bis 4 Kohlenstoffatomen, —$COOR^I$, Acylamino mit 2 bis 9 Kohlenstoffatomen, gegebenenfalls ein- bis fünffach, gleich oder verschieden substituiertes Phenyl, wobei als Substituenten die bei $R^1$ bereits genannten Phenylsubstituenten infrage kommen ; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen ; $R^2$ steht ferner für jeweils gegebenenfalls ein- bis dreifach, gleich oder verschieden substituiertes, geradkettiges oder verzweigtes Alkenyl oder Alkinyl mit 2 bis 6 Kohlenstoffatomen, wobei als Substituenten genannt seien : gegebenenfalls ein- bis fünffach, gleich oder verschieden durch Halogen und Alkyl mit 1 bis 4 Kohlenstoffatomen substituiertes Phenyl ; $R^2$ steht außerdem für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ; $R^2$ steht weiterhin für gegebenenfalls ein- bis fünffach, gleich oder verschieden substituiertes Phenyl, wobei als Substituenten die bei $R^1$ bereits genannten Phenylsubstituenten infrage kommen ; $R^2$ steht ferner für gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen ;

$R^3$ für Wasserstoff oder für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Fluor, Chlor, Brom, lod, Cyano, Alkoxy, Alkylthio, Alkylsulfinyl und Alkylsulfonyl mit jeweils 1 bis 4 Kohlenstoffatomen, Acyloxy und Acylamino mit jeweils 2 bis 9 Kohlenstoffatomen, —$COOR^I$, gegebenenfalls ein-bis fünffach, gleich oder verschieden substituiertes Phenyl, wobei als Substituenten die bei $R^1$ bereits genannten Phenylsubstituenten infrage kommen ; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen sowie gegebenenfalls ein-bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen ; $R^3$ steht ferner für jeweils gegebenenfalls ein- bis dreifach, gleich oder verschieden substituiertes, geradkettiges oder verzweigtes Alkenyl oder Alkinyl mit jeweils 2 bis 6 Kohlenstoffatomen, wobei als Substituenten genannt seien :

gegebenenfalls ein- bis fünffach, gleich oder verschieden durch Halogen und Alkyl mit 1 bis 4 Kohlenstoffatomen substituiertes Phenyl ; $R^3$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis fünffach, gleich oder verschieden substituiertes Cycloalkyl mit 3 bis 6 Kohlenstoffatomen oder Cycloalkenyl mit 5 bis 7 Kohlenstoffatomen, wobei als Substituenten genannt seien : Halogen, Alkyl und Alkoxy mit jeweils

1 bis 4 Kohlenstoffatomen, Acyloxy und Acylamino mit jeweils 2 bis 5 Kohlenstoffatomen, die Oxogruppe, Phenyl, Hydroxycarbonyl und Alkoxycarbonyl mit 1 bis 4 Kohlenstoffatomen im Alkoxyteil ; $R^3$ steht außerdem für gegebenenfalls mit 1 oder 2 Benzol- oder Cyclohexanringen anelliertes, gegebenenfalls ein- bis fünffach, gleich oder verschieden substituiertes Phenyl, wobei als Substituenten genannt seien : Halogen, Hydroxy, Nitro, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen, Acylamino und Acylalkylamino mit jeweils 2 bis 5 Kohlenstoffatomen im Acylteil und 1 bis 4 Kohlenstoffatomen im Alkylteil, Hydroxycarbonyl und Alkoxycarbonyl mit 1 bis 4 Kohlenstoffatomen im Alkoxyteil ; $R^3$ steht ferner für gegebenenfalls mit 1 oder 2 Benzol-, Cyclopentan oder Cyclohexanringen anelliertes, gegebenenfalls ein- bis fünffach, gleich oder verschieden substituiertes 5- oder 6-gliedriges Heterocyclyl mit 1 bis 3 gleichen oder verschiedenen Heteroatomen, wobei als Substituenten genannt seien : Halogen, Alkyl mit 1 bis 4 Kohlenstoffatomen, Acyl mit 2 bis 9 Kohlenstoffatomen, Phenyl, die Oxogruppe und Hydroxy ; $R^3$ steht schließlich auch für Alkoxycarbonyl mit 1 bis 4 Kohlenstoffatomen im Alkoxyteil, für $—OR^4$, $—SR^4$ oder $—NR^5R^6$ ;

$R^4$ für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen : Halogen, $—OR^{IV}$, $—SR^{IV}$, $—COOR^{I}$, $—CONR^{II}R^{III}$, CN, $—NR^{II}R^{III}$, Acyl mit 2 bis 9 Kohlenstoffatomen, jeweils gegebenenfalls einfach bis fünffach, gleich oder verschieden durch Halogen und Alkyl mit 1 bis 4 Kohlenstoffatomen substituiertes Phenyl oder Phenoxy, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen, gegebenenfalls ein- bis fünffach, gleich oder verschieden durch Halogen und Alkyl mit 1 bis 4 Kohlenstoffatomensubstituiertes 3- bis 6-gliedriges Heterocyclyl mit 1 bis 3 gleichen oder verschiedenen Heteroatomen ; $R^4$ steht ferner für Alkenyl oder Alkinyl mit jeweils 2 bis 6 Kohlenstoffatomen, für jeweils gegebenenfalls ein-bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ;

$R^5$ für Wasserstoff oder für gegebenenfalls gleich oder verschieden, ein- oder zweifach substituiertes, geradkettiges oder verzweigtes Alkyl mit 1 bis 4 Kohlenstoffatomen, wobei als Substituenten genannt seien : Halogen, Cyano, $—COOR^{I}$, $—CONR^{II}R^{III}$, $—NR^{II}R^{III}$, $—OR^{IV}$, $—S(O)_nR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, gleich oder verschieden durch Halogen, Alkyl und Alkoxy mit jeweils 1 bis 4 Kohlenstoffatomen substituierter 5- oder 6-gliedriger Heterocyclus mit 1 bis 3 gleichen oder verschiedenen Heteroatomen, jeweils gegebenenfalls ein- bis fünffach, gleich oder verschieden durch Alkyl mit 1 bis 4 Kohlenstoffatomen substituiertes Cycloalkyl mit 3 bis 6 Kohlenatoffatomen oder Cycloalkenyl mit 5 bis 7 Kohlenstoffatomen ; $R^5$ steht ferner für geradkettiges oder verzweigtes Alkenyl oder Alkinyl mit jeweils 2 bis 6 Kohlenstoffatomen ; für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden substituiertes Cycloalkyl mit 3 bis 6 Kohlenstoffatomen, wobei als Substituenten genannt seien : Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden durch Halogen und Alkyl mit 1 bis 4 Kohlenstoffatomen substituiertes Phenyl oder Pyrrolidon ; $R^5$ steht weiterhin für gegebenenfalls ein- bis fünffach, gleich oder verschieden durch Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis fünffach, gleich oder verschieden substituierten 5- oder 6-gliedrigen Heterocyclus mit 1 bis 3 gleichen oder verschiedenen Heteroatomen ; als Substituenten seien genannt : Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil ;

$R^6$ für die Bedeutungen von $R^5$ oder die Gruppierung $—OR^{IV}$ ;

oder

$R^5$ und $R^6$ gemeinsam mit dem Stickstoffatom, an das sie gebunden sind, für einen gegebenenfalls ein- bis fünffach, gleich oder verschieden substituierten mono-, bi- oder tricyclischen Heterocyclus oder Spiroheterocyclus mit ein bis drei weiteren, gleichen oder verschiedenen Heteroatomen, wobei als Substituenten genannt seien :

geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen im

geradkettigen oder verzweigten Alkylteil, jeweils gegebenenfalls ein- oder zweifach, gleich oder verschieden durch Halogen oder geradkettiges oder verzweigtes Alkyl oder Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil ;

$R^I$ für Wasserstoff oder geradkettiges oder verzweigtes Alkyl mit 1 bis 6 Kohlenstoffatomen,

$R^{II}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten genannt seien : Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 2 bis 5 gleichen oder verschiedenen Halogenatomen ; $R^{II}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen,

$R^{III}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten die bei $R^{II}$ genannten Phenylsubstituenten infrage kommen ; $R^{III}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen ;

$R^{IV}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten die bei $R^{II}$ genannten Phenylsubstituenten infrage kommen ; $R^{IV}$ steht ferner für Acyl mit 2 bis 9 Kohlenstoffatomen ;

n für die Zahlen 0, 1 oder 2 und

X für Sauerstoff oder Schwefel.

2. N,N'Diacylaminale gemäß Anspruch 1, wo in der Formel (I)

$R^1$ für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen : Fluor, Chlor, Brom, Iod, Cyano, —$COOR^I$, —$CONR^{II}R^{III}$, —$OR^{IV}$, Acyl mit 2 bis 9 Kohlenstoffatomen, gegebenenfalls ein- bis dreifach, gleich oder verschieden durch Fluor, Chlor, Methyl und Methoxy substituiertes Phenyl, jeweils gegebenenfalls ein- bis dreifach durch Methyl substituiertes Cyclopropyl oder Cyclohexyl, gegebenenfalls ein- bis dreifach, gleich oder verschieden durch Alkyl mit 1 oder 2 Kohlenstoffatomen substituierte Heterocyclen der Formeln

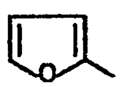 , 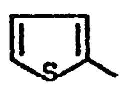 , 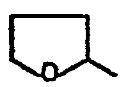 , 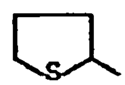 , 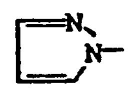

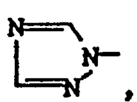 , 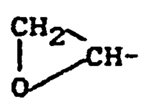 , 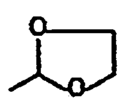 und 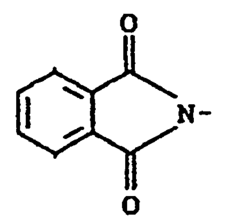 ;

$R^1$ steht ferner für gegebenenfalls ein- oder zweifach durch Methyl substituiertes Allyl oder Propargyl, für jeweils gegebenenfalls ein- bis dreifach durch Methyl substituiertes Cyclopropyl, Cyclohexyl, Cyclopentenyl oder Cyclohexenyl ;

$R^2$ für Wasserstoff oder für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen : Cyano, Methoxy, Ethoxy, Methylthio, Ethylthio, —$COOR^I$, Acylamino mit 2 bis 9 Kohlenstoffatomen, gegebenenfalls ein- bis dreifach, gleich oder verschieden durch Halogen, Methyl und Methoxy substituiertes Phenyl, jeweils gegebenenfalls ein- bis dreifach durch Methyl substituiertes Cyclopropyl oder Cyclohexyl sowie gegebenenfalls ein- bis dreifach, gleich oder verschieden durch Alkyl mit 1 oder 2 Kohlenstoffatomen substituierte Heterocyclen der Formeln

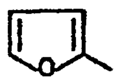 , 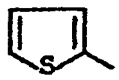 , 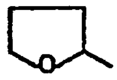 , 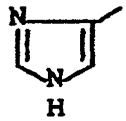 , 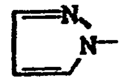

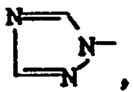 , 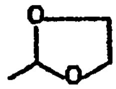 und 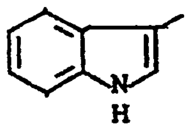 ;

$R^2$ steht ferner für jeweils gegebenenfalls durch Phenyl, das gegebenenfalls ein- bis dreifach, gleich oder verschieden durch Halogen und Methyl substituiert sein kann, substituiertes Allyl, Allenyl, Vinyl, Propargyl oder Ethinyl ; $R^2$ steht außerdem für Cyclopropyl, Cyclohexyl, Cyclopentenyl oder Cyclohexenyl ; $R^2$ steht weiterhin für gegebenenfalls ein- bis dreifach, gleich oder verschieden durch Halogen, Methyl und Methoxy substituiertes Phenyl, für gegebenenfalls ein- bis dreifach, gleich oder verschieden durch Alkyl mit 1 oder 2 Kohlenstoffatomen substituierte Heterocyclen der Formeln

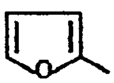 , 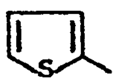 , 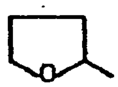 , 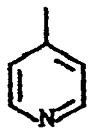 , 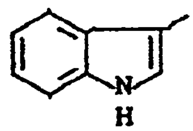 ,

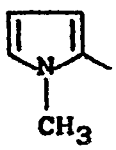 , 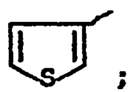 ;

$R^3$ für Wasserstoff oder für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen : Fluor, Chlor, Brom, Iod, Cyano, Alkoxy, Alkylthio, Alkylsulfinyl und Alkylsulfonyl mit jeweils 1 oder 2 Kohlenstoffatomen, Acyloxy und Acylamino mit jeweils 2 bis 9 Kohlenstoffatomen, —COOR', gegebenenfalls ein- bis dreifach, gleich oder verschieden durch Halogen und Methyl substituiertes Phenyl, Cyclopropyl, Cyclopentyl oder Cyclohexyl, gegebenenfalls ein- bis dreifach, gleich oder verschieden durch Alkyl mit 1 oder 2 Kohlenstoffatomen substituierte Heterocyclen der Formeln

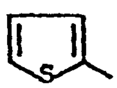 , 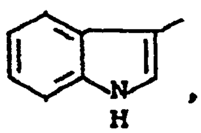 , 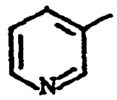 , 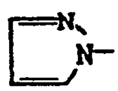 ,

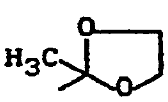 , 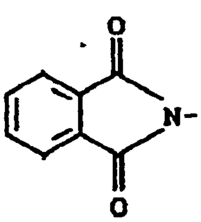 , 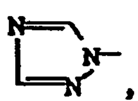 , 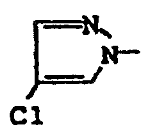 ,

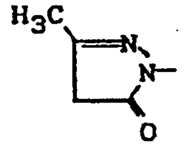 , 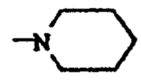 , 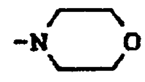 ;

$R^3$ steht ferner für jeweils gegebenenfalls durch Phenyl, das gegebenenfalls ein- bis dreifach, gleich oder verschieden durch Halogen und Methyl substituiert sein kann, oder durch Methyl substituiertes Vinyl oder Ethinyl ; $R^3$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis dreifach, gleich oder verschieden substituiertes Cyclopropyl, Cyclopentyl, Cyclohexyl, Cyclopentenyl oder Cyclohexenyl, wobei als Substituenten genannt seien : Halogen, Methyl, Methoxy, Acyloxy und Acylamino mit jeweils 2 bis 5 Kohlenstoffatomen, die Oxogruppe, Phenyl, Hydroxycarbonyl, Methoxy- und Ethoxycarbonyl ; $R^3$ steht außerdem für gegebenenfalls mit 1 oder 2 Benzol- oder Cyclohexanringen anelliertes, gegebenenfalls ein- bis dreifach, gleich oder verschieden substituiertes Phenyl, wobei als Substituenten genannt seien : Fluor, Chlor, Hydroxy, Nitro, Methyl, Methoxy, Acylamino und N-Alkyl-acyl-amino mit jeweils 2 bis 5 Kohlenstoffatomen im Acylteil und 1 oder 2 Kohlenstoffatomen im Alkylteil, Hydroxycarbonyl, Methoxy- und Ethoxycarbonyl ; $R^3$ steht ferner für gegebenenfalls mit 1 oder 2 Benzol- oder Cyclohexanringen anelliertes, gegebenenfalls ein-bis dreifach, gleich oder verschieden substituiertes 5- oder 6-gliedriges Heterocyclyl mit 1 bis 3 gleichen oder verschiedenen Heteroatomen, wobei als Substituenten genannt seien : Acyl mit 2 bis 5 Kohlenstoffatomen, Chlor, Brom, Methyl, Ethyl, Phenyl, Oxo und Hydroxy ; $R^3$ steht schließlich auch für Alkoxycarbonyl mit 1 bis 4 Kohlenstoffatomen im Alkoxyteil, für $—OR^4$, $—SR^4$ oder $—NR^5R^6$ ;

$R^4$ für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen ; Fluor, Chlor, $—OR^{IV}$, $—SR^{IV}$, $—COOR^{I}$, $—CONR^{II}R^{III}$, CN, $NR^{II}R^{III}$, Alkylcarbonyl mit 1 bis 4 Kohlenstoffatomen im geradkettigen oder verzweigten Alkylteil, jeweils gegebenenfalls ein- bis dreifach, gleich oder verschieden durch Halogen und Methyl substituiertes Phenyl oder Phenoxy, jeweils gegebenenfalls ein- bis dreifach durch Methyl substituiertes Cyclopropyl, Cyclohexyl, Cyclopentenyl oder Cyclohexenyl, gegebenenfalls ein- bis dreifach, gleich oder verschieden durch Halogen, Methyl und Ethyl substituiertes 3- bis 6-gliedriges Heterocyclyl mit 1 bis 3 gleichen oder verschiedenen Heteroatomen, wie Stickstoff-, Sauerstoff- und Schwefelatomen ; $R^4$ steht ferner für gebebenenfalls ein- oder zweifach durch Methyl substituiertes Allyl oder Propargyl sowie für jeweils gegebenenfalls ein- bis dreifach durch Methyl substituiertes Cyclopropyl, Cyclohexyl, Cyclopentenyl oder Cyclohexenyl ;

$R^5$ für Wasserstoff oder für gegebenenfalls ein- oder zweifach, gleich oder verschieden substituiertes geradkettiges oder verzweigtes Alkyl mit 1 bis 4 Kohlenstoffatomen steht, wobei als Substituenten vorzugsweise genannt seien : Halogen, Cyano, $—COOR^{I}$, $—CONR^{II}R^{III}$, $NR^{II}R^{III}$, $—OR^{IV}$, $—S(O)_nR^{IV}$, Acyl mit 2 bis 9 Kohlenstoffatomen, gegebenenfalls ein- bis dreifach, bleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituierter 5- oder 6-gliedrigen Heterocyclus mit 1 bis 3 gleichen oder verschiedenen Heteroatomen und jeweils gegebenenfalls durch geradkettiges oder verzweigtes Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ; $R^5$ steht ferner für geradkettiges oder verzweigtes Alkenyl oder Alkinyl mit jeweils 2 bis 6 Kohlenstoffatomen, für gegebenenfalls durch geradkettiges oder verzweigtes Alkyl mit 1 bis 4 Kohlenstoffatomen substituiertes Cycloalkenyl mit 5 bis 7 Kohlenstoffatomen, für gegebenenfalls substituiertes Cycloalkyl mit 3 bis 6 Kohlenstoffatomen, wobei als Substituenten genannt seien : Halogen, geradkettiges oder verzweigtes Alkyl und Alkoxy mit jeweils 1 bis 4 Kohlenstoffatomen, Cyano, Amino, Carbamoyl, jeweils geradkettiges oder verzweigtes Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden durch Halogen und Alkyl mit 1 bis 4 Kohlenstoffatomen substituiertes Phenyl oder Pyrrolidon ; $R^5$ steht weiterhin für gegebenenfalls ein- bis dreifach, gleich oder verschieden durch Halogen, geradkettiges oder verzweigtes Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis dreifach, gleich oder verschieden substituierten 5- oder 6-gliedrigen Heterocyclus mit 1 bis 3 gleichen oder verschiedenen Heteroatomen ; als Substituenten für die Heterocyclen seien genannt : Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkyl ;

$R^6$ für die Bedeutungen von $R^5$ oder die Gruppierung —$OR^{IV}$ steht,

oder

$R^5$ und $R^6$ gemeinsam mit dem Stickstoffatom, an das sie gebunden sind, für einen gegebenenfalls ein- bis fünffach, gleich oder verschieden substituierten mono-, bi- oder tricyclischen Heterocyclus oder Spiroheterocyclus stehen, wobei als Heterocyclen genannt seien :

Oxazolidin, Pyrrolidin, Imidazolidin, Piperidin, Piperazin, Morpholin, Thiomorpholin, 1,3-Oxazan oder 1,3-Diazan ; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt :

geradkettiges oder verzweigtes Alkyl oder Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem Alkylteil, ferner jeweils gegebenenfals ein- oder zweifach, gleich oder verschieden durch Halogen und geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

$R^I$ für geradkettiges oder verzweigtes Alkyl mit 1 bis 4 Kohlenstoffatomen steht,

$R^{II}$ für Wasserstoff, geradkettiges oder verzweigtes Alkyl mit 1 bis 4 Kohlenstoffatomen, jeweils gegebenenfalls ein-bis dreifach, gleich oder verschieden substituiertes Benzyl oder Phenethyl steht, wobei als Phenylsubstituenten jeweils genannt seien : Fluor, Chlor, Methyl, Methoxy und Trifluormethyl ; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl oder Dialkylcarbamoylalkyl mit jeweils 1 oder 2 Kohlenstoffatomen in jedem Alkylteil, oder für gegebenenfalls ein- bis dreifach, gleich oder verschieden durch Methyl und Ethyl substituiertes Cycloalkyl mit 3 bis 6 Kohlenstoffatomen steht,

$R^{III}$ für die Bedeutungen von $R^{II}$ steht,

$R^{IV}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien : Fluor, Chlor, Methyl, Methoxy und Trifluormethyl ; $R^{IV}$ steht ferner für Acyl mit 2 bis 9 Kohlenstoffatomen ;

n für die Zahlen 0, 1 oder 2 steht und

X für Sauerstoff oder Schwefel steht.

3. N,N'-Diacylaminale gemäß Anspruch 1, wobei in der Formel (I)

$R^1$ für gegebenenfalls ein- oder zweifach, gleich oder verschieden substituiertes Alkyl mit 1 oder 2 Kohlenstoffatomen steht, wobei folgende Substituenten infrage kommen : Fluor, Chlor, Cyano, —$COOR^I$, —$CONR^{II}R^{III}$, —$OR^{IV}$, Alkylcarbonyl mit 1 bis 4 Kohlenstoffatomen im geradkettigen oder verzweigten Alkylteil, gegebenenfalls ein- bis dreifach, gleich oder verschieden durch Fluor, Chlor und Methyl substituiertes Phenyl, jeweils gegebenenfalls ein- bis dreifach durch Methyl substituiertes Cyclopropyl oder Cyclohexyl und die folgenden Heterocyclen :

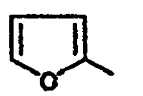 , 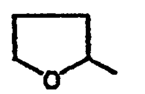 , 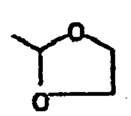 , 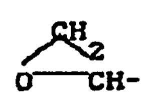

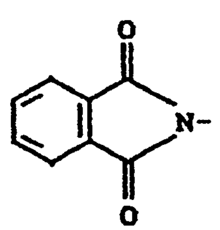 , 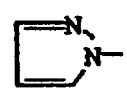 und 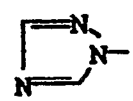 ;

$R^1$ steht ferner für Allyl oder Propargyl, für jeweils gegebenenfalls ein- bis dreifach durch Methyl substituiertes Cyclopropyl, Cyclohexyl, Cyclopentenyl oder Cyclohexenyl ;

$R^2$ für Wasserstoff oder für gegebenenfalls ein- oder zweifach, gleich oder verschieden substituiertes Alkyl mit 1 bis 4 Kohlenstoffatomen steht, wobei folgende Substituenten infrage kommen : Cyano, Methoxy, Ethoxy,

Methylthio, Ethylthio, —COOR$^1$, Alkylcarbonylamino mit 1 bis 4 Kohlenstoffatomen im geradkettigen oder verzweigten Alkylteil, gegebenenfalls ein- bis dreifach, gleich oder verschieden durch Halogen, Methyl und Methoxy substituiertes Phenyl, jeweils gegebenenfalls ein- bis dreifach durch Methyl substituiertes Cyclopropyl oder Cyclohexyl und die folgenden Heterocyclen :

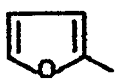 , 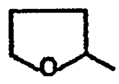 , 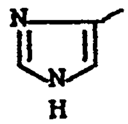 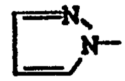 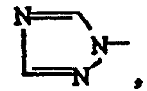 ,

und 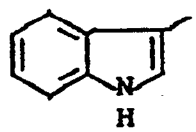 ;

R$^2$ steht ferner für jeweils gegebenenfalls durch Phenyl, das gegebenenfalls ein- bis dreifach, gleich oder verschieden durch Halogen und Methyl substituiert sein kann, substituiertes Vinyl, Allyl, Allenyl, Ethinyl oder Propargyl ;

R$^2$ steht außerdem für Cyclopropyl, Cyclohexyl, Cyclopentenyl oder Cyclohexenyl ;

R$^2$ steht weiterhin für gegebenenfalls ein- bis dreifach, gleich oder verschieden durch Halogen, Methyl und Methoxy substituiertes Phenyl oder für die folgenden Heterocyclen :

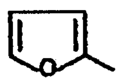 , 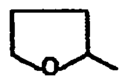 , 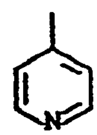 , 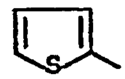 und 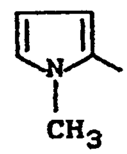 ;

R$^3$ für Wasserstoff oder für gegebenenfalls ein- oder zweifach, gleich oder verschieden substituiertes, geradkettiges oder verzweigtes Alkyl mit 1 bis 4 Kohlenstoffatomen steht, wobei folgende Substituenten infrage kommen : Fluor, Chlor, Brom, Iod, Cyano, Alkoxy, Alkylthio, Alkylsulfinyl und Alkylsulfonyl mit jeweils 1 oder 2 Kohlenstoffatomen, Alkylcarbonyloxy mit 1 bis 4 Kohlenstoffatomen im geradkettigen oder verzweigten Alkylteil, Formamido, Alkylcarbonylamino mit 1 oder 2 Kohlenstoffatomen im gegebenenfalls durch Fluor substituierten Alkylteil, gegebenenfalls durch Chlor substituiertes Benzoylamino, Alkoxycarbonylamino mit 1 bis 4 Kohlenstoffatomen im geradkettigen oder verzweigten Alkoxyteil, —COOR$^1$, gegebenenfalls ein- bis dreifach, gleich oder verschieden durch Halogen und Methyl substituiertes Phenyl, Cyclopropyl, Cyclopentyl oder Cyclohexyl und die folgenden Heterocyclen :

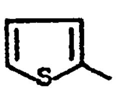 , 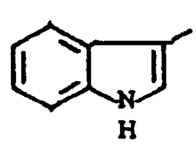 , 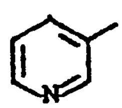 , 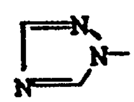 , 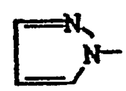 ,

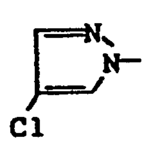 , 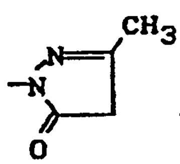 und 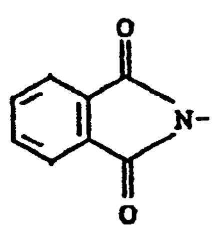 ;

R$^3$ steht ferner für jeweils gegebenenfalls einfach oder zweifach, gleich oder verschieden durch Phenyl, das gegebenenfalls ein- bis dreifach, gleich oder verschieden durch Halogen und Methyl substituiert sein kann, oder durch Methyl substituiertes Vinyl oder Ethinyl ;

$R^3$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis dreifach, gleich oder verschieden substituiertes Cyclopropyl, Cyclopentyl, Cyclohexyl, Cyclopentenyl oder Cyclohexenyl, wobei als Substituenten genannt seien ; Halogen, Methyl, die Oxogruppe, Phenyl, Hydroxycarbonyl, Methoxy- und Ethoxycarbonyl ; im einzelnen seien als Ringe genannt :

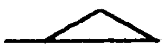 , 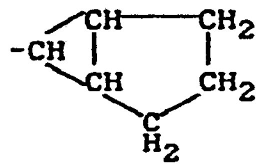 , 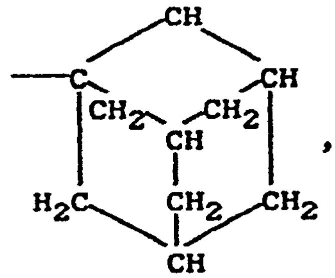 ,

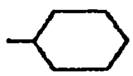 , 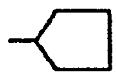 , 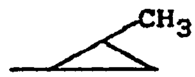 , 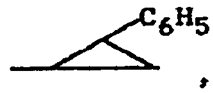 ,

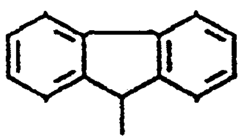 , 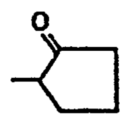 , 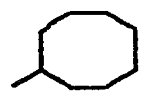 ,

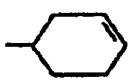 , 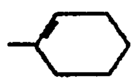 ;

$R^3$ steht außerdem für gegebenenfalls mit 1 oder 2 Benzol-, Cyclopentan- oder Cyclohexanringen anelliertes, gegebenenfalls ein- bis dreifach, gleich oder verschieden substituiertes Phenyl, wobei als Substituenten genannt seien : Fluor, Chlor, Hydroxy, Nitro, Methyl, Methoxy, Acetoxy, Acetamido, Hydroxycarbonyl, Methoxy- und Ethoxycarbonyl ;

$R^3$ steht ferner für gegebenenfalls mit 1 oder 2 Benzol-, Cyclopentan- oder Cyclohexanringen anelliertes, gegebenenfalls ein- bis dreifach, gleich oder verschieden substituiertes 5- oder 6-gliedriges Heterocyclyl mit 1 bis 3 gleichen oder verschiedenen Heteroatomen, wobei als Substituenten genannt seien : Acyl mit 2 bis 5 Kohlenstoffatomen, Chlor, Brom, Methyl, Ethyl, Phenyl, die Oxogruppe und Hydroxy ; im einzelnen seien als Heterocyclen genannt :

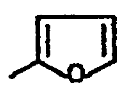 , 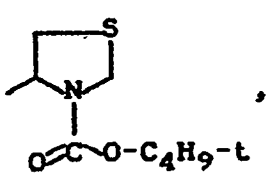 , 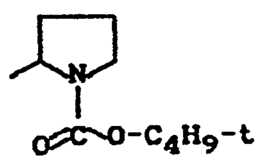 ,

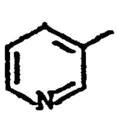 , 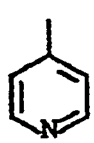 , 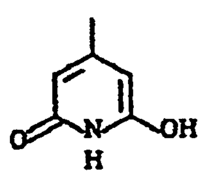 , 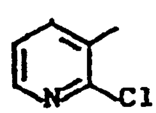 ,

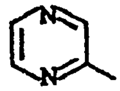 , 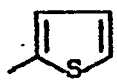 , 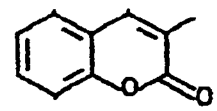 , 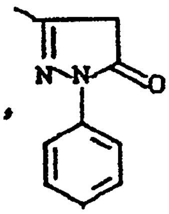 ,

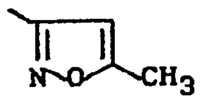 ;

$R^3$ steht schließlich auch für Alkoxycarbonyl mit 1 bis 4 Kohlenstoffatomen im geradkettigen oder verzweigten Alkoxyteil, für —$OR^4$, —$SR^4$ oder —$NR^5R^6$ ;

$R^4$ für gegebenenfalls ein- oder zweifach, gleich oder verschieden substituiertes, geradkettiges oder verzweigtes Alkyl mit 1 oder 2 Kohlenstoffatomen steht, wobei folgende Substituenten infrage kommen : Fluor, Chlor, Methoxy, Ethoxy, Methylthio, Alkoxycarbonyl mit 1 bis 4 Kohlenstoffatomen im geradkettigen oder verzweigten Alkylteil, Carbamoyl, Methylcarbamoyl, Dimethylcarbamoyl, Cyano, Dimethylamino, Diethylamino, Acetyl, Pivaloyl, jeweils gegebenenfalls ein- bis dreifach, gleich oder verschieden durch Halogen und Methyl substituiertes Phenyl oder Phenoxy, jeweils gegebenenfalls ein-bis dreifach durch Methyl substituiertes Cyclopropyl oder Cyclohexyl ;

$R^4$ steht ferner für gegebenenfalls ein- bis dreifach, gleich oder verschieden durch Halogen, Methyl und Ethyl substituiertes 3- bis 6-gliedriges Heterocyclyl mit 1 bis 3 gleichen oder verschiedenen Heteroatomen ; im einzelnen seien als Heterocyclen genannt :

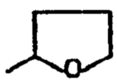 , 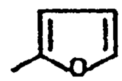 , 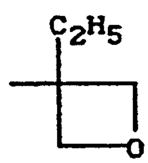 , 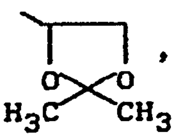 , 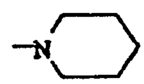 ,

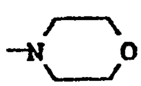 , 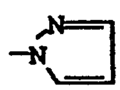 , 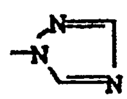 ;

$R^4$ steht außerdem für jeweils gegebenenfalls ein-oder zweifach durch Methyl substituiertes Allyl oder Propargyl oder für jeweils gegebenenfalls ein- bis dreifach durch Methyl substituiertes Cyclohexyl ;

$R^5$ für Wasserstoff, geradkettiges oder verzweigtes Alkyl mit 1 bis 4 Kohlenstoffatomen oder ein- oder zweifach, gleich oder verschieden substituiertes Alkyl mit 1 bis 3 Kohlenstoffatomen steht, wobei als Substituenten genannt seien : Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, Alkoxycarbonyl mit 1 bis 4 Kohlenstoffatomen im geradkettigen oder verzweigten Alkoxyteil, Carbamoyl, Methylcarbamoyl, Dimethylcarbamoyl, Cyano, Dimethylamino, Diethylamino, gegebenenfalls ein- bis dreifach, gleich oder verschieden durch Chlor und Methyl substituiertes Phenyl, 2-Furyl, 2-Pyridyl, 1-Morpholino, Cyclopropyl oder Cyclohexyl ;

$R^5$ steht ferner für Allyl oder Propargyl, für 1-Cyclohexenyl oder für ein- bis dreifach, gleich oder verschieden substituiertes Cyclohexyl, wobei als Substituenten genannt seien : Chlor, Methyl, Ethyl, Methoxy, Cyano, Amino, Alkyl- und Dialkylamino mit jeweils 1 oder 2 Kohlenstoffatomen in jedem Alkylteil, Carbamoyl, Alkyl- und Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil oder 1-Pyrrolidin-2-on ;

$R^5$ steht außerdem für gegebenenfalls ein-bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

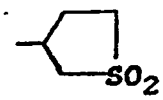 ,

R⁶ für Wasserstoff, geradkettiges oder verzweigtes Alkyl mit 1 bis 4 Kohlenstoffatomen oder für ein- oder zweifach, gleich oder verschieden substituiertes Alkyl mit 1 bis 3 Kohlenstoffatomen steht, wobei als Substituenten genannt seien : Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, Alkoxycarbonyl mit 1 bis 4 Kohlenstoffatomen im geradkettigen oder verzweigten Alkoxyteil, Carbamoyl, Methylcarbamoyl, Dimethylcarbamoyl, Cyano, Dimethylamino, Diethylamino, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl ;

R⁶ steht ferner für Allyl oder Propargyl, für 1-Cyclohexenyl oder für ein-bis dreifach, gleich oder verschieden substituiertes Cyclohexyl, wobei als Substituenten genannt seien : Chlor, Methyl, Ethyl, Methoxy, Cyano, Amino, Alkyl- oder Dialkylamino mit jeweils 1 oder 2 Kohlenstoffatomen in jedem Alkylteil, Carbamoyl, Alkyl- oder Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil oder 1-Pyrrolidin-2-on ;

R⁶ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

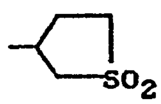 ,

R⁶ steht weiterhin für Hydroxy, Alkoxy mit 1 oder 2 Kohlenstoffatomen, für gegebenenfalls ein- bis dreifach, gleich oder verschieden durch Chlor und Methyl substituiertes Benzyloxy, für 1-Adamantyl, 2-Norbornyl, 1- oder 2-Decalyl oder 1-oder 2-Tetralyl ;

oder

R⁵ und R⁶ gemeinsam mit dem Stickstoffatom, an das sie gebunden sind, für folgende mono-, bi- oder tricyclischen Heterocyclen oder Spiroheterocyclen stehen :

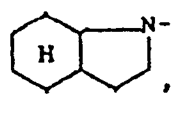 , 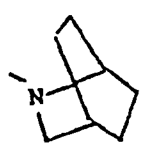 , 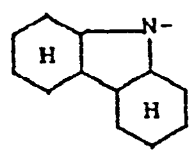 ,

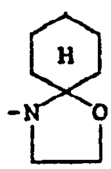 , 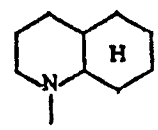 ,

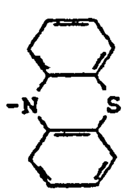 , 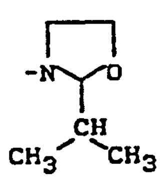 , 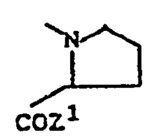

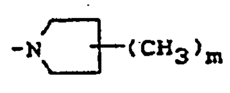 , 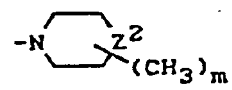

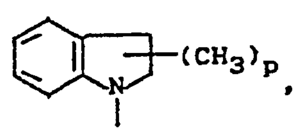 , 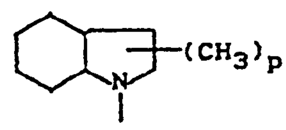 .

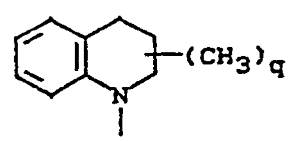 , 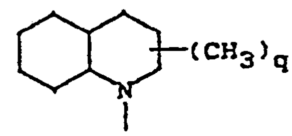 ,

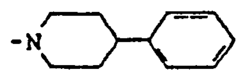 , 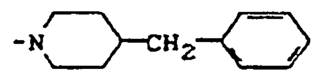 ,

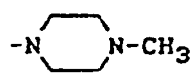 , 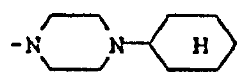 , 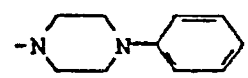 ,

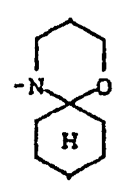 , 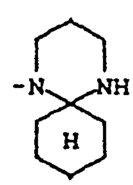 , 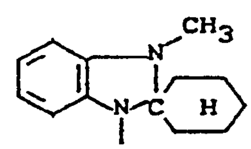 ,

wobei

$Z^1$ für Hydroxy, Methoxy, Ethoxy, Amino, Methylamino, Ethylamino, Dimethylamino oder Ethylmethylamino steht,

$Z^2$ für Sauerstoff oder die $CH_2$-Gruppe steht,

m für die Zahlen 0, 1, 2, 3 oder 4 steht,

p für die Zahlen 0, 1 oder 2 steht,

q für die Zahlen 0, 1, 2 oder 3 steht,

$R^I$ für Methyl, Ethyl, n- oder i-Propyl und n-, i-, s- oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen,

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien; und

X für Sauerstoff oder Schwefel steht.

4. Verfahren zu Herstellung von N,N'-Diacylaminalen der allgemeinen Formel (I),

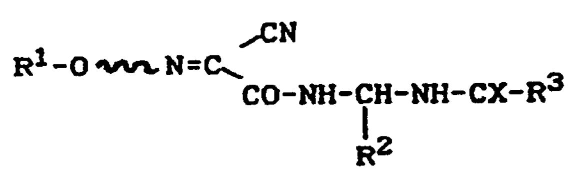 (I)

in welcher

$R^1$, $R^2$, $R^3$ und X die in Anspruch 1 angegebene Bedeutung haben,

deren geometrische und optische Isomere und Isomerengemische, dadurch gekennzeichnet, daß man

a) N-(2-Cyan-2-oximino-acetyl)-aminale der Formel (II)

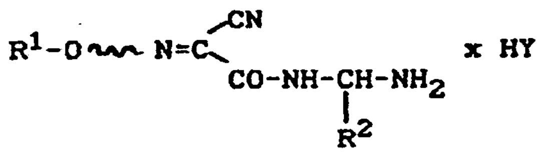

(II)

in welcher

$R^1$ und $R^2$ die oben angegebene Bedeutung haben und

HY für das Äquivalent einer anorganischen oder organischen Säure steht,

mit einem Acylierungsreagenz der Formeln (IIIa) oder (IIIb)

$$Z\text{-}CX\text{-}R^3 \qquad \text{(IIIa)}$$

$$X{=}C{=}N\text{-}R^6 \qquad \text{(IIIb)}$$

in welchen

Z für eine übliche Abgangsgruppe steht, wie Halogen, —O-CO-$R^3$, —O-CO-$OR^4$, —$OR^4$, —$SR^4$, Carboxymethoxy oder Carboxymethylthio und

$R^3$, $R^4$, $R^6$ und X die im Anspruch 1 angegebene Bedeutung haben,

gegebenenfalls in Gegenwart einer Base und in Gegenwart eines Verdünnungsmittels und gegebenenfalls in Gegenwart eines Katalysators umsetzt;

oder

b) Isocyanate der Formel (IV),

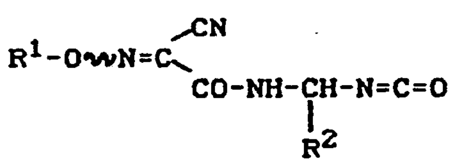

(IV)

in welcher

$R^1$ und $R^2$ die oben angegebene Bedeutung haben,

mit einem protischen Nukleophil der Formeln (Va) oder (Vb)

$$HO\text{-}CO\text{-}R^3 \qquad \text{(Va)}$$

$$H - R^7 \qquad \text{(Vb)}$$

in welchen

$R^7$ für —$OR^4$, —$SR^4$ oder —$NR^5R^6$ steht und

$R^3$, $R^4$, $R^5$ und $R^6$ die im Anspruch 1 angegebene Bedeutung haben,

in Gegenwart eines Verdünnungsmittels und gegebenenfalls in Gegenwart eines Katalysators umsetzt.

5. Schädlingsbekämpfungsmittel, gekennzeichnet durch einen Gehalt an mindestens einem N,N'-Diacylaminal der Formel (I) nach den Ansprüchen 1 und 4.

6. Verwendung von N,N'-Diacylaminalen der Formel (I) nach den Ansprüchen 1 und 4 zur Bekämpfung von Schädlingen.

7. Verfahren zur Bekämpfung von Schädlingen, dadurch gekennzeichnet, daß man N,N'-Diacylaminale der Formel (I) nach den Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man N,N'-Diacylaminale der Formel (I) nach den Ansprüchen 1 und 4 mit Streckmitteln und/oder oberflächenaktiven Mitteln vermischt.

9. N-(2-Cyan-2-oximino-acetyl)-aminale der allgemeinen Formel (II),

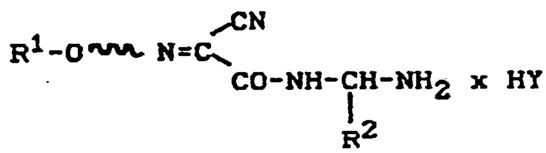

(II)

in welcher

$R^1$ und $R^2$ die in Anspruch 1 angegebene Bedeutung haben und

HY für das Äquivalent einer anorganischen oder organischen Säure steht.

10. Verfahren zur Herstellung von N-(2-Cyan-2-oximino-acetyl)-aminalen der allgemeinen Formel (II),

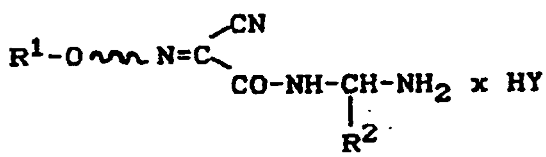

(II)

in welcher

$R^1$, $R^2$ und HY die in Anspruch 9 angegebene Bedeutung haben, dadurch gekennzeichnet, daß man Isocyanate der Formel (IV),

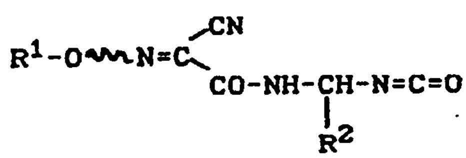

(IV)

in welcher

$R^1$ und $R^2$ die oben angegebene Bedeutung haben, in üblicher Weise hydrolisiert und das entstehende Amin in üblicher Weise als Salz isoliert.

11. Isocyanate der Formel (IV),

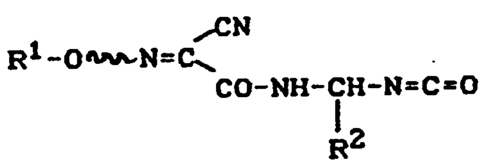

(IV)

in welcher

$R^1$ und $R^2$ die in Anspruch 1 angegebene Bedeutung haben.

12. Verfahren zur Herstellung von Isocyanaten der allgemeinen formel (IV),

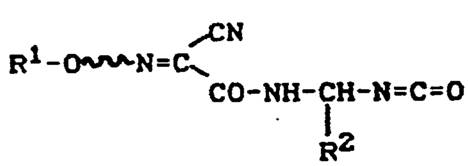

(IV)

in welcher

$R^1$ und $R^2$ die in Anspruch 1 angegebene Bedeutung haben, dadurch gekennzeichnet, daß man

α) Amide der allgemeinen Formel (VI),

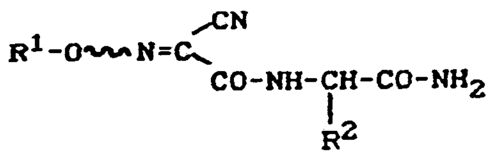

(VI)

in welcher

$R^1$ und $R^2$ die oben angegebene Bedeutung haben, in üblicher Weise, gegebenenfalls in Gegenwart eines Verdünnungsmittels oxidiert,

oder

β) Azide der allgemeinen Formel (VII),

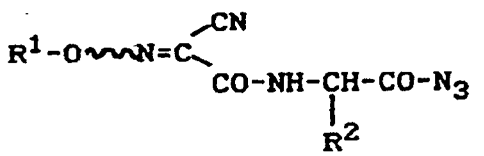

(VII)

in welcher

$R^1$ und $R^2$ die oben angegebene Bedeutung haben, in Gegenwart eines Verdünnungsmittels erwärmt.

**Patentansprüche für folgenden Vertragsstaat : ES**

1. Verfahren zu Herstellung von N,N'-Diacylaminalen der allgemeinen Formel (I),

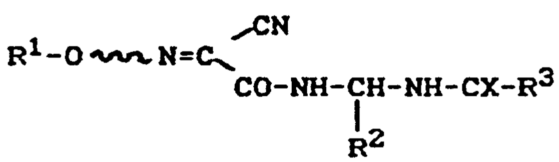

(I)

in welcher

$R^1$ für gegebenenfalls ein- bis fünffach, gleich oder verschieden substituiertes, geradkettiges oder verzweigtes Alkyl mit 1 bis 6 Kohlenstoffatomen steht, wobei folgende Substituenten infrage kommen : Fluor, Chlor, Brom, Iod, Cyano, —$COOR^I$, —$CONR^{II}R^{III}$, —$OR^{IV}$, Acyl mit 2 bis 9 Kohlenstoffatomen, gegebenenfalls ein- bis fünffach, gleich oder verschieden substituiertes Phenyl, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen ; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen ; $R^1$ steht ferner für jeweils gegebenenfalls ein- bis dreifach, gleich oder verschieden substituiertes, geradkettiges oder verzweigtes Alkenyl oder Alkinyl mit 2 bis 6 Kohlenstoffatomen, wobei als Substituenten genannt seien : gegebenenfalls ein-bis fünffach, gleich oder verschieden durch Halogen und Alkyl mit 1 bis 4 Kohlenstoffatomen substituiertes Phenyl ; $R^1$ steht außerdem für jeweils gegebenenfalls ein-bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ;

$R^2$ für Wasserstoff oder für gegebenenfalls ein- bis fünffach, gleich oder verschieden substituiertes, geradkettiges oder verzweigtes Alkyl mit 1 bis 6 Kohlenstoffatomen steht, wobei folgende Substituenten infrage kommen : Cyano, Alkoxy und Alkylthio mit jeweils 1 bis 4 Kohlenstoffatomen, —$COOR^I$, Acylamino mit 2 bis 9 Kohlenstoffatomen, gegebenenfalls ein- bis fünffach, gleich oder verschieden substituiertes Phenyl, wobei als Substituenten die bei $R^1$ bereits genannten Phenylsubstituenten infrage kommen ; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen ; $R^2$ steht ferner für jeweils gegebenenfalls ein- bis dreifach, gleich oder verschieden substituiertes, geradkettiges oder verzweigtes Alkenyl oder Alkinyl mit 2 bis 6 Kohlenstoffatomen, wobei als Substituenten genannt seien : gegebenenfalls ein- bis fünffach, gleich oder verschieden

durch Halogen und Alkyl mit 1 bis 4 Kohlenstoffatomen substituiertes Phenyl ; $R^2$ steht außerdem für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ; $R^2$ steht weiterhin, für gegebenenfalls ein- bis fünffach, gleich oder verschieden substituiertes Phenyl, wobei als Substituenten die bei $R^1$ bereits genannten Phenylsubstituenten infrage kommen ; $R^2$ steht ferner für gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen ;

$R^3$ für Wasserstoff oder für gegebenenfalls ein- bis fünffach, gleich oder verschieden substituiertes, geradkettiges oder verzweigtes Alkyl mit 1 bis 6 Kohlenstoffatomen steht, wobei folgende Substituenten infrage kommen ; Fluor, Chlor, Brom, Iod, Cyano, Alkoxy, Alkylthio, Alkylsulfinyl und Alkylsulfonyl mit jeweils 1 bis 4 Kohlenstoffatomen, Acyloxy und Acylamino mit jeweils 2 bis 9 Kohlenstoffatomen, $—COOR^I$, gegebenenfalls ein-bis fünffach, gleich oder verschieden substituiertes Phenyl, wobei als Substituenten die bei $R^1$ bereits genannten Phenylsubstituenten infrage kommen ; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen sowie gegebenenfalls ein-bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen ; $R^3$ steht ferner für jeweils gegebenenfalls ein- bis dreifach, gleich oder verschieden substituiertes, geradkettiges oder verzweigtes Alkenyl oder Alkinyl mit jeweils 2 bis 6 Kohlenstoffatomen, wobei als Substituenten genannt seien :
gegebenenfalls ein- bis fünffach, gleich oder verschieden durch Halogen und Alkyl mit 1 bis 4 Kohlenstoffatomen substituiertes Phenyl ; $R^3$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls einbis fünffach, gleich oder verschieden substituiertes Cycloalkyl mit 3 bis 6 Kohlenstoffatomen oder Cycloalkenyl mit 5 bis 7 Kohlenstoffatomen, wobei als Substituenten genannt seien : Halogen, Alkyl und Alkoxy mit jeweils 1 bis 4 Kohlenstoffatomen, Acyloxy und Acylamino mit jeweils 2 bis 5 Kohlenstoffatomen, die Oxogruppe, Phenyl, Hydroxycarbonyl und Alkoxycarbonyl mit 1 bis 4 Kohlenstoffatomen im Alkoxyteil ; $R^3$ steht außerdem für gegebenenfalls mit 1 oder 2 Benzol- oder Cyclohexanringen anelliertes, gegebenenfalls ein- bis fünffach, gleich oder verschieden substituiertes Phenyl, wobei als Substituenten genannt seien : Halogen, Hydroxy, Nitro, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen, Acylamino und Acylalkylamino mit jeweils 2 bis 5 Kohlenstoffatomen im Acylteil und 1 bis 4 Kohlenstoffatomen im Alkylteil, Hydroxycarbonyl und Alkoxycarbonyl mit 1 bis 4 Kohlenstoffatomen im Alkoxyteil ; $R^3$ steht ferner für gegebenenfalls mit 1 oder 2 Benzol-, Cyclopentan oder Cyclohexanringen anelliertes, gegebenenfalls ein- bis fünffach, gleich oder verschieden substituiertes 5- oder 6-gliedriges Heterocyclyl mit 1 bis 3 gleichen oder verschiedenen Heteroatomen, wobei als Substituenten genannt seien : Halogen, Alkyl mit 1 bis 4 Kohlenstoffatomen, Acyl mit 2 bis 9 Kohlenatoffatomen, Phenyl, die Oxogruppe und Hydroxy ; $R^3$ steht schließlich auch für Alkoxycarbonyl mit 1 bis 4 Kohlenatoffatomen im Alkoxyteil, für $—OR^4$, $—SR^4$ oder $—NR^5R^6$ ;

$R^4$ für gegebenenfalls ein- bis fünffach, gleich oder verschieden substituiertes, geradkettiges oder verzweigtes Alkyl mit 1 bis 6 Kohlenstoffatomen steht, wobei folgende Substituenten infrage kommen : Halogen, $—OR^{IV}$, $—SR^{IV}$, $—COOR^I$, $—CONR^{II}R^{III}$, CN, $—NR^{II}R^{III}$, Acyl mit 2 bis 9 Kohlenstoffatomen, jeweils gegebenenfalls einfach bis fünffach, gleich oder verschieden durch Halogen und Alkyl mit 1 bis 4 Kohlenstoffatomen substituiertes Phenyl oder Phenoxy, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen ; $R^4$ steht ferner für Alkenyl oder Alkinyl mit jeweils 2 bis 6 Kohlenstoffatomen, für jeweils gegebenenfalls ein-bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ;

$R^5$ für Wasserstoff oder für gegebenenfalls gleich oder verschieden, ein- oder zweifach substituiertes, geradkettiges oder verzweigtes Alkyl mit 1 bis 4 Kohlenstoffatomen steht, wobei als Substituenten genannt seien : Halogen, Cyano, $—COOR^I$, $—CONR^{II}R^{III}$, $—NR^{II}R^{III}$, $—OR^{IV}$, $—S(O)_nR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, gleich oder verschieden durch Halogen, Alkyl und Alkoxy mit jeweils 1 bis 4 Kohlenstoffatomen substituierter 5- oder 6-gliedriger Heterocyclus mit 1 bis 3 gleichen oder verschiedenen Heteroatomen, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ; $R^5$ steht ferner für geradkettiges oder verzweigtes Alkenyl oder Alkinyl mit jeweils 2 bis 6 Kohlenstoffatomen ; für gegebenenfalls ein- bis fünffach, gleich oder verschieden

durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden substituiertes Cycloalkyl mit 3 bis 6 Kohlenstoffatomen, wobei als Substituenten genannt seien ; Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden durch Halogen und Alkyl mit 1 bis 4 Kohlenstoffatomen substituiertes Phenyl oder Pyrrolidon ; $R^5$ steht weiterhin für gegebenenfalls ein- bis fünffach, gleich oder verschieden durch Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis fünffach, gleich oder verschieden substituierten 5- oder 6-gliedrigen Heterocyclus mit 1 bis 3 gleichen oder verschiedenen Heteroatomen ; als Substituenten seien genannt : Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil ;

$R^6$ für die Bedeutung von $R^5$ oder die Gruppierung —$OR^{IV}$ steht ;

oder

$R^5$ und $R^6$ gemeinsam mit dem Stickstoffatom, an das sie gebunden sind, für einen gegebenenfalls ein- bis fünffach, gleich oder verschieden substituierten mono-, bi- oder tricyclischen Heterocyclus oder Spiroheterocyclus mit ein bis drei weiteren, gleichen oder verschiedenen Heteroatomen stehen, wobei als Substituenten genannt seien :

geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen im geradkettigen oder verzweigten Alkylteil, jeweils gegebenenfalls ein- oder zweifach, gleich oder verschieden durch Halogen oder geradkettiges oder verzweigtes Alkyl oder Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil ;

$R^I$ für Wasserstoff oder geradkettiges oder verzweigtes Alkyl mit 1 bis 6 Kohlenstoffatomen steht,

$R^{II}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten genannt seien : Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 2 bis 5 gleichen oder verschiedenen Halogenatomen ; $R^{II}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen,

$R^{III}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen ; $R^{III}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen ;

$R^{IV}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen ; $R^{IV}$ steht ferner für Acyl mit 2 bis 9 Kohlenstoffatomen ;

n für die Zahlen 0, 1 oder 2 steht und

X für Sauerstoff oder Schwefel steht, geometrische und optische Isomere und Isomerengemische, dadurch gekennzeichnet, daß man

a) N-(2-Cyan-2-oximino-acetyl)-aminale der Formel (II)

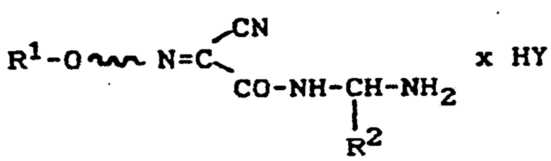

(II)

in welcher

$R^1$ und $R^2$ die oben angegebene Bedeutung haben und

HY für das Äquivalent einer anorganischen oder organischen Säure steht,

mit einem Acylierungsreagenz der Formeln (IIIa) oder (IIIb)

$$Z\text{-}CX\text{-}R^3 \qquad (IIIa)$$

$$X{=}C{=}N\text{-}R^6 \qquad (IIIb)$$

in welchen

Z für eine übliche Abgangsgruppe steht, wie Halogen, $-O\text{-}CO\text{-}R^3$, $-O\text{-}CO\text{-}OR^4$, $-OR^4$, $-SR^4$, Carboxymethoxy oder Carboxymethylthio und

$R^3$, $R^4$, $R^6$ und X die oben angegebene Bedeutung haben,

gegebenenfalls in Gegenwart einer Base und in Gegenwart eines Verdünnungsmittels und gegebenenfalls in Gegenwart eines Katalysators umsetzt;

oder

b) Isocyanate der Formel (IV),

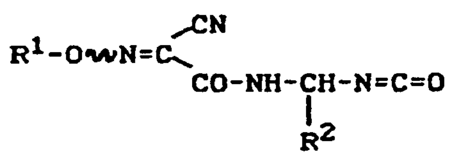

(IV)

in welcher

$R^1$ und $R^2$ die oben angegebene Bedeutung haben,

mit einem protischen Nukleophil der Formeln (Va) oder (Vb)

$$HO\text{-}CO\text{-}R^3 \qquad (Va)$$

$$H - R^7 \qquad (Vb)$$

in welchen

$R^7$ für $-OR^4$, $-SR^4$ oder $-NR^5R^6$ steht und

$R^3$, $R^4$, $R^5$ und $R^6$ die oben angegebene Bedeutung haben,

in Gegenwart eines Verdünnungsmittels und gegebenenfalls in Gegenwart eines Katalysators umsetzt.

2. Verfahren gemäß Anspruch 1, wo in der Formel (I)

$R^1$ für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, Cyano, $-COOR^I$, $-CONR^{II}R^{III}$, $-OR^{IV}$, Acyl mit 2 bis 9 Kohlenstoffatomen, gegebenenfalls ein- bis dreifach, gleich oder verschieden durch Fluor, Chlor, Methyl und Methoxy substituiertes Phenyl, jeweils gegebenenfalls ein- bis dreifach durch Methyl substituiertes Cyclopropyl oder Cyclohexyl, gegebenenfalls ein- bis dreifach, gleich oder verschieden durch Alkyl mit 1 oder 2 Kohlenstoffatomen substituierte Heterocyclen der Formeln

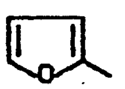 , 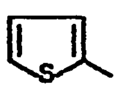 , 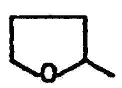 , 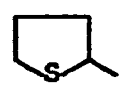 , 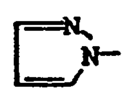

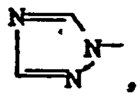, 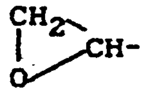, 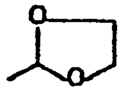 und 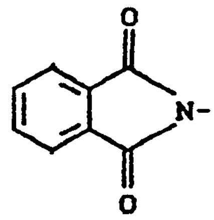 ;

$R^1$ steht ferner für gegebenenfalls ein- oder zweifach durch Methyl substituiertes Allyl oder Propargyl, für jeweils gegebenenfalls ein- bis dreifach durch Methyl substituiertes Cyclopropyl, Cyclohexyl, Cyclopentenyl oder Cyclohexenyl ;

$R^2$ für Wasserstoff oder für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen : Cyano, Methoxy, Ethoxy, Methylthio, Ethylthio, —COOR', Acylamino mit 2 bis 9 Kohlenstoffatomen, gegebenenfalls ein- bis dreifach, gleich oder verschieden durch Halogen, Methyl und Methoxy substituiertes Phenyl, jeweils gegebenenfalls ein- bis dreifach durch Methyl substituiertes Cyclopropyl oder Cyclohexyl sowie gegebenenfalls ein- bis dreifach, gleich oder verschieden durch Alkyl mit 1 oder 2 Kohlenstoffatomen substituierte Heterocyclen der Formeln

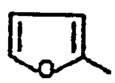, 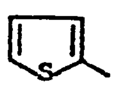, 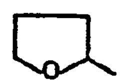, 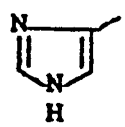, 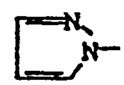

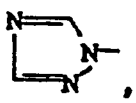, 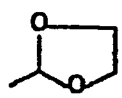 und 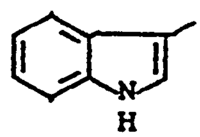 ;

$R^2$ steht ferner für jeweils gegebenenfalls durch Phenyl, das gegebenenfalls ein- bis dreifach, gleich oder verschieden durch Halogen und Methyl substituiert sein kann, substituiertes Allyl, Allenyl, Vinyl, Propargyl oder Ethinyl ; $R^2$ steht außerdem für Cyclopropyl, Cyclohexyl, Cyclopentenyl oder Cyclohexenyl ; $R^2$ steht weiterhin für gegebenenfalls ein- bis dreifach, gleich oder verschieden durch Halogen, Methyl und Methoxy substituiertes Phenyl, für gegebenenfalls ein- bis dreifach, gleich oder verschieden durch Alkyl mit 1 oder 2 Kohlenstoffatomen substituierte Heterocyclen der Formeln

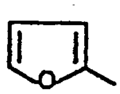, 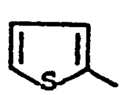, 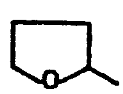, 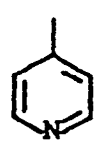, 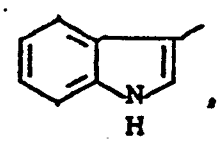,

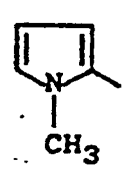, 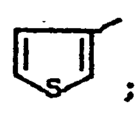 ;

$R^3$ für Wasserstoff oder für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen : Fluor, Chlor, Brom, Iod, Cyano, Alkoxy, Alkylthio, Alkylsulfinyl und Alkylsulfonyl mit jeweils 1 oder 2 Kohlenstoffatomen, Acyloxy und Acylamino mit jeweils 2 bis 9 Kohlenstoffatomen, —COOR', gegebenenfalls ein- bis dreifach, gleich oder verschieden durch Halogen und Methyl substituiertes Phenyl, Cyclopropyl, Cyclopentyl oder Cyclohexyl, gegebenenfalls ein- bis dreifach, gleich oder verschieden durch Alkyl mit 1 oder 2 Kohlenstoffatomen substituierte Heterocyclen der Formeln

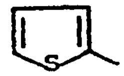 , 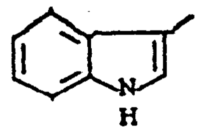 , 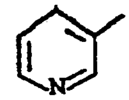 , 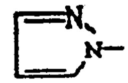 ,

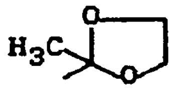 , 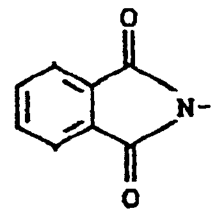 , 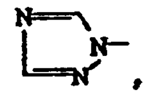 , 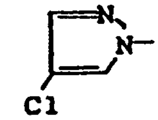 ,

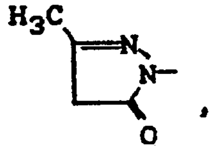 , 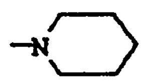 , 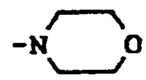 ;

$R^3$ steht ferner für jeweils gegebenenfalls durch Phenyl, das gegebenenfalls ein- bis dreifach, gleich oder verschieden durch Halogen und Methyl substituiert sein kann, oder durch Methyl substituiertes Vinyl oder Ethinyl ; $R^3$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis dreifach, gleich oder verschieden substituiertes Cyclopropyl, Cyclopentyl, Cyclohexyl, Cyclopentenyl oder Cyclohexenyl, wobei als Substituenten genannt seien : Halogen, Methyl, Methoxy, Acyloxy und Acylamino mit jeweils 2 bis 5 Kohlenstoffatomen, die Oxogruppe, Phenyl, Hydroxycarbonyl, Methoxy- und Ethoxycarbonyl ; $R^3$ steht außerdem für gegebenenfalls mit 1 oder 2 Benzol- oder Cyclohexanringen anelliertes, gegebenenfalls ein- bis dreifach, gleich oder verschieden substituiertes Phenyl, wobei als Substituenten genannt seien : Fluor, Chlor, Hydroxy, Nitro, Methyl, Methoxy, Acylamino und N-Alkyl-acyl-amino mit jeweils 2 bis 5 Kohlenstoffatomen im Acylteil und 1 oder 2 Kohlenstoffatomen im Alkylteil, Hydroxycarbonyl, Methoxy- und Ethoxycarbonyl ; $R^3$ steht ferner für gegebenenfalls mit 1 oder 2 Benzol- oder Cyclohexanringen anelliertes, gegebenenfalls ein-bis dreifach, gleich oder verschieden substituiertes 5- oder 6-gliedriges Heterocyclyl mit 1 bis 3 gleichen oder verschiedenen Heteroatomen, wobei als Substituenten genannt seien : Acyl mit 2 bis 5 Kohlenstoffatomen, Chlor, Brom, Methyl, Ethyl, Phenyl, Oxo und Hydroxy ; $R^3$ steht schließlich auch für Alkoxycarbonyl mit 1 bis 4 Kohlenstoffatomen im Alkoxyteil, für $—OR^4$, $—SR^4$ oder $—NR^5R^6$ ;

$R^4$ für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen : Fluor, Chlor, $—OR^{IV}$, $—SR^{IV}$, $—COOR^{I}$, $—CONR^{II}R^{III}$, CN, $NR^{II}R^{III}$, Alkylcarbonyl mit 1 bis 4 Kohlenstoffatomen im geradkettigen oder verzweigten Alkylteil, jeweils gegebenenfalls ein- bis dreifach, gleich oder verschieden durch Halogen und Methyl substituiertes Phenyl oder Phenoxy, jeweils gegebenenfalls ein- bis dreifach durch Methyl substituiertes Cyclopropyl, Cyclohexyl, Cyclopentenyl oder Cyclohexenyl, gegebenenfalls ein- bis dreifach, gleich oder verschieden durch Halogen, Methyl und Ethyl substituiertes 3- bis 6-gliedriges Heterocyclyl mit 1 bis 3 gleichen oder verschiedenen Heteroatomen, wie Stickstoff-, Sauerstoff- und Schwefelatomen ; $R^4$ steht ferner für gegebenenfalls ein- oder zweifach durch Methyl substituiertes Allyl oder Propargyl sowie für jeweils gegebenenfalls ein- bis dreifach durch Methyl substituiertes Cyclopropyl, Cyclohexyl, Cyclopentenyl oder Cyclohexenyl ;

$R^5$ für Wasserstoff oder für gegebenenfalls ein- oder zweifach, gleich oder verschieden substituiertes geradkettiges oder verzweigtes Alkyl mit 1 bis 4 Kohlenstoffatomen steht, wobei als Substituenten vorzugsweise genannt seien : Halogen, Cyano, $—COOR^{I}$, $—CONR^{II}R^{III}$, $NR^{II}R^{III}$, $—OR^{IV}$, $—S(O)_nR^{IV}$, Acyl mit 2 bis 9 Kohlenstoffatomen, gegebenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituierter 5- oder 6-gliedrigen Heterocyclus mit 1 bis 3 gleichen oder verschiedenen Heteroatomen und jeweils gegebenenfalls durch geradkettiges oder verzweigtes Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen ; $R^5$ steht ferner besonders bevorzugt für geradkettiges oder verzweigtes Alkenyl oder Alkinyl mit jeweils 2 bis 6 Kohlenstoffatomen, für gegebenenfalls durch geradkettiges oder verzweigtes Alkyl mit 1 bis 4 Kohlenstoffatomen substituiertes

Cycloalkenyl mit 5 bis 7 Kohlenstoffatomen, für gegebenenfalls substituiertes Cycloalkyl mit 3 bis 6 Kohlenstoffatomen, wobei als Substituenten vorzugsweise genannt seien ; Halogen, geradkettiges oder verzweigtes Alkyl und Alkoxy mit jeweils 1 bis 4 Kohlenstoffatomen, Cyano, Amino, Carbamoyl, jeweils geradkettiges oder verzweigtes Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden durch Halogen und Alkyl mit 1 bis 4 Kohlenstoffatomen substituiertes Phenyl oder Pyrrolidon; $R^5$ steht weiterhin besonders bevorzugt für gegebenenfalls ein- bis dreifach, gleich oder verschieden durch Halogen, geradkettiges oder verzweigtes Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis dreifach, gleich oder verschieden substituierten 5- oder 6-gliedrigen Heterocyclus mit 1 bis 3 gleichen oder verschiedenen Heteroatomen ; als Substituenten für die Heterocyclen seien genannt : Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkyl ;

$R^6$ für die Bedeutungen von $R^5$ oder die Gruppierung $—OR^{IV}$ steht,

oder

$R^5$ und $R^6$ gemeinsam mit dem Stickstoffatom, an das sie gebunden sind, für einen gegebenenfalls ein-bis fünffach, gleich oder verschieden substituierten mono-, bi- oder tricyclischen Heterocyclus oder Spiroheterocyclus stehen, wobei als Heterocyclen genannt seien :

Oxazolidin, Pyrrolidin, Imidazolidin, Piperidin, Piperazin, Morpholin, Thiomorpholin, 1,3-Oxazan oder 1,3-Diazan ; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt :

geradkettiges oder verzweigtes Alkyl oder Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem Alkylteil, ferner jeweils gegebenenfals ein- oder zweifach, gleich oder verschieden durch Halogen und geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

$R^I$ für geradkettiges oder verzweigtes Alkyl mit 1 bis 4 Kohlenstoffatomen steht,

$R^{II}$ für Wasserstoff, geradkettiges oder verzweigtes Alkyl mit 1 bis 4 Kohlenstoffatomen, jeweils gegebenenfalls ein-bis dreifach, gleich oder verschieden substituiertes Benzyl oder Phenethyl steht, wobei als Phenylsubstituenten jeweils genannt seien : Fluor, Chlor, Methyl, Methoxy und Trifluormethyl ; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl oder Dialkylcarbamoylalkyl mit jeweils 1 oder 2 Kohlenstoffatomen in jedem Alkylteil, oder für gegebenenfalls ein- bis dreifach, gleich oder verschieden durch Methyl und Ethyl substituiertes Cycloalkyl mit 3 bis 6 Kohlenstoffatomen steht,

$R^{III}$ für die Bedeutungen von $R^{II}$ steht,

$R^{IV}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien : Fluor, Chlor, Methyl, Methoxy und Trifluormethyl ; $R^{IV}$ steht ferner für Acyl mit 2 bis 9 Kohlenstoffatomen ;

n für die Zahlen 0, 1 oder 2 steht und

X für Sauerstoff oder Schwefel steht.

3. Verfahren gemäß Anspruch 1, wobei in der Formel (I)

$R^1$ für gegebenenfalls ein- oder zweifach, gleich oder verschieden substituiertes Alkyl mit 1 oder 2 Kohlenstoffatomen steht, wobei folgende Substituenten infrage kommen : Fluor, Chlor, Cyano, $—COOR^I$, $—CONR^{II}R^{III}$, $—OR^{IV}$, Alkylcarbonyl mit 1 bis 4 Kohlenstoffatomen im geradkettigen oder verzweigten Alkylteil, gegebenenfalls ein- bis dreifach, gleich oder verschieden durch Fluor, Chlor und Methyl substituiertes Phenyl, jeweils gegebenenfalls ein- bis dreifach durch Methyl substituiertes Cyclopropyl oder Cyclohexyl und die folgenden Heterocyclen :

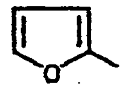, 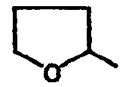, 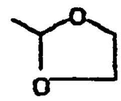, 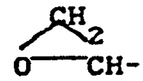,

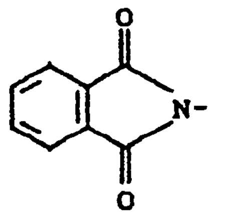, 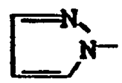 und 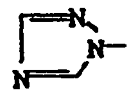 ;

$R^1$ steht ferner für Allyl oder Propargyl, für jeweils gegebenenfalls ein- bis dreifach durch Methyl substituiertes Cyclopropyl, Cyclohexyl, Cyclopentenyl oder Cyclohexenyl ;

$R^2$ für Wasserstoff oder für gegebenenfalls ein- oder zweifach, gleich oder verschieden substituiertes Alkyl mit 1 bis 4 Kohlenstoffatomen steht, wobei folgende Substituenten infrage kommen : Cyano, Methoxy, Ethoxy, Methylthio, Ethylthio, $-COOR^1$, Alkylcarbonylamino mit 1 bis 4 Kohlenstoffatomen im geradkettigen oder verzweigten Alkylteil, gegebenenfalls ein- bis dreifach, gleich oder verschieden durch Halogen, Methyl und Methoxy substituiertes Phenyl, jeweils gegebenenfalls ein- bis dreifach durch Methyl substituiertes Cyclopropyl oder Cyclohexyl und die folgenden Heterocyclen :

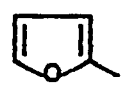, 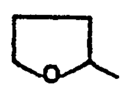, 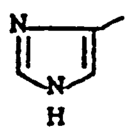 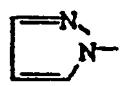 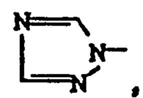,

und 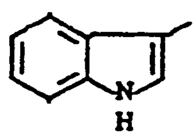 ;

$R^2$ steht ferner für jeweils gegebenenfalls durch Phenyl, das gegebenenfalls ein- bis dreifach, gleich oder verschieden durch Halogen und Methyl substituiert sein kann, substituiertes Vinyl, Allyl, Allenyl, Ethinyl oder Propargyl ;

$R^2$ steht außerdem für Cyclopropyl, Cyclohexyl, Cyclopentenyl oder Cyclohexenyl ;

$R^2$ steht weiterhin für gegebenenfalls ein- bis dreifach, gleich oder verschieden durch Halogen, Methyl und Methoxy substituiertes Phenyl oder für die folgenden Heterocyclen :

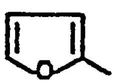, 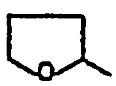, 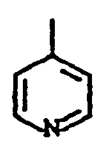, 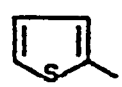 und 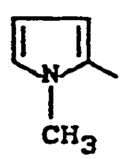 ;

$R^3$ für Wasserstoff oder für gegebenenfalls ein- oder zweifach, gleich oder verschieden substituiertes, geradkettiges oder verzweigtes Alkyl mit 1 bis 4 Kohlenstoffatomen steht, wobei folgende Substituenten infrage kommen : Fluor, Chlor, Brom, Iod, Cyano, Alkoxy, Alkylthio, Alkylsulfinyl und Alkylsulfonyl mit jeweils 1 oder 2 Kohlenstoffatomen, Alkylcarbonyloxy mit 1 bis 4 Kohlenstoffatomen im geradkettigen oder verzweigten Alkylteil, Formamido, Alkylcarbonylamino mit 1 oder 2 Kohlenstoffatomen im gegebenenfalls durch Fluor substituierten Alkylteil, gegebenenfalls durch Chlor substituiertes Benzoylamino, Alkoxycarbonylamino mit 1 bis 4 Kohlenstoffatomen im geradkettigen oder verzweigten Alkoxyteil, $-COOR^1$, gegebenenfalls ein- bis dreifach, gleich oder verschieden durch Halogen und Methyl substituiertes Phenyl, Cyclopropyl, Cyclopentyl oder Cyclohexyl und die folgenden Heterocyclen :

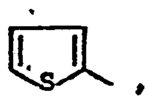 , 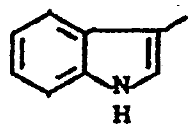 , 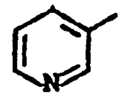 , 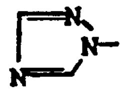 , 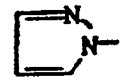 ,

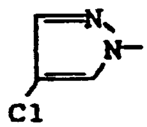 , 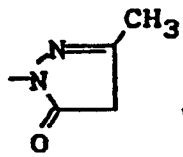 und 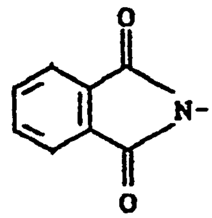 ;

$R^3$ steht ferner für jeweils gegebenenfalls einfach oder zweifach, gleich oder verschieden durch Phenyl, das gegebenenfalls ein- bis dreifach, gleich oder verschieden durch Halogen und Methyl substituiert sein kann, oder durch Methyl substituiertes Vinyl oder Ethinyl ;

$R^3$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis dreifach, gleich oder verschieden substituiertes Cyclopropyl, Cyclopentyl, Cyclohexyl, Cyclopentenyl oder Cyclohexenyl, wobei als Substituenten genannt seien : Halogen, Methyl, die Oxogruppe, Phenyl, Hydroxycarbonyl, Methoxy- und Ethoxycarbonyl ; im einzelnen seien als Ringe genannt :

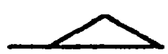 , 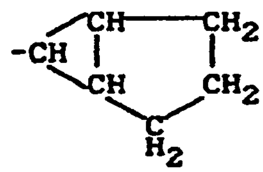 , 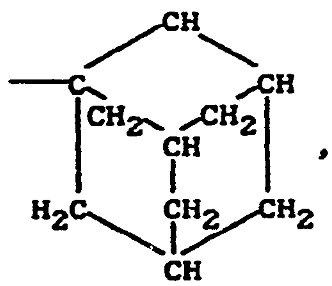 ,

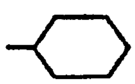 , 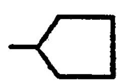 , 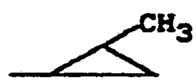 , 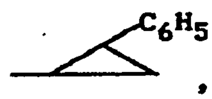 ,

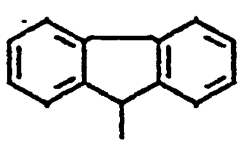 , 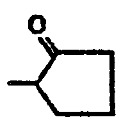 , 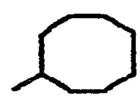 ,

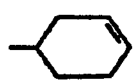 , 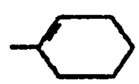 ;

$R^3$ steht außerdem für gegebenenfalls mit 1 oder 2 Benzol-, Cyclopentan- oder Cyclohexanringen anelliertes, gegebenenfalls ein- bis dreifach, gleich oder verschieden substituiertes Phenyl, wobei als Substituenten genannt seien : Fluor, Chlor, Hydroxy, Nitro, Methyl, Methoxy, Acetoxy, Acetamido, Hydroxycarbonyl, Methoxy- und Ethoxycarbonyl ;

$R^3$ steht ferner für gegebenenfalls mit 1 oder 2 Benzol-, Cyclopentan- oder Cyclohexanringen anelliertes, gegebenenfalls ein- bis dreifach, gleich oder verschieden substituiertes 5- oder 6-gliedriges Heterocyclyl mit 1 bis 3 gleichen oder verschiedenen Heteroatomen, wobei als Substituenten genannt seien : Acyl mit 2 bis 5 Kohlenstoffatomen, Chlor, Brom, Methyl, Ethyl, Phenyl, die Oxogruppe und Hydroxy ; im einzelnen seien als Heterocyclen genannt :

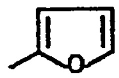 , 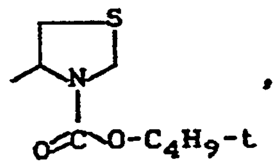 , 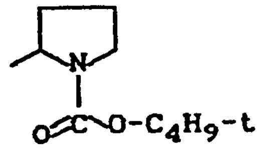 ,

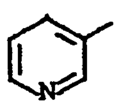 , 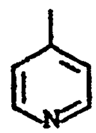 , 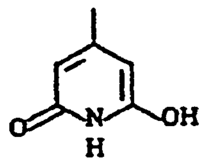 , 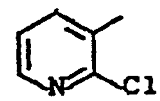 ,

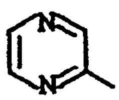 , 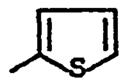 , 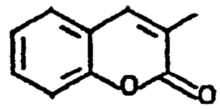 , 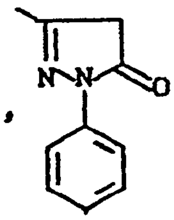 ,

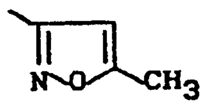 ;

$R^3$ steht schließlich auch für Alkoxycarbonyl mit 1 bis 4 Kohlenstoffatomen im geradkettigen oder verzweigten Alkoxyteil, für —$OR^4$, —$SR^4$ oder —$NR^5R^6$ ;

$R^4$ für gegebenenfalls ein- oder zweifach, gleich oder verschieden substituiertes, geradkettiges oder verzweigtes Alkyl mit 1 oder 2 Kohlenstoffatomen steht, wobei folgende Substituenten infrage kommen : Fluor, Chlor, Methoxy, Ethoxy, Methylthio, Alkoxycarbonyl mit 1 bis 4 Kohlenstoffatomen im geradkettigen oder verzweigten Alkylteil, Carbamoyl, Methylcarbamoyl, Dimethylcarbamoyl, Cyano, Dimethylamino, Diethylamino, Acetyl, Pivaloyl, jeweils gegebenenfalls ein- bis dreifach, gleich oder verschieden durch Halogen und Methyl substituiertes Phenyl oder Phenoxy, jeweils gegebenenfalls ein-bis dreifach durch Methyl substituiertes Cyclopropyl oder Cyclohexyl ;

$R^4$ steht ferner für gegebenenfalls ein- bis dreifach, gleich oder verschieden durch Halogen, Methyl und Ethyl substituiertes 3- bis 6-gliedriges Heterocyclyl mit 1 bis 3 gleichen oder verschiedenen Heteroatomen ; im einzelnen seien als Heterocyclen genannt :

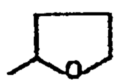 , 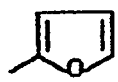 , 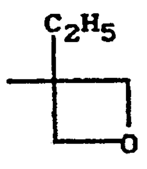 , 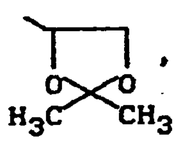 , 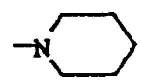 ,

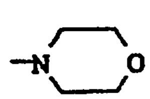 , 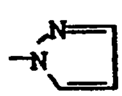 , 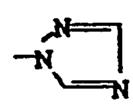 ;

$R^4$ steht außerdem für jeweils gegebenenfalls ein-oder zweifach durch Methyl substituiertes Allyl oder Propargyl oder für jeweils gegebenenfalls ein- bis dreifach durch Methyl substituiertes Cyclohexyl ;

$R^5$ für Wasserstoff, geradkettiges oder verzweigtes Alkyl mit 1 bis 4 Kohlenstoffatomen oder ein- oder zweifach, gleich oder verschieden substituiertes Alkyl mit 1 bis 3 Kohlenstoffatomen steht, wobei als Substituenten genannt seien : Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, Alkoxycarbonyl mit 1 bis 4 Kohlenstoffatomen im geradkettigen oder verzweigten Alkoxyteil, Carbamoyl, Methylcarbamoyl, Dimethylcarbamoyl, Cyano, Dimethylamino, Diethylamino, gegebenenfalls ein- bis dreifach, gleich oder verschieden durch Chlor und Methyl substituiertes Phenyl, 2-Furyl, 2-Pyridyl, 1-Morpholino, Cyclopropyl oder Cyclohexyl ;

$R^5$ steht ferner für Allyl oder Propargyl, für 1-Cyclohexenyl oder für ein- bis dreifach, gleich oder verschieden substituiertes Cyclohexyl, wobei als Substituenten genannt seien : Chlor, Methyl, Ethyl, Methoxy, Cyano, Amino, Alkyl- und Dialkylamino mit jeweils 1 oder 2 Kohlenstoffatomen in jedem Alkylteil, Carbamoyl, Alkyl- und Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil oder 1-Pyrrolidin-2-on ;

$R^5$ steht außerdem für gegebenenfalls ein-bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

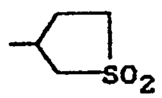 ,

$R^6$ für Wasserstoff, geradkettiges oder verzweigtes Alkyl mit 1 bis 4 Kohlenstoffatomen oder für ein- oder zweifach, gleich oder verschieden substituiertes Alkyl mit 1 bis 3 Kohlenstoffatomen steht, wobei als Substituenten genannt seien : Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, Alkoxycarbonyl mit 1 bis 4 Kohlenstoffatomen im geradkettigen oder verzweigten Alkoxyteil, Carbamoyl, Methylcarbamoyl, Dimethylcarbamoyl, Cyano, Dimethylamino, Diethylamino, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl ;

$R^6$ steht ferner für Allyl oder Propargyl, für 1-Cyclohexenyl oder für ein-bis dreifach, gleich oder verschieden substituiertes Cyclohexyl, wobei als Substituenten genannt seien : Chlor, Methyl, Ethyl, Methoxy, Cyano, Amino, Alkyl- oder Dialkylamino mit jeweils 1 oder 2 Kohlenstoffatomen in jedem Alkylteil, Carbamoyl, Alkyl- oder Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil oder 1-Pyrrolidin-2-on ;

$R^6$ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

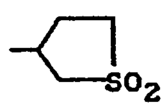 ,

$R^6$ steht weiterhin für Hydroxy, Alkoxy mit 1 oder 2 Kohlenstoffatomen, für gegebenenfalls ein- bis dreifach, gleich oder verschieden durch Chlor und Methyl substituiertes Benzyloxy, für 1-Adamantyl, 2-Norbornyl, 1- oder 2-Decalyl oder 1- oder 2-Tetralyl ;

oder

$R^5$ und $R^6$ gemeinsam mit dem Stickstoffatom, an das sie gebunden sind, für folgende mono-, bi- oder tricyclischen Heterocyclen oder Spiroheterocyclen stehen :

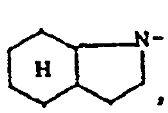 , 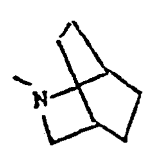 , 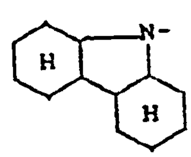 ,

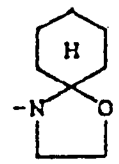 , 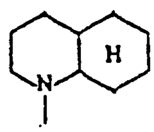 ,

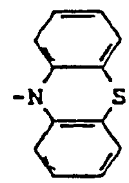 , 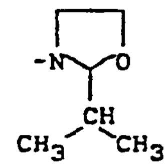 , 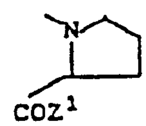

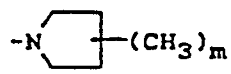 , 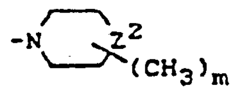

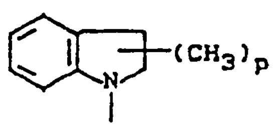 , 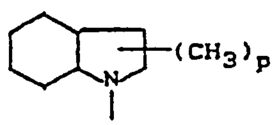 .

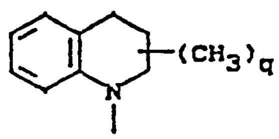 , 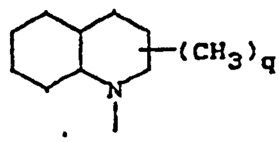 ,

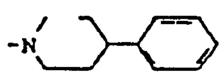 , 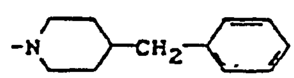 ,

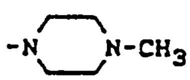 , 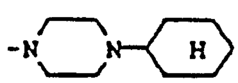 , 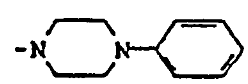 ,

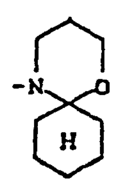 , 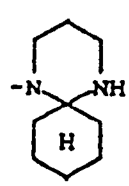 , 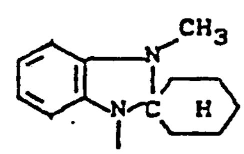 ,

wobei

$Z^1$ für Hydroxy, Methoxy, Ethoxy, Amino, Methylamino, Ethylamino, Dimethylamino oder Ethylmethylamino steht,

$Z^2$ für Sauerstoff oder die $CH_2$-Gruppe steht,

m für die Zahlen 0, 1, 2, 3 oder 4 steht,

p für die Zahlen 0, 1 oder 2 steht,

q für die Zahlen 0, 1, 2 oder 3 steht,

$R^I$ für Methyl, Ethyl, n- oder i-Propyl und n-, i-, s-oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für

gegebenenfalls ein- bis dreifach durch Methyl substituiertes Cyclohexyl stehen,

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien; und

X für Sauerstoff oder Schwefel steht.

4. Schädlingsbekämpfungsmittel, gekennzeichnet durch einen Gehalt an mindestens einem N,N'-Diacylaminal der Formel (I) nach Anspruch 1.

5. Verwendung von N,N'-Diacylaminalen der Formel (I) nach Anspruch 1 zur Bekämpfung von Schädlingen.

6. Verfahren zur Bekämpfung von Schädlingen, dadurch gekennzeichnet, daß man N,N'-Diacylaminale der Formel (I) nach Anspruch 1 auf Schädlinge und/oder ihren Lebensraum einwirken läßt.

7. Verfahren zur Herstellung von Schädlingsbekämpfungsmitteln, dadurch gekennzeichnet, daß man N,N'-Diacylaminale der Formel (I) nach Anspruch 1 mit Streckmitteln und/oder oberflächenaktiven Mitteln vermischt.

8. Verfahren zur Herstellung von N-(2-Cyan-2-oximinoacetyl)-aminalen der allgemeinen Formel (II),

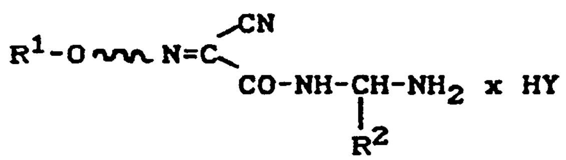
(II)

in welcher

$R^1$, $R^2$ und HY die in Anspruch 1 angegebene Bedeutung haben, dadurch gekennzeichnet, daß man Isocyanate der Formel (IV),

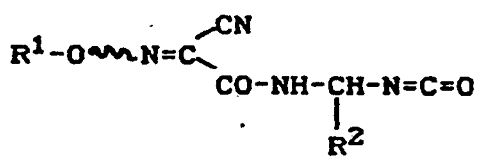
(IV)

in welcher

$R^1$ und $R^2$ die oben angegebene Bedeutung haben, in üblicher Weise hydrolisiert und daß entstehende Amin in üblicher Weise als Salz isoliert.

9. Verfahren zur Herstellung von Isocyanaten der allgemeinen formel (IV),

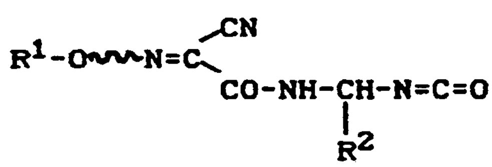
(IV)

in welcher

$R^1$ und $R^2$ die in Anspruch 1 angegebene Bedeutung haben, dadurch gekennzeichnet, daß man

α) Amide der allgemeinen Formel (VI),

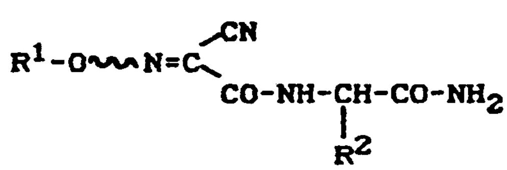
(VI)

in welcher

$R^1$ und $R^2$ die oben angegebene Bedeutung haben, in üblicher Weise, gegebenenfalls in Gegenwart eines Verdünnungsmittels oxidiert,

oder

β) Azide der allgemeinen Formel (VII),

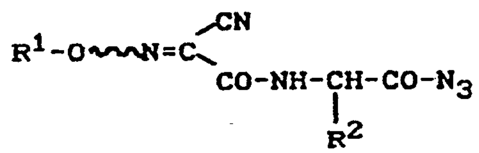

(VII)

in welcher

$R^1$ und $R^2$ die oben angegebene Bedeutung haben, in Gegenwart eines Verdünnungsmittels erwärmt.

## Claims

### Claims for the following Contracting States : BE, CH, DE, FR, GB, IT, LI, NL

1. N,N'-Diacylaminals of the general formula (I)

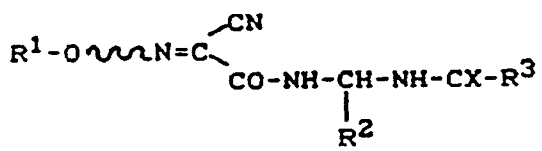

(I)

in which

$R^1$ represents straight-chain or branched alkyl having 1 to 6 carbon atoms which is optionally mono-substituted to pentasubstituted by identical or different substituents, the following substituents being suitable : fluorine, chlorine, bromine, iodine, cyano, —$COOR^{I}$, —$CONR^{II}R^{III}$, —$OR^{IV}$, acyl having 2 to 9 carbon atoms, phenyl which is optionally monosubstituted to pentasubstituted by identical or different substitutents, phenyl substituents which may be mentioned being : halogen, alkyl and alkoxy each having 1 to 4 carbon atoms, halogenoalkyl and halogenoalkoxy each having 1 or 2 carbon atoms and 1 to 5 identical or different halogen atoms ; further alkyl substituents are cycloalkyl having 3 to 6 carbon atoms which is optionally monosubstituted to pentasubstituted by identical or different alkyl having 1 to 4 carbon atoms, and 3- to 6-membered heterocyclyl having 1 to 3 identical or different hetero atoms which is optionally monosubstituted to pentasubstituted by identical or different substituents in the series comprising halogen and alkyl having 1 to 4 carbon atoms ; $R^1$ furthermore represents straight-chain or branched alkenyl or alkinyl, each of which has 2 to 6 carbon atoms and is optionally monosubstituted, disubstituted or trisubstituted by identical or different substituents, substituents which may be mentioned being : phenyl which is optionally monosubstituted to pentasubstituted by identical or different substituents from the series comprising halogen and alkyl having 1 to 4 carbon atoms ; $R^1$ in addition represents cycloalkyl having 3 to 6 carbon atoms or cycloalkenyl having 5 to 7 carbon atoms, each of which is optionally monosubstituted to pentasubstituted by identical or different alkyl having 1 to 4 carbon atoms ;

$R^2$ represents hydrogen or straight-chain or branched alkyl having 1 to 6 carbon atoms which is optionally monosubstituted to pentasubstituted by identical or different substituents, suitable substituents being the following : cyano, alkoxy and alkylthio, each having 1 to 4 carbon atoms, —$COOR^{I}$, acylamino having 2 to 9 carbon atoms, phenyl which is optionally monosubstituted to pentasubstituted by identical or different substituents, suitable substituents being the phenyl substituents mentioned above in the case of $R^1$ ; further alkyl substituents are cycloalkyl having 3 to 6 carbon atoms which is optionally monosubstituted to pentasubstituted by identical or different alkyl having 1 to 4 carbon atoms, and 3- to 6-membered heterocyclyl having 1 to 3 identical or different hetero atoms which is optionally monosubstituted to pentasubstituted by identical or different substituents from the series comprising halogen and alkyl having 1 to 4 carbon atoms ; $R^2$ furthermore represents straight-chain or branched alkenyl or alkinyl, each of which has 2 to 6 carbon atoms and is optionally monosubstituted, disubstituted or trisubstituted by identical or different substituents, substituents which may be mentioned being: phenyl which is optionally monosubstituted to pentasubstituted by identical or different substituents from the series comprising halogen and alkyl having 1 to 4 carbon atoms ; $R^2$ in addition represents cycloalkyl having 3 to 6 carbon atoms or cycloalkenyl having 5 to 7 carbon atoms, each of which is optionally monosubstituted to pentasubstituted by identical or different alkyl having 1 to 4 carbon atoms ; $R^2$ additionally represents phenyl which is optionally monosubstituted to pentasubstituted by identical or different substituents, suitable substituents being the phenyl substituents mentioned above in the case of $R^1$ ; $R^2$ furthermore represents 3- to 6-membered heterocyclyl having 1 to 3 identical or different hetero atoms which is optionally monosubstituted to pentasubstituted by identical or different substituents from the series comprising halogen and alkyl having

1 to 4 carbon atoms ;

$R^3$ represents hydrogen or straight-chain or branched alkyl having 1 to 6 carbon atoms which is optionally monosubstituted to pentasubstituted by identical or different substituents, suitable substituents being the following : fluorine, chlorine, bromine, iodine, cyano, alkoxy, alkylthio, alkylsulphinyl and alkylsulphonyl each having 1 to 4 carbon atoms, acyloxy and acylamino each having 2 to 9 carbon atoms, —COOR$^I$, phenyl which is optionally monosubstituted to pentasubstituted by identical or different substituents, suitable substituents being the phenyl substituents mentioned above in the case of $R^1$ ; further alkyl substituents are cycloalkyl having 3 to 6 carbon atoms which is optionally monosubstituted to pentasubstituted by identical or different alkyl having 1 to 4 carbon atoms, and 3- to 6-membered heterocyclyl having 1 to 3 identical or different hetero atoms which is optionally monosubstituted to pentasubstituted by identical or different substituents from the series comprising halogen and alkyl having 1 to 4 carbon atoms ; $R^3$ furthermore represents straight-chain or branched alkenyl or alkinyl, each of which has 2 to 6 carbon atoms and is optionally monosubstituted, disubstituted or trisubstituted by identical or different substituents, substituents which may be mentioned being : phenyl which is optionally monosubstituted to pentasubstituted by identical or different substituents from the series comprising halogen and alkyl having 1 to 4 carbon atoms ; $R^3$ furthermore represents cycloalkyl having 3 to 6 carbon atoms or cycloalkenyl having 5 to 7 carbon atoms, each of which is optionally bridged by methylene or ethylene and/or fused to 1 or 2 benzene, cyclopentane or cyclohexane rings and is optionally monosubstituted to pentasubstituted by identical or different substituents, substituents which may be mentioned being : halogen, alkyl and alkoxy each having 1 to 4 carbon atoms, acyloxy and acylamino each having 2 to 5 carbon atoms, the oxo group, phenyl, hydroxycarbonyl and alkoxycarbonyl having 1 to 4 carbon atoms in the alkoxy part ; $R^3$ in addition represents phenyl which is optionally fused to 1 or 2 benzene or cyclohexane rings and is optionally monosubstituted to pentasubstituted by identical or different substituents, substituents which may be mentioned being : halogen, hydroxyl, nitro, alkyl and alkoxy each having 1 to 4 carbon atoms, halogenoalkyl and halogenoalkoxy each having 1 or 2 carbon atoms and 2 to 5 identical or different halogen atoms, acylamino and acylalkylamino each having 2 to 5 carbon atoms in the acyl part and 1 to 4 carbon atoms in the alkyl part, hydroxycarbonyl and alkoxycarbonyl having 1 to 4 carbon atoms in the alkoxy part ; $R^3$ furthermore represents 5- or 6-membered heterocyclyl having 1 to 3 identical or different hetero atoms which is optionally fused to 1 or 2 benzene, cyclopentane or cyclohexane rings and is optionally monosubstituted to pentasubstituted by identical or different substituents, substituents which may be mentioned being : halogen, alkyl having 1 to 4 carbon atoms, acyl having 2 to 9 carbon atoms, phenyl, the oxo group and hydroxyl ; finally, $R^3$ alternatively represents alkoxycarbonyl having 1 to 4 carbon atoms in the alkoxy part, or represents —OR$^4$, —SR$^4$, or —NR$^5$R$^6$ ;

$R^4$ represents straight-chain or branched alkyl having 1 to 6 carbon atoms which is optionally monosubstituted to pentasubstituted by identical or different substituents, suitable substituents being the following : halogen, —OR$^{IV}$, —SR$^{IV}$, —COOR$^I$, —CONR$^{II}$R$^{III}$, CN, —NR$^{II}$R$^{III}$, acyl having 2 to 9 carbon atoms, phenyl or phenoxy, each of which is optionally monosubstituted to pentasubstituted by identical or different substituents from the series comprising halogen and alkyl having 1 to 4 carbon atoms, cycloalkyl having 3 to 6 carbon atoms or cycloalkenyl having 5 to 7 carbon atoms, each of which is optionally monosubstituted to pentasubstituted by identical or different alkyl having 1 to 4 carbon atoms, and 3- to 6-membered heterocyclyl having 1 to 3 identical or different hetero atoms which is optionally monosubstituted to pentasubstituted by identical or different substituents from the series comprising halogen and alkyl having 1 to 4 carbon atoms ; $R^4$ furthermore represents alkenyl or alkinyl each having 2 to 6 carbon atoms or cycloalkenyl having 3 to 6 carbon atoms or cycloalkenyl having 5 to 7 carbon atoms, each of which is optionally monosubstituted to pentasubstituted by identical or different alkyl having 1 to 4 carbon atoms ;

$R^5$ represents hydrogen or straight-chain or branched alkyl having 1 to 4 carbon atoms which is optionally monosubstituted or disubstituted by identical or different substituents, substituents which may be mentioned being : halogen, cyano, —COOR$^I$, —CONR$^{II}$R$^{III}$, —NR$^{II}$R$^{III}$, —OR$^{IV}$, —S(O)$_n$R$^{IV}$, acyl having 2 to 9 carbon atoms, phenyl which is optionally monosubstituted to pentasubstituted by identical or different substituents from the series comprising halogen and alkyl having 1 to 4 carbon atoms, a 5- or 6-membered heterocyclic ring having 1 to 3 identical or different hetero atoms, which is optionally monosubstituted to pentasubstituted by identical or different substituents from the series comprising halogen, alkyl and alkoxy each having 1 to 4 carbon atoms, or cycloalkyl having 3 to 6 carbon atoms or cycloalkenyl having 5 to 7 carbon atoms, each of which is optionally monosubstituted to pentasubstituted by identical or different alkyl having 1 to 4 carbon atoms ; $R^5$ furthermore represents straight-chain or branched alkenyl or alkinyl each having 2 to 6 carbon atoms ; represents cycloalkenyl having 5 to 7 carbon atoms which is optionally monosubstituted to pentasubstituted by identical or different alkyl having 1 to 4 carbon atoms, or represents cycloalkyl having 3 to 6 carbon atoms which is optionally monosubstituted to pentasubstituted by identical or different substituents, substituents which may be mentioned being : halogen ; alkyl and alkoxy each having 1 to 4 carbon atoms, cyano, amino, carbamoyl, alkylamino, dialkylamino, alkylcarbamoyl and dialkylcarbamoyl each having 1 to 4 carbon atoms in each alkyl part, Alkoxycarbonylamino having 1 to 4 carbon atoms in the alkoxy part, cycloalkyl and cycloalkylalkyl having 5 or 6 carbon atoms in the cycloalkyl part and 1 to 4 carbon atoms in the straight-chain or branched alkyl part, or phenyl or pyrrolidone, each of which is optionally monosubstituted to pentasubstituted by identical or different substituents from the series comprising halogen and alkyl having 1 to 4 carbon atoms ; $R^5$ additionally represents phenyl which is optionally monosubstituted to pentasubstituted by identical or different substituents from the series comprising halogen, alkyl or alkoxy each having 1 to 4 carbon atoms, or halogenoalkyl or halogenoalkoxy each having 1 or 2 carbon atoms and 1 to 5 identical or different halogen atoms, or represents a 5- or 6-membered heterocyclic ring having 1 to 3 identical or different hetero atoms which is optionally monosubstituted to pentasubstituted by identical or different substituents ; substituents which may be mentioned are : halogen, mercapto, phenyl, straight-chain or branched alkyl, alkoxy, alkylthio, alkylsulphinyl and alkylsulphonyl having 1 to 4 carbon atoms in each alkyl part ;

$R^6$ represents the meanings of $R^5$ or the —$OR^{IV}$ group ;

or

$R^5$ and $R^6$, together with the nitrogen atom to which they are bonded, represent a monocyclic, bicyclic or tricyclic heterocyclic ring or spiroheterocyclic ring having one to three further identical or different hetero atoms which is optionally monosubstituted to pentasubstituted by identical or different substituents, substituents which may be mentioned being : straight-chain or branched alkyl or alkoxy each having 1 to 4 carbon atoms, cycloalkyl having 3 to 6 carbon atoms, the hydroxyl or the oxo group, straight-chain or branched alkenyl having 2 to 4 carbon atoms, hydroxycarbonyl, alkoxycarbonyl having 1 to 4 carbon atoms in the straight-chain or branched alkyl part, carbamoyl, alkylcarbamoyl or dialkylcarbamoyl each having 1 to 4 carbon atoms in the straight-chain or branched alkyl part, or phenyl or phenylalkyl which has 1 to 4 carbon atoms in the straight-chain or branched alkyl part and each of which is optionally monosubstituted to pentasubstituted by identical or different substituents from the series comprising halogen or straight-chain or branched alkyl or alkoxy each having 1 to 4 carbon atoms ;

$R^I$ represents hydrogen or straight-chain or branched alkyl having 1 to 6 carbon atoms,

$R^{II}$ represents hydrogen, straight-chain or branched alkyl having 1 to 6 carbon atoms, phenylalkyl which has 1 to 4 carbon atoms in the straight-chain or branched alkyl part and is optionally monosubstituted to pentasubstituted by identical or different substituents, phenyl substituents which may be mentioned being : halogen, alkyl or alkoxy each having 1 to 4 carbon atoms, or halogenoalkyl or halogenoalkoxy each having 1 or 2 carbon atoms and 2 to 5 identical or different halogen atoms ; $R^{II}$ furthermore represents alkoxycarbonylalkyl, carbamoylalkyl, alkylcarbamoylalkyl or dialkylcarbamoylalkyl each having 1 to 4 carbon atoms in each alkyl part, or for cycloalkyl having 3 to 6 carbon atoms which is optionally substituted by straight-chain or branched alkyl having 1 to 4 carbon atoms,

$R^{III}$ represents hydrogen, straight-chain or branched alkyl having 1 to 6 carbon atoms, phenylalkyl which has 1 to 4 carbon atoms in the straight-chain or branched alkyl part and is optionally monosubstituted to pentasubstituted by identical or different substituents, suitable phenyl substituents being the phenyl substituents mentioned in the case of $R^{II}$ ; $R^{III}$ furthermore represents alkoxycarbonylalkyl, carbamoylalkyl, alkylcarbamoylalkyl or dialkylcarbamoylalkyl each having 1 to 4 carbon atoms in each alkyl part, or for cycloalkyl having 3 to 6 carbon atoms which is optionally substituted by straight-chain or branched alkyl having 1 to 4 carbon atoms ;

$R^{IV}$ represents hydrogen, straight-chain or branched alkyl having 1 to 6 carbon atoms, phenylalkyl which has 1 to 4 carbon atoms in the straight-chain or branched alkyl part and is optionally monosubstituted to pentasubstituted by identical or different substituents, suitable phenyl substituents being the phenyl substituents mentioned in the case of $R^{II}$ ; or $R^{IV}$ furthermore represents acyl having 2 to 9 carbon atoms ;

n represents the numbers 0, 1 or 2, and

X represents oxygen or sulphur.

2. N,N'-Diacylaminals according to Claim 1, where, in the formula (I),

$R^1$ represents straight-chain or branched alkyl having 1 to 4 carbon atoms which is optionally monosubstituted, disubstituted or trisubstituted by identical or different substituents, suitable substituents being the following : fluorine, chlorine, bromine, iodine, cyano, —$COOR^I$, —$CONR^{II}R^{III}$, —$OR^{IV}$, acyl having 2 to 9 carbon atoms, phenyl which is optionally monosubstituted, disubstituted or trisubstituted by identical or different substituents from the series comprising fluorine, chlorine, methyl and methoxy, cyclopropyl or cyclohexyl, each of which is optionally monosubstituted, disubstituted or trisubstituted by methyl, or heterocyclic rings of the formulae

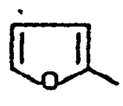, 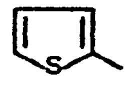, 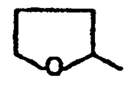, 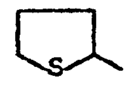, 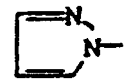

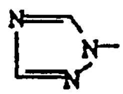, 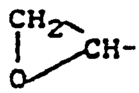, 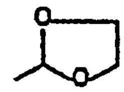 and 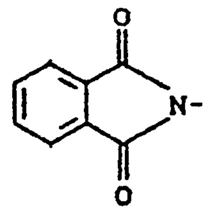 ;

which are optionally monosubstituted, disubstituted or trisubstituted by identical or different alkyl having 1 or 2 carbon atoms ; $R^1$ furthermore represents allyl or propargyl, each of which is optionally monosubstituted or disubstituted by methyl, or represents cyclopropyl, cyclohexyl, cyclopentenyl or cyclohexenyl, each of which is optionally monosubstituted, disubstituted or trisubstituted by methyl ;

$R^2$ represents hydrogen or straight-chain or branched alkyl having 1 to 4 carbon atoms which is optionally monosubstituted, disubstituted or trisubstituted by identical or different substituents, suitable substituents being the following : cyano, methoxy, ethoxy, methylthio, ethylthio, —COOR$^I$, acylamino having 2 to 9 carbon atoms, phenyl which is optionally monosubstituted, disubstituted or trisubstituted by identical or different substituents from the series comprising halogen, methyl and methoxy, cyclopropyl or cyclohexyl, each of which is optionally monosubstituted, disubstituted or trisubstituted by methyl, or heterocyclic rings of the formulae

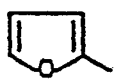, 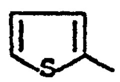, 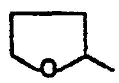, 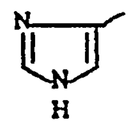, 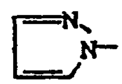

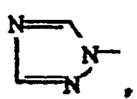, 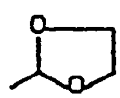 and 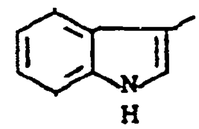 ;

each of which is optionally monosubstituted, disubstituted or trisubstituted by identical or different alkyl having 1 or 2 carbon atoms ; $R^2$ furthermore represents allyl, allenyl, vinyl, propargyl or ethinyl, each of which is optionally substituted by phenyl which may optionally be monosubstituted, disubstituted or trisubstituted by identical or different substituents from the series comprising halogen and methyl ; $R^2$ in addition represents cyclopropyl, cyclohexyl, cyclopentenyl or cyclohexenyl ; $R^2$ additionally represents phenyl which is optionally monosubstituted, disubstituted or trisubstituted by identical or different substituents from the series comprising halogen, methyl and methoxy, or heterocyclic rings of the formulae

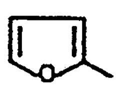, 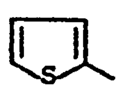, 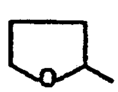, 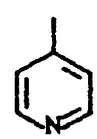, 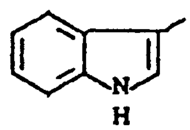,

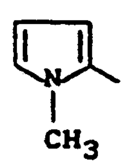, 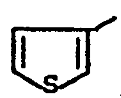 ;

each of which is optionally monosubstituted, disubstituted or trisubstituted by identical or different alkyl having 1 or 2 carbon atoms ;

$R^3$ represents hydrogen or straight-chain or branched alkyl having 1 to 4 carbon atoms which is optionally

monosubstituted, disubstituted or trisubstituted by identical or different substituents, suitable substituents being the following : fluorine, chlorine, bromine, iodine, cyano, alkoxy, alkylthio, alkylsulphinyl and alkylsulphonyl each having 1 or 2 carbon atoms, acyloxy and acylamino each having 2 to 9 carbon atoms, —COOR$^I$, phenyl, cyclopropyl, cyclopentyl or cyclohexyl which is optionally monosubstituted, disubstituted or trisubstituted by identical or different substituents from the series comprising halogen and methyl, or heterocyclic rings of the formulae

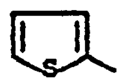,
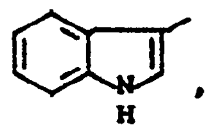,
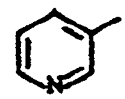,
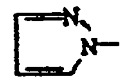,

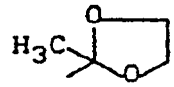,
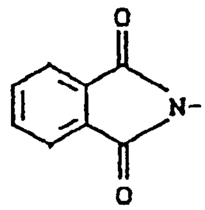,
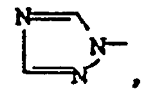,
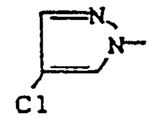,

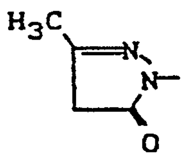,
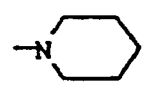,
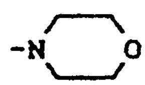;

each of which is optionally monosubstituted, disubstituted or trisubstituted by identical or different alkyl having 1 or 2 carbon atoms ; R$^3$ furthermore represents vinyl or ethinyl, each of which is optionally substituted by phenyl which may optionally be monosubstituted, disubstituted or trisubstituted by identical or different alkyl having 1 or 2 carbon atoms ; R$^3$ furthermore represents vinyl or ethinyl, each of which is optionally substituted by phenyl which may optionally monosubstituted, disubstituted or trisubstituted by identical or different substituents from the series comprising halogen and methyl, or by methyl ; R$^3$ additionally represents cyclopropyl, cyclopentyl, cyclohexyl, cyclopentenyl or cyclohexenyl, each of which is optionally bridged by methylene or ethylene and/or fused to 1 or 2 benzene, cyclopentane or cyclohexane rings and each of which is optionally monosubstituted, disubstituted or trisubstituted by identical or different substituents, substituents which may be mentioned being: halogen, methyl, methoxy, acyloxy and acylamino each having 2 to 5 carbon atoms, the oxo group, phenyl, hydroxycarbonyl, methoxycarbonyl and ethoxycarbonyl ; R$^3$ in addition represents phenyl which is optionally fused to 1 or 2 benzene or cyclohexane rings and is optionally monosubstituted, disubstituted or trisubstituted by identical or different substituents, substituents which may be mentioned being : fluorine, chlorine, hydroxyl, nitro, methyl, methoxy, acylamino and N-alkyl-acyl-amino each having 2 to 5 carbon atoms in the acyl part and 1 or 2 carbon atoms in the alkyl part, hydroxycarbonyl, methoxycarbonyl and ethoxycarbonyl ; R$^3$ furthermore represents 5- or 6-membered heterocyclyl having 1 to 3 identical or different hetero atoms which is optionally fused to 1 or 2 benzene or cyclohexane rings and is optionally monosubstituted, disubstituted or trisubstituted by identical or different substituents, substituents which may be mentioned being : acyl having 2 to 5 carbon atoms, chlorine, bromine, methyl, ethyl, phenyl, oxo and hydroxyl ; finally, R$^3$ alternatively represents alkoxycarbonyl having 1 to 4 carbon atoms in the alkoxy part, or represents —OR$^4$, —SR$^4$ or —NR$^5$R$^6$ ;

R$^4$ represents straight-chain or branched alkyl having 1 to 4 carbon atoms which is optionally monosubstituted, disubstituted or trisubstituted by identical or different substituents, suitable substituents being the following : fluorine, chlorine, —OR$^{IV}$, —SR$^{IV}$, —COOR$^I$, —CONR$^{II}$R$^{III}$, CN, NR$^{II}$R$^{III}$, alkylcarbonyl having 1 to 4 carbon atoms in the straight-chain or branched alkyl part, phenyl or phenoxy, each of which is optionally monosubstituted, disubstituted or trisubstituted by identical or different substituents from the series comprising halogen and methyl, cyclopropyl, cyclohexyl, cyclopentenyl or cyclohexenyl, each of which is optionally monosubstituted, disubstituted or trisubstituted by methyl, or 3- to 6-membered heterocyclyl having 1 to 3 identical or different hetero atoms, such as nitrogen, oxygen or sulphur atoms, which is optionally monosubstituted, disubstituted or trisubstituted by identical or different substituents from the series comprising halogen, methyl and ethyl ; R$^4$ furthermore represents allyl or propargyl, each of which is optionally monosubstituted or disubstituted by methyl, or represents cyclopropyl,cyclohexyl,cyclopentenyl or cyclohexenyl, each of which is

optionally monosubstituted, disubstituted or trisubstituted by methyl ;

$R^5$ represents hydrogen or represents straight-chain or branched alkyl having 1 to 4 carbon atoms which is optionally monosubstituted or disubstituted by identical or different substituents, substituents which may preferably be mentioned being : halogen, cyano, $-COOR^I$, $-CONR^{II}R^{III}$, $NR^{II}R^{III}$, $-OR^{IV}$, $-S(O)_nR^{IV}$, acyl having 2 to 9 carbon atoms, phenyl which is optionally monosubstituted, disubstituted or trisubstituted by identical or different substituents from the series comprising halogen and alkyl having 1 to 4 carbon atoms, a 5- or 6-membered heterocyclic ring having 1 to 3 identical or different hetero atoms which is optionally monosubstituted, disubstituted or trisubstituted by identical or different substituents from the series comprising halogen and straight-chain or branched alkyl and alkoxy each having 1 to 4 carbon atoms, or cycloalkyl having 3 to 6 carbon atoms or cycloalkenyl having 5 to 7 carbon atoms, each of which is optionally substituted by straight-chain or branched alkyl having 1 to 4 carbon atoms ; $R^5$ furthermore represents straight-chain or branched alkenyl or alkinyl each having 2 to 6 carbon atoms, represents cycloalkenyl having 5 to 7 carbon atoms which is optionally substituted by straight-chain or branched alkyl having 1 to 4 carbon atoms, represents optionally substituted cycloalkyl having 3 to 6 carbon atoms, substituents which may be mentioned being : halogen, straight-chain or branched alkyl and alkoxy each having 1 to 4 carbon atoms, cyano, amino, carbamoyl, in each case straight-chain or branched alkylamino, dialkylamino, alkylcarbamoyl and dialkylcarbamoyl each having 1 to 4 carbon atome in each alkyl part, alkoxycarbonylamino having 1 to 4 carbon atoms in the straight-chain or branched alkoxy part, cycloalkyl and cycloalkylalkyl each having 5 or 6 carbon atoms in the cycloalkyl part and 1 to 4 carbon atoms in the straight-chain or branched alkyl part, or phenyl or pyrrolidone, each of which is optionally monosubstituted, disubstituted or trisubstituted by identical or different substituents from the series comprising halogen and alkyl having 1 to 4 carbon atoms ; $R^5$ additionally represents phenyl which is optionally monosubstituted, disubstituted or trisubstituted by identical or different substituents from the series comprising halogen, straight-chain or branched alkyl or alkoxy each having 1 to 4 carbon atoms, and halogenoalkyl or halogenoalkoxy each having 1 or 2 carbon atoms and 1 to 5 identical or different halogen atoms, or represents a 5- or 6-membered heterocyclic ring having 1 to 3 identical or different hetero atoms which is optionally monosubstituted, disubstituted or trisubstituted by identical or different substituents ; substituents which may be mentioned for the heterocyclic rings are : halogen, mercapto, phenyl, straight-chain or branched alkyl, alkoxy, alkylthio, alkylsulphinyl and alkylsulphonyl having 1 to 4 carbon atoms per alkyl ;

$R^6$ represents the meanings of $R^5$ or the $-OR^{IV}$ group,

or

$R^5$ and $R^6$, together which the nitrogen atom to which they are bonded, represent a monocyclic, bicyclic or tricyclic heterocyclic ring or spiroheterocyclic ring which is optionally monosubstituted to pentasubstituted by identical or different substituents, heterocyclic rings which may be mentioned being : oxazolidine, pyrrolidine, imidazolidine, piperidine, piperazine, morpholine, thiomorpholine, 1,3-oxazane or 1,3-diazane ; each of these heterocyclic rings may optionally be fused to 1 or 2 benzene or cyclohexane rings or optionally bridged by methylene or ethylene. Substituents which may be mentioned for all hetero systems are : straight-chain or branched alkyl or cycloalkyl having 3 to 6 carbon atoms, the hydroxyl or the oxo group, straight-chain or branched alkenyl having 2 to 4 carbon atoms, hydroxycarbonyl, alkoxycarbonyl having 1 to 4 carbon atoms in the straight-chain or branched alkyl part, carbamoyl, alkylcarbamoyl or dialkylcarbamoyl each having 1 to 4 carbon atoms in each alkyl part, and furthermore phenyl or phenylalkyl which has 1 to 4 carbon atoms in the straight-chain or branched alkyl part and each of which is optionally monosubstituted or disubstituted by identical or different substituents from the series comprising halogen and straight-chain or branched alkyl and alkoxy each having 1 to 4 carbon atoms,

$R^I$ represents straight-chain or branched alkyl having 1 to 4 carbon atoms,

$R^{II}$ represents hydrogen, straight-chain or branched alkyl having 1 to 4 carbon atoms, benzyl or phenethyl, each of which is optionally monosubstituted, disubstituted or trisubstituted by identical or different substituents, phenyl substituents which may be mentioned in each case being : fluorine, chlorine, methyl, methoxy and trifluormethyl ; $R^{II}$ furthermore represents alkoxycarbonylalkyl having 1 to 4 carbon atoms in the alkoxy part and 1 or 2 carbon atoms in the alkyl part, carbamoylalkyl having 1 or 2 carbon atoms in the alkyl part, alkylcarbamoylalkyl or dialkylcarbamoylalkyl each having 1 or 2 carbon atoms in each alkyl part, or cycloalkyl having 3 to 6 carbon atoms which is optionally monosubstituted, disubstituted or trisubstituted by identical or different substituents from the series comprising methyl and ethyl,

$R^{III}$ represents the meanings of $R^{II}$,

$R^{IV}$ represents hydrogen, straight-chain or branched alkyl having 1 to 4 carbon atoms, or benzyl or phenethyl, each of which is optionally monosubstituted, disubstituted or trisubstituted by identical or different substituents, phenyl substituents which may be mentioned in each case being : fluorine, chlorine, methyl, methoxy and trifluoromethyl ; $R^{IV}$ furthermore represents acyl having 2 to 9 carbon atoms ;

n represents the numbers 0, 1 or 2, and

X represents oxygen or sulphur.

3. N,N'-Diacylaminals according to Claim 1, where, in the formula (I),

$R^1$ represents alkyl having 1 or 2 carbon atoms which is optionally monosubstituted or disubstituted by identical or different substituents, suitable substituents being the following : fluorine, chlorine, cyano, —$COOR^I$, —$CONR^{II}R^{III}$, —$OR^{IV}$, alkylcarbonyl having 1 to 4 carbon atoms in the straight-chain or branched alkyl part, phenyl which is optionally monosubstituted, disubstituted or trisubstituted by identical or different substituents from the series comprising fluorine, chlorine and methyl, cyclopropyl or cyclohexyl, each of which is optionally monosubstituted, disubstituted or trisubstituted by methyl, and the following heterocyclic rings :

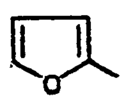 , 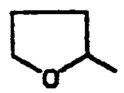 , 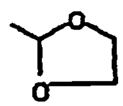 , 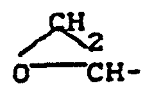

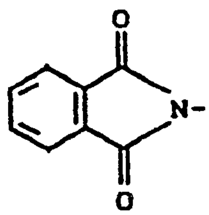 , 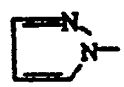 and 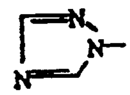 ;

$R^1$ furthermore represents allyl or propargyl, or for cyclopropyl, cyclohexyl, cyclopentenyl or cyclohexenyl, each of which is optionally monosubstituted, disubstituted or trisubstituted by methyl ;

$R^2$ represents hydrogen or represents alkyl having 1 to 4 carbon atoms which is optionally monosubstituted or disubstituted by identical or different substituents, suitable substituents being the following : cyano, methoxy, ethoxy, methylthio, ethylthio, —$COOR^I$, alkylcarbonylamino having 1 to 4 carbon atoms in the straight-chain or branched alkyl part, phenyl which is optionally monosubstituted, disubstituted or trisubstituted by identical or different substituents from the series comprising halogen, methyl and methoxy, cyclopropyl or cyclohexyl, each of which is optionally monosubstituted, disubstituted or trisubstituted by methyl, and the following heterocyclic rings :

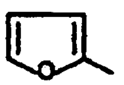 , 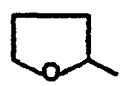 , 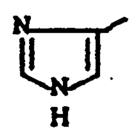 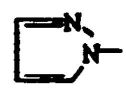 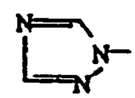 ,

and 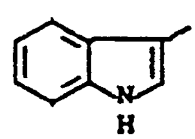 ;

$R^2$ furthermore represents vinyl, allyl, allenyl, ethinyl or propargyl, each of which is optionally substituted by phenyl which may optionally be monosubstituted, disubstituted or trisubstituted by identical or different substituents from the series comprising halogen and methyl ;

$R^2$ in addition represents cyclopropyl, cyclohexyl, cyclopentenyl or cyclohexenyl ;

$R^2$ additionally represents phenyl which is optionally monosubstituted, disubstituted or trisubstituted by identical or different substituents from the series comprising halogen, methyl and methoxy, or represents the following heterocyclic rings :

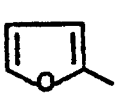 , 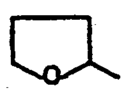 , 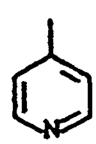 , 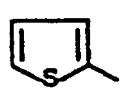 and 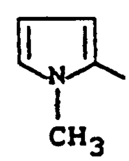 ;

$R^3$ represents hydrogen or represents straight-chain or branched alkyl having 1 to 4 carbon atoms which is optionally monosubstituted or disubstituted by identical or different substituents, suitable substituents being the following : fluorine, chlorine, bromine, iodine, cyano, alkoxy, alkylthio, alkylsulphinyl and alkylsulphonyl each having 1 or 2 carbon atoms, alkylcarbonyloxy having 1 to 4 carbon atoms in the straight-chain or branched alkyl part, formamido, alkylcarbonylamino having 1 or 2 carbon atoms in the alkyl part, which is optionally substituted by fluorine, benzoylamino which is optionally substituted by chlorine, alkoxycarbonylamino having 1 to 4 carbon atoms in the straight-chain or branched alkoxy part,—COOR[I], phenyl, cyclopropyl, cyclopentyl or cyclohexyl which is optionally monosubstituted, disubstituted or trisubstituted by identical or different substituents from the series comprising halogen and methyl, and the following heterocyclic rings :

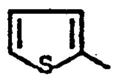 , 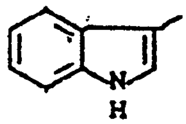 , 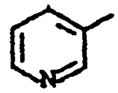 , 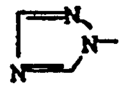 , 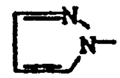 ,

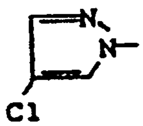 , 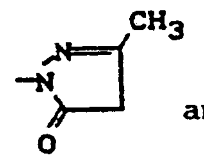 and 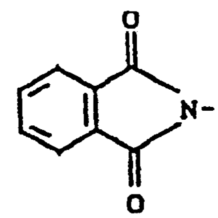 ;

$R^3$ furthermore represents vinyl or ethinyl, each of which is optionally monosubstituted or disubstituted by identical or different substituents from the series comprising phenyl which may optionally be monosubstituted, disubstituted or trisubstituted by identical or different substituents from the series comprising halogen and methyl, or by methyl ;

$R^3$ additionally represents cyclopropyl, cyclopentyl, cyclohexyl, cyclopentenyl or cyclohexenyl, each of which is optionally bridged by methylene or ethylene and/or fused to 1 or 2 benzene, cyclopentane or cyclohexane rings and each of which is optionally monosubstituted, disubstituted or trisubstituted by identical or different substituents, substituents which may be mentioned being : halogen, methyl, the oxo group, phenyl, hydroxycarbonyl, methoxycarbonyl and ethoxycarbonyl ; the following rings may be mentioned individually :

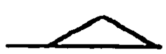 , 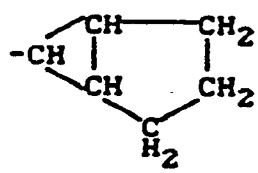 , 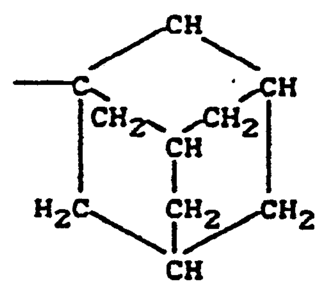 ,

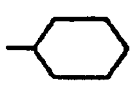 , 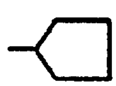 , 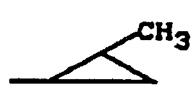 , 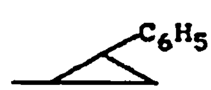 ,

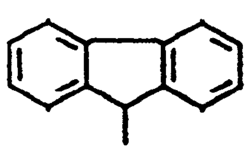 , 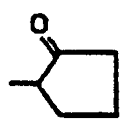 , 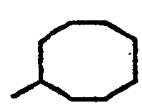 ,

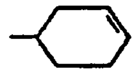 , 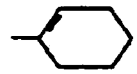 ;

$R^3$ in addition represents phenyl which is optionally fused to 1 or 2 benzene, cyclopentane or cyclohexane rings and is optionally monosubstituted, disubstituted or trisubstituted by identical or different substituents, substituents which may be mentioned being: fluorine, chlorine, hydroxyl, nitro, methyl, methoxy, acetoxy, acetamido, hydroxycarbonyl, methoxycarbonyl and ethoxycarbonyl ;

$R^3$ furthermore represents 5- or 6-membered heterocyclyl having 1 to 3 identical or different hetero atoms, which is optionally fused to 1 or 2 benzene, cyclopentane or cyclohexane rings and is optionally monosubstituted, disubstituted or trisubstituted by identical or different substituents, substituents which may be mentioned being : acyl having 2 to 5 carbon atoms, chlorine, bromine, methyl, ethyl, phenyl, the oxo group and hydroxyl; the following heterocyclic rings may be mentioned individually :

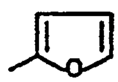 , 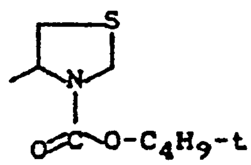 , 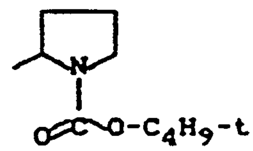 ,

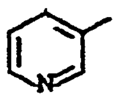 , 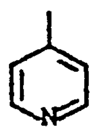 , 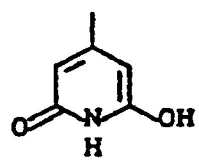 , 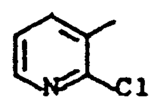 ,

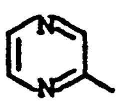 , 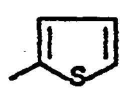 , 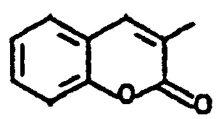 , 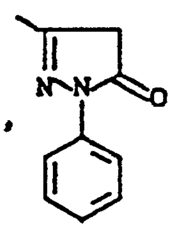 ,

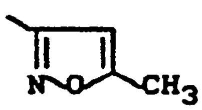 ;

Finally, $R^3$ alternatively represents alkoxycarbonyl having 1 to 4 carbon atoms in the straight-chain or branched alkoxy part, or for —$OR^4$, —$SR^4$ or —$NR^5R^6$ ;

$R^4$ represents straight-chain or branched alkyl having 1 or 2 carbon atoms which is optionally monosubstituted or disubstituted by identical or different substituents, suitable substituents being the following : fluorine, chlorine, methoxy, ethoxy, methylthio, alkoxycarbonyl having 1 to 4 carbon atoms in the straight-chain or branched alkyl part, carbamoyl, methylcarbamoyl, dimethylcarbamoyl, cyano, dimethylamino, diethylamino, acetyl, pivaloyl, or phenyl or phenoxy, each of which is optionally monosubstituted, disubstituted or trisubstituted by identical or different substituents from the series comprising halogen and methyl, and cyclopropyl or cyclohexyl, each of which is optionally monosubstituted, disubstituted or trisubstituted by methyl ;

$R^4$ furthermore represents 3- to 6-membered heterocyclyl having 1 to 3 identical or different hetero atoms which is optionally monosubstituted, disubstituted or trisubstituted by identical or different substituents from the series comprising halogen, methyl and ethyl ; the following heterocyclic rings may be mentioned individually :

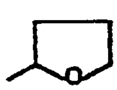 , 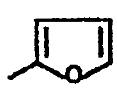 , 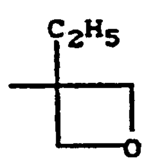 , 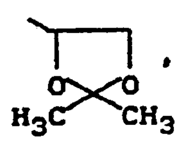 , 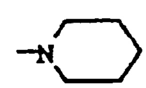 ,

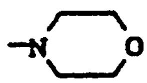 , 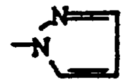 , 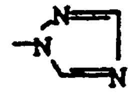 ;

R⁴ in addition represents allyl or propargyl, each of which is optionally monosubstituted or disubstituted by methyl, or represents cyclohexyl which is optionally monosubstituted, disubstituted or trisubstituted by methyl;

$R^5$ represents hydrogen, straight-chain or branched alkyl having 1 to 4 carbon atoms, or alkyl having 1 to 3 carbon atoms which is monosubstituted or disubstituted by identical or different substituents, suitable substituents being : chlorine, hydroxyl, alkylcarbonyloxy having 1 to 4 carbon atoms in the straight-chain or branched alkyl part, alkylcarbonyl having 1 to 4 carbon atoms in the straight-chain or branched alkyl part, alkoxy and alkylthio each having 1 or 2 carbon atoms, alkoxycarbonyl having 1 to 4 carbon atoms in the straight-chain or branched alkoxy part, carbamoyl, methylcarbamoyl, dimethylcarbamoyl, cyano, dimethylamino, diethylamino, phenyl, 2-furyl, 2-pyridyl, 1-morpholino, cyclopropyl or cyclohexyl which is optionally monosubstituted, disubstituted or trisubstituted by identical or different substituents from the series comprising chlorine and methyl ;

$R^5$ furthermore represents allyl or propargyl, represents 1-cyclohexenyl, or represents cyclohexyl which is monosubstituted, disubstituted or trisubstituted by identical or different substituents, suitable substituents being : chlorine, methyl, ethyl, methoxy, cyano, amino, alkylamino and dialkylamino each having 1 or 2 carbon atoms in each alkyl part, carbamoyl, alkylcarbamoyl and dialkylcarbamoyl each having 1 or 2 carbon atoms in each alkyl part, cyclohexyl, cyclohexylalkyl having 1 or 2 carbon atoms in the alkyl part, or 1-pyrrolidin-2-one;

$R^5$ in addition represents phenyl which is optionally monosubstituted, disubstituted or trisubstituted by identical or different substituents from the series comprising chlorine and methyl, for 2-pyridyl or 2-pyrimidinyl, each of which is optionally monosubstituted, disubstituted or trisubstituted by methyl, for 2-thiazolyl which is optionally substituted by phenyl, for 2-benzothiazolyl which is optionally mono-substituted, disubstituted or trisubstituted by identical or different substituents from the series comprising chlorine, methyl and methoxy, for 1,2,4-triazol-3-yl, for 1,2,4-thiadiazol-5-yl which is optionally substituted by phenyl, for 1,3,4-thiadiazol-5-yl which is optionally monosubstituted, disubstituted or trisubstituted by identical or different substituents from the series comprising mercapto, straight-chain or branched alkyl having 1 to 4 carbon atoms, and in each case straight-chain or branched alkylthio, alkylsulphinyl and alkylsulphonyl each having 1 to 4 carbon atoms, or represents the

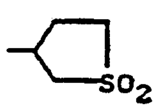 ,

group,

$R^6$ represents hydrogen, straight-chain or branched alkyl having 1 to 4 carbon atoms, or alkyl having 1 to 3 carbon atoms which is monosubstituted or disubstituted by identical or different substituents, substituents which may be mentioned being : chlorine, hydroxyl, alkylcarbonyloxy having 1 to 4 carbon atoms in the straight-chain or branched alkyl part, alkylcarbonyl having 1 to 4 carbon atoms in the straight-chain or branched alkyl part, alkoxy and alkylthio each having 1 or 2 carbon atoms, alkoxycarbonyl having 1 to 4 carbon atoms in the straight-chain or branched alkoxy part, carbamoyl, methylcarbamoyl, dimethylcarbamoyl, cyano, dimethylamino, diethylamino, or phenyl, 2-furyl, 2-pyridyl, 1-morpholino, cyclopropyl and cyclohexyl which is optionally monosubstituted, disubstituted or trisubstituted by identical or different substituents from the series comprising chlorine and methyl ;

$R^6$ furthermore represents allyl or propargyl, represents 1-cyclohexenyl, or represents cyclohexyl which is monosubstituted, disubstituted or trisubstituted by identical or different substituents, substituents which may be mentioned being : chlorine, methyl, ethyl, methoxy, cyano, amino, alkylamino or dialkylamino each having 1 or 2 carbon atoms in each alkyl part, carbamoyl, alkylcarbamoyl or dialkylcarbamoyl each having 1 or 2 carbon atoms in each alkyl part, cyclohexyl, cyclohexylalkyl having 1 or 2 carbon atoms in the alkyl part, or 1-pyrrolidin-2-one ;

$R^6$ in addition represents phenyl which is optionally monosubstituted, disubstituted or trisubstituted by identical or different substituents from the series comprising chlorine and methyl, represents 2-pyridyl or 2-pyrimidinyl, each of which is optionally monosubstituted, disubstituted or trisubstituted by methyl, for 2-thiazolyl which is optionally substituted by phenyl, for 2-benzothiazolyl which is optionally monosubstituted, disubstituted or trisubstituted by identical or different substituents from the series comprising chlorine, methyl and methoxy, for 1,2,4-triazol-3-yl, for 1,2,4-thiadiazol-5-yl which is optionally substituted by phenyl, for 1,3,4-thiadiazol-5-yl which is optionally monosubstituted, disubstituted or trisubstituted by identical or different substituents from the

series comprising mercapto, straight-chain or branched alkyl having 1 to 4 carbon atoms, and in each case straight-chain or branched alkylthio, alkylsulphinyl and alkylsulphonyl each having 1 to 4 carbon atoms, or represents the

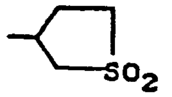,

group,

$R^6$ additionally represents hydroxyl, alkoxy having 1 or 2 carbon atoms, represents benzyloxy which is optionally monosubstituted, disubstituted or trisubstituted by identical or different substituents from the series comprising chlorine and methyl, for 1-adamantyl, 2-norbornyl, 1- or 2-decalyl or 1- or 2-tetralyl ;

or

$R^5$ and $R^6$, together with the nitrogen atom to which they are bonded, represent the following monocyclic, bicyclic or tricyclic heterocyclic rings or spiroheterocyclic rings :

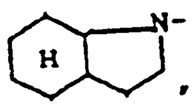, 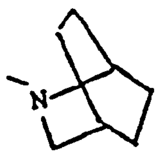, 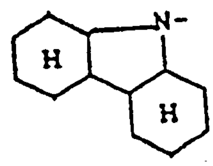

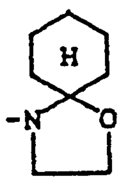, 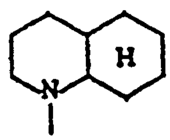,

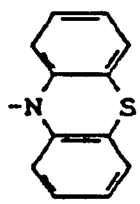, 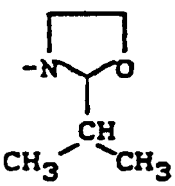, 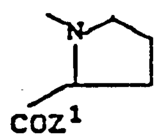

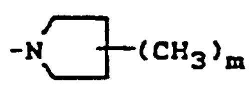, 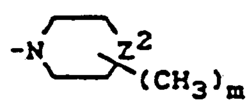

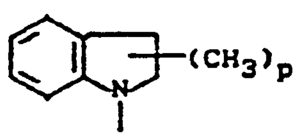, 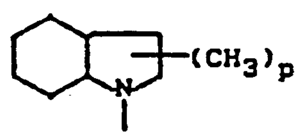.

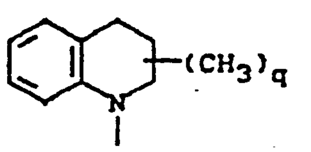, 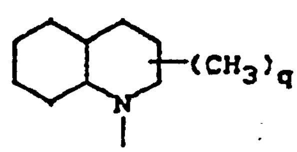,

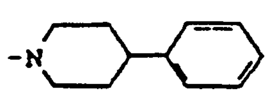, 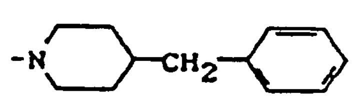,

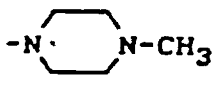 , 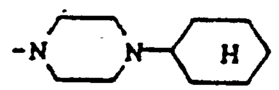 , 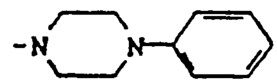 ,

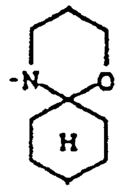 , 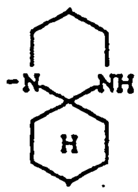 , 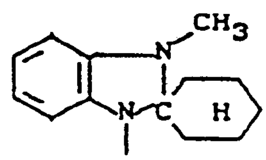 ,

where

$Z^1$ represents hydroxyl, methoxy, ethoxy, amino, methylamino, ethylamino, dimethylamino or ethylmethylamino,

$Z^2$ represents oxygen or the $CH_2$ group,

m represents the numbers 0, 1, 2, 3 or 4,

p represents the numbers 0, 1 or 2,

q represents the numbers 0, 1, 2 or 3

$R^I$ represents methyl, ethyl, n- or i-propyl and n-, i-, s- or t-butyl,

$R^{II}$ and $R^{III}$ are identical or different and represent hydrogen, methyl, ethyl, n- or i-propyl, represent benzyl which is optionally monosubstituted, disubstituted or trisubstituted by identical or different substituents, with chlorine and methyl as substituents, represent alkoxycarbonylalkyl having 1 or 2 carbon atoms in each alkyl part, represent carbamoylalkyl, alkylcarbamoylalkyl or dialkylcarbamoylalkyl each having 1 or 2 carbon atoms in each alkyl part, or represent cyclohexyl which is optionally monosubstituted, disubstituted or trisubstituted by methyl,

$R^{IV}$ represents hydrogen, alkyl having 1 or 2 carbon atoms, represents benzyl which is optionally monosubstituted, disubstituted or trisubstituted by identical or different substituents, phenyl substituents which may be mentioned being fluorine, chlorine and methyl ; and

X represents oxygen or sulphur.

4. Process for the preparation of N,N′-diacylaminals of the general formula (I)

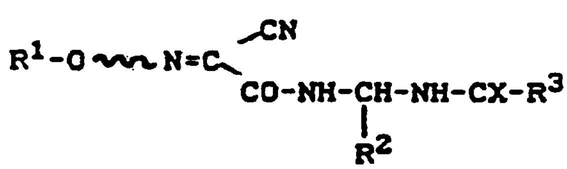 (I)

in which

$R^1$, $R^2$, $R^3$ and X have the meanings given in Claim 1, their geometrical and optical isomers and isomer mixtures, characterised in that

a) N-(2-cyano-2-oximino-acetyl)-aminals of the formula (II)

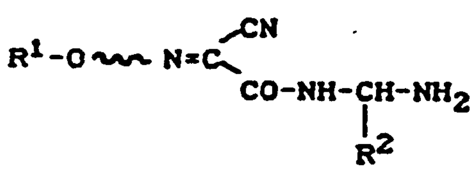 x HY (II)

in which

$R^1$ and $R^2$ have the abovementioned meaning, and

HY represents the equivalent of an inorganic or organic acid,

are reacted with an acylating reagent of the formula (IIIa) or (IIIb)

$$Z-CX-R^3 \quad (IIIa)$$

$X{=}C{=}N{-}R^6$ (IIIb)

in which

Z represents a customary leaving groups such as halogen, —O-CO-$R^3$, —O-CO-$OR^4$, —$OR^4$, —$SR^4$, carboxymethoxy or carboxymethylthio, and

$R^3$, $R^4$, $R^6$ and X have the meaning given in Claim 1, if appropriate in the presence of a base and in the presence of a diluent and if appropriate in the presence of a catalyst;

or

b) isocyanates of the formula (IV)

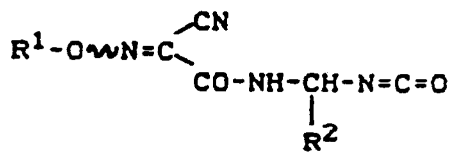

(IV)

in which

$R^1$ and $R^2$ have the abovementioned meaning, are reacted with a protic nucleophile of the formula (Va) or (Vb)

$HO{-}CO{-}R^3$ (Va)

$H{-}R^7$ (Vb)

in which

$R^7$ represents —$OR^4$, —$SR^4$ or —$NR^5R^6$, and $R^3$, $R^4$, $R^5$ and $R^6$ have the meaning given in Claim 1, in the presence of a diluent and if appropriate in the presence of a catalyst.

5. Pesticides, characterised in that they contain at least one N,N'-diacylaminal of the formula (I) according to Claims 1 and 4 for combating pests.

6. Use of N,N'-diacylaminals of the formula (I) according to Claims 1 and 4 for combating pests.

7. Method of combating pests, characterised in that N,N'-diacylaminals of the formula (I) according to Claims 1 and 4 are allowed to act on the pests and/or their environment.

8. Process for the production of pesticides, characterised in that N,N'-diacylaminals of the formula (I) according to Claims 1 and 4 are mixed with extenders and/or surface-active agents.

9. N-(2-Cyano-2-oximino-acetyl)-aminals of the general formula (II)

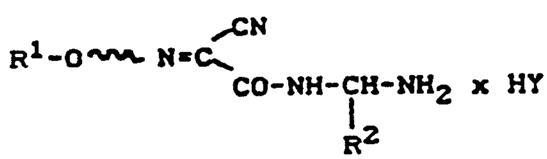

(II)

in which

$R^1$ and $R^2$ have the meaning given in Claim 1, and

HY represents the equivalent of an inorganic or organic acid.

10. Process for the preparation of N-(2-cyano-2-oximinoacetyl)-aminals of the general formula (II)

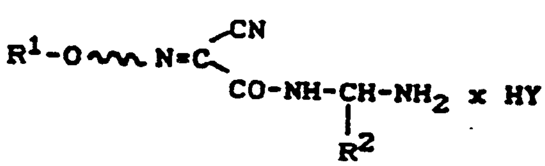

(II)

in which

$R^1$, $R^2$ and HY have the meaning given in Claim 9, characterised in that isocyanates of the formula (IV)

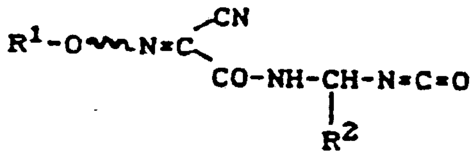

(IV)

in which

$R^1$ and $R^2$ have the abovementioned meaning, are hydrolyzed in a customary manner, and the resultant amine is isolated as a salt in a customary manner.

11. Isocyanates of the formula (IV)

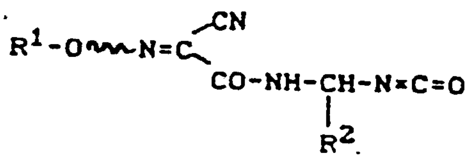

(IV)

in which

$R^1$ and $R^2$ have the meaning given in Claim 1.

12. Procees for the preparation of isocyanates of the general formula (IV)

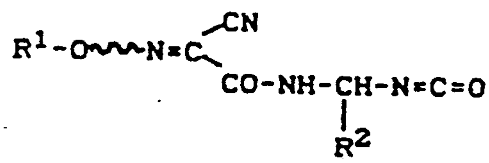

(IV)

in which

$R^1$ and $R^2$ have the meaning given in Claim 1, characterised in that

α) amides of the general formula (VI),

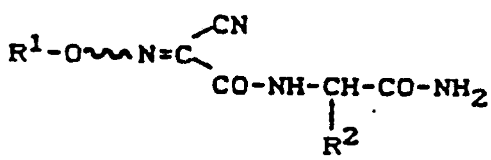

(VI)

in which

$R^1$ and $R^2$ have the abovementioned meaning,

are oxidized in a customary manner, if appropriate in the presence of a diluent,

or

β) azides of the general formula (VII)

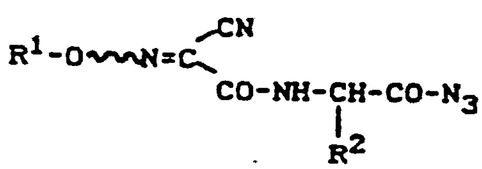

(VII)

in which

$R^1$ and $R^2$ have the abovementioned meaning, are warmed in the presence of a diluent.

**Claims for the following Contracting States : ES**

1. Process for the preparation of N,N′-Diacylaminals of the general formula (I),

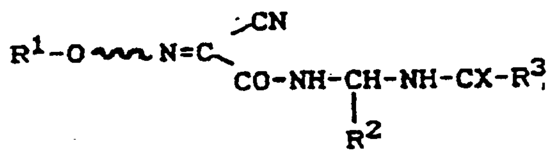 (I)

in which

$R^1$ represents straight-chain or branched alkyl having 1 to 6 carbon atoms which is optionally mono-substituted to pentasubstituted by identical or different substituents, the following substituents being suitable: fluorine, chlorine, bromine, iodine, cyano, —$COOR^I$, —$CONR^{II}R^{III}$, —$OR^{IV}$, acyl having 2 to 9 carbon atoms, phenyl which is optionally monosubstituted to pentasubstituted by identical or different substitutents, phenyl substituents which may be mentioned being: halogen, alkyl and alkoxy each having 1 to 4 carbon atoms, halogenoalkyl and halogenoalkoxy each having 1 or 2 carbon atoms and 1 to 5 identical or different halogen atoms; further alkyl substituents are cycloalkyl having 3 to 6 carbon atoms which is optionally monosubstituted to pentasubstituted by identical or different alkyl having 1 to 4 carbon atoms, and 3- to 6-membered heterocyclyl having 1 to 3 identical or different hetero atoms which is optionally monosubstituted to pentasubstituted by identical or different substituents in the series comprising halogen and alkyl having 1 to 4 carbon atoms; $R^1$ furthermore represents straight-chain or branched alkenyl or alkinyl, each of which has 2 to 6 carbon atoms and is optionally monosubstituted, disubstituted or trisubstituted by identical or different substituents, substituents which may be mentioned being: phenyl which is optionally monosubstituted to pentasubstituted by identical or different substituents from the series comprising halogen and alkyl having 1 to 4 carbon atoms; $R^1$ in addition represents cycloalkyl having 3 to 6 carbon atoms or cycloalkenyl having 5 to 7 carbon atoms, each of which is optionally monosubstituted to pentasubstituted by identical or different alkyl having 1 to 4 carbon atoms;

$R^2$ represents hydrogen or straight-chain or branched alkyl having 1 to 6 carbon atoms which is optionally monosubstituted to pentasubstituted by identical or different substituents, suitable substituents being the following: cyano, alkoxy and alkylthio, each having 1 to 4 carbon atoms, —$COOR^I$, acylamino having 2 to 9 carbon atoms, phenyl which is optionally monosubstituted to pentasubstituted by identical or different substituents, suitable substituents being the phenyl substituents mentioned above in the case of $R^1$; further alkyl substituents are cycloalkyl having 3 to 6 carbon atoms which is optionally monosubstituted to pentasubstituted by identical or different alkyl having 1 to 4 carbon atoms, and 3- to 6-membered heterocyclyl having 1 to 3 identical or different hetero atoms which is optionally monosubstituted to pentasubstituted by identical or different substituents from the series comprising halogen and alkyl having 1 to 4 carbon atoms; $R^2$ furthermore represents straight-chain or branched alkenyl or alkinyl, each of which has 2 to 6 carbon atoms and is optionally monosubstituted, disubstituted or trisubstituted by identical or different substituents, substituents which may be mentioned being: phenyl which is optionally monosubstituted to pentasubstituted by identical or different substituents from the series comprising halogen and alkyl having 1 to 4 carbon atoms; $R^2$ represents in addition cycloalkyl having 3 to 6 carbon atoms or cycloalkenyl having 5 to 7 carbon atoms, each of which is optionally monosubstituted to pentasubstituted by identical or different alkyl having 1 to 4 carbon atoms; $R^2$ additionally represents phenyl which is optionally monosubstituted to pentasubstituted by identical or different substituents, suitable substituents being the phenyl substituents mentioned above in the case of $R^1$; $R^2$ furthermore represents 3- to 6-membered heterocyclyl having 1 to 3 identical or different hetero atoms which is optionally monosubstituted to pentasubstituted by identical or different substituents from the series comprising halogen and alkyl having 1 to 4 carbon atoms;

$R^3$ represents hydrogen or straight-chain or branched alkyl having 1 to 6 carbon atoms which is optionally monosubstituted to pentasubstituted by identical or different substituents, suitable substituents being the following: fluorine, chlorine, bromine, iodine, cyano, alkoxy, alkylthio, alkylsulphinyl and alkylsulphonyl each having 1 to 4 carbon atoms, acyloxy and acylamino each having 2 to 9 carbon atoms, —$COOR^I$, phenyl which is optionally monosubstituted to pentasubstituted by identical or different substituents, suitable substituents being the phenyl substituents mentioned above in the case of $R^1$; further alkyl substituents are cycloalkyl having 3 to 6 carbon atoms which is optionally monosubstituted to pentasubstituted by identical or different alkyl having 1 to 4 carbon atoms, and 3- to 6-membered heterocyclyl having 1 to 3 identical or different hetero atoms which is optionally monosubstituted to pentasubstituted by identical or different substituents from the series comprising halogen and alkyl having 1 to 4 carbon atoms; $R^3$ furthermore represents straight-chain or branched alkenyl or alkinyl, each of which has 2 to 6 carbon atoms and is optionally monosubstituted, disubstituted or trisubstituted by identical or different substituents, substituents which may be mentioned being: phenyl which is optionally monosubstituted to pentasubstituted by identical or different substituents from the series comprising halogen and alkyl having 1 to 4 carbon atoms; $R^3$ furthermore represents cycloalkyl having 3 to 6 carbon atoms or cycloalkenyl having 5 to 7 carbon atoms, each of which is optionally bridged by methylene or ethylene and/or

fused to 1 or 2 benzene, cyclopentane or cyclohexane rings and is optionally monosubstituted to pentasubstituted by identical or different substituents, substituents which may be mentioned being : halogen, alkyl and alkoxy each having 1 to 4 carbon atoms, acyloxy and acylamino each having 2 to 5 carbon atoms, the oxo group, phenyl, hydroxycarbonyl and alkoxycarbonyl having 1 to 4 carbon atoms in the alkoxy part ; $R^3$ in addition represents phenyl which is optionally fused to 1 or 2 benzene or cyclohexane rings and is optionally mono-substituted to pentasubstituted by identical or different substituents, substituents which may be mentioned being: halogen, hydroxyl, nitro, alkyl and alkoxy each having 1 to 4 carbon atoms, halogenoalkyl and halogenoalkoxy each having 1 or 2 carbon atoms and 2 to 5 identical or different halogen atoms, acylamino and acylalkylamino each having 2 to 5 carbon atoms in the acyl part and 1 to 4 carbon atoms in the alkyl part, hydroxycarbonyl and alkoxycarbonyl having 1 to 4 carbon atoms in the alkoxy part ; $R^3$ furthermore represents 5- or 6-membered heterocyclyl having 1 to 3 identical or different hetero atoms which is optionally fused to 1 or 2 benzene, cyclopentane or cyclohexane rings and is optionally monosubstituted to pentasubstituted by identical or different substituents, substituents which may be mentioned being : halogen, alkyl having 1 to 4 carbon atoms, acyl having 2 to 9 carbon atoms, phenyl, the oxo group and hydroxyl ; finally, $R^3$ alternatively represents alkoxycarbonyl having 1 to 4 carbon atoms in the alkoxy part, or represents —$OR^4$, —$SR^4$, or —$NR^5R^6$ ;

$R^4$ represents straight-chain or branched alkyl having 1 to 6 carbon atoms which is optionally monosubstituted to pentasubstituted by identical or different substituents, suitable substituents being the following : halogen, —$OR^{IV}$, —$SR^{IV}$, —$COOR^{I}$, —$CONR^{II}R^{III}$, CN, —$NR^{II}R^{III}$, acyl having 2 to 9 carbon atoms, phenyl or phenoxy, each of which is optionally monosubstituted to pentasubstituted by identical or different substituents from the series comprising halogen and alkyl having 1 to 4 carbon atoms, cycloalkyl having 3 to 6 carbon atoms or cycloalkenyl having 5 to 7 carbon atoms, each of which is optionally monosubstituted to pentasubstituted by identical or different alkyl having 1 to 4 carbon atoms, and 3- to 6-membered heterocyclyl having 1 to 3 identical or different hetero atoms which is optionally monosubstituted to pentasubstituted by identical or different substituents from the series comprising halogen and alkyl having 1 to 4 carbon atoms ; $R^4$ furthermore represents alkenyl or alkinyl each having 2 to 6 carbon atoms or cycloalkenyl having 3 to 6 carbon atoms or cycloalkenyl having 5 to 7 carbon atoms, each of which is optionally monosubstituted to pentasubstituted by identical or different alkyl having 1 to 4 carbon atoms ;

$R^5$ represents hydrogen or straight-chain or branched alkyl having 1 to 4 carbon atoms which is optionally monosubstituted or disubstituted by identical or different substituents, substituents which may be mentioned being : halogen, cyano, —$COOR^{I}$, —$CONR^{II}R^{III}$, —$NR^{II}R^{III}$, —$OR^{IV}$, —$S(O)_nR^{IV}$, acyl having 2 to 9 carbon atoms, phenyl which is optionally monosubstituted to pentasubstituted by identical or different substituents from the series comprising halogen and alkyl having 1 to 4 carbon atoms, a 5- or 6-membered heterocyclic ring having 1 to 3 identical or different hetero atoms, which is optionally monosubstituted to pentasubstituted by identical or different substituents from the series comprising halogen, alkyl and alkoxy each having 1 to 4 carbon atoms, or cycloalkyl having 3 to 6 carbon atoms or cycloalkenyl having 5 to 7 carbon atoms, each of which is optionally monosubstituted to pentasubstituted by identical or different alkyl having 1 to 4 carbon atoms ; $R^5$ furthermore represents straight-chain or branched alkenyl or alkinyl each having 2 to 6 carbon atoms ; represents cycloalkenyl having 5 to 7 carbon atoms which is optionally monosubstituted to pentasubstituted by identical or different alkyl having 1 to 4 carbon atoms, or represents cycloalkyl having 3 to 6 carbon atoms which is optionally monosubstituted to pentasubstituted by identical or different substituents, substituents which may be mentioned being : halogen, alkyl and alkoxy each having 1 to 4 carbon atoms, cyano, amino, carbamoyl, alkylamino, dialkylamino, alkylcarbamoyl and dialkylcarbamoyl each having 1 to 4 carbon atoms in each alkyl part, alkoxycarbonylamino having 1 to 4 carbon atoms in the alkoxy part, cycloalkyl and cycloalkylalkyl having 5 or 6 carbon atoms in the cycloalkyl part and 1 to 4 carbon atoms in the straight-chain or branched alkyl part, or phenyl or pyrrolidone, each of which is optionally monosubstituted to pentasubstituted by identical or different substituents from the series comprising halogen and alkyl having 1 to 4 carbon atoms ; $R^5$ additionally represents phenyl which is optionally monosubstituted to pentasubstituted by identical or different substituents from the series comprising halogen, alkyl or alkoxy each having 1 to 4 carbon atoms, or halogenoalkyl or halogenoalkoxy each having 1 or 2 carbon atoms and 1 to 5 identical or different halogen atoms, or represents a 5-or 6-membered heterocyclic ring having 1 to 3 identical or different hetero atoms which is optionally monosubstituted to pentasubstituted by identical or different substituents ; substituents which may be mentioned are : halogen, mercapto, phenyl, straight-chain or branched alkyl, alkoxy, alkylthio, alkylsulphinyl and alkylsulphonyl having 1 to 4 carbon atoms in each alkyl part ;

$R^6$ represents the meanings of $R^5$ or the —$OR^{IV}$ group ;

or

$R^5$ and $R^6$, together with the nitrogen atom to which they are bonded, represent a monocyclic, bicyclic or tricyclic heterocyclic ring or spiroheterocyclic ring having one to three further identical or different hetero atoms which is optionally monosubstituted to pentasubstituted by identical or different substituents, substituents which

may be mentioned being : straight-chain or branched alkyl or alkoxy each having 1 to 4 carbon atoms, cycloalkyl having 3 to 6 carbon atoms, the hydroxyl or the oxo group, straight-chain or branched alkenyl having 2 to 4 carbon atoms, hydroxycarbonyl, alkoxycarbonyl having 1 to 4 carbon atoms in the straight-chain or branched alkyl part, carbamoyl, alkylcarbamoyl or dialkylcarbamoyl each having 1 to 4 carbon atoms in the straight-chain or branched alkyl part, or phenyl or phenylalkyl which has 1 to 4 carbon atoms in the straight-chain or branched alkyl part and each of which is optionally monosubstituted to pentasubstituted by identical or different substituents from the series comprising halogen or straight-chain or branched alkyl or alkoxy each having 1 to 4 carbon atoms ;

$R^{I}$ represents hydrogen or straight-chain or branched alkyl having 1 to 6 carbon atoms,

$R^{II}$ represents hydrogen, straight-chain or branched alkyl having 1 to 6 carbon atoms, phenylalkyl which has 1 to 4 carbon atoms in the straight-chain or branched alkyl part and is optionally monosubstituted to pentasubstituted by identical or different substituents, phenyl substituents which may be mentioned being : halogen, alkyl or alkoxy each having 1 to 4 carbon atoms, or halogenoalkyl or halogenoalkoxy each having 1 or 2 carbon atoms and 2 to 5 identical or different halogen atoms ; $R^{II}$ furthermore represents alkoxycarbonylalkyl, carbamoylalkyl, alkylcarbamoylalkyl or dialkylcarbamoylalkyl each having 1 to 4 carbon atoms in each alkyl part, or for cycloalkyl having 3 to 6 carbon atoms which is optionally substituted by straight-chain or branched alkyl having 1 to 4 carbon atoms ;

$R^{III}$ represents hydrogen, straight-chain or branched alkyl having 1 to 6 carbon atoms, phenylalkyl which has 1 to 4 carbon atoms in the straight-chain or branched alkyl part and is optionally monosubstituted to pentasubstituted by identical or different substituents, suitable phenyl substituents being the phenyl substituents mentioned in the case of $R^{II}$ ; $R^{III}$ furthermore represents alkoxycarbonylalkyl, carbamoylalkyl, alkylcarbamoylalkyl or dialkylcarbamoylalkyl each having 1 to 4 carbon atoms in each alkyl part, or for cycloalkyl having 3 to 6 carbon atoms which is optionally substituted by straight-chain or branched alkyl having 1 to 4 carbon atoms ;

$R^{IV}$ represents hydrogen, straight-chain or branched alkyl having 1 to 6 carbon atoms, phenylalkyl which has 1 to 4 carbon atoms in the straight-chain or branched alkyl part and is optionally monosubstituted to pentasubstituted by identical or different substituents, suitable phenyl substituents being the phenyl substituents mentioned in the case of $R^{II}$ ; or $R^{IV}$ furthermore represents acyl having 1 to 9 carbon atoms ;

n represents the numbers 0, 1 or 2, and

X represents oxygen or sulphur, their geometrical and optical isomers and isomer mixtures, characterised in that

a) n-(2-cyano-2-oximino-acetyl)-aminals of the formula (II)

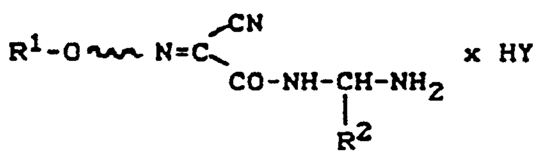

(II)

in which

$R^1$ and $R^2$ have the abovementioned meaning, and

HY represents the equivalent of an inorganic or organic acid,

are reacted with an acylating reagent of the formula (IIIa) or (IIIb)

$$Z\text{-}CX\text{-}R^3 \qquad \text{(IIIa)}$$

$$X{=}C{=}N\text{-}R^6 \qquad \text{(IIIb)}$$

in which

Z represents a customary leaving group, such as halogen, —O-CO-$R^3$, —O-CO-O$R^4$, —O$R^4$, —S$R^4$, carboxymethoxy or carboxymethylthio, and

$R^3$, $R^4$, $R^6$ and X have the abovementioned meaning, if appropriate in the presence of a base and in the presence of a diluent and if appropriate in the presence of a catalyst ;

or

b) isocyanates of the formula (IV)

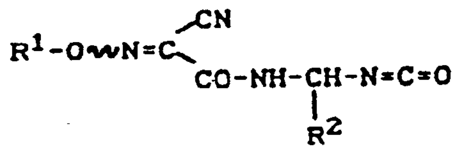 (IV)

in which

$R^1$ and $R^2$ have the abovementioned meaning, are reacted with a protic nucleophile of the formula (Va) or (Vb)

$$HO{-}CO{-}R^3 \qquad (Va)$$

$$H{-}R^7 \qquad (Vb)$$

in which

$R^7$ represents —$OR^4$, —$SR^4$ or —$NR^5R^6$, and

$R^3$, $R^4$, $R^5$ and $R^6$ have the abovementioned meaning,

in the presence of a diluent and if appropriate in the presence of a catalyst.

2. Process according to Claim 1, where, in the formula I,

$R^1$ represents straight-chain or branched alkyl having 1 to 4 carbon atoms which is optionally monosubstituted, disubstituted or trisubstituted by identical or different substituents, suitable substituents being the following : fluorine, chlorine, bromine, iodine, cyano, —$COOR^I$, —$CONR^{II}R^{III}$, —$OR^{IV}$, acyl having 2 to 9 carbon atoms, phenyl which is optionally monosubstituted, disubstituted or trisubstituted by identical or different substituents from the series comprising fluorine, chlorine, methyl and methoxy, cyclopropyl or cyclohexyl, each of which is optionally monosubstituted, disubstituted or trisubstituted by methyl, or heterocyclic rings of the formulae

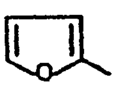 , 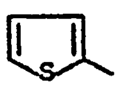 , 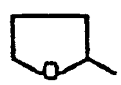 , 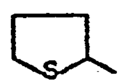 , 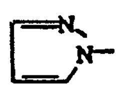

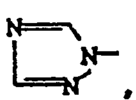 , 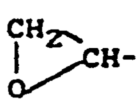 , 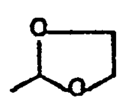 and 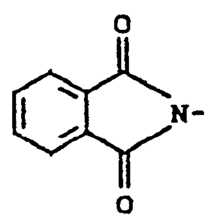 ;

which are optionally monosubstituted, disubstituted or trisubstituted by identical or different alkyl having 1 or 2 carbon atoms ; $R^1$ furthermore represents allyl or propargyl, each of which is optionally monosubstituted or disubstituted by methyl, or represents cyclopropyl, cyclohexyl, cyclopentenyl or cyclohexenyl, each of which is optionally monosubstituted, disubstituted or trisubstituted by methyl ;

$R^2$ represents hydrogen or straight-chain or branched alkyl having 1 to 4 carbon atoms which is optionally monosubstituted, disubstituted or trisubstituted by identical or different substituents, suitable substituents being the following : cyano, methoxy, ethoxy, methylthio, ethylthio, —$COOR^I$, acylamino having 2 to 9 carbon atoms, phenyl which is optionally monosubstituted, disubstituted or trisubstituted by identical or different substituents from the series comprising halogen, methyl and methoxy, cyclopropyl or cyclohexyl, each of which is optionally monosubstituted, disubstituted or trisubstituted by methyl, or heterocyclic rings of the formulae

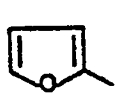 , 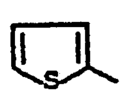 , 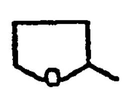 , 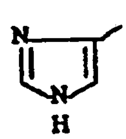 , 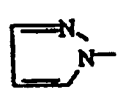

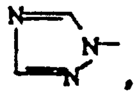, 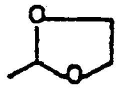 and 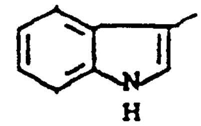 ;

each of which is optionally monosubstituted, disubstituted or trisubstituted by identical or different alkyl having 1 or 2 carbon atoms ; $R^2$ furthermore represents allyl, allenyl, vinyl, propargyl or ethinyl, each of which is optionally substituted by phenyl which may optionally be monosubstituted, disubstituted or trisubstituted by identical or different substituents from the series comprising halogen and methyl ; $R^2$ in addition represents cyclopropyl, cyclohexyl, cyclopentenyl or cyclohexenyl ; $R^2$ additionally represents phenyl which is optionally monosubstituted, disubstituted or trisubstituted by identical or different substituents from the series comprising halogen, methyl and methoxy, or heterocyclic rings of the formulae

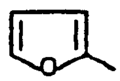, 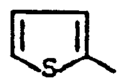, 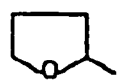, 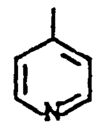, 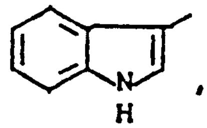,

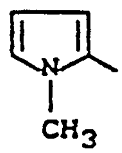, 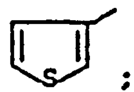 ;

each of which is optionally monosubstituted, disubstituted or trisubstituted by identical or different alkyl having 1 or 2 carbon atoms ;

$R^3$ represents hydrogen or straight-chain or branched alkyl having 1 to 4 carbon atoms which is optionally monosubstituted, disubstituted or trisubstituted by identical or different substituents, suitable substituents being the following : fluorine, chlorine, bromine, iodine, cyano, alkoxy, alkylthio, alkylsulphinyl and alkylsulphonyl each having 1 or 2 carbon atoms, acyloxy and acylamino each having 2 to 9 carbon atoms, —COOR$^I$, phenyl, cyclopropyl, cyclopentyl or cyclohexyl which is optionally monosubstituted, disubstituted or trisubstituted by identical or different substituents from the series comprising halogen and methyl, or heterocyclic rings of the formulae

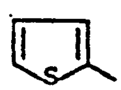, 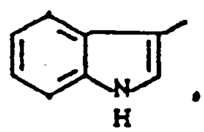, 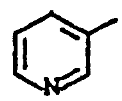, 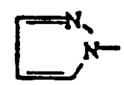,

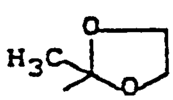, 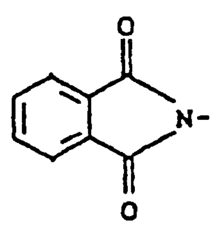, 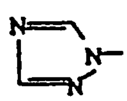, 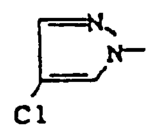,

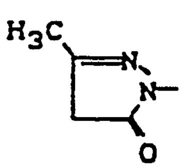, 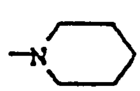, 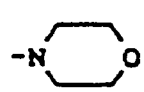 ;

each of which is optionally monosubstituted, disubstituted or trisubstituted by identical or different alkyl having 1 or 2 carbon atoms ; $R^3$ furthermore represents vinyl or ethinyl, each of which is optionally substituted by phenyl which may optionally be monosubstituted, disubstituted or trisubstituted by identical or different alkyl having 1 or 2 carbon atoms ; $R^3$ furthermore represents vinyl or ethinyl, each of which is optionally substituted by phenyl which may optionally monosubstituted, disubstituted or trisubstituted by identical or different substituents from

the series comprising halogen and methyl, or by methyl ; $R^3$ additionally represents cyclopropyl, cyclopentyl, cyclohexyl, cyclopentenyl or cyclohexenyl, each of which is optionally bridged by methylene or ethylene and/or fused to 1 or 2 benzene, cyclopentane or cyclohexane rings and each of which is optionally monosubstituted, disubstituted or trisubstituted by identical or different substituents, substituents which may be mentioned being: halogen, methyl, methoxy, acyloxy and acylamino each having 2 to 5 carbon atoms, the oxo group, phenyl, hydroxycarbonyl, methoxycarbonyl and ethoxycarbonyl ; $R^3$ in addition represents phenyl which is optionally fused to 1 or 2 benzene or cyclohexane rings and is optionally monosubstituted, disubstituted or trisubstituted by identical or different substituents, substituents which may be mentioned being : fluorine, chlorine, hydroxyl, nitro, methyl, methoxy, acylamino and N-alkyl-acyl-amino each having 2 to 5 carbon atoms in the acyl part and 1 or 2 carbon atoms in the alkyl part, hydroxycarbonyl, methoxycarbonyl and ethoxycarbonyl ; $R^3$ furthermore represents 5- or 6-membered heterocyclyl having 1 to 3 identical or different hetero atoms which is optionally fused to 1 or 2 benzene or cyclohexane rings and is optionally monosubstituted, disubstituted or trisubstituted by identical or different substituents, substituents which may be mentioned being : acyl having 2 to 5 carbon atoms, chlorine, bromine, methyl, ethyl, phenyl, oxo and hydroxyl ; finally, $R^3$ alternatively represents alkoxycarbonyl having 1 to 4 carbon atoms in the alkoxy part, or represents $—OR^4$, $—SR^4$ or $—NR^5R^6$ ;

$R^4$ represents straight-chain or branched alkyl having 1 to 4 carbon atoms which is optionally monosubstituted, disubstituted or trisubstituted by identical or different substituents, suitable substituents being the following : fluorine, chlorine, $—OR^{IV}$, $—SR^{IV}$, $—COOR^{I}$, $—CONR^{II}R^{III}$, CN, $NR^{II}R^{III}$, alkylcarbonyl having 1 to 4 carbon atoms in the straight-chain or branched alkyl part, phenyl or phenoxy, each of which is optionally monosubstituted, disubstituted or trisubstituted by identical or different substituents from the series comprising halogen and methyl, cyclopropyl, cyclohexyl, cyclopentenyl or cyclohexenyl, each of which is optionally monosubstituted, disubstituted or trisubstituted by methyl, or 3- to 6-membered heterocyclyl having 1 to 3 identical or different hetero atoms, such as nitrogen, oxygen or sulphur atoms, which is optionally monosubstituted, disubstituted or trisubstituted by identical or different substituents from the series comprising halogen, methyl and ethyl ; $R^4$ furthermore represents allyl or propargyl, each of which is optionally mono-substituted or disubstituted by methyl, or represents cyclopropyl, cycloheyxl, cyclopentenyl or cyclohexenyl, each of which is optionally monosubstituted, disubstituted or trisubstituted by methyl ;

$R^5$ represents hydrogen or represents straight-chain or branched alkyl having 1 to 4 carbon atoms which is optionally monosubstituted or disubstituted by identical or different substituents, substituents which may preferably be mentioned being : halogen, cyano, $—COOR^{I}$, $—CONR^{II}R^{III}$, $NR^{II}R^{III}$, $—OR^{IV}$, $—S(O)_nR^{IV}$, acyl having 2 to 9 carbon atoms, phenyl which is optionally monosubstituted, disubstituted or trisubstituted by identical or different substituents from the series comprising halogen and alkyl having 1 to 4 carbon atoms, a 5- or 6-membered heterocyclic ring having 1 to 3 identical or different hetero atoms which is optionally monosubstituted, disubstituted or trisubstituted by identical or different substituents from the series comprising halogen and straight-chain or branched alkyl and alkoxy each having 1 to 4 carbon atoms, or cycloalkyl having 3 to 6 carbon atoms or cycloalkenyl having 5 to 7 carbon atoms, each of which is optionally substituted by straight-chain or branched alkyl having 1 to 4 carbon atoms ; $R^5$ furthermore particularly preferably represents straight-chain or branched alkenyl or alkinyl each having 2 to 6 carbon atoms, represents cycloalkenyl having 5 to 7 carbon atoms which is optionally substituted by straight-chain or branched alkyl having 1 to 4 carbon atoms, represents optionally substituted cycloalkyl having 3 to 6 carbon atoms, substituents which may preferably be mentioned being : halogen, straight-chain or branched alkyl and alkoxy each having 1 to 4 carbon atoms, cyano, amino, carbamoyl, in each case straight-chain or branched alkylamino, dialkylamino, alkylcarbamoyl and dialkylcarbamoyl each having 1 to 4 carbon atoms in each alkyl part, alkoxycarbonylamino having 1 to 4 carbon atoms in the straight-chain or branched alkoxy part, cycloalkyl and cycloalkylalkyl each having 5 or 6 carbon atoms in the cycloalkyl part and 1 to 4 carbon atoms in the straight-chain or branched alkyl part, or phenyl or pyrrolidone, each of which is optionally monosubstituted, disubstituted or trisubstituted by identical or different substituents from the series comprising halogen and alkyl having 1 to 4 carbon atoms ; $R^5$ additionally particularly preferably represents phenyl which is optionally monosubstituted, disubstituted or trisubstituted by identical or different substituents from the series comprising halogen, straight-chain or branched alkyl or alkoxy each having 1 to 4 carbon atoms, and halogenoalkyl or halogenoalkoxy each having 1 or 2 carbon atoms and 1 to 5 identical or different halogen atoms, or represents a 5- or 6-membered heterocyclic ring having 1 to 3 identical or different hetero atoms which is optionally monosubstituted, disubstituted or trisubstituted by identical or different substituents ; substituents which may be mentioned for the heterocyclic rings are : halogen, mercapto, phenyl, straight-chain or branched alkyl, alkoxy, alkylthio, alkylsulphinyl and alkylsulphonyl having 1 to 4 carbon atoms per alkyl ;

$R^6$ represents the meanings of $R^5$ or the $—OR^{IV}$ group,

or

$R^5$ and $R^6$, together which the nitrogen atom to which they are bonded, represent a monocyclic, bicyclic

or tricyclic heterocyclic ring or spiroheterocyclic ring which is optionally monosubstituted to pentasubstituted by identical or different substituents, heterocyclic rings which may be mentioned being : oxazolidine, pyrrolidine, imidazolidine, piperidine, piperazine, morpholine, thiomorpholine, 1,3-oxazane or 1,3-diazane ; each of these heterocyclic rings may optionally be fused to 1 or 2 benzene or cyclohexane rings or optionally bridged by methylene or ethylene. Substituents which may be mentioned for all hetero systems are : straight-chain or branched alkyl or cycloalkyl having 3 to 6 carbon atoms, the hydroxyl or the oxo group, straight-chain or branched alkenyl having 2 to 4 carbon atoms, hydroxycarbonyl, alkoxycarbonyl having 1 to 4 carbon atoms in the straight-chain or branched alkyl part, carbamoyl, alkylcarbamoyl or dialkylcarbamoyl each having 1 to 4 carbon atoms in each alkyl part, and furthermore phenyl or phenylalkyl which has 1 to 4 carbon atoms in the straight-chain or branched alkyl part and each of which is optionally monosubstituted or disubstituted by identical or different substituents from the series comprising halogen and straight-chain or branched alkyl and alkoxy each having 1 to 4 carbon atoms,

$R^{I}$ represents straight-chain or branched alkyl having 1 to 4 carbon atoms,

$R^{II}$ represents hydrogen, straight-chain or branched alkyl having 1 to 4 carbon atoms, benzyl or phenethyl, each of which is optionally monosubstituted, disubstituted or trisubstituted by identical or different substituents, phenyl substituents which may be mentioned in each case being : fluorine, chlorine, methyl, methoxy and trifluormethyl ; $R^{II}$ furthermore represents alkoxycarbonylalkyl having 1 to 4 carbon atoms in the alkoxy part and 1 or 2 carbon atoms in the alkyl part, carbamoylalkyl having 1 or 2 carbon atoms in the alkyl part, alkylcarbamoylalkyl or dialkylcarbamoylalkyl each having 1 or 2 carbon atoms in each alkyl part, or cycloalkyl having 3 to 6 carbon atoms which is optionally monosubstituted, disubstituted or trisubstituted by identical or different substituents from the series comprising methyl and ethyl,

$R^{III}$ represents the meanings of $R^{II}$,

$R^{IV}$ represents hydrogen, straight-chain or branched alkyl having 1 to 4 carbon atoms, or benzyl or phenethyl, each of which is optionally monosubstituted, disubstituted or trisubstituted by identical or different substituents, phenyl substituents which may be mentioned in each case being : fluorine, chlorine, methyl, methoxy and trifluoromethyl ; $R^{IV}$ furthermore represents acyl having 2 to 9 carbon atoms ;

n represents the numbers 0, 1 or 2, and

X represents oxygen or sulphur.

3. Process according to Claim 1, where, in the formula (I),

$R^1$ represents alkyl having 1 or 2 carbon atoms which is optionally monosubstituted or disubstituted by identical or different substituents, suitable substituents being the following : fluorine, chlorine, cyano, $-COOR^{I}$, $-CONR^{II}R^{III}$, $-OR^{IV}$, alkylcarbonyl having 1 to 4 carbon atoms in the straight-chain or branched alkyl part, phenyl which is optionally monosubstituted, disubstituted or trisubstituted by identical or different substituents from the series comprising fluorine, chlorine and methyl, cyclopropyl or cyclohexyl, each of which is optionally monosubstituted, disubstituted or trisubstituted by methyl, and the following heterocyclic rings :

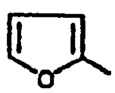 , 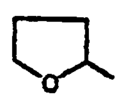 , 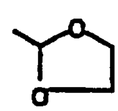 , 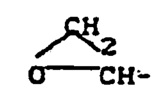

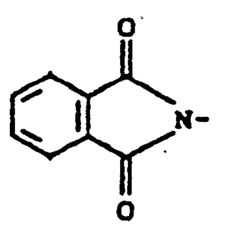 , 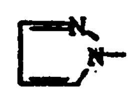 and 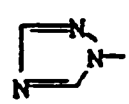 ;

$R^1$ furthermore represents allyl or propargyl, or for cyclopropyl, cyclohexyl, cyclopentenyl or cyclohexenyl, each of which is optionally monosubstituted, disubstituted or trisubstituted by methyl ;

$R^2$ represents hydrogen or represents alkyl having 1 to 4 carbon atoms which is optionally monosubstituted or disubstituted by identical or different substituents, suitable substituents being the following : cyano, methoxy, ethoxy, methylthio, ethylthio, $-COOR^{I}$, alkylcarbonylamino having 1 to 4 carbon atoms in the straight-chain or branched alkyl part, phenyl which is optionally monosubstituted, disubstituted or trisubstituted by identical or different substituents from the series comprising halogen, methyl and methoxy, cyclopropyl or cyclohexyl, each of which is optionally monosubstituted, disubstituted or trisubstituted by methyl, and the following heterocyclic rings :

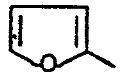 , 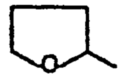 , 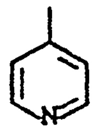 , 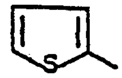 and 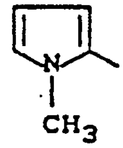 ;

and 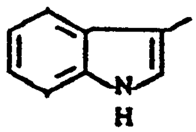 ;

$R^2$ furthermore represents vinyl, allyl, allenyl, ethinyl or propargyl, each of which is optionally substituted by phenyl which may optionally be monosubstituted, disubstituted or trisubstituted by identical or different substituents from the series comprising halogen and methyl ;

$R^2$ in addition represents cyclopropyl, cyclohexyl, cyclopentenyl or cyclohexenyl ;

$R^2$ additionally represents phenyl which is optionally monosubstituted, disubstituted or trisubstituted by identical or different substituents from the series comprising halogen, methyl and methoxy, or represents the following heterocyclic rings :

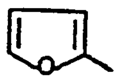 , 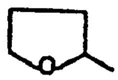 , 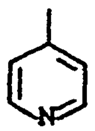 , 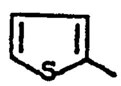 and 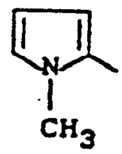 ;

$R^3$ represents hydrogen or represents straight-chain or branched alkyl having 1 to 4 carbon atoms which is optionally monosubstituted or disubstituted by identical or different substituents, suitable substituents being the following : fluorine, chlorine, bromine, iodine, cyano, alkoxy, alkylthio, alkylsulphinyl and alkylsulphonyl each having 1 or 2 carbon atoms, alkylcarbonyloxy having 1 to 4 carbon atoms in the straight-chain or branched alkyl part, formamido, alkylcarbonylamino having 1 or 2 carbon atoms in the alkyl part, which is optionally substituted by fluorine, benzoylamino which is optionally substituted by chlorine, alkoxycarbonylamino having 1 to 4 carbon atoms in the straight-chain or branched alkoxy part, —$COOR^1$, phenyl, cyclopropyl, cyclopentyl or cyclohexyl which is optionally monosubstituted, disubstituted or tri-substituted by identical or different substituents from the series comprising halogen and methyl, and the following heterocyclic rings :

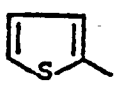 , 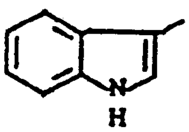 , 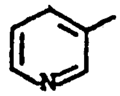 , 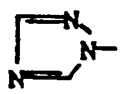 , 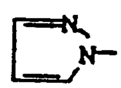 ,

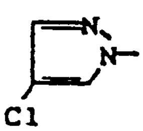 , 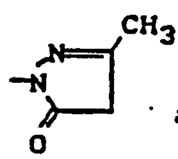 and 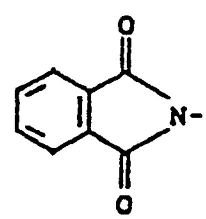 ;

$R^3$ furthermore represents vinyl or ethinyl, each of which is optionally monosubstituted or disubstituted by identical or different substituents from the series comprising phenyl which may optionally be monosubstituted, disubstituted or trisubstituted by identical or different substituents from the series comprising halogen and methyl, or by methyl ;

$R^3$ additionally represents cyclopropyl, cyclopentyl, cyclohexyl, cyclopentenyl or cyclohexenyl, each of which is optionally bridged by methylene or ethylene and/or fused to 1 or 2 benzene, cyclopentane or cyclohexane rings and each of which is optionally monosubstituted, disubstituted or trisubstituted by identical or different substituents, substituents which may be mentioned being : halogen, methyl, the oxo group, phenyl, hydroxycarbonyl, methoxycarbonyl and ethoxycarbonyl ; the following rings may be mentioned individually :

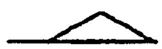 , 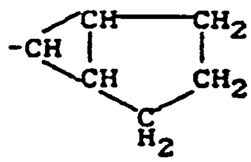 , 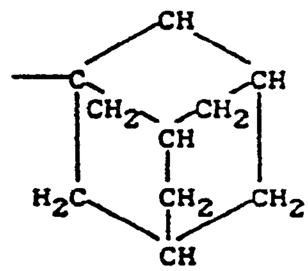 ,

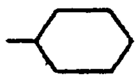 , 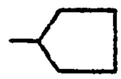 , 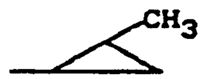 , 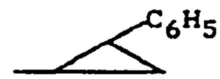 ,

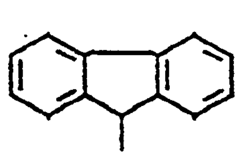 , 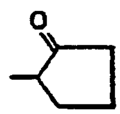 , 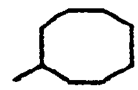 ,

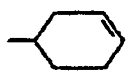 , 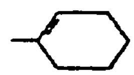 ;

$R^3$ in addition represents phenyl which is optionally fused to 1 or 2 benzene, cyclopentane or cyclohexane rings and is optionally monosubstituted, disubstituted or trisubstituted by identical or different substituents, substituents which may be mentioned being : fluorine, chlorine, hydroxyl, nitro, methyl, methoxy, acetoxy, acetamido, hydroxycarbonyl, methoxycarbonyl and ethoxycarbonyl ;

$R^3$ furthermore represents 5- or 6-membered heterocyclyl having 1 to 3 identical or different hetero atoms, which is optionally fused to 1 or 2 benzene, cyclopentane or cyclohexane rings and is optionally monosubstituted, disubstituted or trisubstituted by identical or different substituents, substituents which may be mentioned being : acyl having 2 to 5 carbon atoms, chlorine, bromine, methyl, ethyl, phenyl, the oxo group and hydroxyl; the following heterocyclic rings may be mentioned individually :

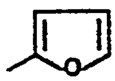 , 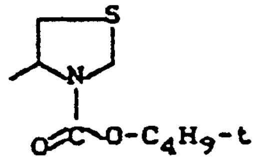 , 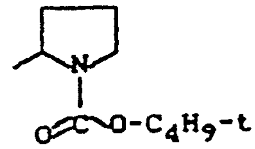 ,

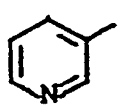 , 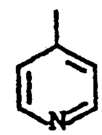 , 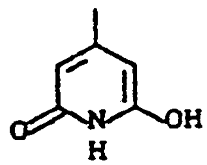 , 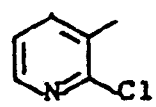 ,

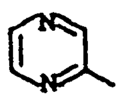 , 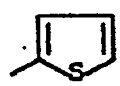 , 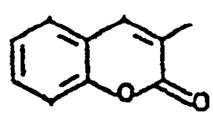 , 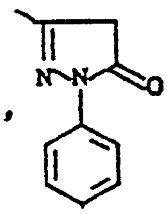 ,

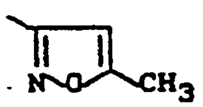 ;

Finally, $R^3$ alternatively represents alkoxycarbonyl having 1 to 4 carbon atoms in the straight-chain or branched alkoxy part, or for $—OR^4$, $—SR^4$ or $—NR^5R^6$ ;

$R^4$ represents straight-chain or branched alkyl having 1 or 2 carbon atoms which is optionally monosubstituted or disubstituted by identical or different substituents, suitable substituents being the following : fluorine, chlorine, methoxy, ethoxy, methylthio, alkoxycarbonyl having 1 to 4 carbon atoms in the straight-chain or branched alkyl part, carbamoyl, methylcarbamoyl, dimethylcarbamoyl, cyano, dimethylamino, diethylamino, acetyl, pivaloyl, or phenyl or phenoxy, each of which is optionally monosubstituted, disubstituted or trisubstituted by identical or different substituents from the series comprising halogen and methyl, and cyclopropyl or cyclohexyl, each of which is optionally monosubstituted, disubstituted or trisubstituted by methyl ;

$R^4$ furthermore represents 3- to 6-membered heterocyclyl having 1 to 3 identical or different hetero atoms which is optionally monosubstituted, disubstituted or trisubstituted by identical or different substituents from the series comprising halogen, methyl and ethyl ; the following heterocyclic rings may be mentioned individually :

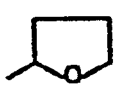 , 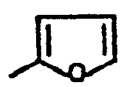 , 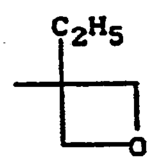 , 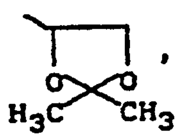 , 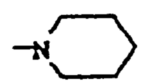 ,

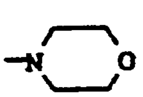 , 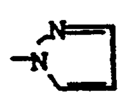 , 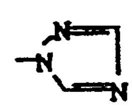 ;

$R^4$ in addition represents allyl or propargyl, each of which is optionally monosubstituted or disubstituted by methyl, or represents cyclohexyl which is optionally monosubstituted, disubstituted or trisubstituted by methyl;

$R^5$ represents hydrogen, straight-chain or branched alkyl having 1 to 4 carbon atoms, or alkyl having 1 to 3 carbon atoms which is monosubstituted or disubstituted by identical or different substituents, suitable substituents being : chlorine, hydroxyl, alkylcarbonyloxy having 1 to 4 carbon atoms in the straight-chain or branched alkyl part, alkylcarbonyl having 1 to 4 carbon atoms in the straight-chain or branched alkyl part, alkoxy and alkylthio each having 1 or 2 carbon atoms, alkoxycarbonyl having 1 to 4 carbon atoms in the straight-chain or branched alkoxy part, carbamoyl, methylcarbamoyl, dimethylcarbamoyl, cyano, dimethylamino, diethylamino, phenyl, 2-furyl, 2-pyridyl, 1-morpholino, cyclopropyl or cyclohexyl which is optionally monosubstituted, disubstituted or trisubstituted by identical or different substituents from the series comprising chlorine and methyl ;

$R^5$ furthermore represents allyl or propargyl, represents 1-cyclohexenyl, or represents cyclohexyl which is monosubstituted, disubstituted or trisubstituted by identical or different substituents, suitable substituents being : chlorine, methyl, ethyl, methoxy, cyano, amino, alkylamino and dialkylamino each having 1 or 2 carbon atoms in each alkyl part, carbamoyl, alkylcarbamoyl and dialkylcarbamoyl each having 1 or 2 carbon atoms in each alkyl part, cyclohexyl, cyclohexylalkyl having 1 or 2 carbon atoms in the alkyl part, or 1-pyrrolidin-2-one;

$R^5$ in addition represents phenyl which is optionally monosubstituted, disubstituted or trisubstituted by identical or different substituents from the series comprising chlorine and methyl, for 2-pyridyl or 2-pyrmidinyl, each of which is optionally monosubstituted, disubstituted or trisubstituted by methyl, for 2-thiazolyl which is optionally substituted by phenyl, for 2-benzothiazolyl which is optionally monosubstituted, disubstituted or trisubstituted by identical or different substituents from the series comprising chlorine, methyl and methoxy, for 1,2,4-triazol-3-yl, for 1,2,4-thiadiazol-5-yl which is optionally substituted by phenyl, for 1,3,4-thiadiazol-5-yl which is optionally monosubstituted, disubstituted or trisubstituted by identical or different substituents from the series comprising mercapto, straight-chain or branched alkyl having 1 to 4 carbon atoms, and in each case straight-chain or branched alkylthio, alkylsulphinyl and alkylsulphonyl each having 1 to 4 carbon atoms, or represents the

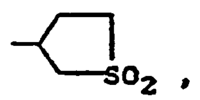

group,

$R^6$ represents hydrogen, straight-chain or branched alkyl having 1 to 4 carbon atoms, or alkyl having 1 to 3 carbon atoms which is monosubstituted or disubstituted by identical or different substituents, substituents which may be mentioned being : chlorine, hydroxyl, alkylcarbonyloxy having 1 to 4 carbon atoms in the straight-chain or branched alkyl part, alkylcarbonyl having 1 to 4 carbon atoms in the straight-chain or branched alkyl part, alkoxy and alkylthio each having 1 or 2 carbon atoms, alkoxycarbonyl having 1 to 4 carbon atoms in the straight-chain or branched alkoxy part, carbamoyl, methylcarbamoyl, dimethylcarbamoyl, cyano, dimethylamino, diethylamino, or phenyl, 2-furyl, 2-pyridyl, 1-morpholino, cyclopropyl and cyclohexyl which is optionally monosubstituted, disubstituted or trisubstituted by identical or different substituents from the series comprising chlorine and methyl ;

$R^6$ furthermore represents allyl or propargyl, represents 1-cyclohexenyl, or represents cyclohexyl which is monosubstituted, disubstituted or trisubstituted by identical or different substituents, substituents which may be mentioned being : chlorine, methyl, ethyl, methoxy, cyano, amino, alkylamino or dialkylamino each having 1 or 2 carbon atoms in each alkyl part, carbamoyl, alkylcarbamoyl or dialkylcarbamoyl each having 1 or 2 carbon atoms in each alkyl part, cyclohexyl, cyclohexylalkyl having 1 or 2 carbon atoms in the alkyl part, or 1-pyrrolidin-2-one ;

$R^6$ in addition represents phenyl which is optionally monosubstituted, disubstituted or trisubstituted by identical or different substituents from the series comprising chlorine and methyl, represents 2-pyridyl or 2-pyrimidinyl, each of which is optionally monosubstituted, disubstituted or trisubstituted by methyl, for 2-thiazolyl which is optionally substituted by phenyl, for 2-benzothiazolyl which is optionally monosubstituted, disubstituted or trisubstituted by identical or different substituents from the series comprising chlorine, methyl and methoxy, for 1,2,4-triazol-3-yl, for 1,2,4-thiadiazol-5-yl which is optionally substituted by phenyl, for 1,3,4-thiadiazol-5-yl which is optionally monosubstituted, disubstituted or trisubstituted by identical or different substituents from the series comprising mercapto, straight-chain or branched alkyl having 1 to 4 carbon atoms, and in each case straight-chain or branched alkylthio, alkylsulphinyl and alkylsulphonyl each having 1 to 4 carbon atoms, or represents the

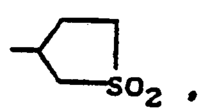

group,

$R^6$ additionally represents hydroxyl, alkoxy having 1 or 2 carbon atoms, represents benzyloxy which is optionally monosubstituted, disubstituted or trisubstituted by identical or different substituents from the series comprising chlorine and methyl, for 1-adamantyl, 2-norbornyl, 1- or 2-decalyl or 1- or 2-tetralyl ;

or

$R^5$ and $R^6$, together with the nitrogen atom to which they are bonded, represent the following monocyclic, bicyclic or tricyclic heterocyclic rings or spiroheterocyclic rings :

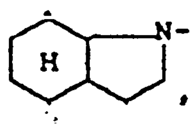 , 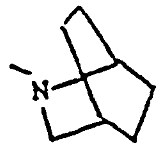 , 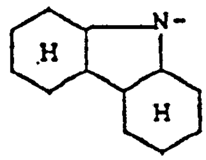 ,

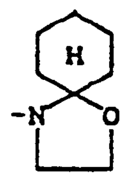 , 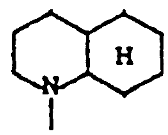 ,

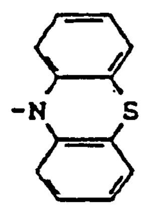 , 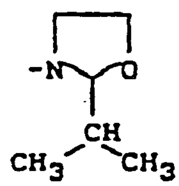 , 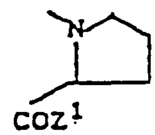

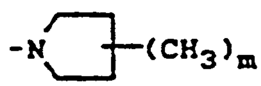 , 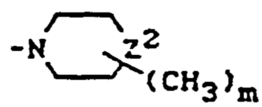

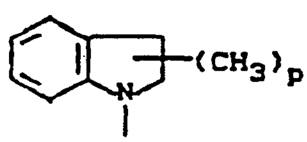 , 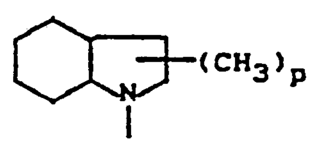 .

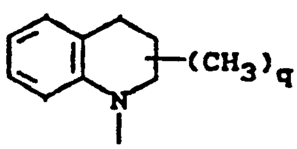 , 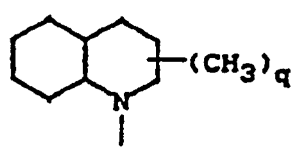 ,

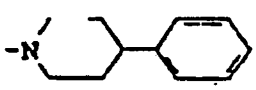 , 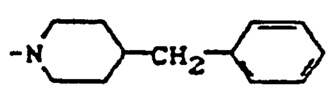 ,

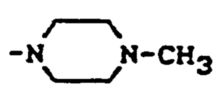 , 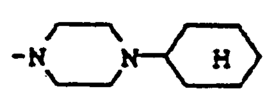 , 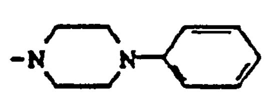 ,

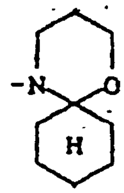, 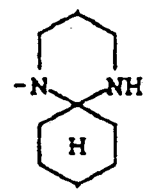, 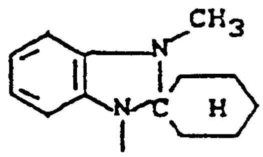,

where

$Z^1$ represents hydroxyl, methoxy, ethoxy, amino, methylamino, ethylamino, dimethylamino or ethylmethylamino,

$Z^2$ represents oxygen or the $CH_2$ group,

m represents the numbers 0, 1, 2, 3 or 4,

p represents the numbers 0, 1 or 2,

q represents the numbers 0, 1, 2 or 3

$R^I$ represents methyl, ethyl, n- or i-propyl and n-, i-, s- or t-butyl,

$R^{II}$ and $R^{III}$ are identical or different and represent hydrogen, methyl, ethyl, n- or i-propyl, represent benzyl which is optionally monosubstituted, disubstituted or trisubstituted by identical or different substituents, with chlorine and methyl as substituents, represent alkoxycarbonylalkyl having 1 or 2 carbon atoms in each alkyl part, represent carbamoylalkyl, alkylcarbamoylalkyl or dialkylcarbamoylalkyl each having 1 or 2 carbon atoms in each alkyl part, or represent cyclohexyl which is optionally monosubstituted, disubstituted or trisubstituted by methyl,

$R^{IV}$ represents hydrogen, alkyl having 1 or 2 carbon atoms, represents benzyl which is optionally monosubstituted, disubstituted or trisubstituted by identical or different substituents, phenyl substituents which may be mentioned being fluorine, chlorine and methyl ; and

X represents oxygen or sulphur.

4. Pesticides, characterised in that they contain at least one N,N'-diacylaminal of the formula (I) according to Claim 1.

5. Use of N,N'-diacylaminals of the formula (I) according to Claim 1 for combating pests.

6. Method of combating pests, characterised in that N,N'-diacylaminals of the formula (I) according to Claim 1 are allowed to act on the pests and/or their environment.

7. Process for the production of pesticides, characterised in that N,N'-diacylaminals of the formula (I) according to Claim 1 are mixed with extenders and/or surface-active agents.

8. Process for the preparation of N-(2-cyano-2-oximinoacetyl)-aminals of the general formula (II)

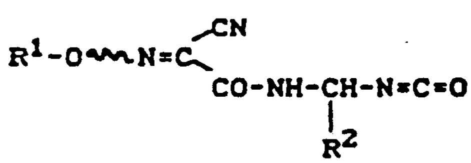

(II)

in which

$R^1$, $R^2$ and HY have the meaning given in Claim 1, characterised in that isocyanates of the formula (IV)

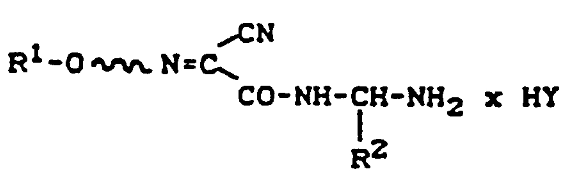

(IV)

in which

$R^1$ and $R^2$ have the abovementioned meaning, are hydrolyzed in a customary manner, and the resultant amine is isolated as a salt in a customary manner.

9. Process for the preparation of isocyanates of the formula (IV)

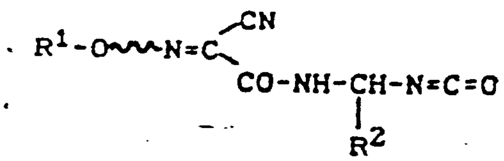

(IV)

in which

$R^1$ and $R^2$ have the meaning given in Claim 1, characterised in that

α) amides of the general formula (VI)

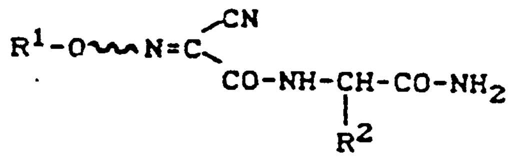

(VI)

in which

$R^1$ and $R^2$ have the abovementioned meaning, are oxidized in a customary manner, if appropriate in the presence of a diluent,

or

β) azides of the general formula (VII)

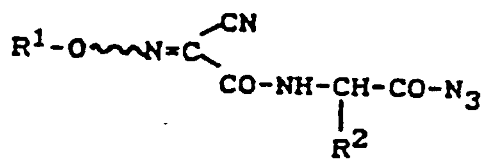

(VII)

in which

$R^1$ and $R^2$ have the abovementioned meaning, are warmed in the presence of a diluent.

**Revendications**

**Revendications pour les Etats contractants suivants : BE, CH, DE, FR, GB, IT, LI, NL**

1. N,N'-diacylaminals de formule générale (I)

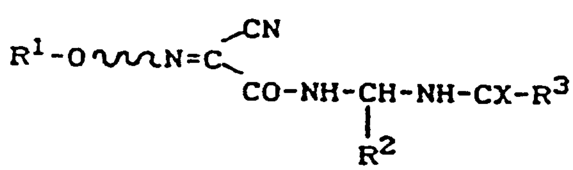

(I)

dans laquelle

$R^1$ représente un groupe alkyle à chaîne droite ou ramifiée ayant 1 à 6 atomes de carbone portant éventuellement un à cinq substituants identiques ou différents, parmi lesquels on considère les substituants suivants : fluor, chlore, brome, iode, radical cyano, —$COOR^I$, —$CONR^{II}R^{III}$, —$OR^{IV}$, acyle ayant 2 à 9 atomes de carbone, phényle portant éventuellement un à cinq substituants identiques ou différents, parmi lesquels on mentionne : un halogène, un radical alkyle et un radical alkoxy ayant chacun 1 à 4 atomes de carbone, un radical halogénalkyle et un radical halogénalkoxy ayant chacun 1 ou 2 atomes de carbone et 1 à 5 atomes d'halogènes identiques ou différents ; d'autres substituants du groupe alkyle sont un radical cycloalkyle de 3 à 6 atomes de carbone portant éventuellement un à cinq substituants alkyle en $C_1$ à $C_4$ identiques ou différents, un radical hétérocyclyle ayant 1 à 3 hétéro-atomes identiques ou différents, portant éventuellement un à cinq substituants identiques ou différents halogéno et alkyle ayant 1 à 4 atomes de carbone ; $R^1$ représente en outre un groupe alcényle ou un groupe alcynyle de 2 à 6 atomes de carbone à chaîne droite ou à chaîne ramifiée portant éventuellement un à trois substituants identiques ou différents, et on mentionne comme substituants, un radical phényle portant éventuellement un à cinq substituants halogéno et alkyle en $C_1$ à $C_4$ identiques ou

différents ; $R^1$ représente en outre un groupe cycloalkyle de 3 à 6 atomes de carbone ou un groupe cycloalcényle de 5 à 7 atomes de carbone portant chacun éventuellement 1 à 5 substituants alkyle en $C_1$ à $C_4$ identiques ou différents ;

$R^2$ représente l'hydrogène ou un groupe alkyle ayant 1 à 6 atomes de carbone à chaîne droite ou à chaîne ramifiée, portant éventuellement un à cinq substituants identiques ou différents, parmi lesquels on mentionne les substituants suivants : cyano, alkoxy et alkylthio ayant chacun 1 à 4 atomes de carbone, —$COOR^I$, acylamino ayant 2 à 9 atomes de carbone, phényle portant éventuellement un à cinq substituants identiques ou différents, et on considère alors comme substituants les substituants du groupe phényle déjà mentionnés pour $R^1$ ; d'autres substituants du groupe alkyle sont un radical cycloalkyle de 3 à 6 atomes de carbone portant éventuellement un à cinq substituants alkyle en $C_1$ à $C_4$ identiques ou différents, un radical hétérocyclyle de 3 à 6 chaînons ayant 1 à 3 hétéro-atomes identiques ou différents, portant éventuellement un à cinq substituants halogéno et alkyle en $C_1$ à $C_4$ identiques ou différents ; $R^2$ représente en outre un groupe alcényle ou un groupe alcynyle de 2 à 6 atomes de carbone à chaîne droite ou ramifiée, portant éventuellement chacun un à trois substituants identiques ou différents, et on mentionne comme substituants : un radical phényle portant éventuellement un à cinq substituants halogéno et alkyle en $C_1$ à $C_4$ identiques ou différents ; $R^2$ représente en outre un groupe cycloalkyle ayant 3 à 6 atomes de carbone ou un groupe cycloalcényle ayant 5 à 7 atomes de carbone portant chacun éventuellement un à cinq substituants alkyle en $C_1$ à $C_4$ identiques ou différents ; $R^2$ représente en outre un groupe phényle portant éventuellement un à cinq substituants identiques ou différents, et on considère comme substituants les substituants du groupe phényle déjà mentionnés pour $R^1$ ; $R^2$ représente en outre un groupe hétérocyclyle de 3 à 6 chaînons ayant 1 à 3 hétéro-atomes identiques ou différents, portant éventuellement un à cinq substituants halogéno et alkyle en $C_1$ à $C_4$ identiques ou différents ;

$R^3$ représente l'hydrogène ou un groupe alkyle de 1 à 6 atomes de carbone à chaîne droite ou ramifiée, portant éventuellement un à cinq substituants identiques ou différents, parmi lesquels on considère les substituants suivants : fluor, chlore, brome, iode, radical cyano, alkoxy, alkylthio, alkylsulfinyle et alkylsulfonyle ayant chacun 1 à 4 atomes de carbone, acyloxy et acylamino ayant chacun 2 à 9 atomes de carbone, —$COOR^I$, phényle portant éventuellement un à cinq substituants identiques ou différents, et on considère alors comme substituants les substituants du groupe phényle déjà mentionnés pour $R^1$ ; d'autres substituants du groupe alkyle sont un radical cycloalkyle de 3 à 6 atomes de carbone portant éventuellement un à cinq substituants alkyle en $C_1$ à $C_4$ identiques ou différents ainsi que, le cas échéant, un radical hétérocyclyle de 3 à 6 chaînons ayant 1 à 3 hétéro-atomes identiques ou différents, portant éventuellement un à cinq substituants halogéno et alkyle en $C_1$ à $C_4$ identiques ou différents ; $R^3$ représente en outre un groupe alcényle ou alcynyle ayant chacun 2 à 6 atomes de carbone, à chaîne droite ou ramifiée, portant chacun éventuellement un à trois substituants identiques ou différents, et on mentionne alors comme substituants : un radical phényle portant éventuellement un à cinq substituants halogéno et alkyle en $C_1$ à $C_4$ identiques ou différents ; $R^3$ représente en outre un groupe cycloalkyle ayant 3 à 6 atomes de carbone ou un groupe cycloalcényle ayant 5 à 7 atomes de carbone présentant chacun le cas échéant un pont méthylène ou éthylène et/ou condensé chacun avec 1 ou 2 noyaux de benzène, de cyclopentane ou de cyclohexane, et portant chacun le cas échéant un à cinq substituants identiques ou différents, parmi lesquels on considère : un halogène, un radical alkyle et un radical alkoxy ayant chacun 1 à 4 atomes de carbone, un radical acyloxy et un radical acylamino ayant chacun 2 à 5 atomes de carbone, le groupe oxo, un radical phényle, hydroxycarbonyle et alkoxycarbonyle ayant 1 à 4 atomes de carbone dans la partie alkoxy ; $R^3$ représente en outre un groupe phényle condensé le cas échéant avec 1 ou 2 noyaux de benzène ou de cyclohexane, portant éventuellement un à cinq substituants identiques ou différents, parmi lesquels on considère : un halogène, un radical hydroxy, nitro, alkyle et alkoxy ayant chacun 1 à 4 atomes de carbone, halogénalkyle et halogénalkoxy ayant chacun 1 ou 2 atomes de carbone et 2 à 5 atomes d'halogènes identiques ou différents, un radical acylamino et un radical acylalkylamino ayant chacun 2 à 5 atomes de carbone dans la partie acyle et 1 à 4 atomes de carbone dans la partie alkyle, un radical hydroxycarbonyle et un radical alkoxycarbonyle ayant 1 à 4 atomes de carbone dans la partie alkoxy ; $R^3$ représente en outre un groupe hétérocyclyle de 5 ou 6 chaînons avec 1 à 3 hétéro-atomes identiques ou différents, éventuellement condensé avec 1 ou 2 noyaux de benzène, de cyclopentane ou de cyclohexane et portant éventuellement un à cinq substituants identiques ou différents, parmi lesquels on mentionne : un halogène, un radical alkyle ayant 1 à 4 atomes de carbone, acyle ayant 2 à 9 atomes de carbone, phényle, le groupe oxo et un radical hydroxy ; $R^3$ représente enfin également un groupe alkoxycarbonyle ayant 1 à 4 atomes de carbone dans la partie alkoxy, un groupe —$OR^4$, —$SR^4$ ou —$NR^5R^6$,

$R^4$ représente un groupe alkyle en $C_1$ à $C_6$ à chaîne droite ou ramifiée portant éventuellement un à cinq substituants identiques ou différents, parmi lesquels on considère : un halogène, un radical —$OR^{IV}$, —$SR^{IV}$, —$COOR^I$, —$CONR^{II}R^{III}$, CN, —$NR^{II}R^{III}$, un radical acyle ayant 2 à 9 atomes de carbone, un radical phényle ou un radical phénoxy portant chacun éventuellement un à cinq substituants halogéno et alkyle en $C_1$ à $C_4$ identiques ou différents, un radical cycloalkyle ayant 3 à 6 atomes de carbone ou un radical cycloalcényle ayant 5

à 7 atomes de carbone portant chacun le cas échéant un à cinq substituants alkyle en $C_1$ à $C_4$ identiques ou différents, un radical hétérocyclyle de 3 à 6 chaînons avec 1 à 3 hétéro-atomes identiques ou différents, portant le cas échéant un à cinq substituants halogéno et alkyle en $C_1$ à $C_4$ identiques ou différents ; $R^4$ représente en outre un groupe alcényle ou un groupe alcynyle ayant chacun 2 à 6 atomes de carbone, un groupe cycloalkyle ayant 3 à 6 atomes de carbone ou un groupe cycloalcényle ayant 5 à 7 atomes de carbone portant chacun éventuellement un à cinq substituants alkyle en $C_1$ à $C_4$ identiques ou différents ;

$R^5$ est l'hydrogène ou un groupe alkyle en $C_1$ à $C_4$ à chaîne droite ou ramifiée portant éventuellement un ou deux substituants identiques ou différents, et on mentionne alors comme substituants : un halogène, un radical cyano, —$COOR^I$, —$CONR^{II}R^{III}$, —$NR^{II}R^{III}$, —$OR^{IV}$, —$S(O)_nR^{IV}$, un radical acyle de 2 à 9 atomes de carbone, un radical phényle portant éventuellement un à cinq substituants halogéno et alkyle en $C_1$ à $C_4$ identiques ou différents, un hétérocycle pentagonal ou hexagonal avec 1 à 3 hétéro-atomes identiques ou différents, portant le cas échéant un à cinq substituants identiques ou différents, halogéno, alkyle et alkoxy ayant chacun 1 à 4 atomes de carbone, un groupe cycloalkyle de 3 à 6 atomes de carbone et un groupe cycloacényle de 5 à 7 atomes de carbone portant chacun le cas échéant un à cinq substituants alkyle en $C_1$ à $C_4$ identiques ou différents ; $R^5$ représente en outre un groupe alcényle ou un groupe alcynyle à chaîne droite ou ramifiée ayant chacun 2 à 6 atomes de carbone ; un groupe cycloalcényle de 5 à 7 atomes de carbone portant éventuellement un à cinq substituants alkyle en $C_1$ à $C_4$ identiques ou différents ou un groupe cycloalkyle de 3 à 6 atomes de carbone portant éventuellement un à cinq substituants identiques ou différents, parmi lesquels on mentionne: un halogène, un radical alkyle et un radical alkoxy ayant chacun 1 à 4 atomes de carbone, un radical cyano, amino, carbamoyle, alkylamino, dialkylamino, alkylcarbamoyle et dialkylcarbamoyle ayant chacun 1 à 4 atomes de carbone dans chaque partie alkyle, un radical alkoxy-carbonylamino ayant 1 à 4 atomes de carbone dans la partie alkoxy, un radical cycloalkyle et un radical cycloalkylalkyle ayant 5 ou 6 atomes de carbone dans la partie cycloalkyle et 1 à 4 atomes de carbone dans la partie alkyle à chaîne droite ou ramifiée, un radical phényle ou pyrrolidone portant chacun un à cinq substituants halogéno et alkyle en $C_1$ à $C_4$ identiques ou différents ; $R^5$ représente en outre un groupe phényle portant éventuellement un à cinq substituants identiques ou différents halogéno, alkyle ou alkoxy ayant chacun 1 à 4 atomes de carbone, halogénalkyle ou halogénalkoxy ayant chacun 1 ou 2 atomes de carbone et 1 à 5 atomes d'halogènes identiques ou différents ou un hétérocycle pentagonal ou hexagonal avec 1 à 3 hétéro-atomes identiques ou différents, portant éventuellement un à cinq substituants identiques ou différents, parmi lesquels on mentionne : un halogène, un radical mercapto, phényle, alkyle à chaîne droite ou ramifiée, alkoxy, alkylthio, alkylsulfinyle et alkylsulfonyle ayant 1 à 4 atomes de carbone par partie alkyle ;

$R^6$ a les définitions données pour $R^5$ ou représente le groupement —$OR^{IV}$ ;

ou bien

$R^5$ et $R^6$ forment conjointement avec l'atome d'azote auquel ils sont liés un hétérocycle ou un spirohétérocycle monocyclique, bicyclique ou tricyclique ayant un à trois autres hétéro-atomes identiques ou différents et portant le cas échéant un à cinq substituants identiques ou différents, parmi lesquels on mentionne :

un radical alkyle ou alkoxy à chaîne droite ou ramifiée ayant chacun 1 à 4 atomes de carbone, un radical cycloalkyle ayant 3 à 6 atomes de carbone, le groupe hydroxy ou le groupe oxo, un radical alcényle à chaîne droite ou ramifiée ayant 2 à 4 atomes de carbone, un radical hydroxycarbonyle, alkoxycarbonyle ayant 1 à 4 atomes de carbone dans la partie alkyle à chaîne droite ou ramifiée, un radical carbamoyle, alkyl- ou dialkylcarbamoyle ayant chacun 1 à 4 atomes de carbone dans la partie alkyle à chaîne droite ou ramifiée, un radical phényle ou phénylalkyle ayant 1 à 4 atomes de carbone dans la partie alkyle à chaîne droite ou ramifiée, portant chacun éventuellement un ou deux substituants, identiques ou différents, halogéno ou alkyle ou alkoxy à chaîne droite ou à chaîne ramifiée ayant chacun 1 à 4 atomes de carbone ;

$R^I$ est l'hydrogène ou un groupe alkyle à chaîne droite ou ramifiée ayant 1 à 6 atomes de carbone,

$R^{II}$ représente l'hydrogène, un groupe alkyle à chaîne droite ou ramifiée ayant 1 à 6 atomes de carbone, un groupe phénylalkyle ayant 1 à 4 atomes de carbone dans la partie alkyle à chaîne droite ou ramifiée, portant éventuellement un à cinq substituants identiques ou différents, et on mentionne comme substituants du groupe phényle : un halogène, un radical alkyle ou alkoxy ayant chacun 1 à 4 atomes de carbone, halogénalkyle ou halogénalkoxy ayant chacun 1 ou 2 atomes de carbone et 2 à 5 atomes d'halogènes identiques ou différents; $R^{II}$ représente en outre un groupe alkoxycarbonylalkyle, carbamoylalkyle, alkylcarbamoylalkyle ou dialkylcarbamoylalkyle ayant chacun 1 à 4 atomes de carbone dans chaque partie alkyle ou un groupe cycloalkyle de 3 à 6 atomes de carbone portant éventuellement un substituant alkyle en $C_1$ à $C_4$ à chaîne droite ou ramifiée,

$R^{III}$ représente l'hydrogène, un groupe alkyle à chaîne droite ou ramifiée ayant 1 à 6 atomes de carbone, un groupe phénylalkyle ayant 1 à 4 atomes de carbone dans la partie alkyle à chaîne droite ou ramifiée portant éventuellement un à cinq substituants identiques ou différents, et on considère comme substituants du groupe phényle les substituants du groupe phényle mentionnés dans le cas de $R^{II}$ ; $R^{III}$ représente en outre un groupe alkoxycarbonylalkyle, carbamoylalkyle, alkylcarbamoylalkyle ou dialkylcarbamoylalkyle ayant chacun 1 à 4 atomes de carbone dans chaque partie alkyle ou un groupe cycloalkyle de 3 à 6 atomes de carbone portant éventuellement un substituant alkyle en $C_1$ à $C_4$ à chaîne droite ou à chaîne ramifiée ;

$R^{IV}$ représente l'hydrogène, un groupe alkyle à chaîne droite ou ramifiée ayant 1 à 6 atomes de carbone, un groupe phénylalkyle ayant 1 à 4 atomes de carbone dans la partie alkyle à chaîne droite ou ramifiée, portant éventuellement un à cinq substituants identiques ou différents, et on considère comme substituants du groupe phényle les substituants du groupe phényle mentionnés pour $R^{II}$ ; $R^{IV}$ représente en outre un groupe acyle ayant 2 à 9 atomes de carbone ;

n représente les nombres 0, 1 ou 2 et

X représente l'oxygène ou le soufre.

2. N,N'-diacylaminals suivant la revendication 1, dans la formule (I) desquels :

$R^1$ est un groupe alkyle ayant 1 à 4 atomes de carbone à chaîne droite ou ramifiée portant éventuellement un à trois substituants identiques ou différents, parmi lesquels on considère les substituants suivants : fluor, chlore, brome, iode, radical cyano, —$COOR^I$, —$CONR^{II}R^{III}$, —$OR^{IV}$, acyle ayant 2 à 9 atomes de carbone, phényle portant éventuellement un à trois substituants fluoro, chloro, méthyle et méthoxy identiques ou différents, un groupe cyclopropyle ou cyclohexyle portant chacun éventuellement un à trois substituants méthyle, des hétérocycles portant éventuellement 1 à 3 substituants alkyle en $C_1$ ou $C_2$ identiques ou différents, de formules

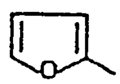 , 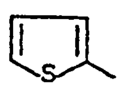 , 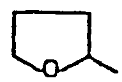 , 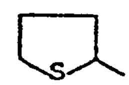 , 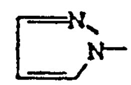

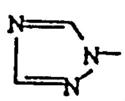 , 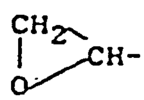 , 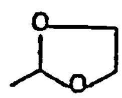 et 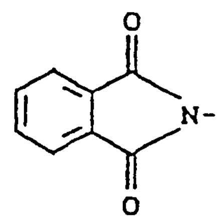 ;

$R^1$ représente en outre un groupe allyle ou propargyle portant éventuellement un ou deux substituants méthyle, un groupe cyclopropyle, cyclohexyle, cyclopentyle ou cyclohexényle portant chacun éventuellement un à trois substituants méthyle ;

$R^2$ est l'hydrogène ou un groupe alkyle ayant 1 à 4 atomes de carbone à chaîne droite ou ramifiée, portant éventuellement un à trois substituants identiques ou différents, parmi lesquels on considère les substituants suivants : cyano, méthoxy, éthoxy, méthylthio, éthylthio, —$COOR^I$, acylamino ayant 2 à 9 atomes de carbone, un groupe phényle portant éventuellement un à trois substituants halogéno, méthyle et méthoxy identiques ou différents, un groupe cyclopropyle ou cyclohexyle portant éventuellement un à trois substituants méthyle, de même que des hétérocycles portant éventuellement un à trois substituants alkyle en $C_1$ ou $C_2$ identiques ou différents, de formules

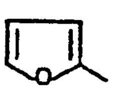 , 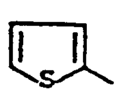 , 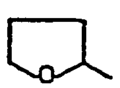 , 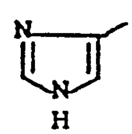 , 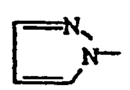 .

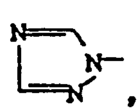 , 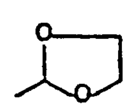 et 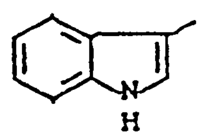 ;

$R^2$ représente en outre un groupe allyle, allényle, vinyle, propargyle ou éthynyle éventuellement substitués chacun par un radical phényle qui peut porter éventuellement un à trois substituants halogéno et méthyle identiques ou différents ; $R^2$ représente en outre un groupe cyclopropyle, cyclohexyle, cyclopentényle ou cyclohexényle ; $R^2$ représente en outre un groupe phényle portant éventuellement un à trois substituants halogéno, méthyle et méthoxy identiques ou différents, des hétérocycles portant éventuellement un à trois substituants alkyle en $C_1$ ou $C_2$ identiques ou différents, de formules :

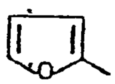 , 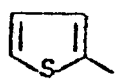 , 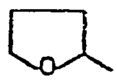 , 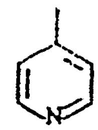 , 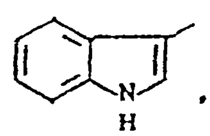 ,

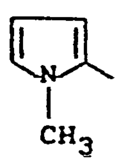 , 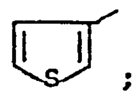 ;

$R^3$ représente l'hydrogène ou un groupe alkyle à chaîne droite ou ramifiée ayant 1 à 4 atomes de carbone, portant éventuellement un ou trois substituants identiques ou différents, parmi lesquels on considère : le fluor, le chlore, le brome, l'iode, un radical cyano, alkoxy, alkylthio, alkylsulfinyle et alkylsulfonyle ayant chacun 1 ou 2 atomes de carbone, acyloxy et acylamino ayant chacun 2 à 9 atomes de carbone, —$COOR^I$, un groupe phényle portant éventuellement un à trois substituants halogéno et méthyle identiques ou différents, un groupe cyclopropyle, cyclopentyle ou cyclohexyle, des hétérocycles portant éventuellement un à trois substituants alkyle en $C_1$ ou $C_2$ identiques ou différents, de formules

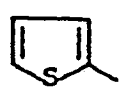 , 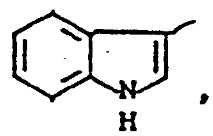 , 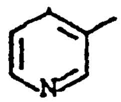 , 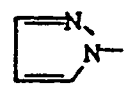 ,

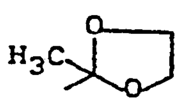 , 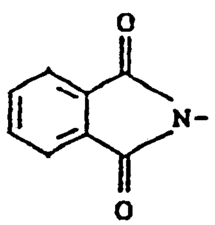 , 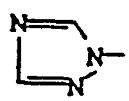 , 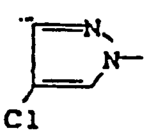 ,

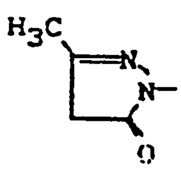 , 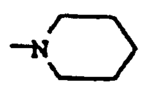 , 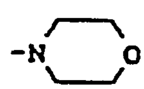 ;

$R^3$ représente en outre un groupe vinyle ou un groupe éthynyle substitué chacun éventuellement par un radical phényle qui peut porter le cas échéant un à trois substituants halogéno et méthyle identiques ou différents ; $R^3$ représente en outre un groupe cyclopropyle, cyclopentyle, cyclohexyle, cyclopenténylе ou cyclohexényle pontés chacun le cas échéant par un groupe méthylène ou éthylène et/ou condensé avec 1 ou 2 noyaux de benzène, de cyclopentane ou de cyclohexane, et portant chacun éventuellement un à trois substituants identiques ou différents, parmi lesquels on mentionne : un halogène, un radical méthyle, méthoxy, acyloxy et acylamino ayant chacun 2 à 5 atomes de carbone, le groupe oxo, un radical phényle, hydroxycarbonyle, méthoxy- et éthoxycarbonyle ; $R^3$ représente en outre un groupe phényle éventuellement condensé avec 1 ou 2 noyaux de benzène ou de cyclohexane, portant éventuellement un à trois substituants identiques ou différents, parmi lesquels on mentionne : le fluor, le chlore, un radical hydroxy, nitro, méthyle, méthoxy, acylamino et N-alkylacylamino avec dans chaque cas 2 à 5 atomes de carbone dans la partie acyle et 1 ou 2 atomes de carbone dans la partie alkyle, un groupe hydroxycarbonyle, méthoxy- et éthoxycarbonyle ; $R^3$ représente en outre un groupe hétérocyclyle pentagonal ou hexagonal avec 1 à 3 hétéro-atomes identiques ou différents, condensé le cas échéant avec 1 ou 2 noyaux de benzène ou de cyclohexane et portant éventuellement un à trois substituants identiques ou différents, parmi lesquels on mentionne : un radical acyle ayant 2 à 5 atomes de carbone, le chlore, le brome, un radical méthyle, éthyle, phényle, oxo et hydroxy ; $R^3$ représente enfin également un groupe alkoxycarbonyle ayant 1 à 4 atomes de carbone dans la partie alkoxy, un groupe —$OR^4$, —$SR^4$ ou —$NR^5R^6$ ;

$R^4$ est un groupe alkyle à chaîne droite ou ramifiée ayant 1 à 4 atomes de carbone, portant éventuellement un à trois substituants identiques ou différents, et on considère alors les substituants suivants : fluor, chlore, radical —$OR^{IV}$, —$SR^{IV}$, —$COOR^I$, —$CONR^{II}R^{III}$, CN, $NR^{II}R^{III}$, alkylcarbonyle ayant 1 à 4 atomes de carbone dans la partie alkyle à chaîne droite ou ramifiée, un groupe phényle ou phénoxy portant chacun le cas échéant un à trois substituants halogéno et méthyle identiques ou différents, un groupe cyclopropyle, cyclohexyle, cyclopentyle ou cyclohexényle portant chacun le cas échéant un à trois substituants méthyle, un groupe hétérocyclyle de 3 à 6 chaînons ayant 1 à 3 hétéro-atomes identiques ou différents tels que des atomes d'azote, d'oxygène et de soufre, portant éventuellement un à trois substituants halogéno, méthyle et éthyle identiques ou différents ; $R^4$ représente en outre un groupe allyle ou propargyle portant éventuellement un ou deux substituants méthyle ainsi qu'un groupe cyclopropyle, cyclohexyle, cyclopentyle ou cyclohexényle portant éventuellement un à trois substituants méthyle ;

$R^5$ représente l'hydrogène ou un groupe alkyle en $C_1$ à $C_4$ à chaîne droite ou à chaîne ramifiée portant éventuellement un ou deux substituants identiques ou différents, parmi lesquels on mentionne de préférence : un halogène, un radical cyano, —$COOR^I$, —$CONR^{II}R^{III}$, $NR^{II}R^{III}$, —$OR^{IV}$, —$S(O)_nR^{IV}$, acyle ayant 2 à 9 atomes de carbone, un groupe phényle portant éventuellement un à trois substituants halogéno et alkyle en $C_1$ à $C_4$ identiques ou différents, un hétérocycle pentagonal ou hexagonal avec 1 à 3 hétéro-atomes identiques ou différents, portant le cas échéant un à trois substituants identiques ou différents halogéno, alkyle et alkoxy à chaîne droite ou ramifiée ayant chacun 1 à 4 atomes de carbone et un groupe cycloalkyle ayant 3 à 6 atomes de carbone ou cycloalcényle ayant 5 à 7 atomes de carbone portant chacun le cas échéant un substituant alkyle à chaîne droite ou ramifiée ayant 1 à 4 atomes de carbone ; $R^5$ représente en outre un groupe alcényle ou alcynyle à chaîne droite ou ramifiée ayant chacun 2 à 6 atomes de carbone, un groupe cycloalcényle de 5 à 7 atomes de carbone portant éventuellement un substituant alkyle à chaîne droite ou ramifiée ayant 1 à 4 atomes de carbone, un groupe cycloalkyle en $C_3$ à $C_8$ éventuellement substitué, et on mentionne alors comme substituants: un halogène, un groupe alkyle et un groupe alkoxy à chaîne droite ou ramifiée ayant chacun 1 à 4 atomes de carbone, un radical cyano, amino, carbamoyle, un radical alkylamino, dialkylamino, alkylcarbamoyle et dialkylcarbamoyle à chaîne droite ou à chaîne ramifiée ayant chacun 1 à 4 atomes de carbone dans chaque partie alkyle, un radical alkoxycarbonylamino ayant 1 à 4 atomes de carbone dans la partie alkoxy à chaîne droite ou ramifiée, un radical cycloalkyle et cycloalkylalkyle ayant chacun 5 ou 6 atomes de carbone dans la partie cycloalkyle et 1 à 4 atomes de carbone dans la partie alkyle à chaîne droite ou ramifiée, un radical phényle ou pyrrolidone portant éventuellement un à trois substituants identiques ou différents halogéno et alkyle ayant 1 à 4 atomes de carbone ; $R^5$ représente en outre un groupe phényle portant éventuellement un à trois substituants, identiques ou différents, halogéno, alkyle ou alkoxy à chaîne droite ou ramifiée ayant chacun 1 à 4 atomes de carbone, halogénalkyle ou halogénalkoxy ayant chacun 1 ou 2 atomes de carbone et 1 à 5 atomes d'halogènes identiques ou différents, ou un hétérocycle pentagonal ou hexagonal ayant 1 à 3 hétéro-atomes identiques ou différents, portant éventuellement un à trois substituants identiques ou différents, et on mentionne comme substituants des hétérocycles : un halogène, un radical mercapto, phényle, alkyle, alkoxy, alkylthio, alkylsulfinyle et alkylsulfonyle à chaîne droite ou ramifiée ayant 1 à 4 atomes de carbone par partie alkyle ;

$R^6$ a les définitions données pour $R^5$ ou représente le groupement—$OR^{IV}$,

ou bien

$R^5$ et $R^6$ forment conjointement avec l'atome d'azote auquel ils sont liés un hétérocycle ou un spirohétérocycle, monocyclique, bicyclique ou tricyclique portant éventuellement un à cinq substituants identiques ou différents, et on mentionne alors comme hétérocycles : l'oxazolidine, la pyrrolidine, l'imidazolidine, la pipéridine, la pipérazine, la morpholine, la thiomorpholine, le 1,3-oxazane ou le 1,3-diazane ; ces hétérocycles peuvent chacun être condensés le cas échéant avec 1 ou 2 noyaux de benzène ou de cyclohexane ou être pontés le cas échéant avec un radical méthylène ou éthylène, les substituants de tous les systèmes hétéro étant les suivants : un radical alkyle à chaîne droite ou ramifiée ou cycloalkyle ayant 3 à 6 atomes de carbone, le groupe hydroxy ou le groupe oxo, un radical alcényle à chaîne droite ou ramifiée ayant 2 à 4 atomes de carbone, un radical hydroxycarbonyle, alkoxycarbonyle ayant 1 à 4 atomes de carbone dans la partie alkyle à chaîne droite ou ramifiée, carbamoyle, alkyl- ou dialkylcarbamoyle ayant chacun 1 à 4 atomes de carbone dans chaque partie alkyle, en outre un groupe phényle ou phénylalkyle ayant 1 à 4 atomes de carbone dans la partie alkyle à chaîne droite ou ramifiée, portant éventuellement un ou deux substituants, identiques ou différents, halogéno et alkyle à chaîne droite ou ramifiée ayant 1 à 4 atomes de carbone,

$R^I$ est un groupe alkyle à chaîne droite ou ramifiée ayant 1 à 4 atomes de carbone,

$R^{II}$ représente l'hydrogène, un groupe alkyle à chaîne droite ou ramifiée ayant 1 à 4 atomes de carbone, un groupe benzyle ou phénéthyle portant éventuellement un à trois substituants identiques ou différents, et on mentionne alors comme substituants du groupe phényle : le fluor, le chlore, un radical méthyle, méthoxy et trifluorométhyle ; $R^{II}$ représente en outre un groupe alkoxycarbonylalkyle ayant 1 à 4 atomes de carbone dans la partie alkoxy et 1 ou 2 atomes de carbone dans la partie alkyle, un groupe carbamoylalkyle ayant 1 ou 2

atomes de carbone dans la partie alkyle, un groupe alkylcarbamoyle ou dialkylcarbamoylalkyle ayant chacun 1 ou 2 atomes de carbone dans chaque partie alkyle, ou un groupe cycloalkyle de 3 à 6 atomes de carbone portant éventuellement un à trois substituants méthyle et éthyle identiques ou différents,

$R^{III}$ a les définitions données pour $R^{II}$,

$R^{IV}$ représente l'hydrogène, un groupe alkyle à chaîne droite ou ramifiée ayant 1 à 4 atomes de carbone, un groupe benzyle ou phénéthyle portant chacun le cas échéant un à trois substituants identiques ou différents, et on mentionne alors comme substituants du groupe phényle : le fluor, le chlore, un radical méthyle, méthoxy et trifluorométhyle ; $R^{IV}$ représente en outre un groupe acyle ayant 2 à 9 atomes de carbone ;

n représente les nombres 0, 1 ou 2 et

X représente l'oxygène ou le soufre.

3. N,N'-diacylaminals suivant la revendication 1, dans la formule (I) desquels

$R^1$ est un groupe alkyle en $C_1$ ou $C_2$ portant éventuellement un ou deux substituants identiques ou différents, et on considère alors les substituants suivants : fluor, chlore, radical cyano, —$COOR^I$, —$CONR^{II}R^{III}$, —$OR^{IV}$, alkylcarbonyle ayant 1 à 4 atomes de carbone dans la partie alkyle à chaîne droite ou ramifiée, phényle portant éventuellement un à trois substituants fluoro, chloro et méthyle identiques ou différents, cyclopropyle ou cyclohexyle portant éventuellement un à trois substituants méthyle, et les hétérocycles suivants :

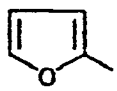 , 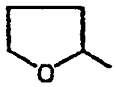 , 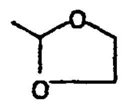 , 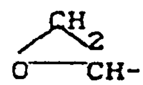

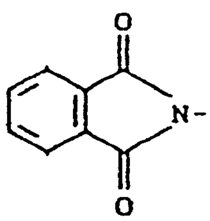 , 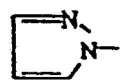 et 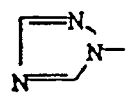 ;

$R^1$ représente en outre un groupe allyle ou propargyle, un groupe cyclopropyle, cyclohexyle, cyclopentényle ou cyclohexényle portant chacun éventuellement un à trois substituants méthyle ;

$R^2$ représente l'hydrogène ou un groupe alkyle en $C_1$ à $C_4$ portant éventuellement un ou deux substituants identiques ou différents, et on considère alors comme substituants : un radical cyano, méthoxy, éthoxy, méthylthio, éthylthio, —$COOR^I$, alkylcarbonylamino ayant 1 à 4 atomes de carbone dans la partie alkyle à chaîne droite ou ramifiée, un groupe phényle portant éventuellement un à trois substituants halogéno, méthyle et méthoxy identiques ou différents, un groupe cyclopropyle ou cyclohexyle portant chacun le cas échéant un à trois substituants méthyle, et les hétérocycles suivants :

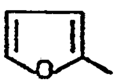 , 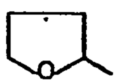 , 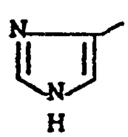 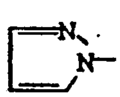 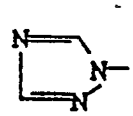 ,

et 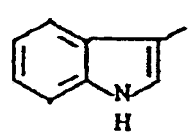 ;

$R^2$ représente en outre un groupe vinyle, allyle, allényle, éthynyle ou propargyle portant éventuellement un substituant phényle qui peut le cas échéant porter un à trois substituants halogéno et méthyle identiques ou différents ;

$R^2$ est en outre un groupe cyclopropyle, cyclohexyle, cyclopentényle ou cyclohexényle ;

$R^2$ représente en outre un groupe phényle portant éventuellement un à trois substituants halogéno, méthyle et méthoxy identiques ou différents ou représente les hétérocycles suivants :

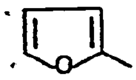, 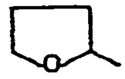, 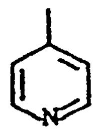, 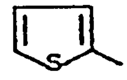 et 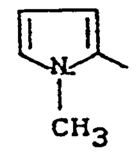 ;

$R^3$ est l'hydrogène ou un groupe alkyle en $C_1$ à $C_4$ à chaîne droite ou ramifiée portant éventuellement un ou deux substituants identiques ou différents, et on considère alors les substituants suivants : fluor, chlore, brome, iode, radical cyano, alkoxy, alkylthio, alkylsulfinyle et alkylsulfonyle ayant chacun 1 ou 2 atomes de carbone, alkylcarbonyloxy ayant 1 à 4 atomes de carbone dans la partie alkyle à chaîne droite ou ramifiée, formamido, alkylcarbonylamino ayant 1 ou 2 atomes de carbone dans la partie alkyle éventuellement substituée par du fluor, un groupe benzoylamino éventuellement substitué par du chlore, alkoxycarbonylamino ayant 1 à 4 atomes de carbone dans la partie alkoxy à chaîne droite ou ramifiée, —COOR', un groupe phényle, cyclopropyle, cyclopentyle ou cyclohexyle portant éventuellement un à trois substituants halogéno et méthyle identiques ou différents, et les hétérocycles suivants :

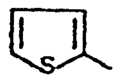, 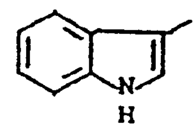, 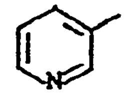, 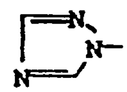, 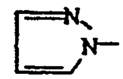,

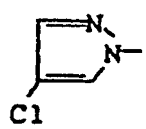, 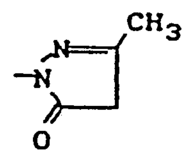 et 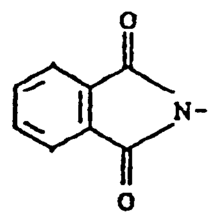 ;

$R^3$ représente en outre un groupe vinyle ou éthynyle portant chacun le cas échéant un ou deux substituants, identiques ou différents, phényle pouvant porter le cas échéant un à trois substituants halogéno et méthyle identiques ou différents, ou méthyle ;

$R^3$ représente en outre un groupe cyclopropyle, cyclopentyle, cyclohexyle, cyclopentényle ou cyclohexényle pontés chacun le cas échéant avec un radical méthylène ou éthylène et/ou condensés avec 1 ou 2 noyaux de benzène, de cyclopentane ou de cyclohexane, portant chacun éventuellement un à trois substituants identiques ou différents, et on considère alors comme substituants : un halogène, un radical méthyle, le groupe oxo, un radical phényle, hydroxycarbonyle, méthoxy- et éthoxycarbonyle ; on mentionne en particulier comme noyaux :

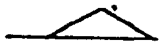, 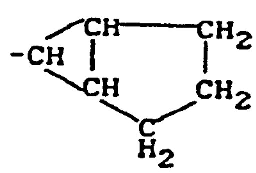, 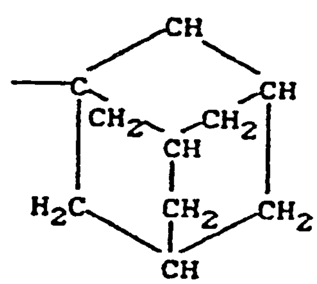,

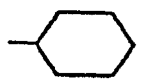, 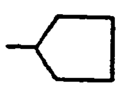, 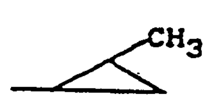, 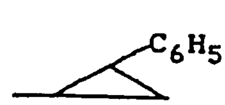,

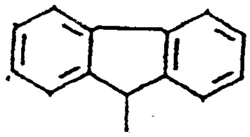 , 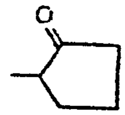 , 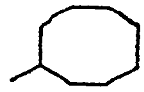 ,

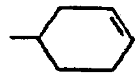 , 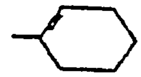 ;

$R^3$ représente en outre un groupe phényle éventuellement condensé avec 1 ou 2 noyaux de benzène, de cyclopentane ou de cyclohexane, portant le cas échéant un à trois substituants identiques ou différents, et on considère alors comme substituants : le fluor, le chlore, un radical hydroxy, nitro, méthyle, méthoxy, acétoxy, acétamido, hydroxycarbonyle, méthoxy- et éthoxycarbonyle ;

$R^3$ représente en outre un groupe hétérocyclyle pentagonal ou hexagonal ayant 1 à 3 hétéro-atomes identiques ou différents, condensé le cas échéant avec 1 ou 2 noyaux de benzène, de cyclopentane ou de cyclohexane et portant le cas échéant un à trois substituants identiques ou différents, et on mentionne alors comme substituants : un radical acyle ayant 2 à 5 atomes de carbone, le chlore, le brome, un radical méthyle, éthyle, phényle, le groupe oxo et un radical hydroxy ; on mentionne en particulier comme hétérocycles :

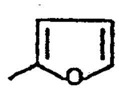 , 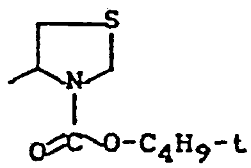 , 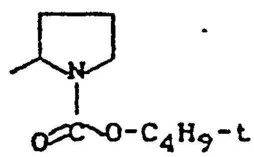 ,

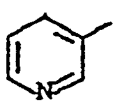 , 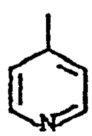 , 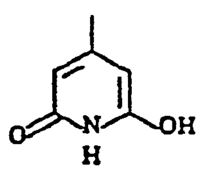 , 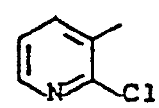 ,

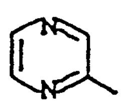 , 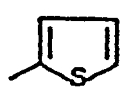 , 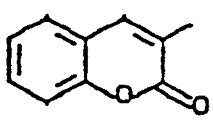 , 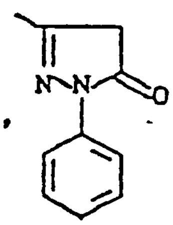 ,

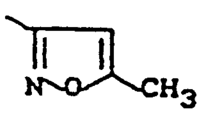 ;

$R^3$ représente enfin également un groupe alkoxycarbonyle ayant 1 à 4 atomes de carbone dans la partie alkoxy à chaîne droite ou ramifiée, un groupe $—OR^4$, $—SR^4$ ou $—NR^5R^6$ ;

$R^4$ représente un groupe alkyle en $C_1$ ou $C_2$ à chaîne droite ou ramifiée portant éventuellement un ou deux substituants identiques ou différents, et on considère alors comme substituants : le fluor, le chlore, un radical méthoxy, éthoxy, méthylthio, alkoxycarbonyle ayant 1 à 4 atomes de carbone dans la partie alkyle à chaîne droite ou ramifiée, carbamoyle, méthylcarbamoyle, diméthylcarbamoyle, cyano, diméthylamino, diéthylamino, acétyle, pivaloyle, un groupe phényle ou phénoxy portant chacun le cas échéant un à trois substituants halogéno et méthyle identiques ou différents, un groupe cyclopropyle ou cyclohexyle portant chacun le cas échéant un à trois substituants méthyle ;

$R^4$ représente en outre un groupe hétérocyclyle de 3 à 6 chaînons ayant 1 à 3 hétéro-atomes identiques ou différents, portant éventuellement un à trois substituants halogéno, méthyle et éthyle identiques ou différents ; on mentionne en particulier comme hétérocycles :

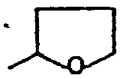, 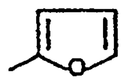, 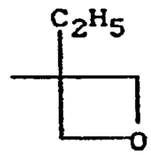, 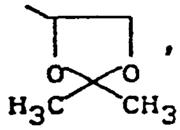, 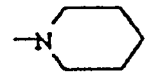,

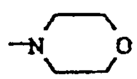, 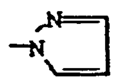, 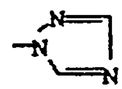 ;

$R^4$ représente en outre un groupe allyle ou propargyle portant chacun le cas échéant un ou deux substituants méthyle ou un groupe cyclohexyle portant un à trois substituants méthyle ;

$R^5$ représente l'hydrogène, un groupe alkyle à chaîne droite ou ramifiée ayant 1 à 4 atomes de carbone ou un groupe alkyle ayant 1 à 3 atomes de carbone portant un ou deux substituants identiques ou différents, et on mentionne comme substituants : le chlore, un radical hydroxy, alkylcarbonyloxy ayant 1 à 4 atomes de carbone dans la partie alkyle à chaîne droite ou ramifiée, un groupe alkylcarbonyle ayant 1 à 4 atomes de carbone dans la partie alkyle à chaîne droite ou ramifiée, un groupe alkoxy et un groupe alkylthio ayant chacun 1 ou 2 atomes de carbone, un groupe alkoxycarbonyle ayant 1 à 4 atomes de carbone dans la partie alkoxy à chaîne droite ou ramifiée, un groupe carbamoyle, méthylcarbamoyle, diméthylcarbamoyle, cyano, diméthylamino, diéthylamino, un groupe phényle portant éventuellement un à trois substituants chloro et méthyle identiques ou différents, un groupe 2-furyle, 2-pyridyle, 1-morpholino, cyclopropyle ou cyclohexyle ;

$R^5$ représente en outre un groupe allyle ou propargyle, un groupe 1-cyclohexényle ou un groupe cyclohexyle portant un à trois substituants identiques ou différents, et on mentionne alors comme substituants : un atome de chlore, un radical méthyle, éthyle, méthoxy, cyano, amino, alkyl- et dialkylamino ayant chacun 1 ou 2 atomes de carbone dans chaque partie alkyle, un groupe carbamoyle, alkyl- et dialkylcarbamoyle ayant chacun 1 ou 2 atomes de carbone dans chaque partie alkyle, un groupe cyclohexyle, cyclohexylalkyle ayant 1 ou 2 atomes de carbone dans la partie alkyle ou un groupe 1-pyrrolidine-2-one ;

$R^5$ représente en outre un groupe phényle portant éventuellement un à trois substituants chloro et méthyle identiques ou différents, un groupe 2-pyridyle ou 2-pyrimidinyle portant chacun le cas échéant un à trois substituants méthyle, un groupe 2-thiazolyle portant éventuellement un groupe phényle, un groupe 2-benzothiazolyle portant éventuellement un à trois substituants chloro, méthyle et méthoxy identiques ou différents, un groupe 1,2,4-triazole-3-yle, un groupe 1,2,4-thiadiazole-5-yle portant éventuellement un substituant phényle, un groupe 1,3,4-thiadiazole-5-yle portant éventuellement un à trois substituants, identiques ou différents, mercapto, alkyle à chaîne droite ou ramifiée ayant 1 à 4 atomes de carbone, alkylthio, alkylsulfinyle et alkylsulfonyle chacun à chaîne droite ou ramifiée et avec chacun 1 à 4 atomes de carbone ou le groupement

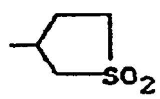 ,

$R^6$ représente l'hydrogène, un groupe alkyle à chaîne droite ou ramifiée ayant 1 à 4 atomes de carbone ou un groupe alkyle ayant 1 à 3 atomes de carbone portant un ou deux substituants identiques ou différents, et on considère alors comme substituants : le chlore, un radical hydroxy, alkylcarbonyloxy ayant 1 à 4 atomes de carbone dans la partie alkyle à chaîne droite ou ramifiée, un groupe alkylcarbonyle ayant 1 à 4 atomes de carbone dans la partie alkyle à chaîne droite ou ramifiée, un groupe alkoxy et un groupe alkylthio ayant chacun 1 ou 2 atomes de carbone, un groupe alkoxycarbonyle ayant 1 à 4 atomes de carbone dans la partie alkoxy à chaîne droite ou ramifiée, un groupe carbamoyle, méthylcarbamoyle, diméthylcarbamoyle, cyano, diméthylamino, diéthylamino, un groupe phényle portant éventuellement un à trois substituants chloro et méthyle identiques ou différents, un groupe 2-furyle, 2-pyridyle, 1-morpholino, cyclopropyle et cyclohexyle ; $R^6$ représente en outre un groupe allyle ou un groupe propargyle, un groupe 1-cyclohexényle ou un groupe cyclohexyle portant un à trois substituants identiques ou différents, et on mentionne alors comme substituants : le chlore, un radical méthyle, éthyle, méthoxy, cyano, amino, alkyl- ou dialkylamino ayant chacun 1 ou 2 atomes de carbone dans chaque partie alkyle, un groupe carbamoyle, alkyl- ou dialkylcarbamoyle ayant chacun 1 ou 2 atomes de carbone dans chaque partie alkyle, un groupe cyclohexyle, cyclohexylalkyle ayant 1 ou 2 atomes de carbone dans la partie alkyle ou un groupe 1-pyrrolidine-2-one ;

$R^6$ représente en outre un groupe phényle portant éventuellement un à trois substituants chloro et méthyle identiques ou différents, un groupe 2-pyridyle ou 2-pyrimidinyle portant chacun le cas échéant un à trois substituants méthyle, un groupe 2-thiazolyle éventuellement substitué par un radical phényle, un groupe 2-benzothiazolyle portant éventuellement un à trois substituants chloro, méthyle et méthoxy identiques ou différents, un groupe 1,2,4-triazole-3-yle, un groupe 1,2,4-thiadiazole-5-yle éventuellement substitué par un radical phényle, un groupe 1,3,4-thiadiazole-5-yle portant éventuellement un à trois substituants, identiques ou différents, mercapto, alkyle à chaîne droite ou ramifiée ayant 1 à 4 atomes de carbone, alkylthio, alkylsulfinyle et alkylsulfonyle chacun à chaîne droite ou ramifiée ayant chacun 1 à 4 atomes de carbone, ou le groupement

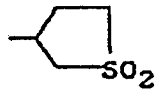 ,

$R^6$ représente en outre un groupe hydroxy, alkoxy ayant 1 ou 2 atomes de carbone, un groupe benzyloxy portant éventuellement un à trois substituants chloro et méthyle, identiques ou différents, un groupe 1-adamantyle, 2-norbornyle ; 1- ou 2-décalyle ou 1- ou 2-tétralyle ;
ou bien

$R^5$ et $R^6$ forment conjointement avec l'atome d'azote auquel ils sont liés, les hétérocycles ou spirohétérocycles monocycliques, bicycliques ou tricycliques suivants :

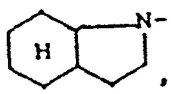 , 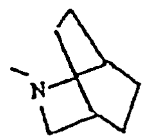 , 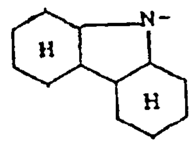 ,

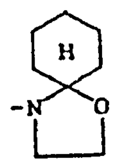 , 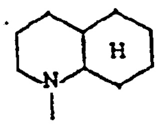 ,

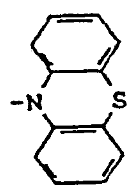 , 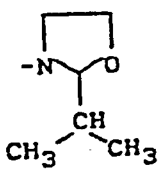 , 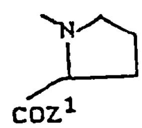

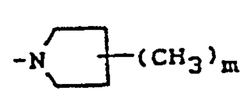 , 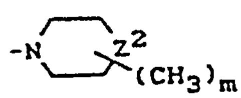

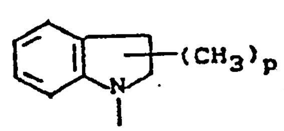 , 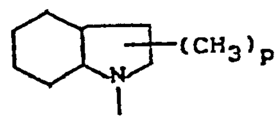 .

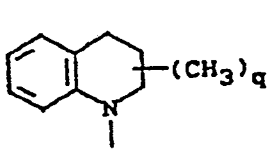 , 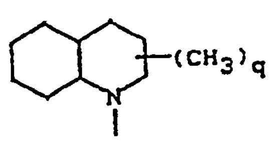 ,

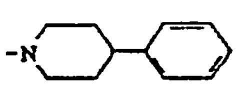 , 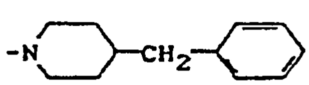 ,

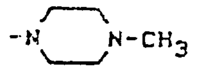 , 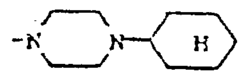 , 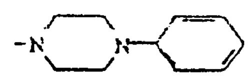 ,

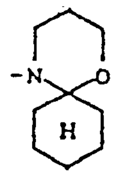 , 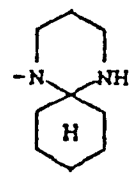 , 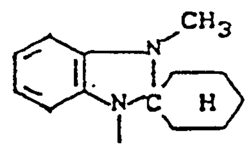 ;

ou

$Z^1$ est un groupe hydroxy, méthoxy, éthoxy, amino, méthylamino, éthylamino, diméthylamino ou éthylméthylamino,

$Z^2$ représente l'oxygène ou le groupe $CH_2$,

m représente les nombres 0, 1, 2, 3 ou 4,

p représente les nombres 0, 1 ou 2,

q représente les nombres 0, 1, 2 ou 3,

$R^I$ est un groupe méthyle, éthyle, n-propyle, isopropyle, n-butyle, isobutyle, sec.butyle ou tertio-butyle,

$R^{II}$ et $R^{III}$ sont identiques ou différents et représentent l'hydrogène, un groupe méthyle, éthyle, n-propyle ou isopropyle, un groupe benzyle portant éventuellement un à trois substituants identiques ou différents avec du chlore et un radical méthyle comme substituants, un groupe alkoxycarbonylalkyle ayant 1 ou 2 atomes de carbone dans chaque partie alkyle, un groupe carbamoylalkyle, alkylcarbamoylalkyle ou dialkylcarbamoylalkyle ayant chacun 1 ou 2 atomes de carbone dans chaque partie alkyle ou un groupe cyclohexyle portant éventuellement un à trois substituants méthyle,

$R^{IV}$ représente l'hydrogène, un groupe alkyle ayant 1 ou 2 atomes de carbone ou un groupe benzyle portant éventuellement un à trois substituants identiques ou différents, et on mentionne alors comme substituants du radical phényle le fluor, le chlore et le radical méthyle ; et

X représente l'oxygène ou le soufre.

4. Procédé de production de N,N'-diacylaminals de formule générale (I)

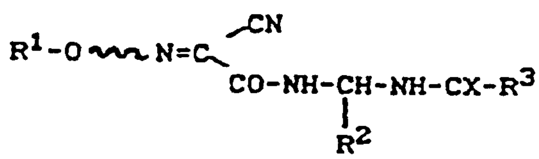 (I)

dans laquelle

$R^1$, $R^2$, $R^3$ et X ont la définition indiquée dans la revendication 1, de leurs isomères géométriques et optiques et de leurs mélanges d'isomères, caractérisé en ce que

a) On fait réagir des N-(2-cyano-2-oximinoacétyl)-aminals de formule (II)

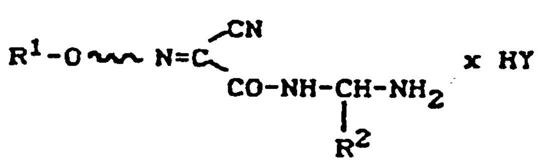 (II)

dans laquelle

$R^1$ et $R^2$ ont la définition indiquée ci-dessus et

HY représente l'équivalent d'un acide inorganique ou organique, avec un réactif d'acylation de formule (IIIa) ou (IIIb)

$$Z-CX-R^3 \qquad (IIIa)$$

$$X=C=N-R^6 \qquad (IIIb)$$

dans lesquelles

Z représente un groupe partant classique tel qu'un halogène, un groupe —O-CO-$R^3$, —O-CO-$OR^4$, —$OR^4$, —$Sr^4$, carboxyméthoxy ou carboxyméthylthio et

$R^3$, $R^4$, $R^6$ et X ont la définition indiquée dans la revendication 1,

le cas échéant en présence d'une base et en présence d'un diluant et le cas échéant en présence d'un catalyseur ;

ou bien

b) On fait réagir des isocyanates de formule (IV),

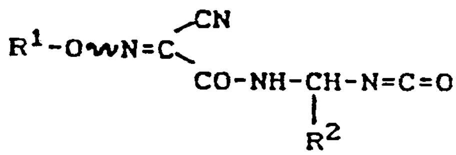

(IV)

dans laquelle

$R^1$ et $R^2$ ont la définition indiquée ci-dessus, avec un nucléophile protique de formules (Va) ou (Vb)

$$HO-CO-R^3 \qquad (Va)$$

$$H - R^7 \qquad (Vb)$$

dans lesquelles

$R^7$ représente —$OR^4$, —$SR^4$ ou —$NR^5R^6$ et $R^3$, $R^4$, $R^5$ et $R^6$ ont la définition indiquée dans la revendication 1, en présence d'un diluant et le cas échéant en présence d'un catalyseur.

5. Compositions pesticides, caractérisées par une teneur en au moins un N,N'-diacylaminal de formule (I) suivant les revendications 1 et 4.

6. Utilisation de N,N'-diacylaminals de formule (I) suivant les revendications 1 et 4 pour combattre des parasites.

7. Procédé pour combattre des parasites, caractérisé en ce qu'on fait agir des N,N'-diacylaminals de formule (I) suivant les revendications 1 et 4 sur des parasites et/ou sur leur habitat.

8. Procédé de préparation de compositions pesticides, caractérisé en ce qu'on mélange des N,N'-diacylaminals de formule (I) selon les revendications 1 et 4 avec des diluants et/ou des agents tensio-actifs.

9. N-(2-cyano-2-oximinoacétyl)aminals de formule générale (II),

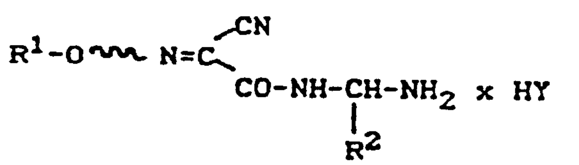

(II)

dans laquelle

$R^1$ et $R^2$ ont la définition indiquée dans la revendication 1 et

HY représente l'équivalent d'un acide inorganique ou organique.

10. Procédé de préparation de N-(2-cyano-2-oximinoacétyl)-aminals de formule générale (II),

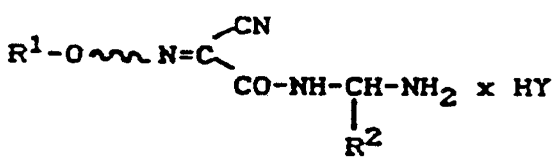

(II)

dans laquelle

$R^1$, $R^2$ et HY ont la définition indiquée dans la revendication 9, caractérisé en ce qu'on hydrolyse d'une manière classique des isocyanates de formule (IV)

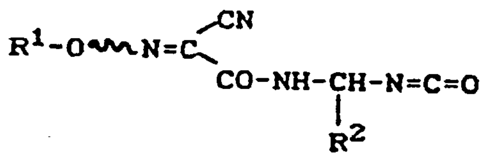 (IV)

dans laquelle

$R^1$ et $R^2$ ont la définition indiquée ci-dessus et on isole comme sel d'une manière classique l'amine formée.

11. Isocyanates de formule (IV),

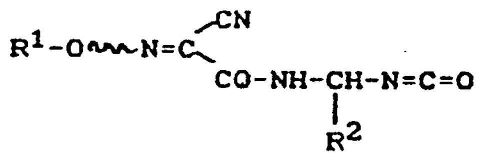 (IV)

dans laquelle

$R^1$ et $R^2$ ont la définition indiquée dans la revendication 1.

12. Procédé de production d'isocyanates de formule générale (IV),

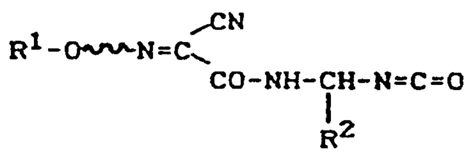 (IV)

dans laquelle

$R^1$ et $R^2$ ont la définition indiquée dans la revendication 1. caractérisé en ce que α) On oxyde des amides de formule générale (VI),

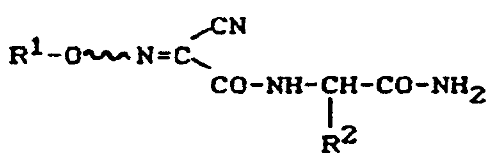 (VI)

dans laquelle

$R^1$ et $R^2$ ont la définition indiquée ci-dessus, d'une manière classique, le cas échéant en présence d'un diluant, ou bien β) On chauffe des azides de formule générale (VII),

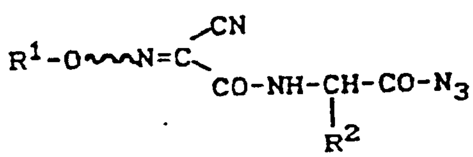 (VII)

dans laquelle

$R^1$ et $R^2$ ont la définition indiquée ci-dessus, en présence d'un diluant.

**Revendications pour l'Etat contractant suivant : ES**

1. Procédé de production de N,N'-diacylaminals de formule générale (I),

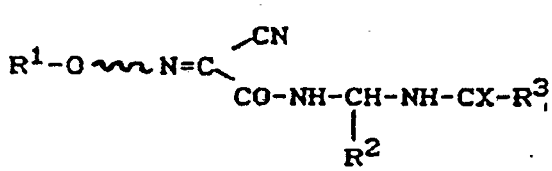 (I)

dans laquelle

$R^1$ représente un groupe alkyle à chaîne droite ou ramifiée ayant 1 à 6 atomes de carbone portant éventuellement un à cinq substituants identiques ou différents, parmi lesquels on considère les substituants suivants : fluor, chlore, brome, iode, radical cyano, —$COOR^I$, —$CONR^{II}R^{III}$, —$OR^{IV}$, acyle ayant 2 à 9 atomes de carbone, phényle portant éventuellement un à cinq substituants identiques ou différents, parmi lesquels on mentionne : un halogène, un radical alkyle et un radical alkoxy ayant chacun 1 à 4 atomes de carbone, un radical halogénalkyle et un radical halogénalkoxy ayant chacun 1 ou 2 atomes de carbone et 1 à 5 atomes d'halogènes identiques ou différents ; d'autres substituants du groupe alkyle sont un radical cycloalkyle de 3 à 6 atomes de carbone portant éventuellement un à cinq substituants alkyle en $C_1$ à $C_4$ identiques ou différents, un radical hétérocyclyle ayant 1 à 3 hétéro-atomes identiques ou différents, portant éventuellement un à cinq substituants identiques ou différents halogéno et alkyle ayant 1 à 4 atomes de carbone ; $R^1$ représente en outre un groupe alcényle ou un groupe alcynyle de 2 à 6 atomes de carbone à chaîne droite ou à chaîne ramifiée portant éventuellement un à trois substituants identiques ou différents, et on mentionne comme substituants, un radical phényle portant éventuellement un à cinq substituants halogéno et alkyle en $C_1$ à $C_4$ identiques ou différents ; $R^1$ représente en outre un groupe cycloalkyle de 3 à 6 atomes de carbone ou un groupe cycloalcényle de 5 à 7 atomes de carbone portant chacun éventuellement 1 à 5 substituants alkyle en $C_1$ à $C_4$ identiques ou différents ;

$R^2$ représente l'hydrogène ou un groupe alkyle ayant 1 à 6 atomes de carbone à chaîne droite ou à chaîne ramifiée, portant éventuellement un à cinq substituants identiques ou différents, parmi lesquels on mentionne les substituants suivants : cyano, alkoxy et alkylthio ayant chacun 1 à 4 atomes de carbone, —$COOR^I$, acylamino ayant 2 à 9 atomes de carbone, phényle portant éventuellement un à cinq substituants identiques ou différents, et on considère alors comme substituants les substituants du groupe phényle déjà mentionnés pour $R^1$ ; d'autres substituants du groupe alkyle sont un radical cycloalkyle de 3 à 6 atomes de carbone portant éventuellement un à cinq substituants alkyle en $C_1$ à $C_4$ identiques ou différents, un radical hétérocyclyle de 3 à 6 chaînons ayant 1 à 3 hétéro-atomes identiques ou différents, portant éventuellement un à cinq substituants halogéno et alkyle en $C_1$ à $C_4$ identiques ou différents ; $R^2$ représente en outre un groupe alcényle ou un groupe alcynyle de 2 à 6 atomes de carbone à chaîne droite ou ramifiée, portant éventuellement chacun un à trois substituants identiques ou différents, et on mentionne comme substituants : un radical phényle portant éventuellement un à cinq substituants halogéno et alkyle en $C_1$ à $C_4$ identiques ou différents ; $R^2$ représente en outre un groupe cycloalkyle ayant 3 à 6 atomes de carbone ou un groupe cycloalcényle ayant 5 à 7 atomes de carbone portant chacun éventuellement un à cinq substituants alkyle en $C_1$ à $C_4$ identiques ou différents ; $R^2$ représente en outre un groupe phényle portant éventuellement un à cinq substituants identiques ou différents, et on considère comme substituants les substituants du groupe phényle déjà mentionnés pour $R^1$ ; $R^2$ représente en outre un groupe hétérocyclyle de 3 à 6 chaînons ayant 1 à 3 hétéro-atomes identiques ou différents, portant éventuellement un à cinq substituants halogéno et alkyle en $C_1$ à $C_4$ identiques ou différents ;

$R^3$ représente l'hydrogène ou un groupe alkyle de 1 à 6 atomes de carbone à chaîne droite ou ramifiée portant éventuellement un à cinq substituants identiques ou différents, parmi lesquels on considère les substituants suivants : fluor, chlore, brome, iode, radical cyano, alkoxy, alkylthio, alkylsulfinyle et alkylsulfonyle ayant chacun 1 à 4 atomes de carbone, acyloxy et acylamino ayant chacun 2 à 9 atomes de carbone, —$COOR^I$, phényle portant éventuellement un à cinq substituants identiques ou différents, et on considère alors comme substituants les substituants du groupe phényle déjà mentionnés pour $R^1$ ; d'autres substituants du groupe alkyle sont un radical cycloalkyle de 3 à 6 atomes de carbone portant éventuellement un à cinq substituants alkyle en $C_1$ à $C_4$ identiques ou différents ainsi que, le cas échéant, un radical hétérocyclyle de 3 à 6 chaînons ayant 1 à 3 hétéro-atomes identiques ou différents, portant éventuellement un à cinq substituants halogéno et alkyle en $C_1$ à $C_4$ identiques ou différents ; $R^3$ représente en outre un groupe alcényle ou alcynyle ayant chacun 2 à 6 atomes de carbone, à chaîne droite ou ramifiée, portant chacun éventuellement un à trois substituants identiques ou différents, et on mentionne alors comme substituants : un radical phényle portant éventuellement un à cinq substituants halogéno et alkyle en $C_1$ à $C_4$ identiques ou différents ; $R^3$ représente en outre un groupe cycloalkyle ayant 3 à 6 atomes de carbone ou un groupe cycloalcényle ayant 5 à 7 atomes de carbone présentant chacun le cas échéant un pont méthylène ou éthylène et/ou condensés chacun avec 1 ou 2 noyaux de benzène, de cyclopentane ou de cyclohexane, et portant chacun le cas échéant un à cinq substituants identiques ou différents, parmi lesquels on considère : un halogène, un radical alkyle et un radical alkoxy ayant chacun 1 à 4 atomes de carbone, un radical acyloxy et un radical acylamino ayant chacun 2 à 5 atomes de carbone, le groupe oxo, un radical phényle, hydroxycarbonyle et alkoxycarbonyle ayant 1 à 4 atomes de carbone dans la partie alkoxy ; $R^3$ représente en outre un groupe phényle condensé le cas échéant avec 1 ou 2 noyaux de benzène ou de cyclohexane, portant éventuellement un à cinq substituants identiques ou différents, parmi lesquels on considère : un halogène, un radical hydroxy, nitro, alkyle et alkoxy ayant chacun 1 à 4 atomes de carbone, halogénalkyle et halogénalkoxy ayant chacun 1 ou 2 atomes de carbone et 2 à 5 atomes d'halogènes identiques ou différents, un radical acylamino et un radical acylalkylamino ayant chacun 2 à 5 atomes de carbone dans la partie acyle et 1 à 4 atomes de carbone dans la partie alkyle, un radical hydroxycarbonyle et un radical alkoxycarbonyle ayant 1 à 4 atomes de carbone dans la partie alkoxy ; $R^3$ représente en outre un groupe hétérocyclyle de 5 ou 6 chaînons avec 1 à 3 hétéro-atomes identiques ou différents, éventuellement condensé avec 1 ou 2 noyaux de benzène, de cyclopentane ou de cyclohexane et portant éventuellement un à cinq substituants identiques ou différents, parmi lesquels on mentionne : un halogène, un radical alkyle ayant 1 à 4 atomes de carbone, acyle ayant 2 à 9 atomes de carbone, phényle, le groupe oxo et un radical hydroxy; $R^3$ représente enfin également un groupe alkoxycarbonyle ayant 1 à 4 atomes de carbone dans la partie alkoxy, un groupe —$OR^4$, —$SR^4$ ou —$NR^5R^6$ ;

$R^4$ représente un groupe alkyle en $C_1$ à $C_8$ à chaîne droite ou ramifiée, portant éventuellement un à cinq substituants identiques ou différents, parmi lesquels on considère : un halogène, un radical —$OR^{IV}$, —$SR^{IV}$, —$COOR^{I}$, —$CONR^{II}R^{III}$, CN, —$NR^{II}R^{III}$, un radical acyle ayant 2 à 9 atomes de carbone, un radical phényle ou un radical phénoxy portant chacun éventuellement un à cinq substituants halogéno et alkyle en $C_1$ à $C_4$ identiques ou différents, un radical cycloalkyle ayant 3 à 6 atomes de carbone ou un radical cycloalcényle ayant 5 à 7 atomes de carbone portant chacun le cas échéant un à cinq substituants alkyle en $C_1$ à $C_4$ identiques ou différents, un radical hétérocyclyle de 3 à 6 chaînons avec 1 à 3 hétéro-atomes identiques ou différents, portant le cas échéant un à cinq substituants halogéno et alkyle en $C_1$ à $C_4$ identiques ou différents ; $R^4$ représente en outre un groupe alcényle ou un groupe alcynyle ayant chacun 2 à 6 atomes de carbone, un groupe cycloalkyle ayant 3 à 6 atomes de carbone ou un groupe cycloalcényle ayant 5 à 7 atomes de carbone portant chacun éventuellement un à cinq substituants alkyle en $C_1$ à $C_4$ identiques ou différents ;

$R^5$ est l'hydrogène ou un groupe alkyle en $C_1$ à $C_4$ à chaîne droite ou ramifiée portant éventuellement un ou deux substituants identiques ou différents, et on mentionne alors comme substituants : un halogène, un radical cyano, —$COOR^{I}$, —$CONR^{II}R^{III}$, —$NR^{II}R^{III}$, —$OR^{IV}$, —$S(O)_nR^{IV}$, un radical acyle de 2 à 9 atomes de carbone, un radical phényle portant éventuellement un à cinq substituants halogéno et alkyle en $C_1$ à $C_4$ identiques ou différents, un hétérocycle pentagonal ou hexagonal avec 1 à 3 hétéro-atomes identiques ou différents, portant le cas échéant un à cinq substituants identiques ou différents, halogéno, alkyle et alkoxy ayant chacun 1 à 4 atomes de carbone, un groupe cycloalkyle de 3 à 6 atomes de carbone ou un groupe cycloacényle de 5 à 7 atomes de carbone portant chacun le cas échéant un à cinq substituants alkyle en $C_1$ à $C_4$ identiques ou différents ; $R^5$ représente en outre un groupe alcényle ou un groupe alcynyle à chaîne droite ou ramifiée ayant chacun 2 à 6 atomes de carbone ; un groupe cycloalcényle de 5 à 7 atomes de carbone portant éventuellement un à cinq substituants alkyle en $C_1$ à $C_4$ identiques ou différents ou un groupe cycloalkyle de 3 à 6 atomes de carbone portant éventuellement un à cinq substituants identiques ou différents, parmi lesquels on mentionne: un halogène, un radical alkyle et un radical alkoxy ayant chacun 1 à 4 atomes de carbone, un radical cyano, amino, carbamoyle, alkylamino, dialkylamino, alkylcarbamoyle et dialkylcarbamoyle ayant chacun 1 à 4 atomes de carbone dans chaque partie alkyle, un radical alkoxy-carbonylamino ayant 1 à 4 atomes de carbone dans la partie alkoxy, un radical cycloalkyle et un radical cycloalkylalkyle ayant 5 ou 6 atomes de carbone dans la partie cycloalkyle et 1 à 4 atomes de carbone dans la partie alkyle à chaîne droite ou ramifiée, un radical phényle ou pyrrolidone portant chacun un à cinq substituants halogéno et alkyle en $C_1$ à $C_4$ identiques ou différents ; $R^5$ représente en outre un groupe phényle portant éventuellement un à cinq substituants identiques ou différents halogéno, alkyle ou alkoxy ayant chacun 1 à 4 atomes de carbone, halogénalkyle ou halogénalkoxy ayant chacun 1 ou 2 atomes de carbone et 1 à 5 atomes d'halogènes identiques ou différents ou un hétérocycle pentagonal ou hexagonal avec 1 à 3 hétéro-atomes identiques ou différents, portant éventuellement un à cinq substituants identiques ou différents, parmi lesquels on mentionne : un halogène, un radical mercapto, phényle, alkyle à chaîne droite ou ramifiée, alkoxy, alkylthio, alkylsulfinyle et alkylsulfonyle ayant 1 à 4 atomes de carbone par partie alkyle ;

$R^6$ a les définitions données pour $R^5$ ou représente le groupement —$OR^{IV}$ ;

ou bien

$R^5$ et $R^6$ forment conjointement avec l'atome d'azote auquel ils sont liés un hétérocycle ou un spirohétérocycle monocyclique, bicyclique ou tricyclique ayant un à trois autres hétéro-atomes identiques ou différents et portant le cas échéant un à cinq substituants identiques ou différents, parmi lesquels on mentionne :

un radical alkyle ou alkoxy à chaîne droite ou ramifiée ayant chacun 1 à 4 atomes de carbone, un radical cycloalkyle ayant 3 à 6 atomes de carbone, le groupe hydroxy ou le groupe oxo, un radical alcényle à chaîne droite ou ramifiée ayant 2 à 4 atomes de carbone, un radical hydroxycarbonyle, alkoxycarbonyle ayant 1 à 4 atomes de carbone dans la partie alkyle à chaîne droite ou ramifiée, un radical carbamoyle, alkyl- ou dialkylcarbamoyle ayant chacun 1 à 4 atomes de carbone dans la partie alkyle à chaîne droite ou ramifiée, un radical phényle ou phénylalkyle ayant 1 à 4 atomes de carbone dans la partie alkyle à chaîne droite ou ramifiée, portant chacun éventuellement un ou deux substituants, identiques ou différents, halogéno ou alkyle ou alkoxy à chaîne droite ou à chaîne ramifiée ayant chacun 1 à 4 atomes de carbone ;

$R^I$ est l'hydrogène ou un groupe alkyle à chaîne droite ou ramifiée ayant 1 à 6 atomes de carbone,

$R^{II}$ représente l'hydrogène, un groupe alkyle à chaîne droite ou ramifiée ayant 1 à 6 atomes de carbone, un groupe phénylalkyle ayant 1 à 4 atomes de carbone dans la partie alkyle à chaîne droite ou ramifiée, portant éventuellement un à cinq substituants identiques ou différents, et on mentionne comme substituants du groupe phényle : un halogène, un radical alkyle ou alkoxy ayant chacun 1 à 4 atomes de carbone, halogénalkyle ou halogénalkoxy ayant chacun 1 ou 2 atomes de carbone et 2 à 5 atomes d'halogènes identiques ou différents; $R^{II}$ représente en outre un groupe alkoxycarbonylalkyle, carbamoylalkyle, alkylcarbamoylalkyle ou dialkylcarbamoylalkyle ayant chacun 1 à 4 atomes de carbone dans chaque partie alkyle ou un groupe cycloalkyle de 3 à 6 atomes de carbone portant éventuellement un substituant alkyle en $C_1$ à $C_4$ à chaîne droite ou ramifiée,

$R^{III}$ représente l'hydrogène, un groupe alkyle à chaîne droite ou ramifiée ayant 1 à 6 atomes de carbone, un groupe phénylalkyle ayant 1 à 4 atomes de carbone dans la partie alkyle à chaîne droite ou ramifiée portant éventuellement un à cinq substituants identiques ou différents, et on considère comme substituants du groupe phényle les substituants du groupe phényle mentionnés dans le cas de $R^{II}$ ; $R^{III}$ représente en outre un groupe alkoxycarbonylalkyle, carbamoylalkyle, alkylcarbamoylalkyle ou dialkylcarbamoylalkyle ayant chacun 1 à 4 atomes de carbone dans chaque partie alkyle ou un groupe cycloalkyle de 3 à 6 atomes de carbone portant éventuellement un substituant alkyle en $C_1$ à $C_4$ à chaîne droite ou à chaîne ramifiée ;

$R^{IV}$ représente l'hydrogène, un groupe alkyle à chaîne droite ou ramifiée ayant 1 à 6 atomes de carbone, un groupe phénylalkyle ayant 1 à 4 atomes de carbone dans la partie alkyle à chaîne droite ou ramifiée, portant éventuellement un à cinq substituants identiques ou différents, et on considère comme substituants du groupe phényle les substituants du groupe phényle mentionnés pour $R^{II}$ ; $R^{IV}$ représente en outre un groupe acyle ayant 2 à 9 atomes de carbone ;

n représente les nombres 0, 1 ou 2 et

X représente l'oxygène ou le soufre, de leurs isomères géométriques et optiques et de leurs mélanges d'isomères, caractérisé en ce qu'on fait réagir des

a) N-(2-cyano-2-oximinoacétyl)-aminals de formule (II)

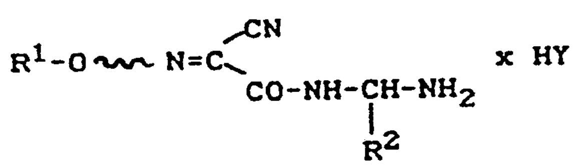

(II)

dans laquelle

$R^1$ et $R^2$ ont la définition indiquée ci-dessus et

HY représente l'équivalent d'un acide inorganique ou organique, avec un réactif d'acylation de formules (IIIa) ou (IIIb)

$$Z\text{-}CX\text{-}R^3 \qquad \text{(IIIa)}$$

$$X{=}C{=}N\text{-}R^6 \qquad \text{(IIIb)}$$

dans lesquelles

Z représente un groupe partant classique tel qu'un halogène, un groupe —O-CO-$R^3$, —O-CO-O$R^4$, —O$R^4$, —S$R^4$, carboxyméthoxy ou carboxyméthylthio et

$R^3$, $R^4$, $R^6$ et X ont la définition indiquée ci-dessus,

éventuellement en présence d'une base et en présence d'un diluant et le cas échéant en présence d'un catalyseur ;

ou bien

b) On fait réagir des isocyanates de formule (IV).

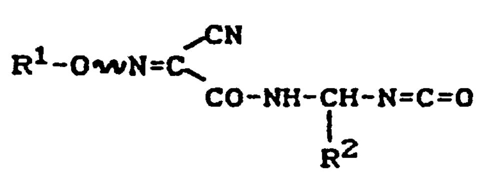

(IV)

dans laquelle

$R^1$ et $R^2$ ont la définition indiquée ci-dessus,

avec un nucléophile protique de formules (Va) ou (Vb)

$$HO-CO-R^3 \qquad (Va)$$

$$H - R^7 \qquad (Vb)$$

dans lesquelles

$R^7$ représente —$OR^4$, —$SR^4$ ou —$NR^5R^6$ et $R^3$, $R^4$, $R^5$ et $R^6$ ont la définition indiquée ci-dessus,

en présence d'un diluant et le cas échéant en présence d'un catalyseur.

2. Procédé suivant la revendication 1, dans lequel, dans la formule (I)

$R^1$ est un groupe alkyle à chaîne droite ou ramifiée en $C_1$ à $C_4$ de carbone à chaîne droite ou ramifiée portant éventuellement un à trois substituants identiques ou différents, parmi lesquels on considère les substituants suivants : fluor, chlore, brome, iode, radical cyano, —$COOR^I$, —$CONR^{II}R^{III}$, —$OR^{IV}$, acyle ayant 2 à 9 atomes de carbone, phényle portant éventuellement un à trois substituants fluoro, chloro, méthyle et méthoxy identiques ou différents, un groupe cyclopropyle ou cyclohexyle portant chacun éventuellement un à trois substituants méthyle, des hétérocycles portant éventuellement 1 ou 3 substituants alkyle en $C_1$ ou $C_2$ identiques ou différents, de formules

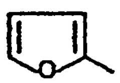, 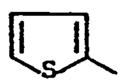, 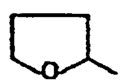, 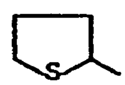, 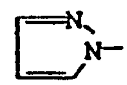

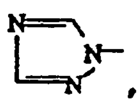, 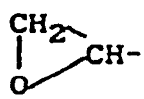, 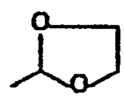 et 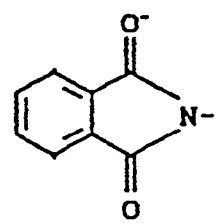 ;

$R^1$ représente en outre un groupe allyle ou propargyle portant éventuellement un ou deux substituants méthyle, un groupe cyclopropyle, cyclohexyle, cyclopentyle ou cyclohexényle portant chacun éventuellement un à trois substituants méthyle ;

$R^2$ est l'hydrogène ou un groupe alkyle ayant 1 à 4 atomes de carbone à chaîne droite ou ramifiée portant éventuellement un à trois substituants identiques ou différents, parmi lesquels on considère les substituants suivants : cyano, méthoxy, éthoxy, méthylthio, éthylthio, —$COOR^I$, acylamino ayant 2 à 9 atomes de carbone, un groupe phényle portant éventuellement un à trois substituants halogéno, méthyle et méthoxy identiques ou différents, un groupe cyclopropyle ou cyclohexyle portant éventuellement un à trois substituants méthyle, de même que des hétérocycles portant éventuellement un à trois substituants alkyle en $C_1$ ou $C_2$ identiques ou différents, de formules

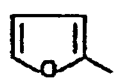, 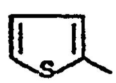, 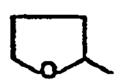, 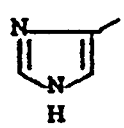, 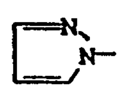

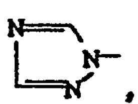, 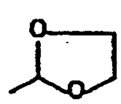 et 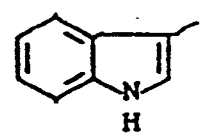 ;

$R^2$ représente en outre un groupe allyle, allényle, vinyle, propargyle ou éthynyle éventuellement substitués chacun par un radical phényle qui peut porter éventuellement un à trois substituants halogéno et méthyle iden-

tiques ou différents ; $R^2$ représente en outre un groupe cyclopropyle, cyclohexyle, cyclopentényle ou cyclohexényle ; $R^2$ représente en outre un groupe phényle portant éventuellement un à trois substituants halogéno, méthyle et méthoxy identiques ou différents, des hétérocycles portant éventuellement un à trois substituants alkyle en $C_1$ ou $C_2$ identiques ou différents, de formules :

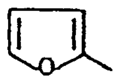 , 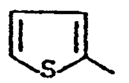 , 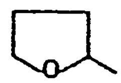 , 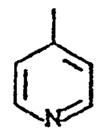 , 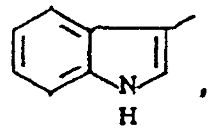 ,

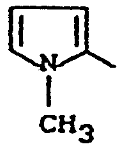 , 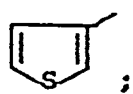 ;

$R^3$ représente l'hydrogène ou un groupe alkyle à chaîne droite ou ramifiée ayant 1 à 4 atomes de carbone, portant éventuellement un ou trois substituants identiques ou différents, parmi lesquels on considère : le fluor, le chlore, le brome, l'iode, un radical cyano, alkoxy, alkylthio, alkylsulfinyle et alkylsulfonyle ayant chacun 1 ou 2 atomes de carbone, acyloxy et acylamino ayant chacun 2 à 9 atomes de carbone, —COOR', un groupe phényle portant éventuellement un à trois substituants halogéno et méthyle identiques ou différents, un groupe cyclopropyle, cyclopentyle ou cyclohexyle, des hétérocycles portant éventuellement un à trois substituants alkyle en $C_1$ ou $C_2$ identiques ou différents, de formules

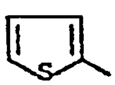 , 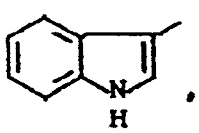 , 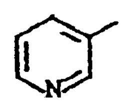 , 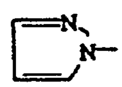 ,

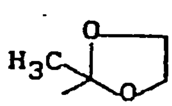 , 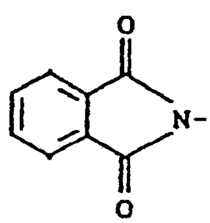 , 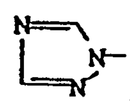 , 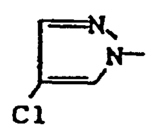 ,

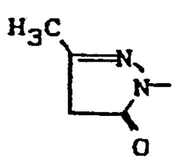 , 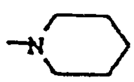 , 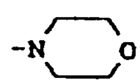 ;

$R^3$ représente en outre un groupe vinyle ou un groupe éthynyle substitué chacun éventuellement par un radical phényle qui peut porter le cas échéant un à trois substituants halogéno et méthyle identiques ou différents ; $R^3$ représente en outre un groupe cyclopropyle, cyclopentyle, cyclohexyle, cyclopentényle ou cyclohexényle pontés chacun le cas échéant par un groupe méthylène ou éthylène et/ou condensé avec 1 ou 2 noyaux de benzène, de cyclopentane ou de cyclohexane, et portant chacun éventuellement un à trois susbtituants identiques ou différents, parmi lesquels on mentionne : un halogène, un radical méthyle, méthoxy, acyloxy et acylamino ayant chacun 2 à 5 atomes de carbone, le groupe oxo, un radical phényle, hydroxycarbonyle, méthoxy- et éthoxycarbonyle ; $R^3$ représente en outre un groupe phényle éventuellement condensé avec 1 ou 2 noyaux de benzène ou de cyclohexane, portant éventuellement un à trois substituants identiques ou différents, parmi lesquels on mentionne : le fluor, le chlore, un radical hydroxy, nitro, méthyle, méthoxy, acylamino et N-alkylacylamino avec dans chaque cas 2 à 5 atomes de carbone dans la partie acyle et 1 ou 2 atomes de carbone dans la partie alkyle, un groupe hydroxycarbonyle, méthoxy- et éthoxycarbonyle ; $R^3$ représente en outre un

groupe hétérocyclyle pentagonal ou hexagonal avec 1 à 3 hétéro-atomes identiques ou différents, condensé le cas échéant avec 1 ou 2 noyaux de benzène ou de cyclohexane et portant éventuellement un à trois substituants identiques ou différents, parmi lesquels on mentionne : un radical acyle ayant 2 à 5 atomes de carbone, le chlore, le brome, un radical méthyle, éthyle, phényle, oxo et hydroxy ; $R^3$ représente enfin également un groupe alkoxycarbonyle ayant 1 à 4 atomes de carbone dans la partie alkoxy, un groupe —$OR^4$, —$SR^4$ ou —$NR^5R^6$ ;

$R^4$ est un groupe alkyle à chaîne droite ou ramifiée ayant 1 à 4 atomes de carbone, portant éventuellement un à trois substituants identiques ou différents, et on considère alors les substituants suivants : fluor, chlore, radical —$OR^{IV}$, —$SR^{IV}$, —$COOR^{I}$, —$CONR^{II}R^{III}$, CN, $NR^{II}R^{III}$, alkylcarbonyle ayant 1 à 4 atomes de carbone dans la partie alkyle à chaîne droite ou ramifiée, un groupe phényle ou phénoxy portant chacun le cas échéant un à trois substituants halogéno et méthyle identiques ou différents, un groupe cyclopropyle, cyclohexyle, cyclopentyle ou cyclohexényle portant chacun le cas échéant un à trois substituants méthyle, un groupe hétérocyclyle de 3 à 6 chaînons ayant 1 à 3 hétéro-atomes identiques ou différents tels que des atomes d'azote, d'oxygène et de soufre, portant éventuellement un à trois substituants halogéno, méthyle et éthyle identiques ou différents ; $R^4$ représente en outre un groupe allyle ou propargyle portant éventuellement un ou deux substituants méthyle ainsi qu'un groupe cyclopropyle, cyclohexyle, cyclopentyle ou cyclohexényle portant éventuellement un à trois substituants méthyle ;

$R^5$ représente l'hydrogène ou un groupe alkyle en $C_1$ à $C_4$ à chaîne droite ou à chaîne ramifiée portant éventuellement un ou deux substituants identiques ou différents, parmi lesquels on mentionne de préférence : un halogène, un radical cyano, —$COOR^{I}$, —$CONR^{II}R^{III}$, $NR^{II}R^{III}$, —$OR^{IV}$, —$S(O)_nR^{IV}$, acyle ayant 2 à 9 atomes de carbone, un groupe phényle portant éventuellement un à trois substituants halogéno et alkyle en $C_1$ à $C_4$ identiques ou différents, un hétérocycle pentagonal ou hexagonal avec 1 à 3 hétéro-atomes identiques ou différents, portant le cas échéant un à trois substituants identiques ou différents halogéno, alkyle et alkoxy à chaîne droite ou ramifiée ayant chacun 1 à 4 atomes de carbone et un groupe cycloalkyle ayant 3 à 6 atomes de carbone ou cycloalcényle ayant 5 à 7 atomes de carbone portant chacun le cas échéant un substituant alkyle à chaîne droite ou ramifiée ayant 1 à 4 atomes de carbone ; $R^5$ représente en outre très préférentiellement un groupe alcényle ou alcynyle à chaîne droite ou ramifiée ayant chacun 2 à 6 atomes de carbone, un groupe cycloalcényle de 5 à 7 atomes de carbone portant éventuellement un substituant alkyle à chaîne droite ou ramifiée ayant 1 à 4 atomes de carbone, un groupe cycloalkyle en $C_3$ à $C_6$ éventuellement substitué, et on mentionne alors comme substituants de préférence : un halogène, un groupe alkyle et un groupe alkoxy à chaîne droite ou ramifiée ayant chacun 1 à 4 atomes de carbone, un radical cyano, amino, carbamoyle, un radical alkylamino, dialkylamino, alkylcarbamoyle et dialkylcarbamoyle à chaîne droite ou à chaîne ramifiée ayant chacun 1 à 4 atomes de carbone dans chaque partie alkyle, un radical alkoxycarbonylamino ayant 1 à 4 atomes de carbone dans la partie alkoxy à chaîne droite ou ramifiée, un radical cycloalkyle et cycloalkylalkyle ayant chacun 5 ou 6 atomes de carbone dans la partie cycloalkyle et 1 à 4 atomes de carbone dans la partie alkyle à chaîne droite ou ramifiée, un radical phényle ou pyrrolidone portant éventuellement un à trois substituants identiques ou différents halogéno et alkyle ayant 1 à 4 atomes de carbone ; $R^5$ représente en outre très préférentiellement un groupe phényle portant éventuellement un à trois substituants, identiques ou différents, halogéno, alkyle ou alkoxy à chaîne droite ou ramifiée ayant chacun 1 à 4 atomes de carbone, halogénalkyle ou halogénalkoxy ayant chacun 1 ou 2 atomes de carbone et 1 à 5 atomes d'halogènes identiques ou différents, ou un hétérocycle pentagonal ou hexagonal ayant 1 à 3 hétéro-atomes identiques ou différents, portant éventuellement un à trois substituants identiques ou différents, et on mentionne comme substituants des hétérocycles : un halogène, un radical mercapto, phényle, alkyle, alkoxy, alkylthio, alkylsulfinyle et alkylsulfonyle à chaîne droite ou ramifiée ayant 1 à 4 atomes de carbone par partie alkyle ;

$R^6$ a les définitions données pour $R^5$ ou représente le groupement —$OR^{IV}$,

ou bien

$R^5$ et $R^6$ forment conjointement avec l'atome d'azote auquel ils sont liés un hétérocycle ou un spirohétérocycle monocyclique, bicyclique ou tricyclique portant éventuellement un à cinq substituants identiques ou différents, et on mentionne alors comme hétérocycles : l'oxazolidine, la pyrrolidine, l'imidazolidine, la pipéridine, la pipérazine, la morpholine, la thiomorpholine, le 1,3-oxazane ou le 1,3-diazane ; ces hétérocycles peuvent chacun être condensés le cas échéant avec 1 ou 2 noyaux de benzène ou de cyclohexane ou être pontés le cas échéant avec un radical méthylène ou éthylène, les substituants de tous les systèmes hétéro étant les suivants : un radical alkyle à chaîne droite ou ramifiée ou cycloalkyle ayant 3 à 6 atomes de carbone, le groupe hydroxy ou le groupe oxo, un radical alcényle à chaîne droite ou ramifiée ayant 2 à 4 atomes de carbone, un radical hydroxycarbonyle, alkoxycarbonyle ayant 1 à 4 atomes de carbone dans la partie alkyle à chaîne droite ou ramifiée, carbamoyle, alkyl- ou dialkylcarbamoyle ayant chacun 1 à 4 atomes de carbone dans chaque partie alkyle, en outre un groupe phényle ou phénylalkyle ayant 1 à 4 atomes de carbone dans la partie alkyle à chaîne droite ou ramifiée, portant éventuellement un ou deux substituants, identiques ou différents, halogéno et alkyle

à chaîne droite ou ramifiée ayant 1 à 4 atomes de carbone,

$R^{I}$ est un groupe alkyle à chaîne droite ou ramifiée ayant 1 à 4 atomes de carbone,

$R^{II}$ représente l'hydrogène, un groupe alkyle à chaîne droite ou ramifiée ayant 1 à 4 atomes de carbone, un groupe benzyle ou phénéthyle portant éventuellement un à trois substituants identiques ou différents, et on mentionne alors comme substituants du groupe phényle : le fluor, le chlore, un radical méthyle, méthoxy et trifluorométhyle ; $R^{II}$ représente en outre un groupe alkoxycarbonylalkyle ayant 1 à 4 atomes de carbone dans la partie alkoxy et 1 ou 2 atomes de carbone dans la partie alkyle, un groupe carbamoylalkyle ayant 1 ou 2 atomes de carbone dans la partie alkyle, un groupe alkylcarbamoyle ou dialkylcarbamoylalkyle ayant chacun 1 ou 2 atomes de carbone dans chaque partie alkyle, ou un groupe cycloalkyle de 3 à 6 atomes de carbone portant éventuellement un à trois substituants méthyle et éthyle identiques ou différents,

$R^{III}$ a les définitions données pour $R^{II}$,

$R^{IV}$ représente l'hydrogène, un groupe alkyle à chaîne droite ou ramifiée ayant 1 à 4 atomes de carbone, un groupe benzyle ou phénéthyle portant chacun le cas échéant un à trois substituants identiques ou différents, et on mentionne alors comme substituants du groupe phényle : le fluor, le chlore, un radical méthyle, méthoxy et trifluorométhyle ; $R^{IV}$ représente en outre un groupe acyle ayant 2 à 9 atomes de carbone ;

n représente les nombres 0, 1 ou 2 et

X représente l'oxygène ou le soufre.

3. Procédé suivant la revendication 1, dans lequel, dans la formule (I)

$R^1$ représente un groupe alkyle ayant 1 ou 2 atomes de carbone portant éventuellement un ou deux substituants identiques ou différents, et on considère alors les substituants suivants : le fluor, le chlore, un radical cyano, —$COOR^{I}$, —$CONR^{II}R^{III}$, —$OR^{IV}$, alkylcarbonyle ayant 1 à 4 atomes de carbone dans la partie alkyle à chaîne droite ou ramifiée, un groupe phényle portant facultativement un à trois substituants fluoro, chloro et méthyle identiques ou différents, un groupe cyclopropyle ou cyclohexyle portant chacun facultativement un à trois substituants méthyle, et les hétérocycles suivants :

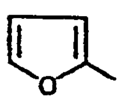 , 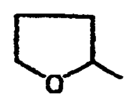 , 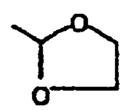 , 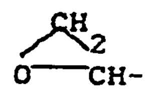

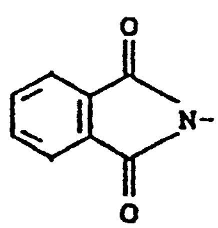 , 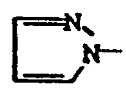 et 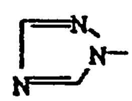 ;

$R^1$ représente en outre un groupe allyle ou propargyle, un groupe cyclopropyle, cyclohexyle, cyclopenténylе ou cyclohexényle portant chacun le cas échéant un à trois substituants méthyle ;

$R^2$ représente l'hydrogène ou un groupe alkyle ayant 1 à 4 atomes de carbone portant éventuellement un ou deux substituants identiques ou différents, et on considère alors les substituants suivants : cyano, méthoxy, éthoxy, méthylthio, éthylthio, —$COOR^{I}$, alkylcarbonylamino ayant 1 à 4 atomes de carbone dans la partie alkyle à chaîne droite ou ramifiée, phényle portant éventuellement un à trois substituants halogéno, méthyle et méthoxy identiques ou différents, un groupe cyclopropyle ou cyclohexyle portant éventuellement un à trois substituants méthyle, et les hétérocycles suivants :

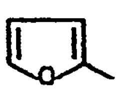 , 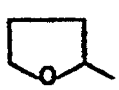 , 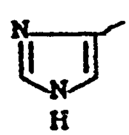 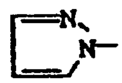 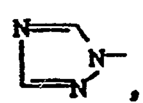 ,

et 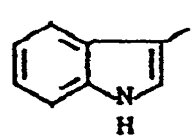 ;

$R^2$ représente en outre un groupe vinyle, allyle, allényle, éthynyle ou propargyle éventuellement substitués par un radical phényle qui peut porter le cas échéant un à trois substituants halogéno et méthyle identiques ou différents ;

$R^2$ représente en outre un groupe cyclopropyle, cyclohexyle, cyclopentényle ou cyclohexényle ;

$R^2$ représente en outre un groupe phényle portant éventuellement un à trois substituants, identiques ou différents, halogéno, méthyle et méthoxy ou les hétérocycles suivants :

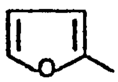 , 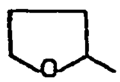 , 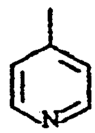 , 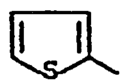 et 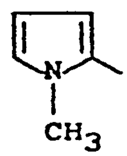 ;

$R^3$ représente l'hydrogène ou un groupe alkyle ayant 1 à 4 atomes de carbone à chaîne droite ou ramifiée portant facultativement un ou deux substituants identiques ou différents, et on considère alors les substituants suivants : fluor, chlore, brome, iode, radicaux cyano, alkoxy, alkylthio, alkylsulfinyle et alkylsulfonyle ayant chacun 1 ou 2 atomes de carbone, alkylcarbonyloxy ayant 1 à 4 atomes de carbone dans la partie alkyle à chaîne droite ou ramifiée, formamido, alkylcarbonylamino ayant 1 ou 2 atomes de carbone dans la partie alkyle éventuellement substituée par du fluor, un groupe benzoylamino éventuellement substitué par du chlore, un groupe alkoxycarbonylamino ayant 1 à 4 atomes de carbone dans la partie alkoxy à chaîne droite ou à chaîne ramifiée, un groupe —COOR', un groupe phényle portant éventuellement un à trois substituants halogéno et méthyle identiques ou différents, un groupe cyclopropyle, cyclopentyle ou cyclohexyle et les hétérocycles suivants :

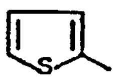 , 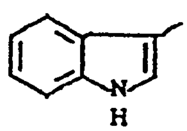 , 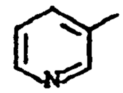 , 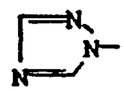 , 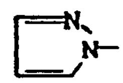 ,

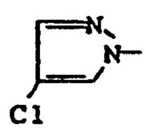 , 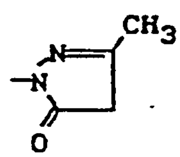 et 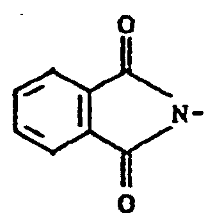 ;

$R^3$ représente en outre un groupe vinyle ou éthynyle portant chacun le cas échéant un ou deux substituants, identiques ou différents, phényle pouvant porter éventuellement un à trois substituants halogéno et méthyle identiques ou différents, ou substitué par un radical méthyle ;

$R^3$ représente en outre un groupe cyclopropyle, cyclopentyle, cyclohexyle, cyclopentényle ou cyclohexényle pontés chacun le cas échéant par un radical méthylène ou éthylène et/ou condensés avec 1 ou 2 noyaux de benzène, de cyclopentane ou de cyclohexane, portant chacun le cas échéant un à trois substituants identiques ou différents, et on mentionne alors comme substituants : un halogène, un radical méthyle, le groupe oxo, un radical phényle, hydroxycarbonyle, méthoxy- et éthoxycarbonyle ; on mentionne en particulier comme noyaux :

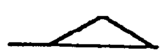 , 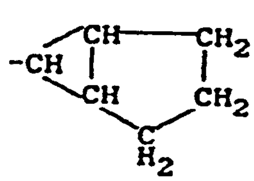 , 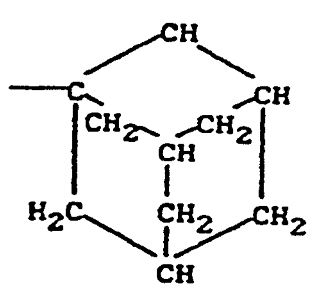 ,

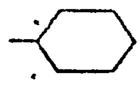 , 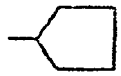 , 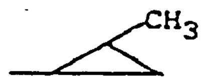 , 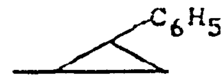 ,

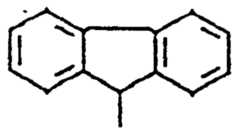 , 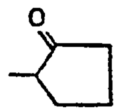 , 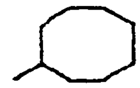 ,

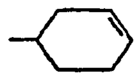 , 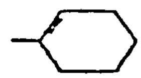 ;

R³ représente en outre un groupe phényle éventuellement condensé avec 1 ou 2 noyaux de benzène, de cyclopentane ou de cyclohexane, portant facultativement un à trois substituants identiques ou différents, et on mentionne alors comme substituants : le fluor, le chlore, un radical hydroxy, nitro, méthyle, méthoxy, acétoxy, acétamido, hydroxycarbonyle, méthoxy- et éthoxycarbonyle ;

R³ représente en outre un groupe hétérocyclyle pentagonal ou hexagonal avec 1 à 3 hétéro-atomes identiques ou différents, condensé le cas échéant avec 1 ou 2 noyaux de benzène, de cyclopentane ou de cyclohexane et portant éventuellement un à trois substituants identiques ou différents, et on mentionne alors comme substituants : un radical acyle ayant 2 à 5 atomes de carbone, le chlore, le brome, un radical méthyle, éthyle, phényle, le groupe oxo et un radical hydroxy ; on mentionne en particulier comme hétérocycles :

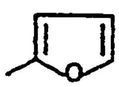 , 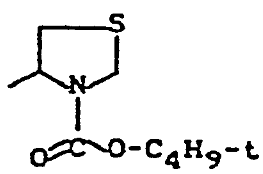 , 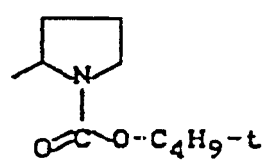 ,

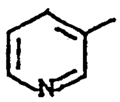 , 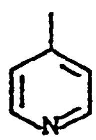 , 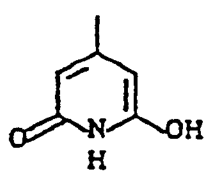 , 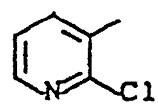 ,

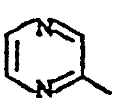 , 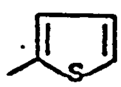 , 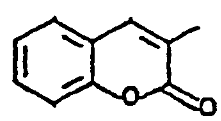 , 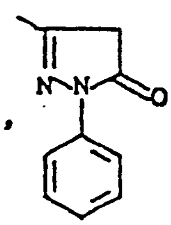 ,

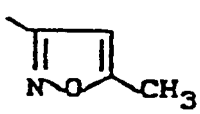 ;

R³ représente enfin également un groupe alkoxycarbonyle ayant 1 à 4 atomes de carbone dans la partie alkoxy à chaîne droite ou à chaîne ramifiée, un groupe —OR⁴, —SR⁴ ou —NR⁵R⁶ ;

R⁴ est un groupe alkyle à chaîne droite ou ramifiée en $C_1$ ou $C_2$ portant éventuellement un ou deux substituants identiques ou différents, et on considère alors les substituants suivants : fluor, chlore, radicaux méthoxy, éthoxy, méthylthio, alkoxycarbonyle ayant 1 à 4 atomes de carbone dans la partie alkyle à chaîne droite ou ramifiée, carbamoyle, méthylcarbamoyle, diméthylcarbamoyle, cyano, diméthylamino, diéthylamino, acétyle, pivaloyle, un groupe phényle ou phénoxy portant chacun le cas échéant un à trois substituants halogéno et méthyle identiques ou différents, un groupe cyclopropyle ou cyclohexyle portant éventuellement un à

trois substituants méthyle ;

$R^4$ représente en outre un groupe hétérocyclyle de 3 à 6 chaînons avec 1 à 3 hétéro-atomes identiques ou différents, portant éventuellement un à trois substituants halogéno, méthyle et éthyle identiques ou différents ; on mentionne en particulier comme hétérocycles :

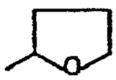 , 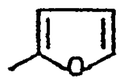 , 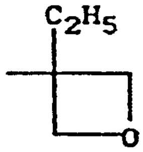 , 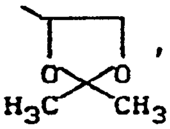 , 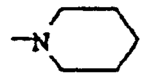 ,

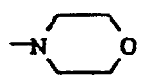 , 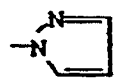 , 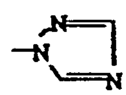 ;

$R^4$ représente en outre un groupe allyle ou propargyle portant chacun le cas échéant un ou deux substituants méthyle ou un groupe cyclohexyle portant éventuellement un à trois substituants méthyle ;

$R^5$ représente l'hydrogène, un groupe alkyle à chaîne droite ou ramifiée ayant 1 à 4 atomes de carbone ou un groupe alkyle de 1 à 3 atomes de carbone portant un ou deux substituants identiques ou différents, et on mentionne alors comme substituants : le chlore, un radical hydroxy, alkylcarbonyloxy ayant 1 à 4 atomes de carbone dans la partie alkyle à chaîne droite ou ramifiée, un groupe alkylcarbonyle ayant 1 à 4 atomes de carbone dans la partie alkyle à chaîne droite ou ramifiée, un groupe alkoxy et un groupe alkylthio ayant chacun 1 ou 2 atomes de carbone, un groupe alkoxycarbonyle ayant 1 à 4 atomes de carbone dans la partie alkoxy à chaîne droite ou ramifiée, un groupe carbamoyle, méthylcarbamoyle, diméthylcarbamoyle, cyano, diméthylamino, diéthylamino, un groupe phényle, 2-furyle, 2-pyridyle, 1-morpholino, cyclopropyle ou cyclohexyle portant éventuellement un à trois substituants chloro et méthyle identiques ou différents ;

$R^5$ représente en outre un groupe allyle ou propargyle, un groupe 1-cyclohexényle ou un groupe cyclohexyle portant un à trois substituants identiques ou différents, et on mentionne alors comme substituants : le chlore, un radical méthyle, éthyle, méthoxy, cyano, amino, alkyl- et dialkylamino ayant chacun 1 ou 2 atomes de carbone dans chaque partie alkyle, un radical carbamoyle, alkyl- et dialkylcarbamoyle ayant chacun 1 ou 2 atomes de carbone dans chaque partie alkyle, un radical cyclohexyle, cyclohexylalkyle ayant 1 ou 2 atomes de carbone dans la partie alkyle ou un groupe 1-pyrrolidine-2-one ;

$R^5$ représente en outre un groupe phényle portant éventuellement un à trois substituants chloro et méthyle identiques ou différents, un groupe 2-pyridyle ou 2-pyrimidinyle portant éventuellement un à trois substituants méthyle, un groupe 2-thiazolyle portant éventuellement un substituant phényle, un groupe 2-benzothiazolyle portant éventuellement un à trois substituants chloro, méthyle et méthoxy identiques ou différents, un groupe 1,2,4-triazole-3-yle, un groupe 1,2,4-thiadiazole-5-yle portant éventuellement un substituant phényle, un groupe alkyle ayant 1 à 4 atomes de carbone à chaîne droite ou ramifiée, portant éventuellement un à trois substituants mercapto identiques ou différents, un groupe 1,3,4-thiadiazole-5-yle portant un substituant alkylthio, alkylsulfinyle et alkylsulfonyle chacun à chaîne droite ou ramifiée, ayant chacun 1 à 4 atomes de carbone, ou le groupement

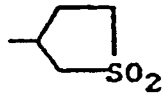 ,

$R^6$ représente l'hydrogène, un groupe alkyyle en $C_1$ à $C_4$ à chaîne droite ou ramifiée ou un groupe alkyle en $C_1$ à $C_3$ portant un ou deux substituants identiques ou différents, et on considère alors comme substituants: le chlore, un radical hydroxy, alkylcarbonyloxy ayant 1 à 4 atomes de carbone dans la partie alkyle à chaîne droite ou ramifiée, un groupe alkylcarbonyle ayant 1 à 4 atomes de carbone dans la partie alkyle à chaîne droite ou ramifiée, un groupe alkoxy et un groupe alkylthio ayant chacun 1 ou 2 atomes de carbone, un groupe alkoxycarbonyle ayant 1 à 4 atomes de carbone dans la partie alkoxy à chaîne droite ou ramifiée, un groupe carbamoyle, méthylcarbamoyle, diméthylcarbamoyle, cyano, diméthylamino, diéthylamino, un groupe phényle portant éventuellement un à trois substituants chloro et méthyle identiques ou différents, un groupe 2-furyle, 2-pyridyle, 1-morpholino, cyclopropyle et cyclohexyle ;

$R^6$ représente en outre un groupe allyle ou un groupe propargyle, un groupe 1-cyclohexényle ou un groupe cyclohexyle portant un à trois substituants identiques ou différents, et on mentionne alors comme substituants:

le chlore, un radical méthyle, éthyle, méthoxy, cyano, amino, alkyl- ou dialkylamino ayant chacun 1 ou 2 atomes de carbone dans chaque partie alkyle, un groupe carbamoyle, alkyl- ou dialkylcarbamoyle ayant chacun 1 ou 2 atomes de carbone dans chaque partie alkyle, un groupe cyclohexyle, cyclohexylalkyle ayant 1 ou 2 atomes de carbone dans la partie alkyle ou un groupe 1-pyrrolidine-2-one ;

$R^6$ représente en outre un groupe phényle portant éventuellement un à trois substituants chloro et méthyle identiques ou différents, un groupe 2-pyridyle ou 2-pyrimidinyle portant chacun le cas échéant un à trois substituants méthyle, un groupe 2-thiazolyle portant éventuellement un substituant phényle, un groupe 2-benzothiazolyle portant éventuellement un à trois substituants chloro, méthyle et méthoxy identiques ou différents, le groupe 1,2,4-triazole-3-yle, un groupe 1,2,4-thiadiazole-5-yle éventuellement substitué par un radical phényle, un groupe 1,3,4-thiadiazole-5-yle portant facultativement un à trois substituants, identiques ou différents, mercapto, alkyle à chaîne droite ou ramifiée ayant 1 à 4 atomes de carbone, alkylthio, alkylsulfinyle et alkylsulfonyle à chaîne droite ou ramifiée ayant chacun 1 à 4 atomes de carbone, ou le groupement

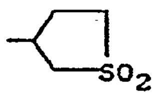 ,

$R^6$ représente en outre un groupe hydroxy, alkoxy ayant 1 ou 2 atomes de carbone, un groupe benzyloxy portant éventuellement un à trois substituants chloro et méthyle, identiques ou différents, un groupe 1-adamantyle, 2-norbornyle, 1- ou 2-décalyle ou 1- ou 2-tétralyle ;
ou bien

$R^5$ et $R^6$ forment conjointement avec l'atome d'azote auquel ils sont liés, les hétérocycles ou spirohétérocycles monocycliques, bicycliques ou tricycliques suivants :

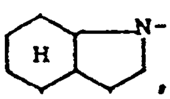 , 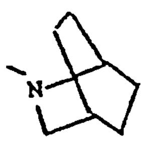 , 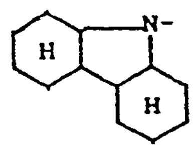 ,

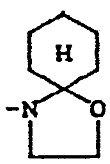 , 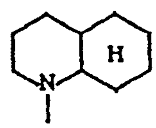 ,

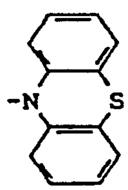 , 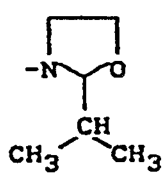 , 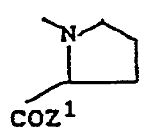

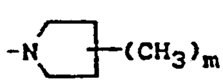 , 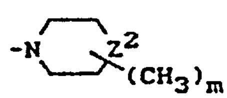

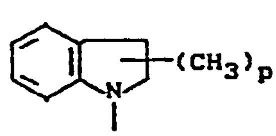 , 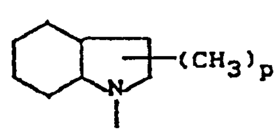 .

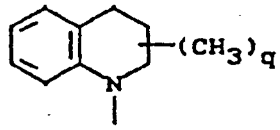 , 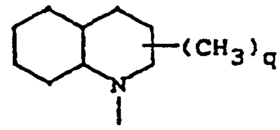 ,

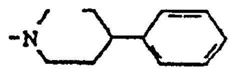 , 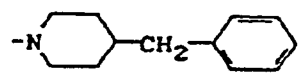 ,

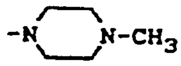 , 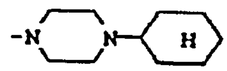 , 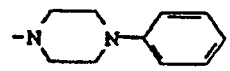 ,

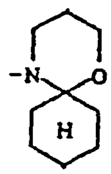 , 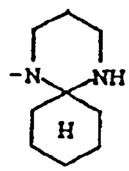 , 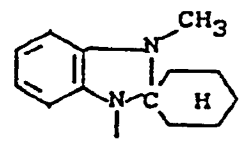

où

$Z^1$ est un groupe hydroxy, méthoxy, éthoxy, amino, méthylamino, éthylamino, diméthylamino ou éthylméthylamino,

$Z^2$ est l'oxygène ou le groupe $CH_2$,

m représente les nombres 0, 1, 2, 3 ou 4,

p représente les nombres 0, 1 ou 2,

q représente les nombres 0, 1, 2 ou 3,

$R^I$ représente un radical méthyle, éthyle, n-propyle, isopropyle, n-butyle, isobutyle, sec.butyle ou tertio-butyle,

$R^{II}$ et $R^{III}$ sont identiques ou différents et représentent l'hydrogène, un radical méthyle, éthyle, n-propyle ou isopropyle, un groupe benzyle portant éventuellement un à trois substituants identiques ou différents avec le chlore et le radical méthyle comme substituants, un groupe alkoxycarbonylalkyle ayant 1 ou 2 atomes de carbone dans chaque partie alkyle, un groupe carbamoylalkyle, alkylcarbamoylalkyle ou dialkylcarbamoylalkyle ayant chacun 1 ou 2 atomes de carbone dans chaque partie alkyle ou un groupe cyclohexyle portant facultativement un à trois substituants méthyle,

$R^{IV}$ représente l'hydrogène, un groupe alkyle ayant 1 ou 2 atomes de carbone ou un groupe benzyle portant éventuellement un à trois substituants identiques ou différents, et on mentionne alors comme substituants du groupe phényle le fluor, le chlore et le radical méthyle ; et

X représente l'oxygène ou le soufre.

4. Compositions pesticides, caractérisées par une teneur en au moins un N,N'-diacylaminal de formule (I) suivant la revendication 1.

5. Utilisation de N,N'-diacylaminals de formule (I) suivant la revendication 1 pour combattre des parasites.

6. Procédé pour combattre des parasites, caractérisé en ce qu'on fait agir des N,N'-diacylaminals de formule (I) suivant la revendication 1 sur les parasites et/ou sur leur habitat.

7. Procédé de préparation de compositions pesticides, caractérisé en ce qu'on mélange des N,N'-diacylaminals de formule (I) suivant la revendication 1 avec des diluants et/ou des agents tensio-actifs.

8. Procédé de préparation de N-(2-cyano-2-oximinoacétyl)aminals de formule générale (II),

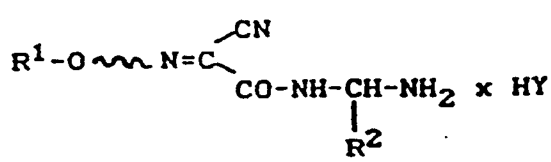 (II)

dans laquelle

$R^1$, $R^2$ et HY ont la définition indiquée dans la revendication 1, caractérisé en ce qu'on hydrolyse d'une manière connue des isocyanates de formule (IV),

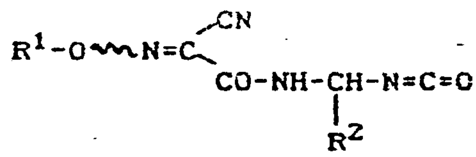

(IV)

dans laquelle

$R^1$ et $R^2$ ont la définition indiquée ci-dessus, et on isole d'une manière classique sous forme de sel l'amine formée.

9. Procédé de production d'isocyanates de formule générale (IV),

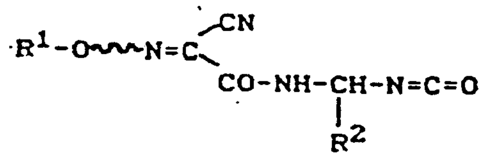

(IV)

dans laquelle

$R^1$ et $R^2$ ont la définition indiquée dans la revendication 1, caractérisé en ce que

α) On oxyde d'une manière connue des amides de formule générale (VI),

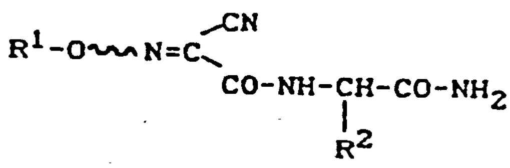

(VI)

dans laquelle

$R^1$ et $R^2$ ont la définition indiquée ci-dessus, le cas échéant en présence d'un diluant, ou bien

β) On chauffe des azides de formule générale (VII),

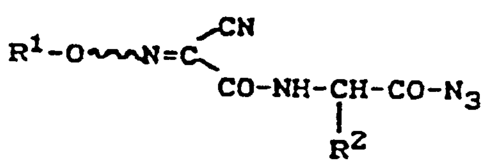

(VII)

dans laquelle

$R^1$ et $R^2$ ont la définition indiquée ci-dessus, en présence d'un diluant.